(19) Europäisches Patentamt
European Patent Office
Office européen des brevets

(11) **EP 3 710 600 B1**

(12) **EUROPEAN PATENT SPECIFICATION**

(45) Date of publication and mention
of the grant of the patent:
**12.03.2025 Bulletin 2025/11**

(21) Application number: **18879014.1**

(22) Date of filing: **16.11.2018**

(51) International Patent Classification (IPC):
*G16B 20/00* (2019.01)    *C12Q 1/6886* (2018.01)
*G16H 50/20* (2018.01)    *G06N 20/00* (2019.01)
*G06N 3/00* (2023.01)

(52) Cooperative Patent Classification (CPC):
**C12Q 1/6886; G16B 20/00; G16H 10/60;**
**G16H 20/10; G16H 20/40; G16H 50/20;**
C12Q 2600/156; G06N 3/044; G06N 3/045;
G06N 3/047; G06N 3/084; G06N 3/088; G06N 5/01;
G06N 7/01; G06N 20/10;                    (Cont.)

(86) International application number:
**PCT/AU2018/051229**

(87) International publication number:
**WO 2019/095017 (23.05.2019 Gazette 2019/21)**

(54) **SYSTEMS AND METHODS FOR PREDICTING THE EFFICACY OF CANCER THERAPY**

SYSTEME UND VERFAHREN ZUR VORHERSAGE DER WIRKSAMKEIT EINER KREBSTHERAPIE

SYSTÈMES ET PROCÉDÉS POUR PRÉDIRE L'EFFICACITÉ DE TRAITEMENT DE CANCER

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.11.2017  AU 2017904663**

(43) Date of publication of application:
**23.09.2020  Bulletin 2020/39**

(73) Proprietor: **GMDx Co Pty Ltd**
**Melbourne, Victoria 3000 (AU)**

(72) Inventors:
• **HALL, Nathan E**
  **Montmorency, Victoria 3094 (AU)**
• **MAMROT, Jared**
  **Clematis, Victoria 3782 (AU)**
• **LINDLEY, Robyn A**
  **Melbourne, Victoria 3000 (AU)**

(74) Representative: **Maschio & Soames IP Ltd**
**30 Carlton Crescent**
**Southampton SO15 2EW (GB)**

(56) References cited:
  WO-A1-2017/031551    WO-A1-2018/039567
  WO-A2-2013/036867    WO-A2-2016/081947
  US-A1- 2012 100 134    US-A1- 2012 252 026

EP 3 710 600 B1

(52) Cooperative Patent Classification (CPC): (Cont.)
G06N 20/20

**Description**

**FIELD OF THE INVENTION**

This invention relates generally to systems and methods for predicting the efficacy of a cancer therapy in a subject. In particular embodiments, the systems and methods of the disclosure can be used to determine a therapy indicator for use in assessing responsiveness to cancer therapy, to determine whether a subject is likely to respond to a new cancer therapy, and/or to determine whether a subject is likely to continue to respond to current cancer therapy, such as a current cancer immunotherapy.

**BACKGROUND OF THE INVENTION**

Cancer therapies have evolved significantly in recent decades. Not only have the traditional treatments of surgery, radiation therapy and chemotherapy become more precise, but newer treatments are also providing a broad range of therapies that target specific molecules, cells and/or pathways associated with cancer development, and which may be particularly effective for different cancers, stages of cancer, and/or populations. Thus, in addition to surgery, radiation therapy and traditional chemotherapy (i.e. non-targeted chemotherapy that involves the use of drugs to kill rapidly dividing cells such as cancer), cancer therapies now also include, for example, hormone therapy, targeted therapy and immunotherapy.

Targeted therapies include drugs and proteins (including antibodies) that interact with molecules that are specific for or associated with a cancer cell or that otherwise are involved, directly or indirectly, with cancer cell proliferation. Targeted therapies typically act in a cytostatic manner to inhibit cancer cell proliferation, although may also be cytotoxic. Targeted therapies include, for example, drugs (i.e. small molecules, such as small molecule kinase inhibitors) and monoclonal antibodies (including chimeric, humanized or fully human antibodies, whether naked or conjugated with a toxic moiety) specific for ABL, Anaplastic lymphoma kinase (ALK), Beta-1,4 N-acetylgalactosaminyltransferase 1 (B4GALNT1), B-cell activating factor (BAFF), B-Raf, Bruton's tyrosine kinase (BTK), CD19, CD20, CD30, CD38, CD52, cytotoxic T-Lymphocyte associated protein 4 (CTLA-4), epidermal growth factor receptor (EGFR), FMS-like tyrosine kinase-3 (FLT3), histone deacetylase (HDAC), human epidermal growth factor receptor 2 (HER-2), isocitrate dehydrogenase 1 (IDH1), IDH2, interleukin 1 beta (IL-1β), c-KIT, MEK, MET, mTOR, Poly (ADP-ribose) polymerase (PARP), programmed cell death protein 1 (PD-1), platelet-derived growth factor receptors (PDGFR), PDGFRβ, programmed death-ligand 1 (PD-L1), phosphatidylinositol-3-kinase delta (PI3Kδ), receptor activator of nuclear factor kappa-B ligand (RANKL), RET, ROS1, signaling lymphocytic activation molecule F7 (SLAMF7), vascular endothelial growth factor (VEGF), VEGF receptor (VEGFR) and VEGFR2. In some instances, these targeted therapies target and/or exploit the patient's immune system and can therefore also be considered cancer immunotherapies.

Cancer immunotherapy functions by exploiting or utilizing the immune system of the patient to treat the cancer. This can be through several mechanisms and by using different strategies, including non-specific stimulation of immune responses by stimulating effector cells and/or inhibiting regulatory cells (e.g. by administration of cytokines such as IL-2 and IFN-a, or drugs such as thalidomide (Thalomid®), ienalidomide (Revlimid®), pomalidomide (Pomalyst®) and miquimod (Zyclara®)), active immunization to stimulate or enhance specific anti-cancer immune responses (e.g. using cancer vaccines such as the HPV vaccines Gardasil® and Cevarix® for the prevention of cervical cancer, Sipuleucel-T (Provenge®) for the treatment of prostate cancer, and the Bacillus Calmette-Guérin (BCG) vaccine for the treatment of bladder cancer), and the passive transfer of antibodies or the passive transfer of activated immune cells (i.e. adoptive cell therapy (ACT), e.g. chimeric antigen receptor (CAR) T-cell therapy). Antibodies that have been developed as cancer immunotherapies include, for example, immune checkpoint inhibitor antibodies (e.g. targeting CTLA-4, PD-1 or PD-L1) and antibodies that target molecules on cancer cells so as to induce an immune response to the cancer cell (e.g. anti-CD52 antibodies).

While the continual development of cancer therapies is providing an expanded tool box for cancer treatment, the new therapies are typically effective in only a subset of patients. Thus, many patients undergo cancer therapy, at significant cost and often with significant side-effects, without any benefit in terms of inhibition of cancer progression or remission. However, predicting which patients will respond to therapy and which patients will not has been very difficult and indeed relatively unsuccessful to date. Thus, there remains a need for effective methods and systems for predicting the likelihood that a subject with cancer will respond to a particular cancer therapy. There also remains a need for methods and systems for predicting which patients will continue to respond to a cancer therapy that they are on, and which patients will develop resistance to the therapy, essentially resulting in relapse.

WO 2016/081947-A2 discloses methods for defining a mutation signature that correlates with responsiveness to therapy with an immune checkpoint modulator. The method comprises determining one or more mutation characteristics in a plurality of samples of tumors sharing a response characteristic to immune checkpoint modulator therapy; comparing the determined one or more mutation characteristics with those in a plurality of samples of tumors that do not share the response characteristic; and identifying a set of mutation characteristics whose presence correlates with the response characteristic. Not disclosed is a machine learning model which accepts as input metrics from a deaminase motif metric group associated with SNVs in one or more deaminase motifs and metrics from a codon context metric group wherein the metrics of this group are associated with a codon context of SNVs.

SUMMARY OF THE INVENTION

The invention is solely defined by the appended independent claims. Preferred embodiments are defined by the dependent claims.

**SUMMARY OF** RELATED DISCLOSURE

The present disclosure is predicated on the determination that the number, percentage or ratio of particular types of single nucleotide variations (SNVs) in the nucleic acid of a subject with cancer who responds to therapy is different to that of a subject who does not respond to therapy. The present disclosure is also predicated on the determination that the number, percentage or ratio of particular types of SNVs in the nucleic acid of a subject with cancer who was responding to therapy and who continues to respond to therapy is different to that of a subject who was responding to therapy but then ceases to respond to therapy, i.e. who develops resistance to therapy. The SNVs include those that might be attributed to the activity of one or more endogenous deaminases, as well as those that may not necessarily be attributed to the activity of one or more endogenous deaminases.

As described herein, SNVs identified in a nucleic acid molecule can be used to determine a plurality of metrics, which can then in turn be used to help distinguish subjects that are likely to respond to cancer therapy from subjects that are unlikely to respond to cancer therapy; and/or subjects that are likely to continue to respond to cancer therapy from subjects that are unlikely to continue to respond to cancer therapy. Thus, a profile can be built based upon this plurality of metrics, whereupon subjects that are likely to respond to cancer therapy typically have a different profile to subjects that are unlikely to respond to cancer therapy; and subjects that are likely to continue to respond to cancer therapy typically have a different profile to subjects that are unlikely to continue to respond to cancer therapy.

In one aspect, provided is a system for generating a therapy indicator for use in assessing responsiveness to cancer therapy for a subject, the system including one or more electronic processing devices that: a) obtain subject data indicative of a sequence of a nucleic acid molecule from the subject; b) analyze the subject data to identify single nucleotide variations (SNVs) within the nucleic acid molecule; c) determine a plurality of metrics using the identified SNVs, the plurality of metrics including metrics from one or more of metric groups including: i) a motif metric group including metrics associated with SNVs in specific motifs; ii) a codon context metric group including metrics associated with a codon context of SNVs; iii) a transition/transversion metric group including metrics associated with SNVs that are transitions or transversions; iv) a synonymous/non-synonymous metric group including metrics associated with SNVs that are synonymous or non-synonymous; v) a strand bias metric group including metrics associated with strand bias of SNVs; vi) a strand specific metric group that includes metrics associated with SNVs on a specific strand; and vii) an AT/GC metric group that includes metrics associated with SNVs in which an adenine and thymine, and/or guanine and cytidine have been targeted; and d) apply the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy. In one embodiment, the plurality of metrics includes metrics from 2, 3, 4, 5, 6 or all of the metric groups.

In another aspect, provided is a system for generating a therapy indicator for use in assessing responsiveness to cancer therapy for a subject, the system including one or more electronic processing devices that: a) obtain subject data indicative of a sequence of a nucleic acid molecule from the subject; b) analyze the subject data to identify single nucleotide variations (SNVs) within the nucleic acid molecule; c) determine a plurality of metrics using the identified SNVs, the plurality of metrics including metrics from three or more metric groups selected from: i) a coding metric group including metrics associated with SNVs in a coding region of the nucleic acid molecule; ii) a non-coding metric group including metrics associated with SNVs in a non-coding region of the nucleic acid molecule; iii) a genomic metric group including metrics associated with SNVs in coding and non-coding regions of the nucleic acid molecule; iv) a codon context metric group including metrics associated with a codon context of SNVs; v) a transition/transversion metric group including metrics associated with SNVs that are transitions or transversions; vi) a synonymous/non-synonymous metric group including metrics associated with SNVs that are synonymous or non-synonymous; vii) a strand bias metric group including metrics associated with strand bias of SNVs; viii) a strand specific metric group that includes metrics associated with SNVs on a specific strand; ix) an AT/GC metric group that includes metrics associated with SNVs in which an adenine and thymine, and/or guanine and cytidine have been targeted; x) a motif metric group including metrics associated with SNVs in specific motifs; and, xi) a motif-independent metric group including metrics associated with SNVs irrespective of motif;

**EP 3 710 600 B1**

and, d) apply the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy. In one embodiment, the the plurality of metrics includes metrics from 4, 5, 6, 7, 8, 9, 10 or all of the metric groups.

In one embodiment, the at least one computational model includes a decision tree. In further embodiments, the at least one computational model includes a plurality of decision trees, and the therapy indicator is generated by aggregating results from the plurality of decision trees. In a particular example, at least one metric is used in multiple ones of the plurality of decision trees.

In further embodiment, the one or more processing devices determine at least one of: a) at least one metric from each available group; and, b) at least two metrics from at least some available groups. In another embodiment, the one or more processing devices determines at least one of: a) at least 2 metrics; b) at least 5 metrics; c) at least 10 metrics; d) at least 20 metrics; e) at least 50 metrics; f) at least 75 metrics; g) at least 100 metrics; and, h) at least 200 metrics. In another example, the one or more processing devices determines at least one of: a) at least 0.1% of all metrics in the metric groups; b) at least 0.2% of all metrics in the metric groups; c) at least 0.3% of all metrics in the metric groups; d) at least 0.4% of all metrics in the metric groups; e) at least 0.5% of all metrics in the metric groups; f) at least 0.75% of all metrics in the metric groups; g) at least 1% of all metrics in the metric groups; h) at least 1.5% of all metrics in the metric groups; and, i) at least 2% of all metrics in the metric groups.

In some embodiments, the one or more processing devices: a) determine one or more subject attributes for the subject; and, b) use the one or more subject attributes to apply the at least one computational model so that the at least one metric is assessed based on reference metrics derived for one or more reference subjects having similar attributes to the subject attributes. In a particular example, the one or more processing devices select a plurality of metrics at least in part using the subject attributes. In a further example, the one or more processing devices select at least one computational model at least in part using the subject attributes, such as one selected from an attribute group including: a) one or more subject characteristics selected from a characteristic group including: i) a subject age; ii) a subject height; iii) a subject weight; iv) a subject sex; and, v) a subject ethnicity; b) one or more body states selected from a body state group including: i) a healthy body state; and ii) an unhealthy body state; c) one or more disease states selected from a disease state group including: i) cancer type; ii) cancer stage; and iii) presence of metastases; d) one or more medical interventions selected from a medical intervention group including i) immunotherapy; ii) radiotherapy; and iii) non-targeted chemotherapy. In some embodiments, the one or more processing devices determine the subject attributes at least one of: a) by querying a subject medical history; b) by receiving sensor data from a sensing device; and, c) in accordance with user input commands.

In further embodiments of the systems of the present disclosure, the one or more processing devices at least one of: a) display a representation of the therapy indicator; b) store the therapy indicator for subsequent retrieval; and, c) provide the therapy indicator to a client device for display.

A further aspect of the present disclosure provides a system for use in calculating at least one computational model, the at least one computational model being used for generating therapy indicator for use in assessing responsiveness to cancer therapy for a subject, the system including one or more electronic processing devices that: a) for each of a plurality of reference subjects: i) obtain reference subject data indicative of: (1) a sequence of a nucleic acid molecule from the reference subject; and, (2) a responsiveness to cancer therapy; ii) analyze the reference subject data to identify single nucleotide variations (SNVs) within the nucleic acid molecule; iii) determine a plurality of metrics using the identified SNVs, the plurality of metrics including metrics from one or more of metric groups including: 1) a motif metric group including metrics associated with SNVs in specific motifs; 2) a codon context metric group including metrics associated with a codon context of SNVs; 3) a transition/transversion metric group including metrics associated with SNVs that are transitions or transversions; 4) a synonymous/non-synonymous metric group including metrics associated with SNVs that are synonymous or non-synonymous; 5) a strand bias metric group including metrics associated with strand bias of SNVs; 6) a strand specific metric group that includes metrics associated with SNVs on a specific strand; and 7) an AT/GC metric group that includes metrics associated with SNVs in which an adenine and thymine, and/or guanine and cytidine have been targeted; and, b) use the plurality of reference metrics and known responsiveness for a number of reference subjects to train at least one computational model, the at least one computational model embodying a relationship between a responsiveness to cancer therapy and the plurality of metrics. In some embodiments, the plurality of metrics includes metrics from 2, 3, 4, 5, 6 or all of the metric groups.

Another aspect of the present disclosure provides a system for use in calculating at least one computational model, the at least one computational model being used for generating a therapy indicator for use in assessing responsiveness to cancer therapy for a subject, the system including one or more electronic processing devices that: a) for each of a plurality of reference subjects: i) obtain reference subject data indicative of: (1) a sequence of a nucleic acid molecule from the reference subject; and, (2) a responsiveness to cancer therapy; ii) analyze the reference subject data to identify single nucleotide variations (SNVs) within the nucleic acid molecule; iii) determine a plurality of metrics using the

5

identified SNVs, the plurality of metrics including metrics from three or more of the metric groups selected from: 1) a coding metric group including metrics associated with SNVs in a coding region of the nucleic acid molecule; 2) a non-coding metric group including metrics associated with SNVs in a non-coding region of the nucleic acid molecule; 3) a genomic metric group including metrics associated with SNVs in coding and non-coding regions of the nucleic acid molecule; 4) a codon context metric group including metrics associated with a codon context of SNVs; 5) a transition/transversion metric group including metrics associated with SNVs that are transitions or transversions; 6) a synonymous/non-synonymous metric group including metrics associated with SNVs that are synonymous or non-synonymous; 7) a strand bias metric group including metrics associated with strand bias of SNVs; 8) a strand specific metric group that includes metrics associated with SNVs on a specific strand; 9) an AT/GC metric group that includes metrics associated with SNVs in which an adenine and thymine, and/or guanine and cytidine have been targeted; 10) a motif metric group including metrics associated with SNVs in specific motifs; and, 11) a motif-independent metric group including metrics associated with SNVs irrespective of motif; and, b) use the plurality of reference metrics and known responsiveness for a number of reference subjects to train at least one computational model, the at least one computational model embodying a relationship between a responsiveness to cancer therapy and the plurality of metrics. In some embodiments, the plurality of metrics includes metrics from 4, 5, 6, 7, 8, 9, 10 or all of the metric groups.

In some embodiments of the system for use in calculating at least one computational model, the one or more processing devices test the at least one computational model to determine a discriminatory performance of the model. The discriminatory performance may be based on at least one of: a) an area under a receiver operating characteristic curve; b) an accuracy; c) a sensitivity; and, d) a specificity. In some examples, the discriminatory performance is at least 70%.

In further embodiments of the system for use in calculating at least one computational model, the one or more processing devices test the at least one computational model using a reference subject data from a subset of the plurality of reference subjects. In additional embodiments, the one or more processing devices: a) select a plurality of reference metrics; b) train at least one computational model using the plurality of reference metrics; c) test the at least one computational model to determine a discriminatory performance of the model; and, d) if the discriminatory performance of the model falls below a threshold, at least one of: i) selectively retrain the at least one computational model using a different plurality of reference metrics; and, ii) train a different computational model.

In other embodiments of the system for use in calculating at least one computational model, the one or more processing devices: a) select a plurality of combinations of reference metrics; b) train a plurality of computational models using each of the combinations; c) test each computational model to determine a discriminatory performance of the model; and, d) selecting the at least one computational model with the highest discriminatory performance for use in determining the therapy indicator. In additional examples, the one or more processing devices: a) determine one or more reference subject attributes; and, b) train the at least one computational model using the one or more reference subject attributes. In a particular example, the one or more processing devices: a) perform clustering using the reference subject attributes to determine clusters of reference subject having similar reference subject attributes; and, b) train the at least one computational model at least in part using the reference subject clusters. The one or more reference subject attributes may be selected from an attribute group including: a) one or more subject characteristics selected from a characteristic group including: i) a subject age; ii) a subject height; iii) a subject weight; iv) a subject sex; and, v) a subject ethnicity; b) one or more body states selected from a body state group including: i) a healthy body state; and ii) an unhealthy body state; c) one or more disease states selected from a disease state group including: i) cancer type; ii) cancer stage; and iii) presence of metastases; d) one or more medical interventions selected from a medical intervention group including i) immunotherapy; ii) radiotherapy; and iii) non-targeted chemotherapy.

In further embodiments of the system for use in calculating at least one computational model, the at least one computational model includes a decision tree. In one example, the at least one computational model includes a plurality of decision trees, and the therapy indicator is generated by aggregating results from the plurality of decision trees. In a particular example, at least one metric is used in multiple ones of the plurality of decision trees.

In additional embodiments of the system for use in calculating at least one computational model the one or more processing devices train the model using at least one of: a) at least 1000 metrics; b) at least 2000 metrics; c) at least 3000 metrics; d) at least 4000 metrics; and, e) at least 5000 metrics. In one example, the resulting model uses at least one of: a) at least 2 metrics; b) at least 5 metrics; c) at least 10 metrics; d) at least 20 metrics; e) at least 50 metrics; f) at least 75 metrics; g) at least 100 metrics; and, h) at least 200 metrics. In a particular example, the resulting model uses at least one of: a) at least 0.1% of all metrics in the metric groups; b) at least 0.2% of all metrics in the metric groups; c) at least 0.3% of all metrics in the metric groups; d) at least 0.4% of all metrics in the metric groups; e) at least 0.5% of all metrics in the metric groups; f) at least 0.75% of all metrics in the metric groups; g) at least 1% of all metrics in the metric groups; g) at least 1.5% of all metrics in the metric groups; and, i) at least 2% of all metrics in the metric groups.

In another aspect, provided is a method for generating a therapy indicator for use in assessing responsiveness to cancer therapy for a subject, the method including, in one or more electronic processing devices: a) obtaining subject data indicative of a sequence of a nucleic acid molecule from the subject; b) analyzing the subject data to identify single nucleotide variations (SNVs) within the nucleic acid molecule; c) determining a plurality of metrics using the identified SNVs, the plurality of metrics including metrics from one or more of metric groups including: i) a motif metric group including metrics associated with SNVs in specific motifs; ii) a codon context metric group including metrics associated with a codon context of SNVs; iii) a transition/transversion metric group including metrics associated with SNVs that are transitions or transversions; iv) a synonymous/non-synonymous metric group including metrics associated with SNVs that are synonymous or non-synonymous; v) a strand bias metric group including metrics associated with strand bias of SNVs; vi) a strand specific metric group that includes metrics associated with SNVs on a specific strand; and vii) an AT/GC metric group that includes metrics associated with SNVs in which an adenine and thymine, and/or guanine and cytidine have been targeted; and, d) applying the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy. In some embodiments, the plurality of metrics includes metrics from 2, 3, 4, 5, 6 or all of the metric groups.

Also provided is a method for generating therapy indicator for use in assessing responsiveness to cancer therapy for a subject, the method including, in one or more electronic processing devices: a) obtaining subject data indicative of a sequence of a nucleic acid molecule from the subject; b) analyzing the subject data to identify single nucleotide variations (SNVs) within the nucleic acid molecule; c) determining a plurality of metrics using the identified SNVs, the plurality of metrics including metrics from three or more of metric groups including: i) a coding metric group including metrics associated with SNVs in a coding region of the nucleic acid molecule; ii) a non-coding metric group including metrics associated with SNVs in a non-coding region of the nucleic acid molecule; iii) a genomic metric group including metrics associated with SNVs in coding and non-coding regions of the nucleic acid molecule; iv) a codon context metric group including metrics associated with a codon context of SNVs; v) a transition/transversion metric group including metrics associated with SNVs that are transitions or transversions; vi) a synonymous/non-synonymous metric group including metrics associated with SNVs that are synonymous or non-synonymous; vii) a strand bias metric group including metrics associated with strand bias of SNVs; viii) a strand specific metric group that includes metrics associated with SNVs on a specific strand; ix) an AT/GC metric group that includes metrics associated with SNVs in which an adenine and thymine, and/or guanine and cytidine have been targeted; x) a motif metric group including metrics associated with SNVs in specific motifs; and, xi) a motif-independent metric group including metrics associated with SNVs irrespective of motif; and, d) applying the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy. In some embodiments, the plurality of metrics includes metrics from 4, 5, 6, 7, 8, 9, 10 or all of the metric groups.

In a further aspect, provided is a computer program product for generating a therapy indicator for use in assessing responsiveness to cancer therapy for a subject, the computer program product including computer executable code, which when executed by one or more suitably programmed electronic processing devices, causes the one or more electronic processing devices to: a) obtain subject data indicative of a sequence of a nucleic acid molecule from the subject; b) analyze the subject data to identify single nucleotide variations (SNVs) within the nucleic acid molecule; c) determine a plurality of metrics using the identified SNVs, the plurality of metrics including metrics from one or more of metric groups including: i) a motif metric group including metrics associated with SNVs in specific motifs; ii) a codon context metric group including metrics associated with a codon context of SNVs; iii) a transition/transversion metric group including metrics associated with SNVs that are transitions or transversions; iv) a synonymous/non-synonymous metric group including metrics associated with SNVs that are synonymous or non-synonymous; v) a strand bias metric group including metrics associated with strand bias of SNVs; vi) a strand specific metric group that includes metrics associated with SNVs on a specific strand; and vii) an AT/GC metric group that includes metrics associated with SNVs in which an adenine and thymine, and/or guanine and cytidine have been targeted; and, d) apply the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy. In one embodiment, the plurality of metrics includes metrics from 2, 3, 4, 5, 6 or all of the metric groups.

Also provided is a computer program product for generating therapy indicator for use in assessing responsiveness to cancer therapy for a subject, the computer program product including computer executable code, which when executed by one or more suitably programmed electronic processing devices, causes the one or more electronic processing devices to: a) obtain subject data indicative of a sequence of a nucleic acid molecule from the subject; b) analyze the subject data to identify single nucleotide variations (SNVs) within the nucleic acid molecule; c) determine a plurality of metrics using the identified SNVs, the plurality of metrics including metrics from one or more of metric groups including: i) a coding metric group including metrics associated with SNVs in a coding region of the nucleic acid molecule; ii) a non-coding metric group including metrics associated with SNVs in a non-coding region of the nucleic acid molecule; iii) a genomic metric group including metrics associated with SNVs in coding and non-coding regions of the nucleic acid molecule; iv) a codon context metric group including metrics associated with a codon context of SNVs; v) a transition/-transversion metric group including metrics associated with SNVs that are transitions or transversions; vi) a synonymous/non-synonymous metric group including metrics associated with SNVs that are synonymous or non-synonymous; vii) a strand bias metric group including metrics associated with strand bias of SNVs; viii) a strand specific metric group that includes metrics associated with SNVs on a specific strand; ix) an AT/GC metric group that includes metrics associated with SNVs in which an adenine and thymine, and/or guanine and cytidine have been targeted; x) a motif metric group including metrics associated with SNVs in specific motifs; and, xi) a motif-independent metric group including metrics associated with SNVs irrespective of motif and, d) apply the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy. In one embodiment, the plurality of metrics includes metrics from 4, 5, 6, 7, 8, 9, 10 or all of the metric groups.

In a further aspect, provided is a computer program product for use in calculating at least one computational model, the at least one computational model being used for generating therapy indicator for use in assessing responsiveness to cancer therapy for a biological subject, the computer program product including computer executable code, which when executed by one or more suitably programmed electronic processing devices, causes the one or more electronic processing devices to: a) for each of a plurality of reference subjects: i) obtain reference subject data indicative of: (1) a sequence of a nucleic acid molecule from the reference subject; and, (2) a responsiveness to cancer therapy; ii) analyze the reference subject data to identify single nucleotide variations (SNVs) within the nucleic acid molecule; iii) determine a plurality of metrics using the identified SNVs, the plurality of metrics including metrics from one or more of metric groups including: 1) a motif metric group including metrics associated with SNVs in specific motifs; 2) a codon context metric group including metrics associated with a codon context of SNVs; 3) a transition/transversion metric group including metrics associated with SNVs that are transitions or transversions; 4) a synonymous/non-synonymous metric group including metrics associated with SNVs that are synonymous or non-synonymous; 5) a strand bias metric group including metrics associated with strand bias of SNVs; 6) a strand specific metric group that includes metrics associated with SNVs on a specific strand; and 7) an AT/GC metric group that includes metrics associated with SNVs in which an adenine and thymine, and/or guanine and cytidine have been targeted; and, d) use the plurality of reference metrics and known responsiveness for a number of reference subjects to train at least one computational model, the at least one computational model embodying a relationship between a responsiveness to cancer therapy and the plurality of metrics. In one embodiment, the plurality of metrics includes metrics from 2, 3, 4, 5, 6 or all of the metric groups.

Another aspect of the present disclosure provides a computer program product for use in calculating at least one computational model, the at least one computational model being used for generating therapy indicator for use in assessing responsiveness to cancer therapy for a biological subject, the computer program product including computer executable code, which when executed by one or more suitably programmed electronic processing devices, causes the one or more electronic processing devices to: a) for each of a plurality of reference subjects: i) obtain reference subject data indicative of: (1) a sequence of a nucleic acid molecule from the reference subject; and, (2) a responsiveness to cancer therapy; ii) analyze the reference subject data to identify single nucleotide variations (SNVs) within the nucleic acid molecule; iii) determine a plurality of metrics using the identified SNVs, the plurality of metrics including metrics from one or more of metric groups including: 1) a coding metric group including metrics associated with SNVs in a coding region of the nucleic acid molecule; 2) a non-coding metric group including metrics associated with SNVs in a non-coding region of the nucleic acid molecule; 3) a genomic metric group including metrics associated with SNVs in coding and non-coding regions of the nucleic acid molecule; 4) a codon context metric group including metrics associated with a codon context of SNVs; 5) a transition/transversion metric group including metrics associated with SNVs that are transitions or transversions; 6) a synonymous/non-synonymous metric group including metrics associated with SNVs that are synonymous or non-synonymous; 7) a strand bias metric group including metrics associated with strand bias of SNVs; 8) a strand specific metric group that includes metrics associated with SNVs on a specific strand; 9) an AT/GC metric group that includes metrics associated with SNVs in which an adenine and thymine, and/or guanine and cytidine have been targeted; 10) a motif metric group including metrics associated with SNVs in specific motifs; and, 11) a motif-independent metric group including metrics associated with SNVs irrespective of motif; and, d) use the plurality of reference metrics and known responsiveness for a number of reference subjects to train at least one computational model, the at least one computational model embodying a relationship between a responsiveness to cancer therapy and the plurality of metrics. In one embodiment, the plurality of metrics includes metrics from 4, 5, 6, 7, 8, 9, 10 or all of the metric groups.

In a further aspect, provided is a method for use in calculating at least one computational model, the at least one computational model being used for generating therapy indicator for use in assessing responsiveness to cancer therapy for a biological subject, the method including, in one or more electronic processing devices: a) for each of a plurality of reference subjects: i) obtaining reference subject data indicative of: (1) a sequence of a nucleic acid molecule from the reference subject; and, (2) a responsiveness to cancer therapy; ii) analyzing the reference subject data to identify single nucleotide variations (SNVs) within the nucleic acid molecule; iii) determining a plurality of metrics using the identified SNVs, the plurality of metrics including metrics from one or more of metric groups including: 1) a motif metric group including metrics associated with SNVs in specific motifs; 2) a codon context metric group including metrics associated with a codon context of SNVs; 3) a transition/transversion metric group including metrics associated with SNVs that are transitions or transversions; 4) a synonymous/non-synonymous metric group including metrics associated with SNVs that are synonymous or non-synonymous; 5) a strand bias metric group including metrics associated with strand bias of SNVs; 6) a strand specific metric group that includes metrics associated with SNVs on a specific strand; and 7) an AT/GC metric group that includes metrics associated with SNVs in which an adenine and thymine, and/or guanine and cytidine have been targeted; and, b) using the plurality of reference metrics and known responsiveness for a number of reference subjects to train at least one computational model, the at least one computational model embodying a relationship between a responsiveness to cancer therapy and the plurality of metrics. In a particular embodiment, the plurality of metrics includes metrics from 2, 3, 4, 5, 6 or all of the metric groups.

In another aspect, provided is a method for use in calculating at least one computational model, the at least one computational model being used for generating therapy indicator for use in assessing responsiveness to cancer therapy for a biological subject, the method including, in one or more electronic processing devices: a) for each of a plurality of reference subjects: i) obtaining reference subject data indicative of: (1) a sequence of a nucleic acid molecule from the reference subject; and, (2) a responsiveness to cancer therapy; ii) analyzing the reference subject data to identify single nucleotide variations (SNVs) within the nucleic acid molecule; iii) determining a plurality of metrics using the identified SNVs, the plurality of metrics including metrics from one or more of metric groups including: 1) a coding metric group including metrics associated with SNVs in a coding region of the nucleic acid molecule; 2) a non-coding metric group including metrics associated with SNVs in a non-coding region of the nucleic acid molecule; 3) a genomic metric group including metrics associated with SNVs in coding and non-coding regions of the nucleic acid molecule; 4) a codon context metric group including metrics associated with a codon context of SNVs; 5) a transition/transversion metric group including metrics associated with SNVs that are transitions or transversions; 6) a synonymous/non-synonymous metric group including metrics associated with SNVs that are synonymous or non-synonymous; 7) a strand bias metric group including metrics associated with strand bias of SNVs; 8) a strand specific metric group that includes metrics associated with SNVs on a specific strand; 9) an AT/GC metric group that includes metrics associated with SNVs in which an adenine and thymine, and/or guanine and cytidine have been targeted; 10) a motif metric group including metrics associated with SNVs in specific motifs; and, 11) a motif-independent metric group including metrics associated with SNVs irrespective of motif; and, b) using the plurality of reference metrics and known responsiveness for a number of reference subjects to train at least one computational model, the at least one computational model embodying a relationship between a responsiveness to cancer therapy and the plurality of metrics. In a particular embodiment, the plurality of metrics includes metrics from 4, 5, 6, 7, 8, 9, 10 or all of the metric groups.

Also provided is a method for determining the likelihood that a subject with cancer will respond to a cancer therapy or will continue to respond to a cancer therapy, the method comprising: analyzing the sequence of a nucleic acid molecule from a subject with cancer to detect SNVs within the nucleic acid molecule; determining a plurality of metrics based on the number and/or type of SNVs detected so as to obtain a subject profile of metrics, wherein the plurality of metrics includes metrics from one or more of the following metric groups: i) a motif metric group including metrics associated with SNVs in specific motifs; ii) a codon context metric group including metrics associated with a codon context of SNVs; iii) a transition/transversion metric group including metrics associated with SNVs that are transitions or transversions; iv) a synonymous/non-synonymous metric group including metrics associated with SNVs that are synonymous or non-synonymous; v) a strand bias metric group including metrics associated with strand bias of SNVs; vi) a strand specific metric group that includes metrics associated with SNVs on a specific strand; and vii) an AT/GC metric group that includes metrics associated with SNVs in which an adenine and thymine, and/or guanine and cytidine have been targeted; and, determining the likelihood of a subject responding to cancer therapy based on a comparison between the subject profile and a reference profile of metrics. In some embodiments, the plurality of metrics includes metrics from 2, 3, 4, 5, 6 or all of the metric groups.

Another aspect of the present disclosure provides a method for determining the likelihood that a subject with cancer will respond to a cancer therapy or will continue to respond to a cancer therapy, the method comprising: analyzing the sequence of a nucleic acid molecule from a subject with cancer to detect SNVs within the nucleic acid molecule; determining a plurality of metrics based on the number and/or type of SNVs detected so as to obtain a subject profile of metrics, wherein the plurality of metrics includes metrics from three or more of the following metric groups: i) a coding metric group including metrics associated with SNVs in a coding region of the nucleic acid molecule; ii) a non-coding metric group including metrics associated with SNVs in a non-coding region of the nucleic acid molecule; iii) a genomic metric group including metrics associated with SNVs in coding and non-coding regions of the nucleic acid molecule; iv) a codon context metric group including metrics associated with a codon context of SNVs; v) a transition/transversion metric group including metrics associated with SNVs that are transitions or transversions; vi) a synonymous/non-synonymous metric group including metrics associated with SNVs that are synonymous or non-synonymous; vii) a strand bias metric group including metrics associated with strand bias of SNVs; viii) a strand specific metric group that includes metrics associated with SNVs on a specific strand; ix) an AT/GC metric group that includes metrics associated with SNVs in which an adenine and thymine, and/or guanine and cytidine have been targeted; x) a motif metric group including metrics associated with SNVs in specific motifs; and, xi) a motif-independent metric group including metrics associated with SNVs irrespective of motif; and, determining the likelihood of a subject responding to cancer therapy based on a comparison between the subject profile and a reference profile of metrics. In some embodiments, the plurality of metrics includes metrics from 4, 5, 6, 7, 8, 9, 10 or all of the metric groups.

In select embodiments of the methods for determining the likelihood that a subject with cancer will respond to a cancer therapy or will continue to respond to a cancer therapy, the reference profile is produced using a computational model. In further embodiments, the subject may be on the cancer therapy and the method is for determining the likelihood that the subject will continue to respond to the cancer therapy. Moreover, the methods can further comprise providing a recommendation to the subject to: begin the cancer therapy if it is determined that the subject is likely to respond to the cancer therapy; continue the cancer therapy if it is determined that the subject is likely to continue responding to the cancer therapy; begin a different cancer therapy if it is determined that the subject is unlikely to respond to the cancer therapy; or cease the cancer therapy if it is determined that the subject is unlikely to continue responding to the cancer therapy.

In some embodiments of any of the systems, computer program products, or methods described above and herein, the plurality of metrics includes metrics from the motif metric group and the codon context metric group. In further examples, the plurality of metrics includes metrics from the motif metric group, the codon context metric group and the transition/transversion metric group. In some instances, the motif metric group comprises a deaminase motif metric group associated with SNVs in one or more deaminase motifs. For example, the deaminase motif metric group may comprise a group selected from among an activation-induced cytidine deaminase (AID), apolipoprotein B mRNA-editing enzyme, catalytic polypeptide-like (APOBEC) 1 cytosine deaminase (APOBEC1), APOBEC3A, APOBEC3B, APOBEC3C, APOBEC3D, APOBEC3F, APOBEC3G, APOBEC3H and an adenine deaminase acting on RNA (ADAR) motif metric group, wherein each group is associated with SNVs in one or more AID, APOBEC1, APOBEC3A, APOBEC3B, APOBEC3C, APOBEC3D, APOBEC3F, APOBEC3G, APOBEC3H or ADAR motifs, respectively. Where the deaminase motif is an AID motif, it may be selected from among WRC/GYW, WRCG/CGYW, WRCGS/SCGYW, WRCY/RGYW, WRCGW/WCGYW, WRCR/YGYW and AGCTNT/ANAGCT. Where the deaminase motif is an ADAR motif, it may be selected from among WA/TW, WAY/RTW, SWAY/RTWS, CWAY/RTWG, CWAA/TTWG, SWA/TWS, WAA/TTW, WAS/STW, RAWA/TWT and SARA/TYTS. Where the deaminase motif is an APOBEC3G motif, it may be selected from among CC/GG, CG/CG, CCGW/WCGG, SCCGW/WCGGS, SCCGS/SCGGS, SCCG/CGGS, CCGS/SCGG, SCGS/SCGS and SGCG/CGCS. Where the deaminase motif is an APOBEC3B motif, it may be selected from among TCW/WGA, TCA/TGA, TCWA/TWGA, RTCA/TGAY, YTCA/TGAR, STCG/CGAS, TCGA/TCGA and WTCG/CGAW. Where the deaminase motif is an APOBEC3F motif, it may be TC/GA. Where the the deaminase motif is an APOBEC1 motif, it may be CA/TG.

In further examples of the systems, computer program products, or methods described above and herein, the motif metric group comprises a 3-mer motif metric group indicative of SNVs in one or more 3-mer motifs. The 3-mer motif metric group may be indicative of SNVs at position 1, 2 and/or 3 of the one or more 3-mer motifs. In other examples, the motif metric group comprises a 5-mer motif metric group indicative of SNVs in one or more 5-mer motifs, and the 5-mer motif metric group may be indicative of SNVs at position 1, 2, 3, 4 and/or 5 of the one or more 5-mer motifs.

In particular instances of the systems, computer program products, or methods described above and herein, the cancer therapy is selected from among radiation therapy, non-targeted chemotherapy, hormone therapy, immunotherapy or targeted therapy.

The immunotherapy or targeted therapy may comprise an antibody, such as one selected from among an antibody specific for CTLA-4, PD-1, PD-L1, CD-52, CD19, CD20, CD27, CD30, CD38, CD137, HER-2, EGFR, VEGF, VEGFR, RANKL, BAFF, Nectin-4, OX40, gpNMB, SLAM7, B4GALNT1, PDGFR$\alpha$, IL-1$\beta$, IL-6 and IL-6R. In particular embodiments, the antibody is specific for PD-1, PD-L1, CTLA-4 or HER2. In further embodiments, the antibody can induce complement dependent cytotoxicity (CDC) or antibody-dependent cellular cytotoxicity (ADCC). Non-limiting examples of antibodies include Ado-trastuzumab emtansine, Alemtuzumab, Atezolizumab, Avelumab, Belimumab, Belinostat, Bevacizumab, Blinatumomab, Brentuximab vedotin, Canakinumab, Cetuximab, Daratumumab, Denosumab, Dinutuximab, Durvalumab, Elotuzumab, Enfortumab), Glembatumumab, GSK3174998, Ibritumomab tiuxetan, Ipilimumab, Necitumumab, Nivolumab, Obinutuzumab, Ofatumumab, Olaratumab, Panitumumab, Pembrolizumab, Pertuzumab, PF-04518600, Pidilizumab, Pogalizumab, Ramucirumab, Rituximab, Siltuximab, Tavolixizumab, Tocilizumab, Tositumomab, Trastuzumab, Tremelimumab, Urelumab and Varlilumab.

In further embodiments, the targeted therapy is a small molecule, such as a tyrosine kinase inhibitor.

In any of the systems, computer program products, or methods described above and herein, the subject can have a cancer selected from among breast, prostate, liver, colorectal, gastrointestinal, pancreatic, skin, thyroid, cervical, lymphoid, haematopoietic, bladder, lung, renal, ovarian, uterine, and head or neck cancer.

Also provided is a use of a cancer therapy for treating a cancer in a subject, wherein the subject is exposed to the cancer therapy on the basis of a determination that the subject is likely to respond to the cancer therapy according to the methods described above and herein. Additionally, provided is a method for treating a cancer in a subject, comprising performing the method described above and herein for for determining the likelihood that a subject with cancer will respond to a cancer therapy or will continue to respond to a cancer therapy, and exposing the subject to the cancer therapy if it is determined that the subject is likely to respond or to continue responding to the cancer therapy.

A further aspect of the disclosure provides a method for treating a cancer in a subject, comprising: (a) sending a biological sample obtained from a subject to a laboratory to (i) conduct the method described above and herein for determining the likelihood that a subject with cancer will respond to a cancer therapy or will continue to respond to a cancer therapy; and (ii) provide the results of the method, wherein the results comprise a determination of whether the subject is likely to respond or to continue responding to the cancer therapy; (b) receiving the results from step (a); and (c) exposing the subject to the cancer therapy if the results comprise a determination that the subject is likely to respond or to continue responding to the cancer therapy.

In a further aspect, provided is a method for determining the likelihood that a subject with cancer will respond to a cancer therapy or will continue to respond to a cancer therapy, the method comprising: analyzing the sequence of a nucleic acid molecule from a subject with cancer to detect SNVs within the nucleic acid molecule; determining a plurality of metrics based on the number and/or type of SNVs detected so as to obtain a subject profile of metrics; and determining the likelihood of a subject responding to cancer therapy based on a comparison between the subject profile and a reference profile of metrics.

In one embodiment, the type and number of SNVs in a subject can be used to measure one or more genetic indicators of deaminase activity. Subjects who respond to therapy have a different profile of genetic indicators of deaminase activity compared to subjects who do not respond to therapy. Without being bound by theory, it is postulated that a profile of genetic indicators of deaminase activity that is associated with responsiveness to therapy is reflective of a deaminase-associated immune response that is functioning effectively, or that is of a quality and/or quantity that is directly or indirectly associated with the ability of the subject to respond to therapy or to continue to respond to therapy. In contrast, subjects who do not respond to therapy or who cease to respond to therapy typically have a different profile of genetic indicators of deaminase activity, and it is postulated that such a profile is reflective of a deaminase-associated immune response that is impaired or dysregulated in these subjects compared to a control group of subjects (i.e. subjects who do respond to therapy or to continue to respond to therapy.

In one embodiment, subjects who respond to therapy or continue to respond to therapy typically have a value (e.g. a percentage, ratio or number) of one or more genetic indicators of deaminase activity that is within a certain range interval (a therapy-responsive range interval). Without being bound by theory, it is postulated that the presence of genetic indicators of deaminase activity within this therapy-responsive range interval is reflective of a deaminase-associated immune response that is functioning effectively, or that is of a quality and/or quantity that is directly or indirectly associated with the ability of the subject to respond to therapy or to continue to respond to therapy. In contrast, subjects who do not respond to therapy or who cease to respond to therapy typically have a value (e.g. a percentage, ratio or number) of one or more genetic indicators of deaminase activity that is outside a therapy-responsive range interval. Again, without being bound by theory, it is postulated that the presence of genetic indicators of deaminase activity outside a therapy-responsive range interval is reflective of a deaminase-associated immune response that is impaired or dysregulated in these subjects compared to a control group of subjects (i.e. subjects who do respond to therapy or to continue to respond to therapy), or is reflective of a deaminase-associated immune response that is of a quality and/or quantity that is not associated with the ability of the subject to respond to therapy.

These genetic indicators of deaminase activity can therefore be used to predict whether or not a subject with cancer will respond to a given cancer therapy, and whether or not a subject will continue to respond to a cancer therapy. The methods disclosed herein thus provide a means for accurately predicting whether a subject is likely to respond to therapy or whether the subject is unlikely to respond to therapy, and whether a subject is likely to continue to respond to therapy or whether the subject is unlikely to continue to respond to therapy (i.e. likely to develop resistance to therapy and thus cease responding to therapy). The methods can also be extended to therapeutic applications, whereby a course of therapy is prescribed based on the information provided by the predictive methods described herein.

In one aspect, the present disclosure provides a method for determining the likelihood that a subject with cancer will respond to a cancer therapy or will continue to respond to a cancer therapy, the method comprising: analyzing the sequence of a nucleic acid molecule from a subject with cancer to detect single nucleotide variations (SNVs); measuring one or more genetic indicators of endogenous deaminase activity based on the number and/or type of SNVs detected; and determining that the subject is likely to respond to the cancer therapy or is likely to continue responding to the cancer therapy when none of the genetic indicators of endogenous deaminase activity are outside a predetermined therapy-responsive range interval for genetic indicators of endogenous deaminase activity; or determining that the subject is unlikely to respond to cancer therapy or is unlikely to continue responding to cancer therapy when at least one of the genetic indicators of endogenous deaminase activity is outside a predetermined therapy-responsive range interval for genetic indicators of endogenous deaminase activity.

A method for determining the likelihood that a subject with cancer will respond to a cancer therapy or will continue to respond to a cancer therapy, the method comprising: analyzing the sequence of a nucleic acid molecule from a subject with cancer to detect SNVs; measuring one or more genetic indicators of endogenous deaminase activity based on the number and/or type of SNVs detected; assigning a score to each genetic indicator of endogenous deaminase activity that is outside a predetermined therapy-responsive range interval for the genetic indicator of endogenous deaminase activity and combining each score to calculate a total score; and determining that the subject is likely to respond to the cancer therapy or is likely to continue responding to the cancer therapy when the total score is equal to or less than a threshold score, or determining that the subject is unlikely to respond to the cancer therapy or is unlikely to continue responding to the cancer therapy when the total score is equal to or more than a threshold score.

In particular embodiments, the endogenous deaminase is any one or more of activation-induced cytosine deaminase (AID), apolipoprotein B mRNA-editing enzyme, catalytic polypeptide-like (APOBEC) 1 cytosine deaminase (APOBEC1), APOBEC3A, APOBEC3B, APOBEC3C, APOBEC3D, APOBEC3F, APOBEC3G, APOBEC3H and an adenine deaminase acting on RNA (ADAR).

In one example, the one or more genetic indicators of endogenous deaminase activity are a measure of the number or percentage of SNVs at a deaminase motif; the number or percentage of SNVs with a specific codon context; the strand bias of SNVs; or the number or percentage of SNVs targeting adenine, thymidine, guanine or cytosine.

In a further example, the one or more genetic indicators of endogenous deaminase activity is selected from among the percentage of SNVs that are at a deaminase motif; the percentage of SNVs at MC-1 sites; the percentage of SNVs at MC-2 sites; the percentage of SNVs at MC-3 sites; the percentage of SNVs resulting from mutation of an adenine nucleotide; the percentage of SNVs resulting from mutation of a thymine nucleotide; the percentage of SNVs resulting from a mutation of a cytosine nucleotide; the percentage of SNVs resulting from mutation of a guanine nucleotide; the ratio of the percentage of SNVs resulting from mutation of a cytosine nucleotide to the percentage of SNVs resulting from a mutation of guanine nucleotide (C:G ratio); the ratio of the percentage of SNVs resulting from mutation of an adenine nucleotide to the percentage of SNVs resulting from a mutation of a thymine nucleotide (A:T ratio); the ratio of the percentage of SNVs resulting from a mutation of an adenine or a thymine nucleotide to the percentage of SNVs resulting from a mutation of a cytosine or a guanine nucleotide (AT:GC ratio); the percentage of the SNVs resulting from a mutation of an adenine nucleotide which occur at a MC-1 site; the percentage of the SNVs resulting from a mutation of an adenine nucleotide which occur at a MC-2 site; the percentage of the SNVs resulting from a mutation of an adenine nucleotide which occur at a MC-3 site; the percentage of the SNVs resulting from a mutation of a cytosine nucleotide which occur at a MC-1 site; the percentage of the SNVs resulting from a mutation of a cytosine nucleotide which occur at a MC-2 site; the percentage of the SNVs resulting from a mutation of a cytosine nucleotide which occur at a MC-3 site; the percentage of the SNVs resulting from a mutation of a guanine nucleotide which occur at a MC-1 site; the percentage of the SNVs resulting from a mutation of a guanine nucleotide which occur at a MC-2 site; the percentage of the SNVs resulting from a mutation of a guanine nucleotide which occur at a MC-3 site; the percentage of the SNVs resulting from a mutation of a thymine nucleotide which occur at a MC-1 site; the percentage of the SNVs resulting from a mutation of a thymine nucleotide which occur at a MC-2 site; the percentage of the SNVs resulting from a mutation of a thymine nucleotide which occur at a MC-3 site; the percentage of the SNVs at the AID motif WRC/GYW which occurred at a MC-2 site; the percentage of the SNVs at an AID motif GYW which involve a G>A mutation and which occur at a MC-3 site; the percentage of the SNVs at the APOBEC3B motif TCA which involve a C>T mutation and which occur at a MC-1 site; the percentage of the SNVs at the APOBEC3B motif TCA which involve a C>T mutation and which occur at a MC-3 site; transition-transversion ratio of SNVs resulting from mutation of a guanine; transition-transversion ratio of SNVs resulting from mutation of a cytosine or guanine; and the ratio of the number of SNVs resulting from mutation of an adenine nucleotide that are not in the deaminase motif WA to the number of SNVs resulting from a mutation of a thymine nucleotide that are not in the deaminase motif TW.

In some embodiments, the deaminase motif is selected from an AID motif, APOBEC1 motif, APOBEC3A motif, APOBEC3B motif, APOBEC3C motif, APOBEC3D motif, APOBEC3F motif, APOBEC3G motif, APOBEC3H motif and an ADAR motif.

In further embodiments, the one or more genetic indicators of endogenous deaminase activity include the percentage of SNVs at an AID motif; the percentage of SNVs at an ADAR motif; the percentage of SNVs at an APOBEC3G motif; and the percentage of SNVs at an APOBEC3B motif.

In a particular embodiment, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of a thymine nucleotide that occur at a MC-3 site; the percentage of the SNVs resulting from a mutation of a guanine nucleotide that occur at a MC-3 site; the percentage of the SNVs resulting from a mutation of a cytosine nucleotide that occur at a MC-1 site; the percentage of the SNVs resulting from a mutation of a cytosine nucleotide that occur at a MC-2 site; the percentage of SNVs at MC-1 sites; the percentage of SNVs at MC-2 sites; the percentage of SNVs at MC-3 sites; the percentage of SNVs resulting from mutation of an adenine nucleotide; the ratio of the percentage of SNVs resulting from mutation of an adenine nucleotide to the percentage of SNVs resulting from a mutation of a thymine nucleotide (A:T ratio); transition-transversion ratio of SNVs resulting from mutation of a cytosine or guanine.

In a further embodiment, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of a cytosine nucleotide which occur at a MC-2 site; the percentage of the SNVs resulting from a mutation of a thymine nucleotide which occur at a MC-1 site; the percentage of the SNVs resulting from a mutation of a thymine nucleotide which occur at a MC-2 site; the percentage of the SNVs resulting from a mutation of a thymine nucleotide which occur at a MC-3 site; the percentage of SNVs at MC-1 sites; the percentage of SNVs at MC-2 sites; the percentage of SNVs at MC-3 sites; the percentage of SNVs resulting from mutation of a guanine nucleotide; the percentage of SNVs resulting from mutation of an adenine nucleotide; the ratio of the percentage of SNVs resulting from mutation of an adenine nucleotide to the percentage of SNVs resulting from a mutation of a thymine nucleotide (A:T ratio); transition-transversion ratio of SNVs resulting from mutation of a cytosine or guanine.

In another embodiment, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of a cytosine nucleotide which occur at a MC-1 site (C_MC1%); the percentage of the SNVs resulting from a mutation of a cytosine nucleotide which occur at a MC-2 site; the percentage of the SNVs resulting from a mutation of a cytosine nucleotide which occur at a MC-3 site; the percentage of SNVs at MC-1 sites; the percentage of SNVs at MC-2 sites; the percentage of SNVs at MC-3 sites; the percentage of SNVs resulting from mutation of a thymine nucleotide; the percentage of SNVs resulting from mutation of an adenine nucleotide; the ratio of the percentage of SNVs resulting from mutation of an adenine nucleotide to the percentage of SNVs resulting from a mutation of a thymine nucleotide (A:T ratio); transition-transversion ratio of SNVs resulting from mutation of a guanine.

In a still further embodiment, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs at the AID motif WRC/GYW which occurred at a MC-2 site; the percentage of the SNVs at an AID motif GYW which involve a G>A mutation and which occur at a MC-3 site; the percentage of the SNVs at the APOBEC3B motif TCA which involve a C>T mutation and which occur at a MC-1 site; the percentage of the SNVs at the APOBEC3B motif TCA which involve a C>T mutation and which occur at a MC-3 site; the percentage of SNVs at MC-1 sites; the percentage of SNVs at MC-2 sites; the percentage of SNVs at MC-3 sites; the percentage of SNVs resulting from mutation of a guanine nucleotide; the percentage of SNVs resulting from mutation of a cytosine nucleotide; the ratio of the number of SNVs resulting from mutation of an adenine nucleotide that are not in the deaminase motif WA to the number of SNVs resulting from a mutation of a thymine nucleotide that are not in the deaminase motif TW; transition-transversion ratio of SNVs resulting from mutation of a guanine or cytosine.

In another embodiment, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of an adenine nucleotide which occur at a MC-1 site (A_MC1%); the percentage of the SNVs resulting from a mutation of an adenine nucleotide which occur at a MC-2 site (A_MC2%); the percentage of the SNVs resulting from a mutation of an adenine nucleotide which occur at a MC-3 site (A_MC3%); the percentage of SNVs at MC-1 sites; the percentage of SNVs at MC-2 sites; the percentage of SNVs at MC-3 sites; the percentage of SNVs resulting from mutation of an thymine nucleotide; the percentage of SNVs resulting from mutation of an adenine nucleotide; the ratio of the percentage of SNVs resulting from mutation of an cytosine nucleotide to the percentage of SNVs resulting from a mutation of a guanine nucleotide; transition-transversion ratio of SNVs resulting from mutation of a guanine or cytosine.

In some examples, the AID motif is selected from among motifs comprising the nucleic acid sequence WRC/GYW, WRCG/CGYW, WRCGS/SCGYW, WRCY/RGYW and WRCGW/WCGYW (wherein the underlined nucleotide is mutated); the ADAR motif is selected from among motifs comprising the nucleic acid sequence WA/TW, WAY/RTW, SWAY/RTWS, CWAY/RTWG, CWAA/TTWG, SARA/TYTS, SWA/TWS, WAA/TTW, WAS/STW, RAWA/TWTY, and SWA/TWS (wherein the underlined nucleotide is mutated); the APOBEC3G motif is selected from among motifs comprising the nucleic acid sequence CC/GG, CG/CG, CG/CG, TCG/CGA, CGG/CCG, CCG/CGG, NCG/CGN, CCGW/WCGG, SCCGW/WCGGS, SCCGS/SCGGS, SCCG/CGGS, CCGS/SCGG, SCGS/SCGS and SGCG/CGCS (wherein the underlined nucleotide is mutated); the deaminase motif is an APOBEC3B motif selected from among motifs comprising the nucleic acid sequence TCA/TGA, TC/GA, TCG/CGA, STCG/CGAS, TCGA/TCGA, WTCG/CGAW and TCWA/TWGA (wherein the underlined nucleotide is mutated); the deaminase motif is an APOBEC3H motif comprising the nucleic acid sequence TCW/WGA (wherein the underlined nucleotide is mutated); the deaminase motif is an APOBEC1 motif selected from among motifs comprising the nucleic acid sequence NCA/TGN, TG/CA and GG/CC (wherein the underlined nucleotide is mutated); and/or the deaminase motif is an APOBEC3A or APOBEC3F motif selected from among motifs comprising the nucleic acid sequence TC/GA and TCW/WGA (wherein the underlined nucleotide is mutated).

In particular embodiments, the subject is on the cancer therapy and the method is for determining the likelihood that the subject will continue to respond to the cancer therapy. In other embodiments, the subject is not on the cancer therapy and the method is for determining the likelihood that the subject will respond to the cancer therapy. The cancer therapy may be selected from, for example, radiation therapy, non-targeted chemotherapy, hormone therapy, immunotherapy or targeted therapy. In some examples, the immunotherapy or targeted therapy comprises an antibody, such as an antibody selected from among an antibody specific for CTLA-4, PD-1, PD-L1, CD-52, CD19, CD20, CD27, CD30, CD38, CD137, HER-2, EGFR, VEGF, VEGFR, RANKL, BAFF, Nectin-4, OX40, gpNMB, SLAM7, B4GALNT1, PDGFRα, PDGFRβ, IL-1β, IL-6 and IL-6R (e.g. Ado-trastuzumab emtansine, Alemtuzumab, Atezolizumab, Avelumab, Belimumab, Belinostat, Bevacizumab, Blinatumomab, Brentuximab vedotin, Canakinumab, Cetuximab, Daratumumab, Denosumab, Dinutuximab, Durvalumab, Elotuzumab, Enfortumab), Glembatumumab, GSK3174998, Ibritumomab tiuxetan, Ipilimumab, Necitumumab, Nivolumab, Obinutuzumab, Ofatumumab, Olaratumab, Panitumumab, Pembrolizumab, Pertuzumab, PF-04518600, Pidilizumab, Pogalizumab, Ramucirumab, Rituximab, Siltuximab, Tavolixizumab, Tocilizumab, Tositumomab, Trastuzumab, Tremelimumab, Urelumab and Varlilumab. In a particular examples, the antibody can induce complement dependent cytotoxicity (CDC) or antibody-dependent cellular cytotoxicity (ADCC). In other examples, the targeted therapy is a small molecule, such as a tyrosine kinase inhibitor.

In some embodiments, the nucleic acid molecule has been obtained from a tumour biopsy or liquid biopsy. In particular embodiments, the methods comprise obtaining a biological sample (e.g. is a tumour biopsy or liquid biopsy) from the subject and extracting the nucleic acid molecule.

In some examples, the methods further comprise providing a recommendation to the subject to: begin the cancer therapy if it is determined that the subject is likely to respond to the cancer therapy; continue the cancer therapy if it is determined that the subject is likely to continue responding to the cancer therapy; begin a different cancer therapy if it is determined that the subject is unlikely to respond to the cancer therapy; or cease the cancer therapy if it is determined that the subject is unlikely to continue responding to the cancer therapy.

The methods of the present disclosure may be useful for assessing subjects with any cancer, including, but not limited to, breast, prostate, liver, colorectal, gastrointestinal, pancreatic, skin, thyroid, cervical, lymphoid, haematopoietic, bladder, lung, renal, ovarian, uterine, and head or neck cancer.

In further aspects, the disclosure provides a use of a cancer therapy for treating a cancer in a subject, wherein the subject is exposed to the cancer therapy on the basis of a determination that the subject is likely to respond to the cancer therapy according to the methods described above and herein.

In a particular aspect, provided is a method for treating a cancer in a subject, comprising performing the method described above and herein and exposing the subject to the cancer therapy if it is determined that the subject is likely to respond or to continue responding to the cancer therapy.

In a further aspect, the disclosure provides a method for treating a cancer in a subject, comprising: (a) sending a biological sample obtained from a subject to a laboratory to (i) conduct the method described above and herein; and (ii) provide the results of the method, wherein the results comprise a determination of whether the subject is likely to respond or to continue responding to the cancer therapy; (b) receiving the results from step (a); and (c) exposing the subject to the cancer therapy if the results comprise a determination that the subject is likely to respond or to continue responding to the cancer therapy.

**BRIEF DESCRIPTION OF THE DRAWINGS**

Various examples and embodiments of the present invention will now be described with reference to the accompanying drawings, in which: -

Figure 1 is a flow chart of an example of a method for generating a therapy indicator for assessing responsiveness to cancer therapy for a biological subject;

Figure 2 is a flow chart of an example of a process for training a computational model;

Figure 3 is a schematic diagram of an example of a network architecture;

Figure 4 is a schematic diagram of an example of a processing system;

Figure 5 is a schematic diagram of an example of a client device;

Figure 6 is a flow chart of a specific example of a method of generating a therapy indicator for assessing responsiveness to cancer therapy for a biological subject;

Figures 7A and 7B are graphs illustrating examples of responders and non-responders for respective metrics;

Figures 8A and 8B are waterfall plots illustrating examples of the cumulative contribution of individual metrics to an overall therapy indicator;

Figure 9A is a graph illustrating examples of responders and non-responders for a particular metric;

Figure 9B is a graph illustrating examples of responders and non-responders for the metric of Figure 9A for a number of different cancer therapies;

Figure 10A is a graph illustrating example therapy indicators calculated using coding metrics for different subjects for a first example dataset;

Figure 10B is a graph illustrating the example impact of different coding metrics for the first example dataset;

Figure 10C is a graph illustrating example therapy indicators calculated using coding and non-coding metrics for different subjects for the first example dataset;

Figure 10D is a graph illustrating the example impact of different coding and non-coding metrics for the first example dataset;

Figure 10E is a graph illustrating example therapy indicators calculated using coding and non-coding metrics for different subjects for the first example dataset excluding non-melanoma datasets from training;

Figure 10F is a graph illustrating the example impact of different coding and non-coding metrics for the first example dataset excluding non-melanoma datasets from training;

Figure 10G is a graph illustrating example therapy indicators calculated using coding and non-coding metrics for different subjects for the first example dataset excluding non-melanoma and outlier datasets from training;

Figure 10H is a graph illustrating the example impact of different coding and non-coding metrics for the first example dataset excluding non-melanoma and outlier datasets from training;

Figure 11A is a graph illustrating example therapy indicators calculated for different subjects for a second example dataset excluding outlier datasets from training;

Figure 11B is a graph illustrating the example impact of different metrics for the second example dataset excluding outlier datasets from training;

Figure 11C is a graph illustrating example therapy indicators calculated using metrics for different subjects for the second example dataset excluding non-melanoma and outlier datasets from training;

Figure 11D is a graph illustrating the example impact of different metrics for the second example dataset excluding non-melanoma and outlier datasets from training;

Figure 12A is a graph illustrating example therapy indicators calculated for different subjects for a third example dataset excluding outlier datasets from training;

Figure 12B is a graph illustrating the example impact of different metrics for the third example dataset excluding outlier datasets from training;

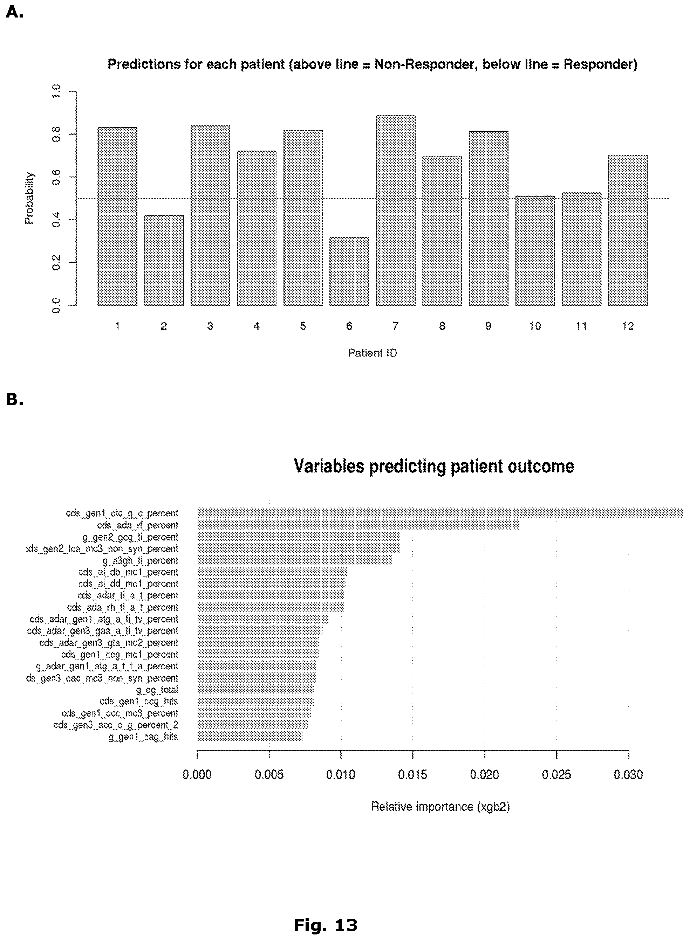

Figure 13A is a graph illustrating example therapy indicators calculated for different subjects for a fourth example dataset; and,

Figure 13B is a graph illustrating the example impact of different metrics for the fourth example dataset.

## DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (*i.e.,* to at least one) of the grammatical object of the article. By way of example, "a telomere" means one telomere or more than one telomere.

As used herein, "and/or" refers to and encompasses any and all possible combinations of one or more of the associated listed items, as well as the lack of combinations when interpreted in the alternative (or).

The term "about", as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. Therefore, about 50% means in the range of 45%-55%. Numerical ranges recited herein by endpoints include all numbers and fractions subsumed within that range (e.g., 1 to 5 includes 1, 1.5, 2, 2.75, 3, 3.90, 4, and 5). It is also to be understood that all numbers and fractions thereof are presumed to be modified by the term "about".

The term "biological sample" as used herein refers to a sample that may be extracted, untreated, treated, diluted or concentrated from a subject or patient. Suitably, the biological sample is selected from any part of a patient's body, including, but not limited to bodily fluids such as saliva or blood, tissue, cells, hair, skin and nails.

As used herein, "cancer therapy" for the purposes of the methods of the present disclosure includes non-targeted chemotherapy, radiation therapy, hormone therapy, targeted therapy and immunotherapy. In particular embodiments, the cancer therapy is targeted therapy or immunotherapy. Cancer therapy may be monovalent (i.e. a single therapy) or combination therapy.

As used herein, the term "codon context" with reference to a mutation refers to the nucleotide position within a codon at which the mutation occurs. For the purposes of the present disclosure, the nucleotide positions within a mutated codon (MC; *i.e.,* a codon containing the mutation) are annotated MC-1, MC-2 and MC-3, and refer to the first, second and third nucleotide positions, respectively, when the sequence of the codon is read 5' to 3'. Accordingly, the phrase "determining the codon context of a mutation" or similar phrase means determining at which nucleotide position within the mutated codon the mutation occurs, *i.e.,* MC-1, MC-2 or MC-3.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements.

The term "control subject", as used in the context of the present disclosure, may refer to a subject known to be responsive to a cancer therapy or known to be continually responsive to a cancer therapy (positive control), or to a subject known to be non-responsive to a cancer therapy or known to develop resistance to a cancer therapy (negative control). It is understood that control subjects can be used to obtain data for use as a standard for multiple studies, *i.e.,* it can be used over and over again for multiple different subjects. In other words, for example, when comparing a subject sample to a control sample, the data from the control sample could have been obtained in a different set of experiments, for example, it could be an average obtained from a number of responsive subjects and not actually obtained at the time the data for the subject was obtained.

The term "correlating" generally refers to determining a relationship between one type of data with another or with a state. In various embodiments, correlating deaminase activity with the likelihood that a subject will be responsive (or non-responsive) to a cancer therapy or with the likelihood that a subject will continue to respond or will develop resistance to a cancer therapy comprises assessing genetic indicators of deaminase activity in a subject and comparing the levels of these indicators to genetic indicators of deaminase activity in persons known to be responsive to that therapy or to predetermined therapy-responsive range intervals for genetic indicators of deaminase activity.

By "gene" is meant a unit of inheritance that occupies a specific locus on a genome and comprises transcriptional and/or translational regulatory sequences and/or a coding region and/or non-translated sequences (*i.e.,* introns, 5' and 3' untranslated sequences).

As used herein, a "genetic indicator of deaminase activity" refers to a number, percentage, ratio and/or type of a single nucleotide variation (SNV) that may be reflective of the activity of one or more endogenous deaminases. The term genetic indicator of deaminase activity may be used interchangeably herein with "indicator" or "metric". Genetic indicators of deaminase activity include without limitation those that are a measure of the number or percentage of SNVs at a deaminase motif, those that are a measure of the number or percentage of SNVs with a specific codon context; those that are a measure of the strand bias of SNVs, and those that are a measure of the number or percentage of SNVs targeting a particular nucleotide type (i.e. adenine, thymidine, guanine or cytosine). Exemplary genetic indicators of deaminase activity include, but are not limited to, the percentage of SNVs that are at a deaminase motif; the percentage of SNVs at MC-1 sites; the percentage of SNVs at MC-2 sites; the percentage of SNVs at MC-3 sites; the percentage of SNVs resulting from mutation of an adenine nucleotide; the percentage of SNVs resulting from mutation of a thymine nucleotide; the percentage of SNVs resulting from a mutation of a cytosine nucleotide; the percentage of SNVs resulting from mutation of a guanine nucleotide; the ratio of the percentage of SNVs resulting from mutation of a cytosine nucleotide to the percentage of SNVs resulting from a mutation of guanine nucleotide (C:G ratio); the ratio of the percentage of SNVs resulting from mutation of an adenine nucleotide to the percentage of SNVs resulting from a mutation of a thymine nucleotide (A:T ratio); the ratio of the percentage of SNVs resulting from a mutation of an adenine or a thymine nucleotide to the percentage of SNVs resulting from a mutation of a cytosine or a guanine nucleotide (AT:GC ratio); the percentage of the SNVs resulting from a mutation of an adenine nucleotide which occur at a MC-1 site; the percentage of the SNVs resulting from a mutation of an adenine nucleotide which occur at a MC-2 site; the percentage of the SNVs resulting from a mutation of an adenine nucleotide which occur at a MC-3 site; the percentage of the SNVs resulting from a mutation of a cytosine nucleotide which occur at a MC-1 site; the percentage of the SNVs resulting from a mutation of a cytosine nucleotide which occur at a MC-2 site; the percentage of the SNVs resulting from a mutation of a cytosine nucleotide which occur at a MC-3 site; the percentage of the SNVs resulting from a mutation of a guanine nucleotide which occur at a MC-1 site; the percentage of the SNVs resulting from a mutation of a guanine nucleotide which occur at a MC-2 site; the percentage of the SNVs resulting from a mutation of a guanine nucleotide which occur at a MC-3 site; the percentage of the SNVs resulting from a mutation of a thymine nucleotide which occur at a MC-1 site; the percentage of the SNVs resulting from a mutation of a thymine nucleotide which occur at a MC-2 site; the percentage of the SNVs resulting from a mutation of a thymine nucleotide which occur at a MC-3 site; the percentage of the SNVs at the AID motif WRC/GYW which occurred at a MC-2 site; the percentage of the SNVs at an AID motif GYW which involve a G>A mutation and which occur at a MC-3 site; the percentage of the SNVs at the APOBEC3B motif TCA which involve a C>T mutation and which occur at a MC-1 site; the percentage of the SNVs at the APOBEC3B motif TCA which involve a C>T mutation and which occur at a MC-3 site; transition-transversion ratio of SNVs resulting from mutation of a guanine; transition-transversion ratio of SNVs resulting from mutation of a cytosine or guanine; and the ratio of the number of SNVs resulting from mutation of an adenine nucleotide that are not in the deaminase motif WA to the number of SNVs resulting from a mutation of a thymine nucleotide that are not in the deaminase motif TW.

As used herein, the term "likelihood" or grammatical variations is used as a measure of whether response or non-response to a cancer therapy will occur; whether continued response to a cancer therapy will occur or whether development of resistance to a cancer therapy and thus relapse while on therapy will occur. In particular, it is used a measure of whether subjects with a genetic indicator of deaminase activity that is within or outside a predetermined therapy-responsive range interval will or will not respond to a cancer therapy, or will continue to respond or will develop resistance to a cancer therapy based on a given mathematical model. An increased likelihood for example may be relative or absolute and may be expressed qualitatively or quantitatively. For instance, an increased likelihood that a subject will respond to therapy, or an increased likelihood that a subject will not respond to therapy, may be expressed as determining whether any genetic indicators of deaminase activity are identified as being outside the normal reference interval (as taught herein) and placing the test subject in an "increased likelihood" category, based upon previous population studies.

In some embodiments, the methods comprise comparing a score based on the number of genetic indicators of deaminase activity that are outside a predetermined therapy-responsive range interval to a "threshold score". The threshold score is one that provides an acceptable ability to predict the likelihood of response or non-response to therapy, or of continued response or development of resistance while on therapy, in a subject, and can be determined by those skilled in the art using any acceptable means. In some examples, receiver operating characteristic (ROC) curves are calculated by plotting the value of a variable versus its relative frequency in two populations in which a first population has a first phenotype or risk and a second population has a second phenotype or risk (called arbitrarily, for example, "response" and "non-response", or "responder" and "non-responder").

A distribution of number of genetic indicators of deaminase activity that are outside a predetermined therapy-responsive range interval in subjects who respond to therapy or continue to respond to therapy and in subjects who do not respond to therapy or who develop resistance to therapy may overlap. Under such conditions, a test does not absolutely distinguish between response and non-response (or continued response and resistance) with 100% accuracy. A threshold is selected, above which the test is considered to be "positive" and below which the test is considered to be "negative." The area under the ROC curve (AUC) provides the C-statistic, which is a measure of the probability that the perceived measurement will allow correct identification of a condition (see, for example, Hanley et al, Radiology 143: 29-36 (1982)). The term "area under the curve" or "AUC" refers to the area under the curve of a receiver operating characteristic (ROC) curve, both of which are well known in the art. AUC measures are useful for comparing the accuracy of a classifier across the complete data range. Classifiers with a greater AUC have a greater capacity to classify unknowns correctly between two groups of interest. ROC curves are useful for plotting the performance of a particular feature in distinguishing or discriminating between two populations (e.g., subjects that are responders and subjects that are non-responders to therapy). Typically, the feature data across the entire population (e.g., the cases and controls) are sorted in ascending order based on the value of a single feature. Then, for each value for that feature, the true positive and false positive rates for the data are calculated. The sensitivity is determined by counting the number of cases above the value for that feature and then dividing by the total number of cases. The specificity is determined by counting the number of controls below the value for that feature and then dividing by the total number of controls. Although this definition refers to scenarios in which a feature is elevated in cases compared to controls, this definition also applies to scenarios in which a feature is lower in cases compared to the controls (in such a scenario, samples below the value for that feature would be counted). ROC curves can be generated for a single feature as well as for other single outputs, for example, a combination of two or more features can be mathematically combined (e.g., added, subtracted, multiplied, etc.) to produce a single value, and this single value can be plotted in a ROC curve. Additionally, any combination of multiple features (e.g., one or more other epigenetic markers), in which the combination derives a single output value, can be plotted in a ROC curve. These combinations of features may comprise a test. The ROC curve is the plot of the sensitivity of a test against the specificity of the test, where sensitivity is traditionally presented on the vertical axis and specificity is traditionally presented on the horizontal axis. Thus, "AUC ROC values" are equal to the probability that a classifier will rank a randomly chosen positive instance higher than a randomly chosen negative one. An AUC ROC value may be thought of as equivalent to the Mann-Whitney U test, which tests for the median difference between scores obtained in the two groups considered if the groups are of continuous data, or to the Wilcoxon test of ranks.

As used herein, "level" with reference to a SNV or genetic indicator of deaminase activity refers to the number, percentage, amount or ratio of SNV or genetic indicator of deaminase activity.

As used herein, a "metric" refers to a number, percentage, ratio and/or type of a single nucleotide variation (SNV). The metrics of the present disclosure are associated with, reflective of or indicative of the number, percentage or ratio of particular SNVs, such as SNVs in the coding region of a nucleic acid molecule (coding metric group); SNVs in the non-coding region of a nucleic acid molecule (non-coding metric group); SNVs in both the coding and non-coding region of a nucleic acid molecule (genomic metric group); SNVs where the coding context of the SNV has been assessed (codon context metric group); SNVs that have been determined to be transitions or transversions (transition/transversion metric group); SNVs that have been determined to be synonymous or non-synonymous (synonomous/non-synonymous metric group); SNVs resulting from or associated with mutational strand bias (strand bias group); SNVs in which an adenine and thymine, and/or a guanine and cytidine have been targeted/mutated (AT/GC metric group); SNVs present in specific motifs (e.g. deaminase, three-mer and/or five-mer motifs) (motif metric group); and SNVs whether present in motifs or not (motif-independent metric group). Typically, the metrics are also genetic indicators of deaminase activity.

As used herein, a "mutation type" refers to the specific nucleotide substitution that comprises the mutation, and is selected from among C to T, C to A, C to G, G to T, G to A, G to C, A to T, A to C, A to G, T to A, T to C and T to G mutations. Thus, for example, a mutation type of C to T refers to a mutation in which the targeted or mutated nucleotide C is replaced with the substituting nucleotide T.

The "nucleic acid" as used herein designates DNA, cDNA, mRNA, RNA, rRNA or cRNA. The term typically refers to polynucleotides greater than 30 nucleotide residues in length.

As used herein, a "predetermined therapy-responsive range interval" or "therapy-responsive range interval" refers to a range of values, with an upper and lower limit, for a genetic indicator of deaminase activity that are reflective of a level or quality of deaminase activity in a subject that responds to a cancer therapy, optionally over a prolonged period (i.e. continues to respond to therapy). The predetermined therapy-responsive range interval can be determined by assessing a genetic indicator of deaminase activity in two or more subjects with cancer that are respective to a given therapy (e.g. subjects that respond to specific types of targeted therapy, immunotherapy etc.). A therapy-responsive range interval for the genetic indicator is then calculated to set the upper and lower limits of what would be considered desirable values for that indicator, e.g. values that would reflect a deaminase activity in individuals that is reflective of, or helps support, response to a therapy. In a particular example, the normal range interval is calculated by measuring the average plus or minus 2 standard deviations, whereby the lower limit of the range interval is the average minus 2 standard deviations and the upper limit of the range interval is the average plus 2 standard deviations. In other examples, less than or more than 2 standard deviations is used to set the upper and lower limits of the interval, such as 0, 0.5, 1, 1.5, 2.5, 3, 3.5 or more standard deviations. In still further examples, the upper and lower limits of the predetermined therapy-responsive range interval are established using receiver operating characteristic (ROC) curves. The subjects used to determine the predetermined therapy-responsive range interval can be of any age, sex or background, or may be of a particular age, sex, ethnic background or other subpopulation. Thus, in some embodiments, two or more predetermined therapy-responsive range intervals can be calculated for the same genetic indicator of deaminase activity, whereby each range interval is specific for a particular subpopulation, e.g. a particular sex, age group, ethnic background and/or other subpopulation. The predetermined therapy-responsive range interval can be determined using any technique know to those skilled in the art, including manual methods of calculation, an algorithm, a neural network, a support vector machine, deep learning, logistic regression with linear models, machine learning, artificial intelligence and/or a Bayesian network.

As used herein, the terms "recur," "recurrence", "relapse" and the like refer to the regrowth of tumour or cancerous cells in a subject after a therapy (i.e. treatment) for the cancer or tumour has been administered. The tumour may recur in the original site or in another part of the body. In one embodiment, a tumour that recurs is of the same type as the original tumour for which the subject was administered a therapy. For example, if a subject had an ovarian cancer tumour, was treated for and subsequently developed another ovarian cancer tumour, the tumour has recurred. In addition, a cancer can recur in or metastasize to a different organ or tissue than the organ or tissue where it originally occurred.

As used herein, "resistance to cancer therapy", "cancer therapy resistance" or grammatical variations thereof refers to non-responsiveness of a cancer to a cancer therapy. The therapy may be a monotherapy or a combination therapy. Resistance to therapy includes an innate or primary resistance to therapy, where there is non-responsiveness to a therapy from the beginning of treatment such that the subject has never been responsive to that therapy, and acquired resistance to therapy where the non-responsiveness to therapy occurs following an initial period of responsiveness to that therapy, i.e. the subject develops resistance to the therapy while on the therapy. Thus, reference to the development of resistance to a cancer therapy in a subject is reference to an acquired resistance to the cancer therapy can be assessed using a range of parameters well known to those skilled in that, including cancer progression and immunological markers and/or responses.

The term "sensitivity", as used herein, refers to the probability that a predictive method or kit of the present disclosure gives a positive result when the biological sample is positive, e.g., having the predicted diagnosis. Sensitivity is calculated as the number of true positive results divided by the sum of the true positives and false negatives. Sensitivity essentially is a measure of how well the present disclosure correctly identifies those who have the predicted diagnosis from those who do not have the predicted diagnosis. The statistical methods and models can be selected such that the sensitivity is at least about 60%, and can be, e.g., at least about 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

As used herein, "single nucleotide variation" refers to a variation occurring in the sequence of a nucleic acid molecule (e.g. a subject nucleic acid molecule) compared to another nucleic acid molecule (e.g. a reference nucleic acid molecule or sequence), wherein the variation is a difference in the identity of a single nucleotide (e.g. A, T, C or G).

The term "somatic mutation" refers to a mutation in the DNA of somatic cells *(i.e.,* not germ cells), occurring after conception. "Somatic mutagenesis" therefore refers to the process by which somatic mutations occur.

The terms "subject", "individual" or "patient", used interchangeably herein, refer to any animal subject, particularly a mammalian subject. By way of an illustrative example, suitable subjects are humans. Typically, the subject presents with clinical signs of cancer as defined herein. As used herein, the term "clinical sign", or simply "sign", refers to objective evidence of cancer present in a subject. Symptoms and/or signs associated with cancer and the evaluation of such signs are routine and known in the art. Examples of signs of cancer may vary depending upon the cancer, but may include tumourigenesis, metastasis, and angiogenesis. Typically, whether a subject has a cancer, and whether a subject is responding to treatment, may be determined by evaluation of signs associated with the cancer.

As used herein, the terms "targeted somatic mutagenesis" and "TSM" refer to the process of somatic mutagenesis resulting from one or more mutagenic agents, wherein mutagenesis occurs at a targeted nucleotide within a motif, the targeted nucleotide is present at a particular position within a codon (e.g., the first, second or third position of the mutated codon reading from 5' to 3', annotated MC-1, MC-2 and MC-3, respectively), and the targeted nucleotide is mutated to a particular substituting nucleotide (*i.e.,* the mutation is of a particular mutation type, *e.g.,* C to T, not C to A or C to G). Thus, a determination that TSM is occurring requires analysis of the type of mutation (*e.g.,* C to T), the motif at which the mutation occurs (*e.g.,* WR<u>C</u>) and codon context of the mutation, *i.e.,* the position within the codon at which the mutation occurs (*e.g.,* MC-1, MC-2 or MC-3). "Targeted somatic mutagen" therefore refers to mutation resulting from TSM.

The terms "treat" and "treating" as used herein, unless otherwise indicated, refer to both therapeutic treatment and prophylactic or preventative measures, wherein the object is to inhibit, either partially or completely, ameliorate or slow down (lessen) recurrence of the targeted condition or disorder (e.g. a cancer), or one or more symptom associated therewith. The terms are also used herein to denote delaying the onset of, inhibiting (e.g., reducing or arresting the growth of), alleviating the effects of, or prolonging the life of a patient suffering from a cancer or tumour. Those in need of treatment include those diagnosed with cancer. In some embodiments, treatment refers to the eradication, removal, modification, or control of primary, regional, or metastatic cancer tissue that results from the administration of one or more therapeutic agents according to the methods of the disclosure. In other embodiments, such terms refer to the minimizing or delaying the spread of cancer resulting from the administration of one or more therapeutic agents to a subject with such a disease. In other embodiments, such terms refer to elimination of disease causing cells. The term "treatment" as used herein, unless otherwise indicated, refers to the act of treating.

As used herein, the term "treatment regimen" refers to a therapeutic regimen (i.e., after the onset of a cancer). The term "treatment regimen" encompasses natural substances and pharmaceutical agents as well as any other treatment regimen including but not limited to chemotherapy, radiotherapy, proton therapy, immunotherapy, hormone therapy, phototherapy, cryotherapy, cryosurgery, toxin therapy or pro-apoptosis therapy, high intensity focused ultrasound, dietary treatments, physical therapy or exercise regimens, surgical interventions, and combinations thereof.

## 2. Abbreviations

The following abbreviations are used throughout the application:

ADAR = adenosine deaminases acting on RNA
AID = activation-induced cytidine deaminase
APOBEC = apolipoprotein B mRNA-editing enzyme, catalytic polypeptide-like (APOBEC) cytidine deaminases
ds = double stranded
h = hours
min = minutes
NTS = non-transcribed strand
SHM = somatic hypermutation
SNV = single nucleotide variation
ss = single stranded
TS = transcribed strand
TSM = targeted somatic mutation

### Table A -Nucleotide Symbols

| A | Adenine |
|---|---|
| C | Cytosine |
| G | Guanine |
| T | Thymine |
| U | Uracil |
| R | Purine - A or G |
| Y | Pyrimidine - C or T |
| S | G or C |
| W | A or T |
| K | G or T |
| M | A or C |
| B | C or G or T |
| D | A or G or T |
| H | A or C or T |
| V | A or C or G |
| N | any base |
| K | G or T |
| - | gap |

## 3. Metrics

As described herein, SNVs identified in a nucleic acid molecule can be used to determine a plurality of metrics, which can then in turn be used to help distinguish subjects that are likely to respond to cancer therapy from subjects that are unlikely to respond to cancer therapy; and/or subjects that are likely to continue to respond to cancer therapy from subjects that are unlikely to continue to respond to cancer therapy. As will be appreciated from the description below, the metrics are determined based on the number or percentage of SNVs in any one or more regions of the nucleic acid molecules, and can include an assessment of the targeted nucleotide (i.e. whether the targeted or mutated nucleotide is an A, T, C or G), the mutation type (e.g. whether the targeted nucleotide has been mutated to an A, T, G or C, whether the mutation is a transition or transversion mutation and/or whether the mutation is synonymous or non-synonymous), the motif in which the targeted nucleotide resides, the codon context of the SNV, and/or the strand on which the SNV occurs. Any single SNV can therefore be used to generate one or more metrics, and multiple SNVs can be used to generate two more metrics, and typically at least 10, 20, 30, 40, 50, 60, 70, 80, 90, 100, 200, 300, 400, 500, 600, 800, 900, 1000, 1500, 2000, 2500, 3000, 3500, 4000, 4500, 5000, 5500 or 6000 metrics. A profile can be built based upon this plurality of metrics, whereupon subjects that are likely to respond to cancer therapy typically have a different profile to subjects that are unlikely to respond to cancer therapy; and subjects that are likely to continue to respond to cancer therapy typically have a different profile to subjects that are unlikely to continue to respond to cancer therapy.

As will be apparent from the disclosure herein, the metrics can be associated with or indicative of deaminase activity, i.e. the metrics reflect a number, percentage, ratio and/or type of a SNV that may be indicative of the activity of one or more endogenous deaminases, e.g. ADAR, AID or an APOBEC deaminase. In such instances, the metrics may be referred to as genetic indicators of deaminase activity.

The metrics may fall into one or more of several groups of metrics, including: i) a coding metric group that includes metrics associated with SNVs in a coding region of the nucleic acid molecule (i.e. any region that encodes a polypeptide); ii) a non-coding metric group that includes metrics associated with SNVs in a non-coding region of the nucleic acid molecule (e.g. an intron, promoter, 5' or 3' untranslated region, intergenic region); iii) a genomic metric group that includes metrics associated with SNVs in all regions of the nucleic acid molecule (i.e. coding and non-coding regions) iv) a codon context metric group that includes metrics associated with the codon context of SNVs; v) a transition/transversion metric group that includes metrics associated with SNVs that are transitions or transversions; vi) a synonymous/non-synonymous metric group that includes metrics associated with SNVs that are synonymous or non-synonymous; vii) a strand bias metric group that includes metrics indicative or associated with strand bias of SNVs; viii) a strand specific metric group that includes metrics associated with SNVs on a specific strand; ix) an AT/GC metric group that includes metrics associated with SNVs in which an adenine and thymine, and/or guanine and cytidine have been targeted (i.e. mutated); x) a motif metric group that includes metrics associated with SNVs in specific motifs; and xi) a motif-independent metric group including metrics associated with SNVs irrespective of motif.

Any one or more of the metrics (or genetic indicators of deaminase activity) can be assessed for the methods of the present disclosure. Typically, multiple metrics are assessed, such as at least 4, 5, 6, 7, 8, 9, 10, 11, 12, 13, 14, 15, 20, 40, 60, 80, 100, 200, 300, 400, 500, 600, 800, 900, 1000, 1500, 2000, 2500, 3000, 3500, 4000, 4500, 5000, 5500, 6000 or more. In some examples, at least 1, 2, 3, 4, 5, 6, 7, 8, 9, 10, 20, 30, 40, 50, 60, 70, 100, 200, 300, 400, 500 or more metrics from each of at least 2, 3, 4, 5, 6, 7, 8, 9 or 10 metric groups identified above are used. The particular combination of metrics (or indicators) used in the methods can be readily determined by the skilled person using the teachings herein.

3.1 Motifs

In instances where the metrics are determined using SNVs identified within a particular motif (i.e. metrics in the motif metric group), motifs may be analysed in pairs: the forward motif and the equivalent reverse complement motif. For example, a forward motif ACG represents a motif in which the underlined C is targeted (or modified), and the reverse motif is CGT, where the underlined G is targeted (or modified). As would be understood, identifying a reverse compliment motif is equivalent to identifying the forward motif on the reverse compliment DNA strand.

Motifs include those that are known or suggested deaminase motifs. Thus, the metric groups of the present disclosure also include a deaminase motif metric group indicative of metrics associated with SNVs in one or more deaminase motifs. As would be appreciated, such metrics can also be referred to as genetic indicators of deaminase activity. Endogenous deaminases are known to be involved in somatic mutagenesis, including somatic hypermutation and class switch recombination of immunoglobulin genes in B cells. In addition, endogenous deaminases are key factors in RNA editing and innate immunity. For example, a number of deaminases have been shown to be involved in editing of viral RNA and DNA as a means to inhibit or reduce viral replication, and have also been implicated in activating other factors of innate immune response to combat viral infection, including (see e.g. Samuel (2012) Curr Top Microbiol Immunol. 353; Vieira and Soares (2013) BioMed Research International, 683095; He et al. (2015) Mol Med Rep. 12(5): 6405-6414). Conversely, in some instances, deaminases appear to have a proviral function (see e.g. Samuel (2011) Virology 411:180-193). Deaminases also cause somatic mutations during oncogenesis and in some instances have been associated with cancer progression and its promotion (Chan et al. (2014) Hepatology 63: 832 - 843; Leonard et al. (2013) Cancer Res. 73:7222-7231; Lindley et al. 2016 Cancer Med. 5: 2629-2640). There is also a growing view that somatic mutation activity such as that resulting from deaminase activity is an early sign of cancer (Tomasetti et al. (2013) Proc. Natl. Acad. Sci. U. S. A. 110:1999-2004; Vogelstein et al. (2013) Science 339: 1456 -1558).

Endogenous deaminases include, for example, activation-induced cytidine deaminase (AID) and apolipoprotein B mRNA-editing enzyme, catalytic polypeptide-like (APOBEC) cytidine deaminases, adenosine deaminases such as adenosine deaminases acting on RNA, and error-prone DNA polymerases such as DNA polymerase eta. These endogenous deaminases preferentially target specific motifs in the nucleic acid. Moreover, there can be both a strand bias and codon context associated with mutation events resulting from these deaminases, as described in, for example, WO 2014/066955 and Lindley et al. (2016) Cancer Med. 2016 Sep; 5(9): 2629-2640.

Activation-induced cytidine deaminase (AID) is an important enzyme in adaptive immunity, involved in somatic hypermutation (SHM) and class switch recombination of immunoglobulin genes in B cells. AID triggers SHM by deaminating cytidines to uracils (C to U) to diversify the immunoglobulin variable region genes (VDJ) and create new antigen-binding sites. If unrepaired, the deamination of C to U by AID gives rise to C to T somatic mutations in DNA. The editing activity of AID is not restricted to Ig loci, nor even to B cells. Rather, it has been shown to play a role in somatic mutagenesis in environments and cells types. AID has been shown to be involved in the innate antiviral immune response, including editing the DNA and RNA of various viruses such as HBV (see e.g. He et al. (2015) Mol Med Rep. 12(5): 6405-6414, Liang et al. Proc Natl Acad Sci U S A. 110(6):2246-51), and has also been implicated in cancer development (Okazaki et al (2007) Adv Immunol. 94:245-73).

In addition to AID, the human genome encodes several homologous APOBEC cytidine deaminases that are known to be involved in innate immunity and RNA editing (Smith et al. (2012) Semin. Cell. Dev. Biol. 23:258-268). In humans, at least APOBEC1, APOBEC3A, APOBEC3B, APOBEC3C, APOBEC3D, APOBEC3F, APOBEC3G and APOBEC3H are involved in providing innate immunity and/or cellular mRNA editing. These APOBEC deaminases have been shown to be involved in the innate antiviral immune response, editing the DNA and RNA of various viruses (including HBV) and disrupting viral replication (see e.g. Turelli et al. (2004) Science 303: 1829; Suspène et al. (2005) Proc Natl Acad Sci USA 102(23): 8321-8326; Nguyen et al. (2007) J. Virol. 81:4465 - 4472; Kock and Blum (2008) J Gen. Virol. 89: 1184-1191; He et al. (2015) Mol Med Rep. 12(5): 6405-6414; Willems et al. (2015) Viruses 7: 2999-3018). APOBEC cytidine deaminases have also been associated with cancer (see e.g. Roberts et al. (2013) Nature Genetics 45, 970-976; Harris et al. (2015) Breast Cancer Res. 17: 8; Nik-Zainal et al. (2012) Cell 149:979-993). Like AID, the APOBEC cytidine deaminases cause somatic point mutations in their target DNA via their DNA editing activity on single stranded DNA usually exposed during transcription and reverse transcription. Deamination of cytosine in DNA to uracil (C-to-U), if left unrepaired by the DNA repair machinery, manifests as C-to-T or reverse complement G-to-A mutations. Thus, these deaminases (along with AID) can leave their own particular C-to-U lesions and abasic sequelae in a motif-specific way.

Double-stranded RNA-specific adenosine deaminases, or ADAR, enzymes, are responsible for the type of RNA editing that is most prevalent in higher eukaryotes, i.e. conversion of adenosine residues into inosine (A-to-I editing). ADAR is an enzyme that is encoded by the ADAR gene in humans. The ADAR1 enzyme destabilizes dsRNA through conversion of adenosine to inosine. The ADAR1 enzyme modifies cellular and viral RNA, including coding and noncoding RNAs. ADAR1 is an RNA editing enzyme, required for hematopoiesis. Regulated levels of ADAR1 expression are critical for embryonic erythropoiesis in the liver. Mutations in the ADAR gene have been associated with dyschromatosis symmetrica hereditaria. Alternate transcriptional splice variants, encoding different isoforms, have been characterized. Like other deaminases, ADARs have been implicated in the innate immune response, including the innate immune response to viruses. However, their precise role is in many instances not well defined, and in some examples, there appears to be both and antiviral and proviral role for ADARs (see e.g. Samuel (2011) Virology 411(2):180-93). ADAR activity has also been associated with cancer, with elevated ADAR expression and activity demonstrated in some cancers (Gallo and Gallardi (2008) RNA Biol. 5:135-139; Galeano et al. (2012) Semin Cell Dev Biol. 23(3):244-50; Amin et al. (2017) Sci. Signal, 10:3941).

Table B sets forth exemplary deaminase motifs, and any one or more of the motifs can be used to generate the metrics of the disclosure. The primary motif for AID is WRC/GYW and there are six secondary motifs (b-g). The primary motif for ADAR is WA/TW, and there are nine secondary motifs (b-j). The primary motif for APOBEC3G (A3G) is CC/GG, and there are eight secondary motifs (b-i). The primary motif for APOBEC3B (A3B) is TCW/WGA, and there are seven secondary motifs (b-i). The motif for APOBEC3F (A3F) is TC/GA and the motif for APOBEC1 (A1) is CA/TG.

Table B. Exemplary deaminase motifs

| Motif Name | Forward Motif | | Reverse Compliment Motif |
|---|---|---|---|
| AID | W R **C** | / | **G** Y W |
| ADAR | W **A** | / | **T** W |
| A3G | C **C** | / | **G** G |
| A3B | T **C** W | / | W **G** A |
| AIDb | W R **C** G | / | C **G** Y W |
| AIDc | W R **C** G S | / | S C **G** Y W |
| AIDd | W R **C** Y | / | R **G** Y W |
| AIDe | W R **C** G W | / | W C **G** Y W |

(continued)

| Motif Name | Forward Motif | | Reverse Compliment Motif |
|---|---|---|---|
| AIDf | W R **C** R | / | Y **G** Y W |
| AIDg | A G **C** T N T | / | A N A **G** C T |
| ADARb | W **A** Y | / | R **T** W |
| ADARc | S W **A** Y | / | R **T** W S |
| ADARd | C W **A** Y | / | R **T** W G |
| ADARe | C W **A** A | / | T **T** W G |
| ADARf | S W **A** | / | **T** W S |
| ADARg | W **A** A | / | T **T** W |
| ADARh | W **A** S | / | S **T** W |
| ADARi | R A W **A** | / | **T** W T Y |
| ADARj | S **A** R A | / | T Y **T** S |
| A3Gb | **C** G | / | C **G** |
| A3Gc | C **C** G W | / | W C **G** G |
| A3Gd | S C **C** G W | / | W C **G** G S |
| A3Ge | S C **C** G S | / | S C **G** G S |
| A3Gf | S C **C** G | / | C **G** G S |
| A3Gg | C **C** G S | / | S C **G** G |
| A3Gh | S **C** G S | / | S C **G** S |
| A3Gi | S G **C** G | / | C **G** C S |
| A3Bb | T **C** A | / | T **G** A |
| A3Bc | T **C** W A | / | T W **G** A |
| A3Bd | R T **C** A | / | T **G** A Y |
| A3Be | Y T **C** A | / | T **G** A R |
| A3Bf | S T **C** G | / | C **G** A S |
| A3Bg | T **C** G A | / | T C **G** A |
| A3Bh | W T **C** G | / | C **G** A W |
| A3F | T **C** | / | **G** A |
| A1 | **C** A | / | T **G** |

Any one or more deaminase motifs can be assessed as described herein to generate the metrics. In some examples at least one ADAR motif (e.g. 1, 2, 3, 4, 5 or 6 ADAR motifs), at least one AID motif (e.g. 1, 2, 3, 4, 5, 6, 7, 8 or 9 AID motifs), at least one APOBEC3G motif (e.g. 1, 2, 3, 4, 5, 6, 7 or 8 APOBEC3G motifs), at least one APOBEC3B motif (e.g. 1, 2, 3, 4, 5, 6 or 7 APOBEC3B motifs), at least one APOBEC3F and/or at least one APOBEC1 motif is assessed to determine the number and/or type of SNVs at that particular motif, and to thereby generate the metrics.

In further examples, the motifs are not necessarily deaminase motifs. Included among such motifs are general three-mer motifs in which a SNV is detected in one of the positions in the three-mer: M1, M2 or M3. For the purposes herein, typically the targeted nucleotide is an A or C, which may represent a deamination event (although does not necessarily do so). For example, the motif M1 M2 M3 represents a motif in which the targeted (underlined) nucleotide at position M1 is A or C, and the nucleotides at positions M2 and M3 are each independently A, T, G or C. The motif M1 M2 M3 represents a motif in which the targeted (underlined) nucleotide at position M2 is A or C, and the nucleotides at non-targeted positions M1 and M3 are each independently A, T, G or C. The motif M1 M2 M3 represents a motif in which the targeted (underlined) nucleotide at position M3 is A or C, and the nucleotides at non- targeted positions M1 and M2 are each independently A, T, G or C. Thus, there are ninety-six (96) possible three-mer forward motifs of this type, with each motif being associated with the corresponding reverse compliment motif. In further embodiments, metrics can be determined using such three-mer motifs but with the nucleotides at the non-targeted positions being any one of A, T, C, G, R, Y, S, W, K, M or N, resulting in 726 possible motifs.

Non-limiting examples of three-mer motifs include those sometimes referred to below (e.g. Table G) as Gen2 motifs: ACA/TGT, TCA/TGA, CCA/TGG, GCA/TGC, ACT/AGT, TCT/AGA, CCT/AGG, GCT/AGC, ACC/GGT, TCC/GGA, CCC/GGG, GCC/GGC, ACG/CGT, TCG/CGA, CCG/CGG, GCG/CGC; those sometimes referred to below (e.g. Table G) as ADAR Gen2 motifs AAA/TTT, TAA/TTA, CAA/TTG, GAA/TTC, AAT/ATT, TAT/ATA, CAT/ATG, GAT/ATC, AAC/GTT, TAC/GTA, CAC/GTG, GAC/GTC, AAG/CTT, TAG/CTC, CAG/CTG, GAG/CTC; those sometimes referred to below (e.g. Table G) as ADAR Gen1 motifs: AAA/TTT, AAT/ATT, AAC/GTT, AAG/CTT, ATA/TCT, ATT/AAT, ATC/GAT, ATG/CAT, ACA/TGT, ACT/AGT, ACC/GGT, ACG/CGT, AGA/TCT, AGT/ACT, AGC/GCT, AGG/CCT; those sometimes referred to below (e.g. Table G) as ADAR Gen3 motifs: AAA/TTT, ATA/TAT, ACA/TGT, AGA/TCT, TAA/TTA, TTA/TAA, TCA/TGA, CAA/TTG, CTA/TAG, CCA/TGG, CGA/TCG, GAA/TTC, GTA/TAC, GCA/TGC, GGA/TCC; those sometimes referred to below (e.g. Table G) as Gen1 motifs: CAA/TTG, CTA/TAG, CCA/TGG, CGA/TCG, CAT/ATG, CTT/AAG, CCT/AGG, CGT/ACG, CAC/GTG, CTC/GAG, CCC/GGG, CGC/GCG, CAG/CTG, CTG/CAG, CCG/CGG, CGG/CCG; and those sometimes referred to below (e.g. Table G) as Gen3 motifs AAC/GTT, ATC/GAT, ACC/GGT, AGC/GCT, TAC/GTA, TTC/GAA, TCC/GGA, TGC/GCA, CAC/GTG, CTC/GAG, CCC/GGG, CGC/GCG, GAC/GTC, GTC/GAC, GCC/GGC, GGC/GCC.

Other motifs include general five-mer motifs in which a SNV is detected in one of the positions in the five-mer: M1, M2, M3, M4 or M5. For the purposes herein, typically the targeted position is M2, M3 or M4 and the nucleotide is an A or C, which may represent a deamination event (although does not necessarily do so). For example, the motif M1 M2 M3 M4 M5 represents a motif in which the targeted (underlined) nucleotide at position M2 is A or C, and the nucleotides at non-targeted positions M1, M3, M4 and M5 are each independently A, T, G or C. The motif M1 M2 M3 M4 M5 represents a motif in which the targeted (underlined) nucleotide at position M3 is A or C, and the nucleotides at non-targeted positions is M1, M2, M4 and M5 are each independently A, T, G or C. The motif M1 M2 M3 M4 M5 represents a motif in which the targeted (underlined) nucleotide at position M4 is A or C, and the nucleotides at non-targeted positions M1, M2, M3 and M5 are each independently A, T, G or C. Thus, there are 1536 possible five-mer forward motifs of this type, with each motif being associated with the corresponding reverse compliment motif. In further embodiments, metrics can be determined using such five-mer motifs but with the nucleotides at the non-targeted positions being any one of A, T, C, G, R, Y, S, W, K, M or N, resulting in 87,846 possible motifs.

The motif metrics may reflect (and thus be generated by assessing) the number or percentage of total SNVs in the nucleic acid molecules that are at a particular motif. In further embodiments, motif metrics can be generated by detecting, and can therefore indicate, the particular type of mutation at the targeted nucleotide, e.g. whether there is an A, C or T substituting a targeted G. Further, the metrics can indicate whether the targeted nucleotide is at any position within the codon (*i.e.* at MC-1, MC-2 or MC-3, as described below). Thus, in some examples, motif metrics can represent a number, percentage or ratio of any SNV at a targeted position in a motif (e.g. a deaminase motif), wherein the targeted nucleotide is at any position within the codon. The percentage of SNVs at the motif is therefore calculated by dividing the total number of SNVs at the motif (regardless of the type of the mutation or codon context of the mutation) by the total number of SNVs in nucleic acid molecule. In other examples, however, only SNVs that are particular types of mutations, such as transition mutations (i.e. C>T, G>A, T>C and A>G), at a motif are considered in the assessment and metric reflects the percentage, number or ratio of such mutations. In still further embodiments, both the codon context and the type of mutation is assessed, as described below.

Exemplary motif metrics are provided in Section 3.7.10 below.

3.2 Codon context

Mutagens, including deaminases, can target nucleotides in a codon context manner (as described in, for example, WO 2014/066955 and Lindley et al. (2016) Cancer Med. 2016 Sep; 5(9): 2629-2640). Specifically, mutagenesis can occur at a targeted nucleotide, wherein the targeted nucleotide is present at a particular position within a codon. For the purposes of the present disclosure, the nucleotide positions within a mutated codon (MC; *i.e.,* a codon containing the mutation) are annotated MC-1, MC-2 and MC-3, and refer to the first, second and third nucleotide positions, respectively, of the codon when the sequence of the codon is read 5' to 3'.

Metrics of the present disclosure can be based, at least in part, on a determination of the codon context of a SNV, i.e. whether the SNV is at the first, second or third position in the mutated codon, i.e. the MC-1, MC-2 or MC-3 site. As noted above, many deaminases have a preference for targeting nucleotides at a particular position within the mutated codon. As such, the number and/or percentage of SNVs that occur at a MC-1, MC-2 or MC-3 site can be a genetic indicator of deaminase activity. As would be appreciated, codon-context metrics are only assessed in the coding region of the nucleic acid molecule.

Metrics based on an assessment of the codon context of a SNV can be motif-independent (i.e. an assessment of the number and/or percentage of SNVs at a particular codon regardless of whether or not the targeted nucleotide is within a particular motif). Thus, these metrics (or genetic indicators of deaminase activity) include the number and/or percentage of total SNVs that occur at a MC-1 site; the number and/or percentage of total SNVs that occur at a MC-2 site; and or the number and/or percentage of total SNVs that occur at a MC-3 site.

In other embodiments, a simultaneous assessment of whether the SNV is at a motif, such as a deaminase motif, three-mer motif or five-mer motif (as described above) is also made. Thus, the metrics include codon-context, motif-dependent metrics that are based on the number and/or percentage of SNVs within in a particular motif and at a MC-1 site, MC-2 site and/or MC-3 site. Where the motifs are deaminase motifs, the metrics can be considered as genetic indicators of deaminase activity, and include the number and/or percentage of SNVs that are attributable to a particular motif at a MC-1 site, MC-2 site and/or MC-3 site, such as the number and/or percentage of SNVs that are attributable AID (i.e. that are at an AID motif) and that occur at a MC-1 site, MC-2 site and/or MC-3 site; the number and/or percentage of SNVs that are attributable to ADAR (i.e. that are at an ADAR motif) and that occur at a MC-1 site, a MC-2 site and/or a MC-3 site; the number and/or percentage of SNVs that are attributable to an APOBEC deaminase (i.e. that are at an APOBEC motif, such as a APOBEC1, APOBEC3A, APOBEC3B, APOBEC3C, APOBEC3D, APOBEC3F, APOBEC3G or APOBEC3H motif) and that occur at a MC-1 site, MC-2 site and/or a MC-3 site.

The codon-context metrics also include those that take into account not only the codon context, but also the nucleotide that is targeted (i.e. the nucleotide that is mutated to another nucleotide to produce the SNV). Thus, the metrics (or genetic indicators of deaminase activity) include the number or percentage of SNVs resulting from mutation of an adenine which are at the MC1 position, MC2 position and/or MC3 position. For example, the number of SNVs resulting from mutation of an adenine may be determined, and the percentage of these that are at a MC-1 site, MC-2 site and/or MC-3 site is then determined to generate the metric. Similarly, the number or percentage of SNVs resulting from mutation of a thymine that occurred at the MC1 position, the MC2 position and/or the MC3 position; the number or percentage of SNVs resulting from mutation of a cytosine that occurred at the MC1 position, the MC2 position, and/or the MC3 position; the number or percentage of SNVs resulting from mutation of a guanine that occurred at the MC1 position, the MC2 position, and/or the MC3 position can be assessed to generate the metrics (or genetic indicators of deaminase activity).

In further embodiments, both the type of mutation that results in the SNV (e.g. C>A, C>T, C>G, G>C, G>T, G>A, A>T, A>G, A>C, T>A, T>C or T>G) and the codon context of the SNV is assessed, so as to determine the number or percentage of a particular type of mutation at a MC-1, MC-2 or MC-3 site. Again, in some embodiments, this is performed without a simultaneous assessment of whether the SNV is at a motif associated with a particular deaminase. Thus, metrics (or genetic indicators of deaminase activity) include, for example, the number or percentage of C>T mutations at the MC1 site (typically indicative of AID, APOBEC3B or APOBEC3G activity); the number or percentage of C>T mutations at the MC2 site (typically indicative of AID, APOBEC3B or APOBEC3G activity); the number or percentage of C>T mutations at the MC3 site (typically indicative of AID, APOBEC3B or APOBEC3G activity); the number or percentage of G>A mutations at the MC1 site (typically indicative of AID, APOBEC3B or APOBEC3G activity); the number or percentage of G>A mutations at the MC2 site (typically indicative of AID, APOBEC3B or APOBEC3G activity); the number or percentage of G>A mutations at the MC3 site (typically indicative of AID, APOBEC3B or APOBEC3G activity); the number or percentage of T>C mutations at the MC1 site (typically indicative of ADAR activity); the number or percentage of T>C mutations at the MC2 site (typically indicative of ADAR activity); the number or percentage of T>C mutations at the MC3 site (typically indicative of ADAR activity); the number or percentage of A>G mutations at the MC1 site (typically indicative of ADAR activity); the number or percentage of A>G mutations at the MC2 site (typically indicative of ADAR activity); and the number or percentage of A>G mutations at the MC3 site (typically indicative of ADAR activity).

In other embodiments, an assessment of whether the SNV is at a motif (e.g. a deaminase, three-mer or five-mer motif), what type of mutation results in the SNV, and also the codon context of the SNV is made to generate the codon context metric. In non-limiting examples, an assessment of the number and/or percentage of SNVs that are a C>T, C>A or A>G mutation at a AID motif; an A>T mutation at an ADAR motif; a C>T or G>A mutation an APOBEC3G motif; a G>A mutation at an APOBEC3H motif; or a G>A or G>T mutation an APOBEC1 motif, is determined to generate the metrics (or genetic indicators of deaminase activity).

Exemplary codon context metrics are provided in Section 3.7.4 below.

3.3 Transitions/transversions

Transitions (Ti) are defined as any mutation of a purine to a purine, or a pyrimidine to a pyrimidine (i.e. C>A, G>T, A>C and T>G, and transversions (Tv) are defined as any mutation of a pyrimidine to a purine or purine to a pyrimidine (i.e. C>T, C>G, G>A, G>C, A>G, A>T, T>C and T>A). The transition/transversion metric group therefore includes metrics determined from or associated with SNVs that are transitions or transversions, and include, for example, the number or percentage of SNVs that are transitions or transversions, or the ratio of transitions to transversions or transversions to transitions).

In some embodiments, the motif, codon context and/or specific mutation type is also assessed. Thus, for example, the transition/transversion metric group can include metrics that reflect the number or percentage of SNVs at a motif that are transitions (or transversions), the number or percentage of SNVs that target a particular nucleotide (e.g. C, G, A or T) that are transitions (or transversions), or assess both the motif and nucleotide. Thus, included are metrics that reflect, for example, the transition-transversion ratio of all SNVs resulting from mutation of an adenine, i.e. ratio of all SNVs resulting from a transition mutation of an adenine (i.e. A>G) to all SNVs resulting from a transversion mutation of an adenine (i.e. A>T or C); the transition-transversion ratio of all SNVs resulting from mutation of a thymine i.e. ratio of all SNVs resulting from a transition mutation of a thymine (i.e. T>C) to all SNVs resulting from a transversion mutation of a thymine (i.e. T>G or A); the transition-transversion ratio of all SNVs resulting from mutation of a cytosine i.e. ratio of all SNVs resulting from a transition mutation of a cytosine (i.e. C>T) to all SNVs resulting from a transversion mutation of a cytosine (i.e. C>G or A) and the transition-transversion ratio of all SNVs resulting from mutation of an guanine, i.e. ratio of all SNVs resulting from a transition mutation of a guanine (i.e. G>A) to all SNVs resulting from a transversion mutation of a guanine (i.e. G>C or T); the transition-transversion ratio of all SNVs resulting from mutation of an adenine or thymine, i.e. ratio of all SNVs resulting from a transition mutation of an adenine (i.e. A>G) or thymine (i.e. T>C) to all SNVs resulting from a transversion mutation of an adenine (i.e. A>T or C) or thymine (i.e. T>G or A); and the transition-transversion ratio of SNVs resulting from mutation of an cytosine or guanine, i.e. ratio of all SNVs resulting from a transition mutation of a guanine (i.e. G>A) or cytosine (i.e. C>T) to all SNVs resulting from a transversion mutation of a guanine (i.e. G>C or T) or cytosine (i.e. C>G or A). In further examples, the motif is also taken into account when assessing the SNVs to generate the metrics, such that the percentage or ratio is of SNVs resulting from transition or transversion of a nucleotide at one or more deaminase motifs.

Exemplary transition/transversion metrics are provided in Section 3.7.5 below.

### 3.4 Synonymous/non-synonymous mutations

Metrics of the present disclosure may also reflect or be based on SNVs that are synonymous or non-synonymous mutations in the nucleic acid molecule. Synonymous mutations are those that have no effect on the encoded polypeptide, while non-synonymous mutations result in a modification of the amino acid sequence of the encoded polypeptide. The synonymous/non-synonymous metric group therefore includes metrics determined from or associated with SNVs that are synonymous or non-synonymous mutations, and include, for example, the number or percentage of SNVs that are synonymous or non-synonymous, or the ratio of synonymous to non-synonymous mutations or non-synonymous to synonymous mutations.

In some embodiments, the motif, codon context and/or specific mutation type is also assessed. Thus, for example, the synonymous/non-synonymous metric group can include metrics that reflect the percentage of SNVs at a motif that are synonymous (or non-synonymous), or the percentage of SNVs at a motif that are synonymous (or non-synonymous) and that targeted a particular nucleotide (e.g. C, G, A or T).

Exemplary synonymous/non-synonymous metrics are provided in Section 3.7.6 below.

### 3.5 Strand bias

As noted above, deaminases can exhibit strand bias in their targeting of nucleotides. Evidence of strand bias of mutations can therefore also be an indicator of deaminase activity, and metrics that reflect strand bias (i.e. metrics in the strand bias metric group) are therefore included in the present disclosure. Strand bias can be assessed, for example, by calculating the percentage of mutations targeting a particular nucleotide, or the various ratios of these mutations. For example, the ratio of the number or percentage of SNVs that include mutation of a cytosine nucleotide to the percentage of SNVs that include mutation of a guanine nucleotide (C:G ratio) can be determined. In another example, the ratio of the number or percentage of SNVs that include mutation of an adenine nucleotide to the percentage of SNVs that include mutation of a thymine nucleotide (A:T ratio) is determined. In some embodiments, the motif or codon context of the SNV is also assessed. Thus, for example, where the motif includes a targeted cytidine nucleotide in the forward motif and thus a guanine nucleotide in the reverse motif, the metric is determined by assessing the percentage of SNVs at the motif that target the C (which essentially then reflects the C:G ratio).

Exemplary strand bias metrics are provided in Section 3.7.7 below.

### 3.6 Strand specificity

Metrics of the present disclosure can also include those based on SNVs identified on just one strand of DNA, i.e. the non-transcribed (or sense or coding) strand or the transcribed (or antisense or template) strand (or "C" or "G" strand, respectively, when mutations of/from C or G are assessed; or "A" or "T" strand, respectively, when mutations of/from A or T are assessed. These strand specific metrics typically include as assessment of the number or percentage of mutations from (or of) a particular targeted (or mutated) nucleotide (e.g. A, T, C or G) on a given strand. Given that particular deaminases can have a preference for targeting a particular nucleotide in a nucleic acid molecule, such metrics can be considered genetic indicators of deaminase activity. For example, adenines are often the target of ADAR, while cytosines are often the target of AID or APOBEC deaminases. Thus, metrics (or genetic indicators of deaminase activity) can represent the number or percentage of SNVs resulting from a mutation of an adenine nucleotide (e.g. detecting the total number of mutations of A>C, A>T and A>G and expressing this total as a percentage of the total number of SNVs detected); the number or percentage of SNVs resulting from a mutation of a thymine nucleotide (e.g. detecting the total number of mutations of T>C, T>A and T>G and expressing this total as a percentage of the total number of SNVs detected); the number or percentage of SNVs resulting from a mutation of a cytosine nucleotide (e.g. detecting the total number of mutations of C>A, C>T and C>G and expressing this total as a percentage of the total number of SNVs detected); and/or the number or percentage of SNVs resulting from a mutation of a guanine nucleotide (e.g. detecting the total number of mutations of G>C, G>T and G>A and expressing this total as a percentage of the total number of SNVs detected). These can also be an indication of strand bias, as they can show an imbalance in the total number of mutations of A, T, G or C nucleotides. In a further example, the nucleotide to which the targeted nucleotide is mutated to is also assessed. For example, the metric may represent the number or percentage of all SNVs that target A that are A>C mutations.

Exemplary strand specific metrics are provided in section 3.7.8 below.

3.7 <u>AT and GC SNVs</u>

Metrics can also include an assessment of combined SNVs mutating or targeting adenine and thymine (AT) and/or combined SNVs mutating or targeting guanine and cytosine (GC). The number and/or percentage of SNVs at AT or GC can be assessed. In further instances, a ratio is calculated, such as a ratio of the number or percentage of SNVs that include mutation of an adenine or a thymine nucleotide to the number or percentage of SNVs that include mutation of a cytosine or a guanine nucleotide (AT:GC ratio) is determined. In further instances, the codon context of the AT or GC SNVs can be taken into consideration to generate the metrics.

Exemplary strand specific metrics are provided in Section 3.7.8 below.

3.8 <u>Exemplary Metrics</u>

**3.8.1 Coding Region Metrics**

Metrics can be determined using SNVs identified in just the coding region (also referred to as the coding sequence or CDS) of a nucleic acid molecule. Exemplary coding region metrics include the mostly motif-associated metrics provided in Table C (with the exception of "CDS variants" which represents the total number of SNVs in the coding region) and the motif-independent metrics provided in Table D. These tables provide the metric name, a brief description of what the metric represents, and how the metric was calculated/determined. Reference to "motif" in the table refers to any one of the motifs described above in section 3.1, including any one of the deaminase, three-mer or five-mer motifs. Metrics at rows 8-19, 21-25 and 26-27 are utilized in the alternative and where relevant. For example, where a motif comprises a C or G at the targeted nucleotide, the metric that assesses SNVs at these G or C nucleotides is used, and where a motif comprises an A or T at the targeted nucleotide, the alternative metric that assesses SNVs at these A or T nucleotides is used (i.e. the metrics in italics).

Typically, multiple coding region metrics are determined and/or used in the methods and systems described herein, such as at least 4, 5, 6, 7, 8, 9, 10, 11, 12, 13, 14, 15, 20, 40, 60, 80, 100, 200, 300, 400, 500, 600, 800, 900, 1000, 1500, 2000, 3000, 4000 or more.

Table C: Motif-associated coding region metrics.

|   | **Metric Name** | **Description of metric** | **Calculation of metric** |
|---|---|---|---|
| 1 | **CDS Variants** | Total number of CDS variants | #CDS |
| 2 | **Motif Hits** | Number of "motif" hits | #motif |
| 3 | **Motif %** | number of "motif" hits/#CDS hits as a % | #motif/#CDS |
| 4 | **Motif Ti %** | number of "motif" hits which are transitions /#CDS hits as a % | #motif_Ti/#CDS |
| 5 | **Motif MC1 %** | % motif hits which are at MC1 | #motif_MC1/#motif |
| 6 | **Motif MC2 %** | % motif hits which are at MC2 | #motif_MC2/#motif |
| 7 | **Motif MC3 %** | % motif hits which are at MC3 | #motif_MC3/#motif |

(continued)

| | | Metric Name | Description of metric | Calculation of metric |
|---|---|---|---|---|
| 8 | | **Motif C>T at MC1 %** | % motif C>T hits which are at MC1 (of all C>T) | #motif_C>T_MC1 / #motif_C>T_all |
| | | *Motif A>G at MC1 %* | *% motif A>G hits which are at MC1 (of all C>T)* | #motif_A>G_MC1 / #motif_A>G_all |
| 9 | | **Motif C>T at MC2 %** | % motif C>T hits which are at MC2 | #motif_C>T_MC2 / #motif_C>T_all |
| | | *Motif A>G at MC2 %* | *% motif A>G hits which are at MC2 (of all A>G)* | #motif_A>G_MC2 / #motif_A>G_all |
| 10 | | **Motif C>T at MC3 %** | % motif C>T hits which are at MC3 | #motif_C>T_MC3 / #motif_C>T_all |
| | | *Motif A>G at MC3 %* | *% motif A>G hits which are at MC3 (of all A>G)* | #motif_A>G_MC3 / #motif_A>G_all |
| 11 | | **Motif G>A at MC1 %** | % motif G>A hits which are at MC1 (of all G>A) | #motif_G>A_MC1 / #motif_G>A_all |
| | | **Motif T>C at MC1 %** | % motif T>C hits which are at MC1 (of all T>C) | #motif_T>C_MC1 / #motif_T>C_all |
| 12 | | **Motif G>A at MC2 %** | % motif G>A hits which are at MC2 | #motif_G>A_MC2 / #motif_G>A_all |
| | | **Motif T>C at MC2 %** | % motif T>C hits which are at MC2 (of all T>C) | #motif_T>C_MC2 / #motif_T>C_all |
| 13 | | **Motif G>A at MC3 %** | % motif G>A hits which are at MC3 | #motif_G>A_MC3 / #motif_G>A_all |
| | | **Motif T>C at MC3 %** | % motif T>C hits which are at MC3 (of all T>C) | #motif_T>C_MC3 / #motif_T>C_all |
| 14 | | **Motif C>T %** | % motif hits that are C>T/ of all C mutations | #motif_C>T/#motif_C |
| | | **Motif A>G %** | % motif hits that are A>G/ of all A mutations | #motif_A>G/#motif_A |
| 15 | | **Motif C>A %** | % motif hits that are C>A/ of all C mutations | #motif_C>A/#motif_C |
| | | **Motif A>C %** | % motif hits that are A>C/ of all A mutations | #motif_A>C/#motif_A |
| 16 | | **Motif C>G %** | % motif hits that are C>G/ of all C mutations | #motif_C>G/#motif_C |
| | | **Motif A>T %** | % motif hits that are A>T/ of all A mutations | #motif_A>T/#motif_A |
| 17 | | **Motif G>A %** | % motif hits that are G>A/ of all G mutations | #motif_G>A/#motif_G |
| | | **Motif T>C %** | % motif hits that are T>C/ of all T mutations | #motif_T>C/#motif_T |
| 18 | | **Motif G>T %** | % motif hits that are G>T/ of all G mutations | #motif_G>T/#motif_G |
| | | **Motif T>G %** | % motif hits that are T>G/ of all T mutations | #motif_T>G/#motif_T |
| 19 | | **Motif G>C %** | % motif hits that are G>C/ of all G mutations | #motif_G>C/#motif_G |
| | | **Motif T>A %** | % motif hits that are T>A/ of all T mutations | #motif_T>A/#motif_T |
| 20 | | **Motif Ti/Tv %** | % motif hits that are transitions | #motif_Ti/#motif |
| 21 | | **Motif C:G %** | % motif hits that are C - strand bias | #motif_C/#motif |
| | | **Motif A:T %** | % motif hits that are A - strand bias | #motif_A/#motif |
| 22 | | **Motif Ti C:G %** | % motif hits - transition only - that are C - strand bias | #motif_C>T/#motif_Ti |
| | | **Motif Ti A:T %** | % motif hits - transition only - that are A - strand bias | #motif_A>G/#motif_Ti |
| 23 | | **Motif non-syn %** | % motifs hits which are non-synonymous protein change | #motif_ns/#motif |

(continued)

| | | Metric Name | Description of metric | Calculation of metric |
|---|---|---|---|---|
| 24 | | **Motif C non-syn %** | % motifs hits - C strand only - which are non-synonymous protein change | #motif_C_ns/#motif |
| | | *Motif A non-syn %* | *% motifs hits - A strand only - which are non-synonymous protein change* | #motif_A_ns/#motif |
| 25 | | **Motif G non-syn %** | % motifs hits - G strand only - which are non-synonymous protein change | #motif_G_ns/#motif |
| | | *Motif T non-syn %* | *% motifs hits - T strand only - which are non-synonymous protein* | #motif_T_ns/#motif |
| 26 | | **Motif MC1 non-syn %** | % non-syn of motif hits at MC1 | #motif_MC1_ns/#motif_MC1 |
| 27 | | **Motif MC2 non-syn %** | % non-syn of motif hits at MC2 | #motif_MC2_ns/#motif_MC2 |
| 28 | | **Motif MC3 non-syn %** | % non-syn of motif hits at MC2 | #motif_MC3_ns/#motif_MC3 |

As can clearly be seen from Table C, motif-associated metrics not only fall within the motif metric group, but can also form part of the codon context metric group, transition/transversion (Ti/Tv) metric group, synonymous/non-synonymous metric group, strand bias group metric group, strand specific metric group, and AT/GC metric group. In some embodiments, any one or more of the motif-associated coding region metrics (or genetic indicators of deaminase activity) set forth in Table C are assessed for the methods and systems of the present disclosure, and for any one or more of the motifs set forth in Table B. As would be appreciated, due to the large number of possible motifs, there Table C represented a large number of possible metrics. For example, with 29 motif-associated coding region metrics and 39 deaminase motifs, there are 1044 possible deaminase motif-associated coding region metrics. Typically, multiple motif-associated coding region metrics are used in the methods and systems described herein, such as at least 4, 5, 6, 7, 8, 9, 10, 11, 12, 13, 14, 15, 20, 40, 60, 80, 100, 200, 300, 400, 500, 600, 800, 900, 1000, 1500, 2000 or more.

Table D. Motif-independent coding region metrics

| | Metric Name | Description of metric | Calculation of metric |
|---|---|---|---|
| 1 | **cds:All A total** | Total number of A CDS variants | #A |
| 2 | **cds:All T total** | Total number of T CDS variants | #T |
| 3 | **cds:All C total** | Total number of C CDS variants | #C |
| 4 | **cds:All G total** | Total number of G CDS variants | #G |
| 5 | **cds:All A %** | number of A variants/#CDS variants % | #A/#CDS |
| 6 | **cds:All T %** | number of T variants/#CDS variants % | #T/#CDS |
| 7 | **cds:All C %** | number of C variants/#CDS variants % | #C/#CDS |
| 8 | **cds:All G %** | number of G variants/#CDS variants % | #G/#CDS |
| 9 | **cds:All MC1 %** | % CDS which are at MC1 | #MC1/#CDS |
| 10 | **cds:All MC2 %** | % CDS which are at MC2 | #MC2/#CDS |
| 11 | **cds:All MC3 %** | % CDS which are at MC3 | #MC3/#CDS |
| 12 | **cds:All A MC1 %** | % A which are at MC1 | #A_MC1/#CDS |
| 13 | **cds:All A MC2 %** | % A which are at MC2 | #A_MC2/#CDS |
| 14 | **cds:All A MC3 %** | % A which are at MC3 | #A_MC3/#CDS |
| 15 | **cds:All T MC1 %** | % T which are at MC1 | #T_MC1/#CDS |
| 16 | **cds:All T MC2 %** | % T which are at MC2 | #T_MC2/#CDS |
| 17 | **cds:All T MC3 %** | % T which are at MC3 | #T_MC3/#CDS |
| 18 | **cds:All C MC1 %** | % C which are at MC1 | #C_MC1/#CDS |
| 19 | **cds:All C MC2 %** | % C which are at MC2 | #C_MC2/#CDS |

(continued)

| | Metric Name | Description of metric | Calculation of metric |
|---|---|---|---|
| 20 | cds:All C MC3 % | % C which are at MC3 | #C_MC3/#CDS |
| 21 | cds:All G MC1 % | % G which are at MC1 | #G_MC1/#CDS |
| 22 | cds:All G MC2 % | % G which are at MC2 | #G_MC2/#CDS |
| 23 | cds:All G MC3 % | % G which are at MC3 | #G_MC3/#CDS |
| 24 | cds:All MC1 A % | % MC1 which are A | #A_MC1/#MC1 |
| 25 | cds:All MC1 T % | % MC1 which are T | #T_MC1/#MC1 |
| 26 | cds:All MC1 C % | % MC1 which are C | #C_MC1/#MC1 |
| 27 | cds:All MC1 G % | % MC1 which are G | #G_MC1/#MC1 |
| 28 | cds:All MC2 A % | % MC2 which are A | #A_MC2/#MC2 |
| 29 | cds:All MC2 T % | % MC2 which are T | #T_MC2/#MC2 |
| 30 | cds:All MC2 C % | % MC2 which are C | #C_MC2/#MC2 |
| 31 | cds:All MC2 G % | % MC2 which are G | #G_MC2/#MC2 |
| 32 | cds:All MC3 A % | % MC3 which are A | #A_MC3/#MC3 |
| 33 | cds:All MC3 T % | % MC3 which are T | #T_MC3/#MC3 |
| 34 | cds:All MC3 C % | % MC3 which are C | #C_MC3/#MC3 |
| 35 | cds:All MC3 G % | % MC3 which are G | #G_MC3/#MC3 |
| 36 | cds:All AT Ti/Tv % | % A and T variants that are transitions | (#A_Ti + #A_Ti )/(#A + #T) |
| 37 | cds:All CG Ti/Tv % | % C and G variants that are transitions | (#C_Ti + #G_Ti )/(#C + #G) |
| 38 | cds:All MC1 Ti/Tv % | % MC1 variants that are transitions | #MC1_Ti/#MC1 |
| 39 | cds:All MC2 Ti/Tv % | % MC2 variants that are transitions | #MC2_Ti/#MC2 |
| 40 | cds:All MC3 Ti/Tv % | % MC3 variants that are transitions | #MC3_Ti/#MC3 |
| 41 | cds:All A MC1 Ti/Tv % | % A MC1 variants that are transitions | #A_MC1_Ti/#A_MC1 |
| 42 | cds:All A MC2 Ti/Tv % | % A MC2 variants that are transitions | #A_MC2_Ti/#A_MC2 |
| 43 | cds:All A MC3 Ti/Tv % | % A MC3 variants that are transitions | #A_MC3_Ti/#A_MC3 |
| 44 | cds:All T MC1 Ti/Tv % | % T MC1 variants that are transitions | #T_MC1_Ti/#T_MC1 |
| 45 | cds:All T MC2 Ti/Tv % | % T MC2 variants that are transitions | #T_MC2_Ti/#T_MC2 |
| 46 | cds:All T MC3 Ti/Tv % | % T MC3 variants that are transitions | #T_MC3_Ti/#T MC3 |
| 47 | cds:All C MC1 Ti/Tv % | % C MC1 variants that are transitions | #C_MC1_Ti/#C_MC1 |
| 48 | cds:All C MC2 Ti/Tv % | % C MC2 variants that are transitions | #C_MC2_Ti/#C_MC2 |
| 49 | cds:All C MC3 Ti/Tv % | % C MC3 variants that are transitions | #C_MC3_Ti/#C_MC3 |
| 50 | cds:All G MC1 Ti/Tv % | % G MC1 variants that are transitions | #G_MC1_Ti/#G_MC1 |
| 51 | cds:All G MC2 Ti/Tv % | % G MC2 variants that are transitions | #G_MC2_Ti/#G_MC2 |
| 52 | cds:All G MC3 Ti/Tv % | % G MC3 variants that are transitions | #G_MC3_Ti/#G_MC3 |
| 53 | cds:All C:G % | % variants that are C - compared to G - strand bias % | #C/(#C + #G) |
| 54 | cds:All A:T % | % variants that are A - compared to T - strand bias % | #A/(#A + #T) |
| 55 | cds:All AT:GC % | % A or T variants -compared to all variants | (#A + #T) /#CDS |
| 56 | cds:All MC1 C:G % | % MC1 variants that are C - compared to G - strand bias % | #C_MC1/(#C_MC1 + #G_MC1) |

(continued)

|  | Metric Name | Description of metric | Calculation of metric |
|---|---|---|---|
| 57 | cds:All MC2 C:G % | % MC2 variants that are C - compared to G - strand bias % | #C_MC2/(#C_MC2 + #G_MC2) |
| 58 | cds:All MC3 C:G % | % MC3 variants that are C - compared to G - strand bias % | #C_MC3/(#C_MC3 + #G_MC3) |
| 59 | cds:All MC1 A:T % | % MC1 variants that are A - compared to T - strand bias % | #A_MC1/(#A_MC1 + #T_MC1) |
| 60 | cds:All MC2 A:T % | % MC2 variants that are A - compared to T - strand bias % | #A_MC2/(#A_MC2 + #T_MC2) |
| 61 | cds:All MC3 A:T % | % MC3 variants that are A - compared to T - strand bias % | #A_MC3/(#A_MC3 + #T_MC3) |
| 62 | cds:All MC1 AT:GC % | % MC1 A or T variants -compared to all variants | (#A_MC1 + #T_MC1) /#CDS_MC1 |
| 63 | cds:All MC2 AT:GC % | % MC2 A or T variants -compared to all variants | (#A_MC2 + #T_MC2) /#CDS_MC2 |
| 64 | cds:All MC3 AT:GC % | % MC3 A or T variants -compared to all variants | (#A_MC2 + #T_MC3) /#CDS_MC3 |
| 65 | cds:All A>G % | % variants that are A>G/ of all A mutations | #A>G/#A |
| 66 | cds:All A>C % | % variants that are A>C/ of all A mutations | #A>C/#A |
| 67 | cds:All A>T % | % variants that are A>T/ of all A mutations | #A>T/#A |
| 68 | cds:All T>C % | % variants that are T>C/ of all T mutations | #T>C/#T |
| 69 | cds:All T>G % | % variants that are T>G/ of all T mutations | #T>G/#T |
| 70 | cds:All T>A % | % variants that are T>A/ of all T mutations | #T>A/#T |
| 71 | cds:All C>T % | % variants that are C>T/ of all C mutations | #C>T/#C |
| 72 | cds:All C>A % | % variants that are C>A/ of all C mutations | #C>A/#C |
| 73 | cds:All C>G % | % variants that are C>G/ of all C mutations | #C>G/#C |
| 74 | cds:All G>A % | % variants that are G>A/ of all G mutations | #G>A/#G |
| 75 | cds:All G>T % | % variants that are G>T/ of all G mutations | #G>T/#G |
| 76 | cds:All G>C % | % variants that are G>C/ of all G mutations | #G>C/#G |
| 77 | cds:All non-syn % | % variants which are non-synonymous | #CDS_ns/#CDS |
| 78 | cds:All A non-syn % | % A variants which are non-synonymous | #A_ns/#A |
| 79 | cds:All T non-syn % | % T variants which are non-synonymous | #T_ns/#T |
| 80 | cds:All C non-syn % | % C variants which are non-synonymous | #C_ns/#C |
| 81 | cds:All G non-syn % | % G variants which are non-synonymous | #G_ns/#G |
| 82 | cds:All MC1 non-syn % | % MC1 variants which are non-synonymous | #MC1_ns/#MC1 |
| 83 | cds:All MC2 non-syn % | % MC2 variants which are non-synonymous | #MC2_ns/#MC2 |
| 84 | cds:All MC3 non-syn % | % MC3 variants which are non-synonymous | #MC3_ns/#MC3 |

In addition to the metrics shown Table D, an additional corresponding set of motif-independent coding region metrics is provided that represent the metrics shown in rows 1-84 of Table D but which are not associated with one of the four primary deaminase motifs (i.e. the AID motif WRC/GYW; the ADAR motif WA/TW, the APOBEC3G motif CC/GG; and the APOBEC3B motif TCW/WGA). Thus, where the metrics in Table D include "all" of the recited metrics in the coding region, including those that fall within one of the four primary deaminase motifs, within one of the secondary deaminase motifs, within a three-mer or five-mer motif, or not within any motif, the corresponding "other" metrics include only those metrics shown in rows 1-84 that fall within one of the four primary deaminase motifs. For example, the metric in row 1 of Table D (cds:All A total) is total number of A CDS variants. The corresponding "other" metric (cds:Other A total) is the total number of cds A variants that are not associated with (or within) one of the four primary deaminase motifs.

As can clearly be seen from Table D and the description of the corresponding "other" metrics, motif-independent metrics can also form part of the codon context metric group, transition/transversion (Ti/Tv) metric group, synonymous/-non-synonymous metric group, strand bias group metric group, strand specific metric group, and AT/GC metric group. Any one or more of the motif-independent coding region metrics (or genetic indicators of deaminase activity) set forth in Table D may be determined and/or used in accordance with the methods and systems of the present disclosure. Typically, at least 2, 3, 4, 5, 6, 7, 8, 9, 10, 11, 12, 13, 14, 15, 20, 40, 60 or 80 of these metrics are determined and/or used.

### 3.8.2 Non-coding region metrics

Metrics based on SNVs identified in the non-coding region (e.g. the 5' and 3' UTRs, promoters, intergenic regions, introns etc.) of a nucleic acid molecule can also be determined. Exemplary non-coding region metrics include, but are not limited to, those set forth in Table E. As would be appreciated, these metrics are only determined and/or used when the nucleic acid sequence analysed contains non-coding regions; these metrics are not used when the nucleic acid sequence is, for example, obtained using whole exome sequencing.

Metrics in rows 11-20 essentially correspond to the metrics in rows 1-10 but which are not associated with one of the four primary deaminase motifs (i.e. the AID motif WRC/GYW; the ADAR motif WA/TW, the APOBEC3G motif CC/GG; and the APOBEC3B motif TCW/WGA). Thus, where the metrics in rows 1-10 of Table E include "all" of the recited metrics in the non-coding region, including those that fall within one of the four primary deaminase motifs, within one of the secondary deaminase motifs, within a three-mer or five-mer motif, or not within any motif, the corresponding "other" metrics include only those metrics shown in rows 1-10 that fall within one of the four primary deaminase motifs.

Table E. Exemplary non-coding region metrics

| | Metric Name | Description of metric | Calculation of metric |
|---|---|---|---|
| 1 | nc:variant total | Number of variants in non-coding region (nc) | #nc |
| 2 | nc:AT total | # total noncoding A and T variants | #nc_A + #nc_T |
| 3 | nc:CG total | # total noncoding C and G variants | #nc_C+ #nc_G |
| 4 | nc:AT:GC % | % noncoding A and T variants | (#nc_A + #nc_T)/ #nc |
| 5 | nc:A>G + T>C % | % A>G and T>C variants of all AT variants | (#nc_A>G + #nc_T>C) / (#nc_A + #nc_T) |
| 6 | nc:A>C + T>G % | % A>C and T>G variants of all AT variants | (#nc_A>C + #nc_T>G) / (#nc_A + #nc_T) |
| 7 | nc:A>T + T>A % | % A>T and T>A variants of all AT variants | (#nc_A>T + #nc_T>A) / (#nc_A + #nc_T) |
| 8 | nc:C>T + G>A % | % C>T and G>A variants of all CG variants | (#nc_C>T + #nc_G>A) / (#nc C + #nc_G) |
| 9 | nc:C>A + G>T % | % C>A and G>T variants of all CG variants | (#nc_C>A + #nc_G>T) / (#nc C + #nc_G) |
| 10 | nc:C>G + G>C % | % C>G and G>C variants of all CG variants | (#nc_C>G + #nc_G>C) / (#nc C + #nc_G) |
| 11 | nc:Other variant total | Number of variants in non-coding region (nc) that are not in a primary deaminase motif | #ncO |
| 12 | nc: Other AT total | # total noncoding A and T variants that are not associated with a primary deaminase motif | #ncO_A + #ncO_T |
| 13 | nc: Other CG total | # total noncoding C and G variants that are not associated with a primary deaminase motif | #ncO_C+ #ncO_G |

(continued)

| | | Metric Name | Description of metric | Calculation of metric |
|---|---|---|---|---|
| | 14 | nc: Other AT:GC % | % noncoding A and T variants that are not associated with a primary deaminase motif | (#ncO_A + #ncO_T)/ #ncO |
| | 15 | nc: Other A>G + T>C % | % A>G and T>C variants of all AT variants that are not associated with a primary deaminase motif | (#ncO_A>G + #ncO_T>C) / (#ncO_A + #ncO_T) |
| | 16 | nc: Other A>C + T>G % | % A>C and T>G variants of all AT variants that are not associated with a primary deaminase motif | (#ncO_A>C + #ncO_T>G) / (#ncO_A + #ncO_T) |
| | 17 | nc: Other A>T + T>A % | % A>T and T>A variants of all AT variants that are not associated with a primary deaminase motif | (#ncO_A>T + #ncO_T>A) / (#ncO_A + #ncO_T) |
| | 18 | nc: Other C>T + G>A % | % C>T and G>A variants of all CG variants that are not associated with a primary deaminase motif | (#ncO_C>T + #ncO_G>A) / (#ncO_C + #ncO_G) |
| | 19 | nc: Other C>A + G>T % | % C>A and G>T variants of all CG variants that are not associated with a primary deaminase motif | (#ncO_C>A + #ncO_G>T) / (#ncO_C + #ncO_G) |
| | 20 | nc: Other C>G + G>C % | % C>G and G>C variants of all CG variants that are not associated with a primary deaminase motif | (#ncO_C>G + #ncO_G>C) / (#ncO_C + #ncO_G) |
| | 21 | nc:Motif Hits | Number of "motif" hits in non-coding region | #nc_motif |
| | 22 | nc:Motif % | number of "motif" hits/#nc hits % | #nc_motif/#nc |
| | 23 | nc:Motif Ti % | number of "motif" hits which are transitions /#nc hits % | #nc_motif Ti/#nc |
| | 24 | nc:Motif C>T + G>A % | % motif hits that are C>T or G>A/ motif hits | (#nc_motif_C>T + *nc_motif_G>A) /#motif |
| | | nc:Motif A>G + T>C % | % motif hits that are A>G or T>C/ motif hits | (#nc_motif_A>G + #nc_motif_T>C) /#motif |
| | 25 | nc:Motif C>A + G>T % | % motif hits that are C>A or G>T/ motif hits | (#nc_motif_C>A + #nc_motif_G>T ) /#nc_motif |
| | | nc:Motif A>C + T>G % | % motif hits that are A>C or T>G/ motif hits | (#nc_motif_A>C + #nc_motif_T>G) /#motif |
| | 26 | nc:Motif C>G + G>C % | % motif hits that are C>G or G>C/ motif hits | (#nc_motif_C>G + #nc_motif_G>C ) /#nc_motif |
| | | nc:Motif A>T + T>A % | % motif hits that are A>T or T>A/ motif hits | (#nc_motif_A>T + #nc_motif_T>A) /#motif |

As would be appreciated, these non-coding metrics can also form part of the motif metric group, motif-independent metric group, transition/transversion metric group, and AT/GC metric group. Any one or more of the non-coding region metrics (or genetic indicators of deaminase activity) set forth in Table E may be determined and/or used in accordance with the methods and systems of the present disclosure. Typically, at least 2, 3, 4, 5, 6, 7, 8, 9, 10, 11, 12, 13, 14, 15, 20, 40, 60, 80, 100 or more of these metrics are determined and/or used.

### 3.8.3 Genomic metrics

Other exemplary metrics include those that are determined across all regions of the genomic nucleic acid sequence are assessed, i.e. regardless of whether the sequence is of a non-coding or coding region. As would be appreciated, these metrics can thus be determined and/or used when the sequence of only a part of the nucleic acid is assessed (e.g. by whole exome sequencing), or whether the sequence of the entire nucleic acid is assessed (e.g. by whole genome sequencing). Exemplary metrics in the genomic metric group include those set forth in Table F. Metrics in rows 11-20 essentially correspond to the metrics in rows 1-10 but which are not associated with one of the four primary deaminase motifs (i.e. the AID motif WRC/GYW; the ADAR motif WA/TW, the APOBEC3G motif CC/GG; and the APOBEC3B motif TCW/WGA). Thus, where the metrics in rows 1-10 of Table F include "all" of the recited metrics in the genomic region, including those that fall within one of the four primary deaminase motifs, within one of the secondary deaminase motifs, within a three-mer or five-mer motif, or not within any motif, the corresponding "other" metrics include only those metrics shown in rows 1-10 that fall within one of the four primary deaminase motifs.

Table F. Exemplary genomic metrics

| | Metric Name | Description of metric | Calculation of metric |
|---|---|---|---|
| 1 | g: variant total | Number of all (genomic (g)) variants | #g |
| 2 | g: AT total | # total genomic A and T variants | #g_A + #g_T |
| 3 | g: CG total | # total genomic C and G variants | #g_C+ #g_G |
| 4 | g: AT:GC % | % genomic A and T variants | (#g_A + #g_T)/ #g |
| 5 | g: A>G + T>C % | % A>G and T>C variants of all AT variants | (#g_A>G + #g_T>C) / (#g_A + #g_T) |
| 6 | g: A>C + T>G % | % A>C and T>G variants of all AT variants | (#g_A>C + #g_T>G) / (#g_A + #g_T) |
| 7 | g: A>T + T>A % | % A>T and T>A variants of all AT variants | (#g_A>T + #g_T>A) / (#g_A + #g T) |
| 8 | g: C>T + G>A % | % C>T and G>A variants of all CG variants | (#g_C>T + #g_G>A) / (#g_C + #g_G) |
| 9 | g: C>A + G>T % | % C>A and G>T variants of all CG variants | (#g_C>A + #g_G>T) / (#g_C + #g_G) |
| 10 | g: C>G + G>C % | % C>G and G>C variants of all CG variants | (#g_C>G + #g_G>C) / (#g_C + #g_G) |
| 11 | g:Other variant total | Number of all (genomic) variants that are not associated with a primary deaminase motif | #gO |
| 12 | g: Other AT total | # total genomic A and T variants that are not associated with a primary deaminase motif | #gO_A + #gO_T |
| 13 | g: Other CG total | # total genomic C and G variants that are not associated with a primary deaminase motif | #gO_C+ #gO_G |
| 14 | g: Other AT:GC % | % genomic A and T that are not associated with a primary deaminase motif | (#gO_A + #gO_T)/ #gO |
| 15 | g: Other A>G + T>C % | % A>G and T>C variants of all AT variants that are not associated with a primary deaminase motif | (#gO_A>G + #gO_T>C) / (#gO_A + #gO_T) |
| 16 | g: Other A>C + T>G % | % A>C and T>G variants of all AT variants that are not associated with a primary deaminase motif | (#gO_A>C + #gO_T>G) / (#gO_A + #gO_T) |
| 17 | g: Other A>T + T>A % | % A>T and T>A variants of all AT variants that are not associated with a primary deaminase motif | (#gO_A>T + #gO_T>A) / (#gO_A + #gO_T) |
| 18 | g: Other C>T + G>A % | % C>T and G>A variants of all CG variants that are not associated with a primary deaminase motif | (#gO_C>T + #gO_G>A) / (#gO_C + #gO_G) |
| 19 | g: Other C>A + G>T % | % C>A and G>T variants of all CG variants that are not associated with a primary deaminase motif | (#gO_C>A + #gO_G>T)/ (#gO_C + #gO_G) |

(continued)

| | | Metric Name | Description of metric | Calculation of metric |
|---|---|---|---|---|
| | 20 | g: Other C>G + G>C % | % C>G and G>C variants of all CG variants that are not associated with a primary deaminase motif | (#gO_C>G + #gO_G>C) / (#gO_C + #gO_G) |
| | 21 | g:Motif Hits | Number of "motif" hits in genome | #g_motif |
| | 22 | g:Motif % | number of "motif" hits/#g hits % | #g_motif/#g |
| | 23 | g:Motif Ti % | number of "motif" hits which are transitions /#g hits % | #g_motif_Ti/#g |
| | 24 | g:Motif C>T + G>A % | % motif hits that are C>T or G>A/ motif hits | (#g_motif_C>T + #g_motif_G>A ) /#g_motif |
| | | g:Motif A>G + T>C % | % motif hits that are A>G or T>C/ motif hits | (*g_motif_A>G + *g_motif_T>C ) /#g_motif |
| | 25 | g:Motif C>A + G>T % | % motif hits that are C>A or G>T/ motif hits | (#g_motif_C>A + *g_motif_G>T ) /#motif |
| | | g:Motif A>C + T>G % | % motif hits that are A>C or T>G/ motif hits | (*g_motif_A>C + *g_motif_T>G ) /#g_motif |
| | 26 | g:Motif C>G + G>C % | % motif hits that are C>G or G>C/ motif hits | (#g_motif_C>G + #g_motif_G>C ) /#g_motif |
| | | g:Motif A>T + T>A % | % motif hits that are A>T or T>A/ motif hits | (*g_motif_A>T + *g_motif_T>A ) /#g_motif |

**As** would be appreciated, these genomic metrics also include metrics in the AT/GC metric group. Any one or more of the genomic metrics (or genetic indicators of deaminase activity) set forth in Table F may be determined and/or used in accordance with the methods and systems of the present disclosure. Typically, at least 2, 3, 4, 5, 6, 7, 8, 9, 10, 11, 12, 13, 14, 15, 20, 40, 60 or 80 of these metrics are determined and/or used.

### 3.8.4 Codon context metrics

Metrics of the present disclosure include those that are associated with the codon context of a SNV, i.e. where the codon context of a SNV is assessed when determining the metric. Codon context metrics therefore include any in which an assessment of whether the SNV is at the MC-1, MC-2 or MC-3 position of a codon has been made. As would be appreciated, such metrics include only those in the coding metric group, and do not include any metrics in the non-coding or genomic metric groups.

In some instances, the codon context metrics are also motif metrics, and include, for example, those set forth in rows 5-13 and 26-28 of Table C, i.e. Motif C>T at MC1 %, Motif A>G at MC1 %, Motif C>T at MC2 %, Motif A>G at MC2 %, Motif C>T at MC3 %, Motif A>G at MC3 %, Motif G>A at MC1 %, Motif T>C at MC1 %, Motif G>A at MC2 %, Motif T>C at MC2 %, Motif G>A at MC3 %, Motif T>C at MC3 %, Motif MC1 non-syn %, Motif MC2 non-syn %, and Motif MC3 non-syn %. In other instances, the codon context metrics are also motif-independent metrics and include, for example, those set forth in rows 9-35, 42-56, 60-68 and 86-88 of Table D, i.e. cds:All MC1 %, cds:All MC2 %, cds:All MC3 %, cds:All A MC1 %, cds:All A MC2 %, cds:All A MC3 %, cds:All T MC1 %, cds:All T MC2 %, cds:All T MC3 %, cds:All C MC1 %, cds:All C MC2 %, cds:All C MC3 %, cds:All G MC1 %, cds:All G MC2 %, cds:All G MC3 %, cds:All MC1 A %, cds:All MC1 T %, cds:All MC1 C %, cds:All MC1 G %, cds:All MC2 A %, cds:All MC2 T %, cds:All MC2 C %, cds:All MC2 G %, cds:All MC3 A %, cds:All MC3 T %, cds:All MC3 C %, cds:All MC3 G %, cds:All MC1 Ti/Tv %, cds:All MC2 Ti/Tv %, cds:All MC3 Ti/Tv %, cds:All A MC1 Ti/Tv %, cds:All A MC2 Ti/Tv %, cds:All A MC3 Ti/Tv %, cds:All T MC1 Ti/Tv %, cds:All T MC2 Ti/Tv %, cds:All T MC3 Ti/Tv %, cds:All C MC1 Ti/Tv %, cds:All C MC2 Ti/Tv %, cds:All C MC3 Ti/Tv %, cds:All G MC1 Ti/Tv %, cds:All G MC2 Ti/Tv %, cds:All G MC3 Ti/Tv %, cds:All MC1 C:G %, cds:All MC2 C:G %, cds:All MC3 C:G %, cds:All MC1 A:T %, cds:All MC2 A:T %, cds:All MC3 A:T %, cds:All MC1 AT:GC %, cds:All MC2 AT:GC %, cds:All MC3 AT:GC %, cds:All MC1 non-syn %, cds:All MC2 non-syn %, and cds:All MC3 non-syn %. As would be appreciated, these codon context metrics also include metrics in the transition/transversion (Ti/Tv) metric group, synonymous/non-synonymous metric group, strand bias group metric group, AT metric group and GC metric group.

Any one or more of the codon context metrics (or genetic indicators of deaminase activity) may be determined and/or used in accordance with the methods and systems of the present disclosure. In some examples, one or more motif-associated codon context metrics, and/or one more motif-independent codon context metrics are determined and/or used. Typically, at least 2, 3, 4, 5, 6, 7, 8, 9, 10, 11, 12, 13, 14, 15, 20, 40, 60, 80, 100, 120, 140, 160, 180, 200 of these metrics are determined and/or used (taking into account the various motifs that might be assessed in conjunction with the codon context of the SNV).

### 3.8.5 Transition/Transversion metrics

Metrics of the present disclosure include those that are associated with SNVs that are either transition (Ti) or transversion (Tv) mutations. i.e. where it is determined whether the SNV is either a transition or transversion. Such metrics typically fall within the coding metric group, although could also be in the non-coding or genomic metric groups.

In some instances, the transition/transversion metrics are also motif metrics, and include, for example, those set forth in rows 4, 8-20 and 20-22 of Table C, i.e. Motif Ti%, Motif C>T at MC1 %, Motif A>G at MC1 %, Motif C>T at MC2 %, Motif A>G at MC2 %, Motif C>T at MC3 %, Motif A>G at MC3 %, Motif G>A at MC1 %, Motif T>C at MC1 %, Motif G>A at MC2 %, Motif T>C at MC2 %, Motif G>A at MC3 %, Motif T>C at MC3 %, Motif C>T %, Motif A>G %, Motif C>A %, Motif A>C %, Motif C>G %, Motif A>T %, Motif G>A %, Motif T>C %, Motif G>T %, Motif T>G %, Motif G>C %, Motif T>A %, Motif Ti/Tv %, and Motif Ti C:G %. In other instances, the transition/transversion metrics are also motif-independent metrics and include, for example, those coding region metrics set forth in 36-52 and 65-76 of Table D, i.e., cds:All AT Ti/Tv %, cds:All CG Ti/Tv %, cds:All MC1 Ti/Tv %, cds:All MC2 Ti/Tv %, cds:All MC3 Ti/Tv %, cds:All A MC1 Ti/Tv %, cds:All A MC2 Ti/Tv %, cds:All A MC3 Ti/Tv %, cds:All T MC1 Ti/Tv %, cds:All T MC2 Ti/Tv %, cds:All T MC3 Ti/Tv %, cds:All C MC1 Ti/Tv %, cds:All C MC2 Ti/Tv %, cds:All C MC3 Ti/Tv %, cds:All G MC1 Ti/Tv %, cds:All G MC2 Ti/Tv %, and cds:All G MC3 Ti/Tv %, cds:All A>G %, cds:All A>C %, cds:All A>T %, cds:All T>C %, cds:All T>G %, cds:All T>A %, cds:All C>T %, cds:All C>A %, cds:All C>G %, cds:All G>A %, cds:All G>T %, cds:All G>C %; those non-coding region metrics set forth in rows 5-10, 15-20 and 24-26 of Table E, i.e. nc:A>G + T>C %, nc:A>C + T>G %, nc:A>T + T>A %, nc:C>T + G>A %, nc:C>A + G>T %, nc:C>G + G>C %, nc:Other A>G + T>C %, nc: Other A>C + T>G %, nc: Other A>T + T>A %, nc: Other C>T + G>A %, nc: Other C>A + G>T %, nc: Other C>G + G>C %, nc:Motif C>T + G>A %, nc:Motif A>G + T>C %, nc:Motif C>A + G>T %, nc:Motif A>C + T>G %, nc:Motif C>G + G>C % and nc:Motif A>T + T>A %; and those genomic metrics set forth in rows 5-10, 15-20 and 14-16 of Table F, i.e. g:A>G + T>C %, g:A>C + T>G %, g:A>T + T>A %, g:C>T + G>A %, g:C>A + G>T %, g:C>G + G>C %, g: Other A>G + T>C %, g: Other A>C + T>G %, g: Other A>T + T>A %, g: Other C>T + G>A %, g: Other C>A + G>T %, g: Other C>G + G>C %g:Motif C>T + G>A %, g:Motif A>G + T>C %, g:Motif C>A + G>T %, g:Motif A>C + T>G %, g:Motif C>G + G>C % and g:Motif A>T + T>A %.

As would be appreciated, these transition/transversion metrics also include metrics in the codon context metric group, synonymous/non-synonymous metric group, strand bias group metric group, strand specific metric group and AT/GC metric group.

Any one or more of the transition/transversion metrics (or genetic indicators of deaminase activity) may be determined and/or used in accordance with the methods and systems of the present disclosure. In some examples, one or more motif-associated transition/transversion metrics, and/or one more motif-independent transition/transversion metrics are determined and/or used. Typically, at least 2, 3, 4, 5, 6, 7, 8, 9, 10, 11, 12, 13, 14, 15, 20, 40, 60, 80, 100, 120, 140, 160, 180, 200 of these metrics are determined and/or used (taking into account the various motifs that might be assessed in conjunction with whether the SNV is a transition or transversion).

### 3.8.6 Synonymous/non-synonymous metrics

Metrics of the present disclosure include those that are associated with SNVs that are either synonymous or non-synonymous mutations. i.e. where it is determined whether the SNV is either a synonymous or non-synonymous mutation. As would be appreciated, these metrics fall solely within the coding metric group.

In some instances, the synonymous/non-synonymous metrics are also motif metrics, and include, for example, those set forth in rows 23-28 of Table C, i.e. Motif non-syn %, Motif C non-syn %, Motif A non-syn %, Motif G non-syn %, Motif T non-syn %, Motif MC1 non-syn %, Motif MC2 non-syn % and Motif MC3 non-syn %. In other instances, the synonymous/non-synonymous metrics are also motif-independent metrics and include, for example, those set forth in 77-84 of Table D, i.e. cds:All non-syn %, cds:All A non-syn %, cds:All T non-syn %, cds:All C non-syn %, cds:All G non-syn %, cds:All MC1 non-syn %, cds:All MC2 non-syn %, and cds:All MC3 non-syn %. As would be appreciated, these synonymous/non-synonymous metrics also include metrics in the codon context metric group, and strand specific metric group.

Any one or more of the synonymous/non-synonymous metrics (or genetic indicators of deaminase activity) may be determined and/or used in accordance with the methods and systems of the present disclosure. In some examples, one or more motif-associated synonymous/non-synonymous metrics, and/or one more motif-independent synonymous/non-synonymous metrics are determined and/or used. Typically, at least 2, 3, 4, 5, 6, 7, 8, 9, 10, 11, 12, 13, 14, 15, 20, 40, 60, 80, 100, 120, 140, 160, 180, 200 of these metrics are determined and/or used (taking into account the various motifs that might be assessed in conjunction with whether the SNV is a synonymous or non-synonymous mutation).

### 3.8.7 Strand bias metrics

Metrics of the present disclosure include those that are associated with strand bias of SNVs. i.e. where it can be inferred whether the SNV is more or less prevalent on the transcribed or untranscribed strand of DNA. These metrics typically fall within the coding metric group, but could also include metrics in the non-coding or genomic metric groups.
In some instances, the strand bias metrics are also motif metrics, and include, for example, those set forth in rows 21 and 22 of Table C, i.e. Motif C:G %, Motif A:T %, Motif Ti C:G % and Motif Ti A:T %. In other instances, the synonymous/non-synonymous metrics are also motif-independent metrics and include, for example, those set forth in 53, 54 and 56-61 of Table D, i.e. cds:All C:G %, cds:All A:T %, cds:All MC1 C:G %, cds:All MC2 C:G %, cds:All MC3 C:G %, cds:All MC1 A:T %, cds:All MC2 A:T %, and cds:All MC3 A:T %. As would be appreciated, these strand bias metrics also include metrics in the codon context metric group and transition/transversion metric group.
Any one or more of the strand bias metrics (or genetic indicators of deaminase activity) may be determined and/or used in accordance with the methods and systems of the present disclosure. In some examples, one or more motif-associated strand bias metrics, and/or one more motif-independent strand bias metrics are determined and/or used. Typically, at least 2, 3, 4, 5, 6, 7, 8, 9, 10, 11, 12, 13, 14, 15, 20, 40, 60, 80, 100, 120, 140, 160, 180, 200 of these metrics are determined and/or used (taking into account the various motifs that might be assessed in conjunction with whether there is strand bias of the SNV).

### 3.8.8 Strand specific metrics

Strand specific metrics (or genetic indicators of deaminase activity) that are associated with SNVs on a particular strand (i.e. the non-transcribed or transcribed strand) are also provided. These metrics include only those in the coding group, where the natrure of the strand can be determined.
Exemplary strand specific metrics include metrics that are also motif metrics, such as those set forth in rows 8-19, 24 and 25 of Table, i.e. Motif C>T at MC1 %, Motif A>G at MC1 %, Motif C>T at MC2 %, Motif A>G at MC2 %, Motif C>T at MC3 %, Motif A>G at MC3 %, Motif G>A at MC1 %, Motif T>C at MC1 %, Motif G>A at MC2 %, Motif T>C at MC2 %, Motif G>A at MC3 %, Motif T>C at MC3 %, Motif C>T %, Motif A>G %, Motif C>A %, Motif A>C %, Motif C>G %, Motif A>T %, Motif G>A %, Motif T>C %, Motif G>T %, Motif T>G %, Motif G>C %, Motif T>A %, Motif C non-syn %, Motif A non-syn %, Motif G non-syn % and Motif T non-syn %. Strand specific metrics also include motif-independent metrics, including the coding metrics set forth in rows 1-8, 12-35, 41-52, 65-76 and 878-81 of Table D, i.e. cds:All A total, cds:All T total, cds:All C total, cds:All G total, cds:All A %, cds:All T %, cds:All C %, cds:All G %, cds:All A MC1 %, cds:All A MC2 %, cds:All A MC3 %, cds:All T MC1 %, cds:All T MC2 %, cds:All T MC3 %, cds:All C MC1 %, cds:All C MC2 %, cds:All C MC3 %, cds:All G MC1 %, cds:All G MC2 %, cds:All G MC3 %, cds:All MC1 A %, cds:All MC1 T %, cds:All MC1 C %, cds:All MC1 G %, cds:All MC2 A %, cds:All MC2 T %, cds:All MC2 C %, cds:All MC2 G %, cds:All MC3 A %, cds:All MC3 T %, cds:All MC3 C %, cds:All MC3 G %, cds:All A MC1 %, cds:All A MC2 %, cds:All A MC3 %, cds:All T MC1 %, cds:All T MC2 %, cds:All T MC3 %, cds:All C MC1 %, cds:All C MC2 %, cds:All C MC3 %, cds:All G MC1 %, cds:All G MC2 %, cds:All G MC3 %, cds:All MC1 A %, cds:All MC1 T %, cds:All MC1 C %, cds:All MC1 G %, cds:All MC2 A %, cds:All MC2 T %, cds:All MC2 C %, cds:All MC2 G %, cds:All MC3 A %, cds:All MC3 T %, cds:All MC3 C %, cds:All MC3 G %, cds:All A>G %, cds:All A>C %, cds:All A>T %, cds:All T>C %, cds:All T>G %, cds:All T>A %, cds:All C>T %, cds:All C>A %, cds:All C>G %, cds:All G>A %, cds:All G>T %, cds:All G>C %, cds:All A non-syn %, cds:All T non-syn %, cds:All C non-syn %, and cds:All G non-syn %.
Any one or more of the strand specific metrics (or genetic indicators of deaminase activity) may be determined and/or used in accordance with the methods and systems of the present disclosure. In some examples, one or more motif-associated strand specific metrics, and/or one more motif-independent strand bias metrics are determined and/or used. Typically, at least 2, 3, 4, 5, 6, 7, 8, 9, 10, 11, 12, 13, 14, 15, 20, 40, 60, 80, 100, 120, 140, 160, 180, 200 of these metrics are determined and/or used (taking into account the various motifs that might be assessed in conjunction with the strand specificity of the SNV).

### 3.8.9 AT/GC metrics

Metrics of the present disclosure include those that reflect the number, percentage or ratio of SNVs that target adenine and thymine (AT), and/or guanine and cytidine (GC). AT/GC metrics include some of those in the coding metric group, non-coding metric group and genomic metric groups.
The AT/GC metrics are motif-independent metrics, and include, for example, coding sequence metrics set forth in rows 36, 37, 55 and 62-64 of Table D, i.e. cds:All AT Ti/Tv %, cds:All CG Ti/Tv %, cds:All AT:GC %, cds:All MC1 AT:GC %, cds:All MC2 AT:GC %, cds:All MC3 AT:GC %; non-coding region metrics set forth in rows 2-4 and 12-14 of Table E, i.e. nc:AT total, nc:CG total, nc:AT:GC %, nc:Other AT total, nc:Other CG total, and nc:Other AT:GC %; and genomic metrics set forth in rows 2-4 and 12-14 of Table F, i.e. g:AT total, g:CG total, g:AT:GC %, g:Other AT total, g:Other CG total, and g:other AT:GC %. As would be appreciated, these AT/GC metrics also include metrics in the codon context metric group and transition/transversion (Ti/Tv) metric group.

**Any** one or more of the AT/GC metrics (or genetic indicators of deaminase activity) may be determined and/or used in accordance with the methods and systems of the present disclosure. In some examples, 2, 3, 4, 5, 6, 7, 8, 9, 10, 11 or 12 of these metrics are determined and/or used.

### 3.8.10 Motif metrics

In particular embodiments, the metrics of the present disclosure are associated with SNVs in specific motifs (i.e. reflect the number or percentage of SNVs in specific motifs). In some instances, these motifs are deaminase motifs, such as an AID, APOBEC1, APOBEC3A, APOBEC3B, APOBEC3C, APOBEC3D, APOBEC3F, APOBEC3G, APOBEC3H or ADAR motif, such as any described above and set forth in Table B. Accordingly, the motif metric group described herein includes APOBEC1, APOBEC3A, APOBEC3B, APOBEC3C, APOBEC3D, APOBEC3F, APOBEC3G, APOBEC3H and ADAR motif groups, wherein each group represents metrics associated with SNVs in the respective deaminase motif(s). The motifs may also be three-mer or five-mer motifs, as described above in section 3.1.

Exemplary motif metrics include the coding region metrics set forth in rows 2-28 of Table C, i.e. Motif Hits, Motif %, Motif Ti %, Motif MC1 %, Motif MC2 %, Motif MC3 %, Motif C>T at MC1 %, Motif C>T at MC2 %, Motif C>T at MC3 %, Motif G>A at MC1 %, Motif G>A at MC2 %, Motif G>A at MC3 %, Motif C>T %, Motif C>A %, Motif C>G %, Motif G>A %, Motif G>T %, Motif G>C %, Motif Ti/Tv %, Motif C:G %, Motif Ti C:G %, Motif non-syn %, Motif C non-syn %, Motif A non-syn %, Motif G non-syn %, Motif T non-syn %, Motif MC1 non-syn %, Motif MC2 non-syn % and Motif MC3 non-syn %; the non-coding region metrics set forth in rows 21-26 of Table E, i.e. nc:Motif Hits, nc:Motif %, nc:Motif Ti %, nc:Motif C>T + G>A %, nc:Motif C>A + G>T % and nc:Motif C>G + G>C %; and the genomic metrics set forth in rows 21-26 of Table F, i.e. g:Motif Hits, g:Motif %, g:Motif Ti %, g:Motif C>T + G>A %, g:Motif C>A + G>T %, g:Motif C>G + G>C %.

As would be appreciated, these motif metrics also include metrics in the codon context metric group, transition/-transversion (Ti/Tv) metric group, synonymous/non-synonymous metric group, strand bias group metric group, AT metric group and GC metric group.

Any one or more of the motif metrics (or genetic indicators of deaminase activity) may be determined and/or used in accordance with the methods and systems of the present disclosure. In some examples, one or more metrics in which the SNV is in an ADAR, AID, APOBEC3B, APOBEC3G, APOBEC3F or APOBEC1 motif is determined and/or used in the methods and systems of the present disclosure. Such metrics are referred to as being in the ADAR, AID, APOBEC3B, APOBEC3G, APOBEC3F or APOBEC1, respectively. Thus, for example, metrics in the ADAR metric group include metrics set forth in rows 2-28 of Table C, rows 21-26 of Table E and rows 21-26 of Table F, wherein the motif is, for example, any one or more of ADAR, ADARb, ADARc, ADARd, ADARe, ADARf, ADARg, ADARh, ADARi or ADARj as set forth in Table B; metrics in the AID metric group include metrics set forth in rows 2-28 of Table C, rows 21-26 of Table E and rows 21-26 of Table F, wherein the motif is, for example, any one or more of AID, AIDb, AIDc, AIDd, AIDe, AIDf and AIDg as set forth in Table B; metrics in the APOBEC3G metric group include metrics set forth in rows 2-28 of Table C, rows 21-26 of Table E and rows 21-26 of Table F, wherein the motif is, for example, any one or more of A3G, A3Gb, A3Gc, A3Gd, A3Ge, A3Gf, A3Gg, A3Gh and A3Gi as set forth in Table B; metrics in the APOBEC3B metric group include metrics set forth in rows 2-28 of Table C, rows 21-26 of Table E and rows 21-26 of Table F, wherein the motif is, for example, any one or more of A3B, A3Bb, A3Bc, A3Bd, A3Be, A3Bf, A3Bg and A3Bh as set forth in Table B; metrics in the APOBEC3F metric group include metrics set forth in rows 2-28 of Table C, rows 21-26 of Table E and rows 21-26 of Table F, wherein the motif is, for example, A3F as set forth in Table B; and metrics in the APOBEC1 metric group include metrics set forth in rows 2-28 of Table C, rows 21-26 of Table E and rows 21-26 of Table F, wherein the motif is, for example, A1 as set forth in Table B. In particular examples, metrics associated with 2, 3, 4, 5, or all of these deaminases are assessed. Typically, at least 2, 3, 4, 5, 6, 7, 8, 9, 10, 11, 12, 13, 14, 15, 20, 40, 60, 80, 100, 120, 140, 160, 180, 200, 300, 400, 500, 1500, 2000, 2500, 3000, 3500, 4000, 4500, 5000, 5500, 6000 or more motif metrics are determined and/or used.

### 3.8.11 Motif independent metrics

Metrics of the present disclosure also include motif-independent metrics which are associated with SNVs irrespective of whether the SNV is in a motif. These metrics include CDS Variants (row 1 of Table C), each of the coding region metrics set forth in Table D, each of the non-coding metrics set forth in rows 1-20 of Table E and each of the genomic metrics set forth in rows 1-20 of Table F. As would be appreciated, these motif-independent metric also include metrics in the codon context metric group, transition/transversion (Ti/Tv) metric group, synonymous/non-synonymous metric group, strand bias group metric group, stand specific metric group and AT/GC metric group.

Typically, at least 2, 3, 4, 5, 6, 7, 8, 9, 10, 11, 12, 13, 14, 15, 20, 40, 60, 80, 100 or more motif-independent metrics are determined and/or used.

### 3.8.12 Exemplary combinations of metrics

Typically, at least 10, 20, 30, 40, 50, 60, 70, 80, 90, 100, 150, 200, 250, 300, 350, 400, 450, 500, 550, 600, 650, 700, 750, 800, 850, 900, 950, 1000, 1200, 1400, 1600, 1800, 2000, 2200, 2400, 2600, 2800, 3000, 3200, 3400, 3600, 3800, 4000, 4200, 4400, 4600, 4800, 5000, 5200, 5500, 5600, 5800, 6000, 6500, or 7000 metrics (or genetic indicators of deaminase activity) are determined and/or used in accordance with the methods and systems of the present disclosure.

In some examples, at least 1, 2, 3, 4, 5, 6, 7, 8, 9, 10, 20, 30, 40, 50, 60, 70, 80, 90 or 100 from at least 2 (e.g. at least 3, 4, 5, 6, 7, 8, 9 or 10 metric groups selected from among i) the coding metric group; ii) the non-coding metric group; iii) the genomic metric group; iv) the codon context metric group; v) the transition/transversion metric group; vi) the synonymous/non-synonymous metric group; vii) the strand bias metric group; viii) the strand specific metric group; ix) the AT/GC metric group; x) the motif metric group; and xi) the motif-independent metric group are determined and/or used. In a particular example, at least 1, 2, 3, 4, 5, 6, 7, 8, 9, 10 or more metrics from each of the metric groups identified in i) through xi) are used.

When metrics from the motif metric group are used, this can include metrics from at least one deaminase motif metric group (e.g. the ADAR motif group, the AID motif group, the APOBEC3G motif group, the APOBEC3B motif group, the APOBEC3F and/or the APOBEC1 motif group), metrics from a three-mer metric group and/or metrics from a five-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In particular examples, a combination of metrics from each of the ADAR motif group, the AID motif group, the APOBEC3G motif group, the APOBEC3B motif group, and the three-mer metric group are determined and/or used.

In some instances, a combination of metrics from the motif metric group and the motif-independent metric group are determined and/or used in accordance with the methods and systems described herein. At least 2, 3, 4, 5, 6, 7, 8, 9, 10, 15, 20, 30, 40, 50, 60, 70, 80, 90 or 100 metrics from each group may be utilised. In some examples, the metrics from the motif metric group include metrics from at least 2 (e.g. 2, 3, 4, 5, or 6) deaminase motif groups selected from among the ADAR motif group, the AID motif group, the APOBEC3G motif group, the APOBEC3B motif group, the APOBEC3F and the APOBEC1 motif group, and also optionally from the three-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In a particular example, the metrics from the motif metric group include at least 2, 3, 4, 5, 6, 7, 8, 9, 10, 15, 20, 30, 40, 50, 60, 70, 80 or 100 metrics from each of the ADAR motif group, the AID motif group, the APOBEC3G motif group, and the APOBEC3B motif group, and optionally each of these deaminase motif metric groups can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B.

In other examples, a combination of metrics from the motif metric group and the codon context group are determined and/or used in accordance with the methods and systems described herein. At least 2, 3, 4, 5, 6, 7, 8, 9, 10, 15, 20, 30, 40, 50, 60, 70, 80, 90 or 100 metrics from each group may be utilised. In some examples, the metrics from the motif metric group include metrics from at least 2 (e.g. 2, 3, 4, 5, or 6) deaminase motif groups selected from among the ADAR motif group, the AID motif group, the APOBEC3G motif group, the APOBEC3B motif group, the APOBEC3F and the APOBEC1 motif group, and also optionally from the three-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In a particular example, the metrics from the motif metric group include at least 2, 3, 4, 5, 6, 7, 8, 9, 10, 15, 20, 30, 40, 50, 60, 70, 80 or 100 metrics from each of the ADAR motif group, the AID motif group, the APOBEC3G motif group, and the APOBEC3B motif group, and optionally each of these deaminase motif metric groups can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In some examples, these metrics are also combined with at least 2, 3, 4, 5, 6, 7, 8, 9, 10, 15, 20, 30, 40, 50, 60, 70, 80, 90 or 100 metrics from the transition/transversion, synonymous/non-synonymous group, and/or strand specific group.

In further instances, a combination of metrics from the motif metric group, the codon context metric group and the motif-independent metric group are determined and/or used in accordance with the methods and systems described herein. At least 2, 3, 4, 5, 6, 7, 8, 9, 10, 15, 20, 30, 40, 50, 60, 70, 80, 90 or 100 metrics from each group may be utilised. In some examples, the metrics from the motif metric group include metrics from at least 2 (e.g. 2, 3, 4, 5, or 6) deaminase motif groups selected from among the ADAR motif group, the AID motif group, the APOBEC3G motif group, the APOBEC3B motif group, the APOBEC3F and the APOBEC1 motif group, and also optionally from the three-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In a particular example, the metrics from the motif metric group include at least 2, 3, 4, 5, 6, 7, 8, 9, 10, 15, 20, 30, 40, 50, 60, 70, 80 or 100 metrics from each of the ADAR motif group, the AID motif group, the APOBEC3G motif group, and the APOBEC3B motif group, and optionally each of these deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In some examples, these metrics are also combined with at least 2, 3, 4, 5, 6, 7, 8, 9, 10, 15, 20, 30, 40, 50, 60, 70, 80, 90 or 100 metrics from the transition/transversion, synonymous/non-synonymous group, and/or strand bias group.

In other examples, a combination of metrics from the motif metric group and the strand specific group are determined and/or used in accordance with the methods and systems described herein. At least 2, 3, 4, 5, 6, 7, 8, 9, 10, 15, 20, 30, 40, 50, 60, 70, 80, 90 or 100 metrics from each group may be utilised. In some examples, the metrics from the motif metric group include metrics from at least 2 (e.g. 2, 3, 4, 5, or 6) deaminase motif groups selected from among the ADAR motif group, the AID motif group, the APOBEC3G motif group, the APOBEC3B motif group, the APOBEC3F and the APOBEC1 motif group, and also optionally from the three-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In a particular example, the metrics from the motif metric group include at least 2, 3, 4, 5, 6, 7, 8, 9, 10, 15, 20, 30, 40, 50, 60, 70, 80 or 100 metrics from each of the ADAR motif group, the AID motif group, the APOBEC3G motif group, and the APOBEC3B motif group, and optionally each of these deaminase motif metric groups can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In some examples, these metrics are also combined with at least 2, 3, 4, 5, 6, 7, 8, 9, 10, 15, 20, 30, 40, 50, 60, 70, 80, 90 or 100 metrics from the transition/transversion, synonymous/non-synonymous group, and/or strand bias group.

In other examples, a combination of metrics from the motif metric group, the codon context group and the strand specific group are determined and/or used in accordance with the methods and systems described herein. At least 2, 3, 4, 5, 6, 7, 8, 9, 10, 15, 20, 30, 40, 50, 60, 70, 80, 90 or 100 metrics from each group may be utilised. In some examples, the metrics from the motif metric group include metrics from at least 2 (e.g. 2, 3, 4, 5, or 6) deaminase motif groups selected from among the ADAR motif group, the AID motif group, the APOBEC3G motif group, the APOBEC3B motif group, the APOBEC3F and the APOBEC1 motif group, and also optionally from the three-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In a particular example, the metrics from the motif metric group include at least 2, 3, 4, 5, 6, 7, 8, 9, 10, 15, 20, 30, 40, 50, 60, 70, 80 or 100 metrics from each of the ADAR motif group, the AID motif group, the APOBEC3G motif group, and the APOBEC3B motif group, and optionally each of these deaminase motif metric groups can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In some examples, these metrics are also combined with at least 2, 3, 4, 5, 6, 7, 8, 9, 10, 15, 20, 30, 40, 50, 60, 70, 80, 90 or 100 metrics from the strand bias, transition/transversion and/or synonymous/non-synonymous group.

In further examples, a combination of metrics from the motif metric group and the transition/transversion group are determined and/or used in accordance with the methods and systems described herein. At least 2, 3, 4, 5, 6, 7, 8, 9, 10, 15, 20, 30, 40, 50, 60, 70, 80, 90 or 100 metrics from each group may be utilised. In some examples, the metrics from the motif metric group include metrics from at least 2 (e.g. 2, 3, 4, 5, or 6) deaminase motif groups selected from among the ADAR motif group, the AID motif group, the APOBEC3G motif group, the APOBEC3B motif group, the APOBEC3F and the APOBEC1 motif group, and also optionally from the three-mer metric group. Moreover, each deaminase motif metric group can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Bf, A3Bg and A3Bh, as set forth in Table B. In a particular example, the metrics from the motif metric group include at least 2, 3, 4, 5, 6, 7, 8, 9, 10, 15, 20, 30, 40, 50, 60, 70, 80 or 100 metrics from each of the ADAR motif group, the AID motif group, the APOBEC3G motif group, and the APOBEC3B motif group, and optionally each of these deaminase motif metric groups can include metrics determined on the basis of 2 or more specific motifs, e.g. 2 or more of AID, AIDb, AIDc, AIDd, AIDe, AIDf and AIDg; 2 or more of ADAR, ADARb, ADARc, ADARd, ADARe, ADARf, ADARg, ADARh, ADARi and ADARj; 2 or more of A3G, A3Gb, A3Gc, A3Gd, A3Ge, A3Gf, A3Gg, A3Gh and A3Gi; and/or 2 or more of A3B, A3Bb, A3Bc, A3Bd, A3Be, A3Bf, A3Bg and A3Bh, as set forth in Table B. In some examples, these metrics are also combined with at least 2, 3, 4, 5, 6, 7, 8, 9, 10, 15, 20, 30, 40, 50, 60, 70, 80, 90 or 100 metrics from the codon context group and/or the strand specific group.

In a particular example, at least 20, 50, 100, 200, 300, 400, 500, 600, 700, 800, 900, 1000, 1200, 1400, 1600, 1800, 2000, 2200, 2400, 2600, 2800, 3000, 3200, 3400, 3600, 3800, 4000, 4200, 4400, 4600, 4800, 5000, 5200, 5400 or 5600, or all, metrics selected from Table G are determined and/or used in the methods and systems of the disclosure. Nomenclature of the metrics in Table G is essentially as described above. For example, "cds AID Hits" is the number of SNVs at the AID primary motif of WRC/GYW in the coding region. Reference to Gen1, Gen2 and Gen3 (including reference to ADAR_Gen1, ADAR_Gen2, and ADAR_Gen3) simply refers to various three-mer motifs as described above in section 3.1.

Table G. Exemplary metrics

| Metric | Metric | Metric |
|---|---|---|
| CDS Variants | cds:AIDf G>A at MC2 % | cds:ADAR_Gen3_ACA A>G at MC1 % |
| Total Variants | cds:AIDf G>A at MC3 % | cds:ADAR_Gen3_ACA A>G at MC2 % |
| cds:AID Hits | cds:AIDf C>T % | cds:ADAR_Gen3_ACA A>G at MC3 % |
| cds:AID % | cds:AIDf C>A % | cds:ADAR_Gen3_ACA T>C at MC1 % |
| cds:AID Ti % | cds:AIDf C>G % | cds:ADAR_Gen3_ACA T>C at MC2 % |
| cds:AID MC1 % | cds:AIDf G>A % | cds:ADAR_Gen3_ACA T>C at MC3 % |
| cds:AID MC2 % | cds:AIDf G>T % | cds:ADAR_Gen3_ACA A>G % |
| cds:AID MC3 % | cds:AIDf G>C % | cds:ADAR_Gen3_ACA A>C % |
| cds:AID C>T at MC1 % | cds:AIDf Ti/Tv % | cds:ADAR_Gen3_ACA A>T % |
| cds:AID C>T at MC2 % | cds:AIDf C:G % | cds:ADAR_Gen3_ACA T>C % |
| cds:AID C>T at MC3 % | cds:AIDf Ti C:G % | cds:ADAR_Gen3_ACA T>G % |
| cds:AID G>A at MC1 % | cds:AIDf non-syn % | cds:ADAR_Gen3_ACA T>A % |
| cds:AID G>A at MC2 % | cds:AIDf C non-syn % | cds:ADAR_Gen3_ACA Ti/Tv % |
| cds:AID G>A at MC3 % | cds:AIDf G non-syn % | cds:ADAR_Gen3_ACA A:T % |
| cds:AID C>T % | cds:AIDf MC1 non-syn % | cds:ADAR_Gen3_ACA Ti A:T % |
| cds:AID C>A % | cds:AIDf MC2 non-syn % | cds:ADAR_Gen3_ACA non-syn % |
| cds:AID C>G % | cds:AIDf MC3 non-syn % | cds:ADAR_Gen3_ACA A non-syn % |
| cds:AID G>A % | g:AIDf Hits | cds:ADAR_Gen3_ACA T non-syn % |
| cds:AID G>T % | g:AIDf % | cds:ADAR_Gen3_ACA MC1 non-syn % |

(continued)

| Metric | Metric | Metric |
|---|---|---|
| cds:AID G>C % | g:AIDf Ti % | cds:ADAR_Gen3_ACA MC2 non-syn % |
| cds:AID Ti/Tv % | g:AIDf C>T + G>A % | cds:ADAR_Gen3_ACA MC3 non-syn % |
| cds:AID C:G % | g:AIDf C>A + G>T % | g:ADAR_Gen3_ACA Hits |
| cds:AID Ti C:G % | g:AIDf C>G + G>C % | g:ADAR_Gen3_ACA % |
| cds:AID non-syn % | nc:AIDf Hits | g:ADAR_Gen3_ACA Ti % |
| cds:AID C non-syn % | nc:AIDf % | g:ADAR_Gen3_ACA A>G + T>C % |
| cds:AID G non-syn % | nc:AIDf Ti % | g:ADAR_Gen3_ACA A>C + T>G % |
| cds:AID MC1 non-syn % | nc:AIDf C>T + G>A % | g:ADAR_Gen3_ACA A>T + T>A % |
| cds:AID MC2 non-syn % | nc:AIDf C>A + G>T % | nc:ADAR_Gen3_ACA Hits |
| cds:AID MC3 non-syn % | nc:AIDf C>G + G>C % | nc:ADAR_Gen3_ACA % |
| g:AID Hits | cds:AIDg Hits | nc:ADAR_Gen3_ACA Ti % |
| g:AID % | cds:AIDg % | nc:ADAR_Gen3_ACA A>G + T>C |
| g:AID Ti % | cds:AIDg Ti % | nc:ADAR_Gen3_ACA A>C + T>G |
| g:AID C>T + G>A % | cds:AIDg MC1 % | nc:ADAR_Gen3_ACA A>T + T>A % |
| g:AID C>A + G>T % | cds:AIDg MC2 % | cds:ADAR_Gen3_AGA Hits |
| g:AID C>G + G>C % | cds:AIDg MC3 % | cds:ADAR_Gen3_AGA % |
| nc:AID Hits | cds:AIDg C>T at MC1 % | cds:ADAR_Gen3_AGA Ti **%** |
| nc:AID % | cds:AIDg C>T at MC2 % | cds:ADAR_Gen3_AGA MC1 % |
| nc:AID Ti % | cds:AIDg C>T at MC3 % | cds:ADAR_Gen3_AGA MC2 % |
| nc:AID C>T + G>A % | cds:AIDg G>A at MC1 % | cds:ADAR_Gen3_AGA MC3 % |
| nc:AID C>A + G>T % | cds:AIDg G>A at MC2 % | cds:ADAR_Gen3_AGA A>G at MC1 % |
| nc:AID C>G + G>C % | cds:AIDg G>A at MC3 % | cds:ADAR_Gen3_AGA A>G at MC2 % |
| cds:ADAR Hits | cds:AIDg C>T % | cds:ADAR_Gen3_AGA A>G at MC3 |
| cds:ADAR % | cds:AIDg C>A % | cds:ADAR_Gen3_AGA T>C at MC1 |
| cds:ADAR Ti % | cds:AIDg C>G % | cds:ADAR_Gen3_AGA T>C at MC2 |
| cds:ADAR MC1 % | cds:AIDg G>A % | cds:ADAR_Gen3_AGA T>C at MC3 |
| cds:ADAR MC2 % | cds:AIDg G>T % | cds:ADAR_Gen3_AGA A>G % |
| cds:ADAR MC3 % | cds:AIDg G>C % | cds:ADAR_Gen3_AGA A>C % |
| cds:ADAR A>G at MC1 % | cds:AIDg Ti/Tv % | cds:ADAR_Gen3_AGA A>T % |
| cds:ADAR A>G at MC2 % | cds:AIDg C:G % | cds:ADAR_Gen3_AGA T>C % |
| cds:ADAR A>G at MC3 % | cds:AIDg Ti C:G % | cds:ADAR_Gen3_AGA T>G % |
| cds:ADAR T>C at MC1 % | cds:AIDg non-syn % | cds:ADAR_Gen3_AGA T>A % |
| cds:ADAR T>C at MC2 % | cds:AIDg C non-syn % | cds:ADAR_Gen3_AGA Ti/Tv % |
| cds:ADAR T>C at MC3 % | cds:AIDg G non-syn % | cds:ADAR_Gen3_AGA A:T % |
| cds:ADAR A>G % | cds:AIDg MC1 non-syn % | cds:ADAR_Gen3_AGA Ti A:T % |
| cds:ADAR A>C % | cds:AIDg MC2 non-syn % | cds:ADAR_Gen3_AGA non-syn % |

(continued)

| Metric | Metric | Metric |
|---|---|---|
| cds:ADAR A>T % | cds:AIDg MC3 non-syn % | cds:ADAR_Gen3_AGA A non-syn % |
| cds:ADAR T>C % | g:AIDg Hits | cds:ADAR_Gen3_AGA T non-syn % |
| cds:ADAR T>G % | g:AIDg % | cds:ADAR_Gen3_AGA MC1 non-syn % |
| cds:ADAR T>A % | g:AIDg Ti % | cds:ADAR_Gen3_AGA MC2 non-syn % |
| cds:ADAR Ti/Tv % | g:AIDg C>T + G>A % | cds:ADAR_Gen3_AGA MC3 non-syn % |
| cds:ADAR A:T % | g:AIDg C>A + G>T % | g:ADAR_Gen3_AGA Hits |
| cds:ADAR Ti A:T % | g:AIDg C>G + G>C % | g:ADAR_Gen3_AGA % |
| cds:ADAR non-syn % | nc:AIDg Hits | g:ADAR_Gen3_AGA Ti **%** |
| cds:ADAR A non-syn % | nc:AIDg % | g:ADAR_Gen3_AGA A>G + T>C % |
| cds:ADAR T non-syn % | nc:AIDg Ti % | g:ADAR_Gen3_AGA A>C + T>G % |
| cds:ADAR MC1 non-syn % | nc:AIDg C>T + G>A % | g:ADAR_Gen3_AGA A>T + T>A % |
| cds:ADAR MC2 non-syn % | nc:AIDg C>A + G>T % | nc:ADAR_Gen3_AGA Hits |
| cds:ADAR MC3 non-syn % | nc:AIDg C>G + G>C % | nc:ADAR_Gen3_AGA % |
| g:ADAR Hits | cds:ADARb Hits | nc:ADAR_Gen3_AGA Ti % |
| g:ADAR % | cds:ADARb % | nc:ADAR_Gen3_AGA A>G + T>C |
| g:ADAR Ti % | cds:ADARb Ti % | nc:ADAR_Gen3_AGA A>C + T>G |
| g:ADAR A>G + T>C % | cds:ADARb MC1 % | nc:ADAR_Gen3_AGA A>T + T>A % |
| g:ADAR A>C + T>G % | cds:ADARb MC2 % | cds:ADAR_Gen3_TAA Hits |
| g:ADAR A>T + T>A % | cds:ADARb MC3 % | cds:ADAR_Gen3_TAA % |
| nc:ADAR Hits | cds:ADARb A>G at MC1 % | cds:ADAR_Gen3_TAA Ti % |
| nc:ADAR % | cds:ADARb A>G at MC2 % | cds:ADAR_Gen3_TAA MC1 % |
| nc:ADAR Ti % | cds:ADARb A>G at MC3 % | cds:ADAR_Gen3_TAA MC2 % |
| nc:ADAR A>G + T>C % | cds:ADARb T>C at MC1 % | cds:ADAR_Gen3_TAA MC3 % |
| nc:ADAR A>C + T>G % | cds:ADARb T>C at MC2 % | cds:ADAR_Gen3_TAA A>G at MC1 % |
| nc:ADAR A>T + T>A % | cds:ADARb T>C at MC3 % | cds:ADAR_Gen3_TAA A>G at MC2 % |
| cds:A3G Hits | cds:ADARb A>G % | cds:ADAR_Gen3_TAA A>G at MC3 % |
| cds:A3G % | cds:ADARb A>C % | cds:ADAR_Gen3_TAA T>C at MC1 % |
| cds:A3G Ti % | cds:ADARb A>T % | cds:ADAR_Gen3_TAA T>C at MC2 % |
| cds:A3G MC1 % | cds:ADARb T>C % | cds:ADAR_Gen3_TAA T>C at MC3 % |
| cds:A3G MC2 % | cds:ADARb T>G % | cds:ADAR_Gen3_TAA A>G % |
| cds:A3G MC3 % | cds:ADARb T>A % | cds:ADAR_Gen3_TAA A>C % |
| cds:A3G C>T at MC1 % | cds:ADARb Ti/Tv % | cds:ADAR_Gen3_TAA A>T % |
| cds:A3G C>T at MC2 % | cds:ADARb A:T % | cds:ADAR_Gen3_TAA T>C % |

(continued)

| Metric | Metric | Metric |
|---|---|---|
| cds:A3G C>T at MC3 % | cds:ADARb Ti A:T % | cds:ADAR_Gen3_TAA T>G % |
| cds:A3G G>A at MC1 % | cds:ADARb non-syn % | cds:ADAR_Gen3_TAA T>A % |
| cds:A3G G>A at MC2 % | cds:ADARb A non-syn % | cds:ADAR_Gen3_TAA Ti/Tv % |
| cds:A3G G>A at MC3 % | cds:ADARb T non-syn % | cds:ADAR_Gen3_TAA A:T % |
| cds:A3G C>T % | cds:ADARb MC1 non-syn % | cds:ADAR_Gen3_TAA Ti A:T % |
| cds:A3G C>A % | cds:ADARb MC2 non-syn % | cds:ADAR_Gen3_TAA non-syn % |
| cds:A3G C>G % | cds:ADARb MC3 non-syn % | cds:ADAR_Gen3_TAA A non-syn % |
| cds:A3G G>A % | g:ADARb Hits | cds:ADAR_Gen3_TAA T non-syn % |
| cds:A3G G>T % | g:ADARb % | cds:ADAR_Gen3_TAA MC1 non-syn % |
| cds:A3G G>C % | g:ADARb Ti % | cds:ADAR_Gen3_TAA MC2 non-syn % |
| cds:A3G Ti/Tv % | g:ADARb A>G + T>C % | cds:ADAR_Gen3_TAA MC3 non-syn % |
| cds:A3G C:G % | g:ADARb A>C + T>G % | g:ADAR_Gen3_TAA Hits |
| cds:A3G Ti C:G % | g:ADARb A>T + T>A % | g:ADAR_Gen3_TAA % |
| cds:A3G non-syn % | nc:ADARb Hits | g:ADAR_Gen3_TAA Ti % |
| cds:A3G C non-syn % | nc:ADARb % | g:ADAR_Gen3_TAA A>G + T>C % |
| cds:A3G G non-syn % | nc:ADARb Ti % | g:ADAR_Gen3_TAA A>C + T>G % |
| cds:A3G MC1 non-syn % | nc:ADARb A>G + T>C % | g:ADAR_Gen3_TAA A>T + T>A % |
| cds:A3G MC2 non-syn % | nc:ADARb A>C + T>G % | nc:ADAR_Gen3_TAA Hits |
| cds:A3G MC3 non-syn % | nc:ADARb A>T + T>A % | nc:ADAR_Gen3_TAA % |
| g:A3G Hits | cds:ADARc Hits | nc:ADAR_Gen3_TAA Ti % |
| g:A3G % | cds:ADARc % | nc:ADAR_Gen3_TAA A>G + T>C |
| g:A3G Ti % | cds:ADARc Ti % | nc:ADAR_Gen3_TAA A>C + T>G |
| g:A3G C>T + G>A % | cds:ADARc MC1 % | nc:ADAR_Gen3_TAA A>T + T>A % |
| g:A3G C>A + G>T % | cds:ADARc MC2 % | cds:ADAR_Gen3_TTA Hits |
| g:A3G C>G + G>C % | cds:ADARc MC3 % | cds:ADAR_Gen3_TTA % |
| nc:A3G Hits | cds:ADARc A>G at MC1 % | cds:ADAR_Gen3_TTA Ti % |
| nc:A3G % | cds:ADARc A>G at MC2 % | cds:ADAR_Gen3_TTA MC1 % |
| nc:A3G Ti % | cds:ADARc A>G at MC3 % | cds:ADAR_Gen3_TTA MC2 % |
| nc:A3G C>T + G>A % | cds:ADARc T>C at MC1 % | cds:ADAR_Gen3_TTA MC3 % |
| nc:A3G C>A + G>T % | cds:ADARc T>C at MC2 % | cds:ADAR_Gen3_TTA A>G at MC1 % |
| nc:A3G C>G + G>C % | cds:ADARc T>C at MC3 % | cds:ADAR_Gen3_TTA A>G at MC2 % |
| cds:A3B Hits | cds:ADARc A>G % | cds:ADAR_Gen3_TTA A>G at MC3 % |
| cds:A3B % | cds:ADARc A>C % | cds:ADAR_Gen3_TTA T>C at MC1 % |
| cds:A3B Ti % | cds:ADARc A>T % | cds:ADAR_Gen3_TTA T>C at MC2 % |

(continued)

| Metric | Metric | Metric |
|---|---|---|
| cds:A3B MC1 % | cds:ADARc T>C % | cds:ADAR_Gen3_TTA T>C at MC3 % |
| cds:A3B MC2 % | cds:ADARc T>G % | cds:ADAR_Gen3_TTA A>G % |
| cds:A3B MC3 % | cds:ADARc T>A % | cds:ADAR_Gen3_TTA A>C % |
| cds:A3B C>T at MC1 % | cds:ADARc Ti/Tv % | cds:ADAR_Gen3_TTA A>T % |
| cds:A3B C>T at MC2 % | cds:ADARc A:T % | cds:ADAR_Gen3_TTA T>C % |
| cds:A3B C>T at MC3 % | cds:ADARc Ti A:T % | cds:ADAR_Gen3_TTA T>G % |
| cds:A3B G>A at MC1 % | cds:ADARc non-syn % | cds:ADAR_Gen3_TTA T>A % |
| cds:A3B G>A at MC2 % | cds:ADARc A non-syn % | cds:ADAR_Gen3_TTA Ti/Tv % |
| cds:A3B G>A at MC3 % | cds:ADARc T non-syn % | cds:ADAR_Gen3_TTA A:T % |
| cds:A3B C>T % | cds:ADARc MC1 non-syn % | cds:ADAR_Gen3_TTA Ti A:T % |
| cds:A3B C>A % | cds:ADARc MC2 non-syn % | cds:ADAR_Gen3_TTA non-syn % |
| cds:A3B C>G % | cds:ADARc MC3 non-syn % | cds:ADAR_Gen3_TTA A non-syn % |
| cds:A3B G>A % | g:ADARc Hits | cds:ADAR_Gen3_TTA T non-syn % |
| cds:A3B G>T % | g:ADARc % | cds:ADAR_Gen3_TTA MC1 non-syn % |
| cds:A3B G>C % | g:ADARc Ti % | cds:ADAR_Gen3_TTA MC2 non-syn % |
| cds:A3B Ti/Tv % | g:ADARc A>G + T>C % | cds:ADAR_Gen3_TTA MC3 non-syn % |
| cds:A3B C:G % | g:ADARc A>C + T>G % | g:ADAR_Gen3_TTA Hits |
| cds:A3B Ti C:G % | g:ADARc A>T + T>A % | g:ADAR_Gen3_TTA % |
| cds:A3B non-syn % | nc:ADARc Hits | g:ADAR_Gen3_TTA Ti % |
| cds:A3B C non-syn % | nc:ADARc % | g:ADAR_Gen3_TTA A>G + T>C % |
| cds:A3B G non-syn % | nc:ADARc Ti % | g:ADAR_Gen3_TTA A>C + T>G % |
| cds:A3B MC1 non-syn % | nc:ADARc A>G + T>C % | g:ADAR_Gen3_TTA A>T + T>A % |
| cds:A3B MC2 non-syn % | nc:ADARc A>C + T>G % | nc:ADAR_Gen3_TTA Hits |
| cds:A3B MC3 non-syn % | nc:ADARc A>T + T>A % | nc:ADAR_Gen3_TTA % |
| cds:Primary Deaminase % | cds:ADARd Hits | nc:ADAR_Gen3_TTA Ti % |
| cds:All A total | cds:ADARd % | nc:ADAR_Gen3_TTA A>G + T>C % |
| cds:All T total | cds:ADARd Ti % | nc:ADAR_Gen3_TTA A>C + T>G % |
| cds:All C total | cds:ADARd MC1 % | nc:ADAR_Gen3_TTA A>T + T>A % |
| cds:All G total | cds:ADARd MC2 % | cds:ADAR_Gen3_TCA Hits |
| cds:All A % | cds:ADARd MC3 % | cds:ADAR_Gen3_TCA % |
| cds:All T % | cds:ADARd A>G at MC1 % | cds:ADAR_Gen3_TCA Ti % |
| cds:All C % | cds:ADARd A>G at MC2 % | cds:ADAR_Gen3_TCA MC1 % |
| cds:All G % | cds:ADARd A>G at MC3 % | cds:ADAR_Gen3_TCA MC2 % |
| cds:All MC1 % | cds:ADARd T>C at MC1 % | cds:ADAR_Gen3_TCA MC3 % |
| cds:All MC2 % | cds:ADARd T>C at MC2 % | cds:ADAR_Gen3_TCA A>G at MC1 % |

(continued)

| Metric | Metric | Metric |
|---|---|---|
| cds:All MC3 % | cds:ADARd T>C at MC3 % | cds:ADAR_Gen3_TCA A>G at MC2 % |
| cds:All A MC1 % | cds:ADARd A>G % | cds:ADAR_Gen3_TCA A>G at MC3 % |
| cds:All A MC2 % | cds:ADARd A>C % | cds:ADAR_Gen3_TCA T>C at MC1 % |
| cds:All A MC3 % | cds:ADARd A>T % | cds:ADAR_Gen3_TCA T>C at MC2 % |
| cds:All T MC1 % | cds:ADARd T>C % | cds:ADAR_Gen3_TCA T>C at MC3 % |
| cds:All T MC2 % | cds:ADARd T>G % | cds:ADAR_Gen3_TCA A>G % |
| cds:All T MC3 % | cds:ADARd T>A % | cds:ADAR_Gen3_TCA A>C % |
| cds:All C MC1 % | cds:ADARd Ti/Tv % | cds:ADAR_Gen3_TCA A>T % |
| cds:All C MC2 % | cds:ADARd A:T % | cds:ADAR_Gen3_TCA T>C % |
| cds:All C MC3 % | cds:ADARd Ti A:T % | cds:ADAR_Gen3_TCA T>G % |
| cds:All G MC1 % | cds:ADARd non-syn % | cds:ADAR_Gen3_TCA T>A % |
| cds:All G MC2 % | cds:ADARd A non-syn % | cds:ADAR_Gen3_TCA Ti/Tv % |
| cds:All G MC3 % | cds:ADARd T non-syn % | cds:ADAR_Gen3_TCA A:T % |
| cds:All MC1 A % | cds:ADARd MC1 non-syn % | cds:ADAR_Gen3_TCA Ti A:T % |
| cds:All MC1 T % | cds:ADARd MC2 non-syn % | cds:ADAR_Gen3_TCA non-syn % |
| cds:All MC1 C % | cds:ADARd MC3 non-syn % | cds:ADAR_Gen3_TCA A non-syn % |
| cds:All MC1 G % | g:ADARd Hits | cds:ADAR_Gen3_TCA T non-syn % |
| cds:All MC2 A % | g:ADARd % | cds:ADAR_Gen3_TCA MC1 non-syn % |
| cds:All MC2 T % | g:ADARd Ti % | cds:ADAR_Gen3_TCA MC2 non-syn % |
| cds:All MC2 C % | g:ADARd A>G + T>C % | cds:ADAR_Gen3_TCA MC3 non-syn % |
| cds:All MC2 G % | g:ADARd A>C + T>G % | g:ADAR_Gen3_TCA Hits |
| cds:All MC3 A % | g:ADARd A>T + T>A % | g:ADAR_Gen3_TCA % |
| cds:All MC3 T % | nc:ADARd Hits | g:ADAR_Gen3_TCA Ti % |
| cds:All MC3 C % | nc:ADARd % | g:ADAR_Gen3_TCA A>G + T>C % |
| cds:All MC3 G % | nc:ADARd Ti % | g:ADAR_Gen3_TCA A>C + T>G % |
| cds:All A Ti/Tv % | nc:ADARd A>G + T>C % | g:ADAR_Gen3_TCA A>T + T>A % |
| cds:All T Ti/Tv % | nc:ADARd A>C + T>G % | nc:ADAR_Gen3_TCA Hits |
| cds:All C Ti/Tv % | nc:ADARd A>T + T>A % | nc:ADAR_Gen3_TCA % |
| cds:All G Ti/Tv % | cds:ADARe Hits | nc:ADAR_Gen3_TCA Ti % |
| cds:All AT Ti/Tv % | cds:ADARe % | nc:ADAR_Gen3_TCA A>G + T>C % |
| cds:All GC Ti/Tv % | cds:ADARe Ti % | nc:ADAR_Gen3_TCA A>C + T>G % |
| cds:All MC1 Ti/Tv % | cds:ADARe MC1 % | nc:ADAR_Gen3_TCA A>T + T>A % |
| cds:All MC2 Ti/Tv % | cds:ADARe MC2 % | cds:ADAR_Gen3_TGA Hits |
| cds:All MC3 Ti/Tv % | cds:ADARe MC3 % | cds:ADAR_Gen3_TGA % |

(continued)

| Metric | Metric | Metric |
|---|---|---|
| cds:All A MC1 Ti/Tv % | cds:ADARe A>G at MC1 % | cds:ADAR_Gen3_TGA Ti % |
| cds:All A MC2 Ti/Tv % | cds:ADARe A>G at MC2 % | cds:ADAR_Gen3_TGA MC1 % |
| cds:All A MC3 Ti/Tv % | cds:ADARe A>G at MC3 % | cds:ADAR_Gen3_TGA MC2 % |
| cds:All T MC1 Ti/Tv % | cds:ADARe T>C at MC1 % | cds:ADAR_Gen3_TGA MC3 % |
| cds:All T MC2 Ti/Tv % | cds:ADARe T>C at MC2 % | cds:ADAR_Gen3_TGA A>G at MC1 |
| cds:All T MC3 Ti/Tv % | cds:ADARe T>C at MC3 % | cds:ADAR_Gen3_TGA A>G at MC2 % |
| cds:All C MC1 Ti/Tv % | cds:ADARe A>G % | cds:ADAR_Gen3_TGA A>G at MC3 % |
| cds:All C MC2 Ti/Tv % | cds:ADARe A>C % | cds:ADAR_Gen3_TGA T>C at MC1 % |
| cds:All C MC3 Ti/Tv % | cds:ADARe A>T % | cds:ADAR_Gen3_TGA T>C at MC2 % |
| cds:All G MC1 Ti/Tv % | cds:ADARe T>C % | cds:ADAR_Gen3_TGA T>C at MC3 % |
| cds:All G MC2 Ti/Tv % | cds:ADARe T>G % | cds:ADAR_Gen3_TGA A>G % |
| cds:All G MC3 Ti/Tv % | cds:ADARe T>A % | cds:ADAR_Gen3_TGA A>C % |
| cds:All C:G % | cds:ADARe Ti/Tv % | cds:ADAR_Gen3_TGA A>T % |
| cds:All A:T % | cds:ADARe A:T % | cds:ADAR_Gen3_TGA T>C % |
| cds:All AT:GC % | cds:ADARe Ti A:T % | cds:ADAR_Gen3_TGA T>G % |
| cds:All MC1 C:G % | cds:ADARe non-syn % | cds:ADAR_Gen3_TGA T>A % |
| cds:All MC2 C:G % | cds:ADARe A non-syn % | cds:ADAR_Gen3_TGA Ti/Tv % |
| cds:All MC3 C:G % | cds:ADARe T non-syn % | cds:ADAR_Gen3_TGA A:T % |
| cds:All MC1 A:T % | cds:ADARe MC1 non-syn % | cds:ADAR_Gen3_TGA Ti A:T % |
| cds:All MC2 A:T % | cds:ADARe MC2 non-syn % | cds:ADAR_Gen3_TGA non-syn % |
| cds:All MC3 A:T % | cds:ADARe MC3 non-syn % | cds:ADAR_Gen3_TGA A non-syn % |
| cds:All MC1 AT:GC % | g:ADARe Hits | cds:ADAR_Gen3_TGA T non-syn % |
| cds:All MC2 AT:GC % | g:ADARe % | cds:ADAR_Gen3_TGA MC1 non-syn % |
| cds:All MC3 AT:GC % | g:ADARe Ti % | cds:ADAR_Gen3_TGA MC2 non-syn % |
| cds:All A>G % | g:ADARe A>G + T>C % | cds:ADAR_Gen3_TGA MC3 non-syn % |
| cds:All A>C % | g:ADARe A>C + T>G % | g:ADAR_Gen3_TGA Hits |
| cds:All A>T % | g:ADARe A>T + T>A % | g:ADAR_Gen3_TGA % |
| cds:All T>C % | nc:ADARe Hits | g:ADAR_Gen3_TGA Ti % |
| cds:All T>G % | nc:ADARe % | g:ADAR_Gen3_TGA A>G + T>C % |
| cds:All T>A % | nc:ADARe Ti % | g:ADAR_Gen3_TGA A>C + T>G % |
| cds:All C>T % | nc:ADARe A>G + T>C % | g:ADAR_Gen3_TGA A>T + T>A % |
| cds:All C>A % | nc:ADARe A>C + T>G % | nc:ADAR_Gen3_TGA Hits |
| cds:All C>G % | nc:ADARe A>T + T>A % | nc:ADAR_Gen3_TGA % |
| cds:All G>A % | cds:ADARf Hits | nc:ADAR_Gen3_TGA Ti % |

(continued)

| Metric | Metric | Metric |
|---|---|---|
| cds:All G>T % | cds:ADARf % | nc:ADAR_Gen3_TGA A>G + T>C % |
| cds:All G>C % | cds:ADARf Ti % | nc:ADAR_Gen3_TGA A>C + T>G % |
| cds:All non-syn % | cds:ADARf MC1 % | nc:ADAR_Gen3_TGA A>T + T>A % |
| cds:All A non-syn % | cds:ADARf MC2 % | cds:ADAR_Gen3_CAA Hits |
| cds:All T non-syn % | cds:ADARf MC3 % | cds:ADAR_Gen3_CAA % |
| cds:All C non-syn % | cds:ADARf A>G at MC1 % | cds:ADAR_Gen3_CAA Ti % |
| cds:All G non-syn % | cds:ADARf A>G at MC2 % | cds:ADAR_Gen3_CAA MC1 % |
| cds:All MC1 non-syn % | cds:ADARf A>G at MC3 % | cds:ADAR_Gen3_CAA MC2 % |
| cds:All MC2 non-syn % | cds:ADARf T>C at MC1 % | cds:ADAR_Gen3_CAA MC3 % |
| cds:All MC3 non-syn % | cds:ADARf T>C at MC2 % | cds:ADAR_Gen3_CAA A>G at MC1 % |
| g:variant total | cds:ADARf T>C at MC3 % | cds:ADAR_Gen3_CAA A>G at MC2 % |
| g:AT total | cds:ADARf A>G % | cds:ADAR_Gen3_CAA A>G at MC3 |
| g:CG total | cds:ADARf A>C % | cds:ADAR_Gen3_CAA T>C at MC1 % |
| g:AT:GC % | cds:ADARf A>T % | cds:ADAR_Gen3_CAA T>C at MC2 |
| g:A>G + T>C % | cds:ADARf T>C % | cds:ADAR_Gen3_CAA T>C at MC3 |
| g:A>C + T>G % | cds:ADARf T>G % | cds:ADAR_Gen3_CAA A>G % |
| g:A>T + T>A % | cds:ADARf T>A % | cds:ADAR_Gen3_CAA A>C % |
| g:C>T + G>A % | cds:ADARf Ti/Tv % | cds:ADAR_Gen3_CAA A>T % |
| g:C>A + G>T % | cds:ADARf A:T % | cds:ADAR_Gen3_CAA T>C % |
| g:C>G + G>C % | cds:ADARf Ti A:T % | cds:ADAR_Gen3_CAA T>G % |
| g:Other variant total | cds:ADARf non-syn % | cds:ADAR_Gen3_CAA T>A % |
| g: Other AT total | cds:ADARf A non-syn % | cds:ADAR_Gen3_CAA Ti/Tv % |
| g: Other CG total | cds:ADARf T non-syn % | cds:ADAR_Gen3_CAA A:T % |
| g: Other AT:GC % | cds:ADARf MC1 non-syn % | cds:ADAR_Gen3_CAA Ti A:T % |
| g: Other A>G + T>C % | cds:ADARf MC2 non-syn % | cds:ADAR_Gen3_CAA non-syn % |
| g: Other A>C + T>G % | cds:ADARf MC3 non-syn % | cds:ADAR_Gen3_CAA A non-syn % |
| g: Other A>T + T>A % | g:ADARf Hits | cds:ADAR_Gen3_CAA T non-syn % |
| g: Other C>T + G>A % | g:ADARf % | cds:ADAR_Gen3_CAA MC1 non-syn % |
| g: Other C>A + G>T % | g:ADARf Ti % | cds:ADAR_Gen3_CAA MC2 non-syn % |
| g: Other C>G + G>C % | g:ADARf A>G + T>C % | cds:ADAR_Gen3_CAA MC3 non-syn % |
| nc:variant total | g:ADARf A>C + T>G % | g:ADAR_Gen3_CAA Hits |
| nc:AT total | g:ADARf A>T + T>A % | g:ADAR_Gen3_CAA % |
| nc:CG total | nc:ADARf Hits | g:ADAR_Gen3_CAA Ti % |
| nc:AT:GC % | nc:ADARf % | g:ADAR_Gen3_CAA A>G + T>C % |
| nc:A>G + T>C % | nc:ADARf Ti % | g:ADAR_Gen3_CAA A>C + T>G % |

(continued)

| Metric | Metric | Metric |
|---|---|---|
| nc:A>C + T>G % | nc:ADARf A>G + T>C % | g:ADAR_Gen3_CAA A>T + T>A % |
| nc:A>T + T>A % | nc:ADARf A>C + T>G % | nc:ADAR_Gen3_CAA Hits |
| nc:C>T + G>A % | nc:ADARf A>T + T>A % | nc:ADAR_Gen3_CAA % |
| nc:C>A + G>T % | cds:ADARg Hits | nc:ADAR_Gen3_CAA Ti % |
| nc:C>G + G>C % | cds:ADARg % | nc:ADAR_Gen3_CAA A>G + T>C |
| nc:Other variant total | cds:ADARg Ti % | nc:ADAR_Gen3_CAA A>C + T>G % |
| nc: Other AT total | cds:ADARg MC1 % | nc:ADAR_Gen3_CAA A>T + T>A % |
| nc: Other CG total | cds:ADARg MC2 % | cds:ADAR_Gen3_CTA Hits |
| nc: Other AT:GC % | cds:ADARg MC3 % | cds:ADAR_Gen3_CTA % |
| nc: Other A>G + T>C % | cds:ADARg A>G at MC1 % | cds:ADAR_Gen3_CTA Ti % |
| nc: Other A>C + T>G % | cds:ADARg A>G at MC2 % | cds:ADAR_Gen3_CTA MC1 % |
| nc: Other A>T + T>A % | cds:ADARg A>G at MC3 % | cds:ADAR_Gen3_CTA MC2 % |
| nc: Other C>T + G>A % | cds:ADARg T>C at MC1 % | cds:ADAR_Gen3_CTA MC3 % |
| nc: Other C>A + G>T % | cds:ADARg T>C at MC2 % | cds:ADAR_Gen3_CTA A>G at MC1 % |
| nc: Other C>G + G>C % | cds:ADARg T>C at MC3 % | cds:ADAR_Gen3_CTA A>G at MC2 % |
| cds:Other deaminase % | cds:ADARg A>G % | cds:ADAR_Gen3_CTA A>G at MC3 % |
| cds:Other A total | cds:ADARg A>C % | cds:ADAR_Gen3_CTA T>C at MC1 % |
| cds:Other T total | cds:ADARg A>T % | cds:ADAR_Gen3_CTA T>C at MC2 % |
| cds:Other C total | cds:ADARg T>C % | cds:ADAR_Gen3_CTA T>C at MC3 % |
| cds:Other G total | cds:ADARg T>G % | cds:ADAR_Gen3_CTA A>G % |
| cds:Other A % | cds:ADARg T>A % | cds:ADAR_Gen3_CTA A>C % |
| cds:Other T % | cds:ADARg Ti/Tv % | cds:ADAR_Gen3_CTA A>T % |
| cds:Other C % | cds:ADARg A:T % | cds:ADAR_Gen3_CTA T>C % |
| cds:Other G % | cds:ADARg Ti A:T % | cds:ADAR_Gen3_CTA T>G % |
| cds:Other MC1 % | cds:ADARg non-syn % | cds:ADAR_Gen3_CTA T>A % |
| cds:Other MC2 % | cds:ADARg A non-syn % | cds:ADAR_Gen3_CTA Ti/Tv % |
| cds:Other MC3 % | cds:ADARg T non-syn % | cds:ADAR_Gen3_CTA A:T % |
| cds:Other A MC1 % | cds:ADARg MC1 non-syn % | cds:ADAR_Gen3_CTA Ti A:T % |
| cds:Other A MC2 % | cds:ADARg MC2 non-syn % | cds:ADAR_Gen3_CTA non-syn % |
| cds:Other A MC3 % | cds:ADARg MC3 non-syn % | cds:ADAR_Gen3_CTA A non-syn % |
| cds:Other T MC1 % | g:ADARg Hits | cds:ADAR_Gen3_CTA T non-syn % |
| cds:Other T MC2 % | g:ADARg % | cds:ADAR_Gen3_CTA MC1 non-syn % |
| cds:Other T MC3 % | g:ADARg Ti % | cds:ADAR_Gen3_CTA MC2 non-syn % |

(continued)

| Metric | Metric | Metric |
|---|---|---|
| cds:Other C MC1 % | g:ADARg A>G + T>C % | cds:ADAR_Gen3_CTA MC3 non-syn % |
| cds:Other C MC2 % | g:ADARg A>C + T>G % | g:ADAR_Gen3_CTA Hits |
| cds:Other C MC3 % | g:ADARg A>T + T>A % | g:ADAR_Gen3_CTA % |
| cds:Other G MC1 % | nc:ADARg Hits | g:ADAR_Gen3_CTA Ti % |
| cds:Other G MC2 % | nc:ADARg % | g:ADAR_Gen3_CTA A>G + T>C % |
| cds:Other G MC3 % | nc:ADARg Ti % | g:ADAR_Gen3_CTA A>C + T>G % |
| cds:Other MC1 A % | nc:ADARg A>G + T>C % | g:ADAR_Gen3_CTA A>T + T>A % |
| cds:Other MC1 T % | nc:ADARg A>C + T>G % | nc:ADAR_Gen3_CTA Hits |
| cds:Other MC1 C % | nc:ADARg A>T + T>A % | nc:ADAR_Gen3_CTA % |
| cds:Other MC1 G % | cds:ADARh Hits | nc:ADAR_Gen3_CTA Ti % |
| cds:Other MC2 A % | cds:ADARh % | nc:ADAR_Gen3_CTA A>G + T>C % |
| cds:Other MC2 T % | cds:ADARh Ti % | nc:ADAR_Gen3_CTA A>C + T>G % |
| cds:Other MC2 C % | cds:ADARh MC1 % | nc:ADAR_Gen3_CTA A>T + T>A % |
| cds:Other MC2 G % | cds:ADARh MC2 % | cds:ADAR_Gen3_CCA Hits |
| cds:Other MC3 A % | cds:ADARh MC3 % | cds:ADAR_Gen3_CCA % |
| cds:Other MC3 T % | cds:ADARh A>G at MC1 % | cds:ADAR_Gen3_CCA Ti % |
| cds:Other MC3 C % | cds:ADARh A>G at MC2 % | cds:ADAR_Gen3_CCA MC1 % |
| cds:Other MC3 G % | cds:ADARh A>G at MC3 % | cds:ADAR_Gen3_CCA MC2 % |
| cds:Other A Ti/Tv % | cds:ADARh T>C at MC1 % | cds:ADAR_Gen3_CCA MC3 % |
| cds:Other T Ti/Tv % | cds:ADARh T>C at MC2 % | cds:ADAR_Gen3_CCA A>G at MC1 % |
| cds:Other C Ti/Tv % | cds:ADARh T>C at MC3 % | cds:ADAR_Gen3_CCA A>G at MC2 % |
| cds:Other G Ti/Tv % | cds:ADARh A>G % | cds:ADAR_Gen3_CCA A>G at MC3 % |
| cds:Other AT Ti/Tv % | cds:ADARh A>C % | cds:ADAR_Gen3_CCA T>C at MC1 % |
| cds:Other GC Ti/Tv % | cds:ADARh A>T % | cds:ADAR_Gen3_CCA T>C at MC2 % |
| cds:Other MC1 Ti/Tv % | cds:ADARh T>C % | cds:ADAR_Gen3_CCA T>C at MC3 % |
| cds:Other MC2 Ti/Tv % | cds:ADARh T>G % | cds:ADAR_Gen3_CCA A>G % |
| cds:Other MC3 Ti/Tv % | cds:ADARh T>A % | cds:ADAR_Gen3_CCA A>C % |
| cds:Other A MC1 Ti/Tv % | cds:ADARh Ti/Tv % | cds:ADAR_Gen3_CCA A>T % |
| cds:Other A MC2 Ti/Tv % | cds:ADARh A:T % | cds:ADAR_Gen3_CCA T>C % |
| cds:Other A MC3 Ti/Tv % | cds:ADARh Ti A:T % | cds:ADAR_Gen3_CCA T>G % |
| cds:Other T MC1 Ti/Tv % | cds:ADARh non-syn % | cds:ADAR_Gen3_CCA T>A % |
| cds:Other T MC2 Ti/Tv % | cds:ADARh A non-syn % | cds:ADAR_Gen3_CCA Ti/Tv % |
| cds:Other T MC3 Ti/Tv % | cds:ADARh T non-syn % | cds:ADAR_Gen3_CCA A:T % |
| cds:Other C MC1 Ti/Tv % | cds:ADARh MC1 non-syn % | cds:ADAR_Gen3_CCA Ti A:T % |

(continued)

| Metric | Metric | Metric |
|---|---|---|
| cds:Other C MC2 Ti/Tv % | cds:ADARh MC2 non-syn % | cds:ADAR_Gen3_CCA non-syn % |
| cds:Other C MC3 Ti/Tv % | cds:ADARh MC3 non-syn % | cds:ADAR_Gen3_CCA A non-syn % |
| cds:Other G MC1 Ti/Tv % | g:ADARh Hits | cds:ADAR_Gen3_CCA T non-syn % |
| cds:Other G MC2 Ti/Tv % | g:ADARh % | cds:ADAR_Gen3_CCA MC1 non-syn % |
| cds:Other G MC3 Ti/Tv % | g:ADARh Ti % | cds:ADAR_Gen3_CCA MC2 non-syn % |
| cds:Other C:G % | g:ADARh A>G + T>C % | cds:ADAR_Gen3_CCA MC3 non-syn % |
| cds:Other A:T % | g:ADARh A>C + T>G % | g:ADAR_Gen3_CCA Hits |
| cds:Other AT:GC % | g:ADARh A>T + T>A % | g:ADAR_Gen3_CCA % |
| cds:Other MC1 C:G % | nc:ADARh Hits | g:ADAR_Gen3_CCA Ti % |
| cds:Other MC2 C:G % | nc:ADARh % | g:ADAR_Gen3_CCA A>G + T>C % |
| cds:Other MC3 C:G % | nc:ADARh Ti % | g:ADAR_Gen3_CCA A>C + T>G % |
| cds:Other MC1 A:T % | nc:ADARh A>G + T>C % | g:ADAR_Gen3_CCA A>T + T>A % |
| cds:Other MC2 A:T % | nc:ADARh A>C + T>G % | nc:ADAR_Gen3_CCA Hits |
| cds:Other MC3 A:T % | nc:ADARh A>T + T>A % | nc:ADAR_Gen3_CCA % |
| cds:Other MC1 AT:GC % | cds:ADARi Hits | nc:ADAR_Gen3_CCA Ti % |
| cds:Other MC2 AT:GC % | cds:ADARi % | nc:ADAR_Gen3_CCA A>G + T>C % |
| cds:Other MC3 AT:GC % | cds:ADARi Ti % | nc:ADAR_Gen3_CCA A>C + T>G % |
| cds:Other A>G % | cds:ADARi MC1 % | nc:ADAR_Gen3_CCA A>T + T>A % |
| cds:Other A>C % | cds:ADARi MC2 % | cds:ADAR_Gen3_CGA Hits |
| cds:Other A>T % | cds:ADARi MC3 % | cds:ADAR_Gen3_CGA % |
| cds:Other T>C % | cds:ADARi A>G at MC1 % | cds:ADAR_Gen3_CGA Ti % |
| cds:Other T>G % | cds:ADARi A>G at MC2 % | cds:ADAR_Gen3_CGA MC1 % |
| cds:Other T>A % | cds:ADARi A>G at MC3 % | cds:ADAR_Gen3_CGA MC2 % |
| cds:Other C>T % | cds:ADARi T>C at MC1 % | cds:ADAR_Gen3_CGA MC3 % |
| cds:Other C>A % | cds:ADARi T>C at MC2 % | cds:ADAR_Gen3_CGA A>G at MC1 % |
| cds:Other C>G % | cds:ADARi T>C at MC3 % | cds:ADAR_Gen3_CGA A>G at MC2 % |
| cds:Other G>A % | cds:ADARi A>G % | cds:ADAR_Gen3_CGA A>G at MC3 % |
| cds:Other G>T % | cds:ADARi A>C % | cds:ADAR_Gen3_CGA T>C at MC1 % |
| cds:Other G>C % | cds:ADARi A>T % | cds:ADAR_Gen3_CGA T>C at MC2 % |
| cds:Other non-syn % | cds:ADARi T>C % | cds:ADAR_Gen3_CGA T>C at MC3 % |
| cds:Other A non-syn % | cds:ADARi T>G % | cds:ADAR_Gen3_CGA A>G % |
| cds:Other T non-syn % | cds:ADARi T>A % | cds:ADAR_Gen3_CGA A>C % |
| cds:Other C non-syn % | cds:ADARi Ti/Tv % | cds:ADAR_Gen3_CGA A>T % |

(continued)

| Metric | Metric | Metric |
|---|---|---|
| cds:Other G non-syn % | cds:ADARi A:T % | cds:ADAR_Gen3_CGA T>C % |
| cds:Other MC1 non-syn % | cds:ADARi Ti A:T % | cds:ADAR_Gen3_CGA T>G % |
| cds:Other MC2 non-syn % | cds:ADARi non-syn % | cds:ADAR_Gen3_CGA T>A % |
| cds:Other MC3 non-syn % | cds:ADARi A non-syn % | cds:ADAR_Gen3_CGA Ti/Tv % |
| g:A3B Hits | cds:ADARi T non-syn % | cds:ADAR_Gen3_CGA A:T % |
| g:A3B % | cds:ADARi MC1 non-syn % | cds:ADAR_Gen3_CGA Ti A:T % |
| g:A3B Ti % | cds:ADARi MC2 non-syn % | cds:ADAR_Gen3_CGA non-syn % |
| g:A3B C>T + G>A % | cds:ADARi MC3 non-syn % | cds:ADAR_Gen3_CGA A non-syn % |
| g:A3B C>A + G>T % | g:ADARi Hits | cds:ADAR_Gen3_CGA T non-syn % |
| g:A3B C>G + G>C % | g:ADARi % | cds:ADAR_Gen3_CGA MC1 non-syn % |
| nc:A3B Hits | g:ADARi Ti % | cds:ADAR_Gen3_CGA MC2 non-syn % |
| nc:A3B % | g:ADARi A>G + T>C % | cds:ADAR_Gen3_CGA MC3 non-syn % |
| nc:A3B Ti % | g:ADARi A>C + T>G % | g:ADAR_Gen3_CGA Hits |
| nc:A3B C>T + G>A % | g:ADARi A>T + T>A % | g:ADAR_Gen3_CGA % |
| nc:A3B C>A + G>T % | nc:ADARi Hits | g:ADAR_Gen3_CGA Ti % |
| nc:A3B C>G + G>C % | nc:ADARi % | g:ADAR_Gen3_CGA A>G + T>C % |
| cds:Gen2_ACA Hits | nc:ADARi Ti % | g:ADAR_Gen3_CGA A>C + T>G % |
| cds:Gen2_ACA % | nc:ADARi A>G + T>C % | g:ADAR_Gen3_CGA A>T + T>A % |
| cds:Gen2_ACA Ti % | nc:ADARi A>C + T>G % | nc:ADAR_Gen3_CGA Hits |
| cds:Gen2_ACA MC1 % | nc:ADARi A>T + T>A % | nc:ADAR_Gen3_CGA % |
| cds:Gen2_ACA MC2 % | cds:ADARj Hits | nc:ADAR_Gen3_CGA Ti % |
| cds:Gen2_ACA MC3 % | cds:ADARj % | nc:ADAR_Gen3_CGA A>G + T>C % |
| cds:Gen2_ACA C>T at MC1 % | cds:ADARj Ti % | nc:ADAR_Gen3_CGA A>C + T>G % |
| cds:Gen2_ACA C>T at MC2 % | cds:ADARj MC1 % | nc:ADAR_Gen3_CGA A>T + T>A % |
| cds:Gen2_ACA C>T at MC3 % | cds:ADARj MC2 % | cds:ADAR_Gen3_GAA Hits |
| cds:Gen2_ACA G>A at MC1 % | cds:ADARj MC3 % | cds:ADAR_Gen3_GAA % |
| cds:Gen2_ACA G>A at MC2 % | cds:ADARj A>G at MC1 % | cds:ADAR_Gen3_GAA Ti % |
| cds:Gen2_ACA G>A at MC3 % | cds:ADARj A>G at MC2 % | cds:ADAR_Gen3_GAA MC1 % |
| cds:Gen2_ACA C>T % | cds:ADARj A>G at MC3 % | cds:ADAR_Gen3_GAA MC2 % |
| cds:Gen2_ACA C>A % | cds:ADARj T>C at MC1 % | cds:ADAR_Gen3_GAA MC3 % |
| cds:Gen2_ACA C>G % | cds:ADARj T>C at MC2 % | cds:ADAR_Gen3_GAA A>G at MC1 % |
| cds:Gen2_ACA G>A % | cds:ADARj T>C at MC3 % | cds:ADAR_Gen3_GAA A>G at MC2 % |
| cds:Gen2_ACA G>T % | cds:ADARj A>G % | cds:ADAR_Gen3_GAA A>G at MC3 % |
| cds:Gen2_ACA G>C % | cds:ADARj A>C % | cds:ADAR_Gen3_GAA T>C at MC1 % |

(continued)

| Metric | Metric | Metric |
|---|---|---|
| cds:Gen2_ACA Ti/Tv % | cds:ADARj A>T % | cds:ADAR_Gen3_GAA T>C at MC2 % |
| cds:Gen2_ACA C:G % | cds:ADARj T>C % | cds:ADAR_Gen3_GAA T>C at MC3 % |
| cds:Gen2_ACA Ti C:G % | cds:ADARj T>G % | cds:ADAR_Gen3_GAA A>G % |
| cds:Gen2_ACA non-syn % | cds:ADARj T>A % | cds:ADAR_Gen3_GAA A>C % |
| cds:Gen2_ACA C non-syn % | cds:ADARj Ti/Tv % | cds:ADAR_Gen3_GAA A>T % |
| cds:Gen2_ACA G non-syn % | cds:ADARj A:T % | cds:ADAR_Gen3_GAA T>C % |
| cds:Gen2_ACA MC1 non-syn % | cds:ADARj Ti A:T % | cds:ADAR_Gen3_GAA T>G % |
| cds:Gen2_ACA MC2 non-syn % | cds:ADARj non-syn % | cds:ADAR_Gen3_GAA T>A % |
| cds:Gen2_ACA MC3 non-syn % | cds:ADARj A non-syn % | cds:ADAR_Gen3_GAA Ti/Tv % |
| g:Gen2_ACA Hits | cds:ADARj T non-syn % | cds:ADAR_Gen3_GAA A:T % |
| g:Gen2_ACA % | cds:ADARj MC1 non-syn % | cds:ADAR_Gen3_GAA Ti A:T % |
| g:Gen2_ACA Ti % | cds:ADARj MC2 non-syn % | cds:ADAR_Gen3_GAA non-syn % |
| g:Gen2_ACA C>T + G>A % | cds:ADARj MC3 non-syn % | cds:ADAR_Gen3_GAA A non-syn % |
| g:Gen2_ACA C>A + G>T % | g:ADARj Hits | cds:ADAR_Gen3_GAA T non-syn % |
| g:Gen2_ACA C>G + G>C % | g:ADARj % | cds:ADAR_Gen3_GAA MC1 non-syn % |
| nc:Gen2_ACA Hits | g:ADARj Ti % | cds:ADAR_Gen3_GAA MC2 non-syn % |
| nc:Gen2_ACA % | g:ADARj A>G + T>C % | cds:ADAR_Gen3_GAA MC3 non-syn % |
| nc:Gen2_ACA Ti % | g:ADARj A>C + T>G % | g:ADAR_Gen3_GAA Hits |
| nc:Gen2_ACA C>T + G>A % | g:ADARj A>T + T>A % | g:ADAR_Gen3_GAA % |
| nc:Gen2_ACA C>A + G>T % | nc:ADARj Hits | g:ADAR_Gen3_GAA Ti **%** |
| nc:Gen2_ACA C>G + G>C % | nc:ADARj % | g:ADAR_Gen3_GAA A>G + T>C % |
| cds:Gen2_TCA Hits | nc:ADARj Ti % | g:ADAR_Gen3_GAA A>C + T>G % |
| cds:Gen2_TCA % | nc:ADARj A>G + T>C % | g:ADAR_Gen3_GAA A>T + T>A % |
| cds:Gen2_TCA Ti % | nc:ADARj A>C + T>G % | nc:ADAR_Gen3_GAA Hits |
| cds:Gen2_TCA MC1 % | nc:ADARj A>T + T>A % | nc:ADAR_Gen3_GAA % |
| cds:Gen2_TCA MC2 % | cds:A3Gb Hits | nc:ADAR_Gen3_GAA Ti % |
| cds:Gen2_TCA MC3 % | cds:A3Gb % | nc:ADAR_Gen3_GAA A>G + T>C % |
| cds:Gen2_TCA C>T at MC1 % | cds:A3Gb Ti % | nc:ADAR_Gen3_GAA A>C + T>G % |
| cds:Gen2_TCA C>T at MC2 % | cds:A3Gb MC1 % | nc:ADAR_Gen3_GAA A>T + T>A % |
| cds:Gen2_TCA C>T at MC3 % | cds:A3Gb MC2 % | cds:ADAR_Gen3_GTA Hits |
| cds:Gen2_TCA G>A at MC1 % | cds:A3Gb MC3 % | cds:ADAR_Gen3_GTA % |
| cds:Gen2_TCA G>A at MC2 % | cds:A3Gb C>T at MC1 % | cds:ADAR_Gen3_GTA Ti % |
| cds:Gen2_TCA G>A at MC3 % | cds:A3Gb C>T at MC2 % | cds:ADAR_Gen3_GTA MC1 % |
| cds:Gen2_TCA C>T % | cds:A3Gb C>T at MC3 % | cds:ADAR_Gen3_GTA MC2 % |
| cds:Gen2_TCA C>A % | cds:A3Gb G>A at MC1 % | cds:ADAR_Gen3_GTA MC3 % |

(continued)

| Metric | Metric | Metric |
|---|---|---|
| cds:Gen2_TCA C>G % | cds:A3Gb G>A at MC2 % | cds:ADAR_Gen3_GTA A>G at MC1 % |
| cds:Gen2_TCA G>A % | cds:A3Gb G>A at MC3 % | cds:ADAR_Gen3_GTA A>G at MC2 % |
| cds:Gen2_TCA G>T % | cds:A3Gb C>T % | cds:ADAR_Gen3_GTA A>G at MC3 % |
| cds:Gen2_TCA G>C % | cds:A3Gb C>A % | cds:ADAR_Gen3_GTA T>C at MC1 % |
| cds:Gen2_TCA Ti/Tv % | cds:A3Gb C>G % | cds:ADAR_Gen3_GTA T>C at MC2 % |
| cds:Gen2_TCA C:G % | cds:A3Gb G>A % | cds:ADAR_Gen3_GTA T>C at MC3 % |
| cds:Gen2_TCA Ti C:G % | cds:A3Gb G>T % | cds:ADAR_Gen3_GTA A>G % |
| cds:Gen2_TCA G non-syn % | cds:A3Gb G>C % | cds:ADAR_Gen3_GTA A>C % |
| cds:Gen2_TCA MC1 non-syn % | cds:A3Gb Ti/Tv % | cds:ADAR_Gen3_GTA A>T % |
| cds:Gen2_TCA MC2 non-syn % | cds:A3Gb C:G % | cds:ADAR_Gen3_GTA T>C % |
| cds:Gen2_TCA MC3 non-syn % | cds:A3Gb Ti C:G % | cds:ADAR_Gen3_GTA T>G % |
| g:Gen2_TCA Hits | cds:A3Gb non-syn % | cds:ADAR_Gen3_GTA T>A % |
| g:Gen2_TCA % | cds:A3Gb C non-syn % | cds:ADAR_Gen3_GTA Ti/Tv % |
| g:Gen2_TCA Ti % | cds:A3Gb G non-syn % | cds:ADAR_Gen3_GTA A:T % |
| g:Gen2_TCA C>T + G>A % | cds:A3Gb MC1 non-syn % | cds:ADAR_Gen3_GTA Ti A:T % |
| g:Gen2_TCA C>A + G>T % | cds:A3Gb MC2 non-syn % | cds:ADAR_Gen3_GTA non-syn % |
| g:Gen2_TCA C>G + G>C % | cds:A3Gb MC3 non-syn % | cds:ADAR_Gen3_GTA A non-syn % |
| nc:Gen2_TCA Hits | g:A3Gb Hits | cds:ADAR_Gen3_GTA T non-syn % |
| nc:Gen2_TCA % | g:A3Gb % | cds:ADAR_Gen3_GTA MC1 non-syn % |
| nc:Gen2_TCA Ti % | g:A3Gb Ti % | cds:ADAR_Gen3_GTA MC2 non-syn % |
| nc:Gen2_TCA C>T + G>A % | g:A3Gb C>T + G>A % | cds:ADAR_Gen3_GTA MC3 non-syn % |
| nc:Gen2_TCA C>A + G>T % | g:A3Gb C>A + G>T % | g:ADAR_Gen3_GTA Hits |
| nc:Gen2_TCA C>G + G>C % | g:A3Gb C>G + G>C % | g:ADAR_Gen3_GTA % |
| cds:Gen2_CCA Hits | nc:A3Gb Hits | g:ADAR_Gen3_GTA Ti % |
| cds:Gen2_CCA % | nc:A3Gb % | g:ADAR_Gen3_GTA A>G + T>C % |
| cds:Gen2_CCA Ti % | nc:A3Gb Ti % | g:ADAR_Gen3_GTA A>C + T>G % |
| cds:Gen2_CCA MC1 % | nc:A3Gb C>T + G>A % | g:ADAR_Gen3_GTA A>T + T>A % |
| cds:Gen2_CCA MC2 % | nc:A3Gb C>A + G>T % | nc:ADAR_Gen3_GTA Hits |
| cds:Gen2_CCA MC3 % | nc:A3Gb C>G + G>C % | nc:ADAR_Gen3_GTA % |
| cds:Gen2_CCA C>T at MC1 % | cds:A3Gc Hits | nc:ADAR_Gen3_GTA Ti % |
| cds:Gen2_CCA C>T at MC2 % | cds:A3Gc % | nc:ADAR_Gen3_GTA A>G + T>C % |
| cds:Gen2_CCA C>T at MC3 % | cds:A3Gc Ti % | nc:ADAR_Gen3_GTA A>C + T>G % |
| cds:Gen2_CCA G>A at MC1 % | cds:A3Gc MC1 % | nc:ADAR_Gen3_GTA A>T + T>A % |

(continued)

| Metric | Metric | Metric |
|---|---|---|
| cds:Gen2_CCA G>A at MC2 % | cds:A3Gc MC2 % | cds:ADAR_Gen3_GCA Hits |
| cds:Gen2_CCA G>A at MC3 % | cds:A3Gc MC3 % | cds:ADAR_Gen3_GCA % |
| cds:Gen2_CCA C>T % | cds:A3Gc C>T at MC1 % | cds:ADAR_Gen3_GCA Ti % |
| cds:Gen2_CCA C>A % | cds:A3Gc C>T at MC2 % | cds:ADAR_Gen3_GCA MC1 % |
| cds:Gen2_CCA C>G % | cds:A3Gc C>T at MC3 % | cds:ADAR_Gen3_GCA MC2 % |
| cds:Gen2_CCA G>A % | cds:A3Gc G>A at MC1 % | cds:ADAR_Gen3_GCA MC3 % |
| cds:Gen2_CCA G>T % | cds:A3Gc G>A at MC2 % | cds:ADAR_Gen3_GCA A>G at MC1 % |
| cds:Gen2_CCA G>C % | cds:A3Gc G>A at MC3 % | cds:ADAR_Gen3_GCA A>G at MC2 % |
| cds:Gen2_CCA Ti/Tv % | cds:A3Gc C>T % | cds:ADAR_Gen3_GCA A>G at MC3 % |
| cds:Gen2_CCA C:G % | cds:A3Gc C>A % | cds:ADAR_Gen3_GCA T>C at MC1 % |
| cds:Gen2_CCA Ti C:G % | cds:A3Gc C>G % | cds:ADAR_Gen3_GCA T>C at MC2 % |
| cds:Gen2_CCA non-syn % | cds:A3Gc G>A % | cds:ADAR_Gen3_GCA T>C at MC3 % |
| cds:Gen2_CCA C non-syn % | cds:A3Gc G>T % | cds:ADAR_Gen3_GCA A>G % |
| cds:Gen2_CCA G non-syn % | cds:A3Gc G>C % | cds:ADAR_Gen3_GCA A>C % |
| cds:Gen2_CCA MC1 non-syn % | cds:A3Gc Ti/Tv % | cds:ADAR_Gen3_GCA A>T % |
| cds:Gen2_CCA MC2 non-syn % | cds:A3Gc C:G % | cds:ADAR_Gen3_GCA T>C % |
| cds:Gen2_CCA MC3 non-syn % | cds:A3Gc Ti C:G % | cds:ADAR_Gen3_GCA T>G % |
| g:Gen2_CCA Hits | cds:A3Gc non-syn % | cds:ADAR_Gen3_GCA T>A % |
| g:Gen2_CCA % | cds:A3Gc C non-syn % | cds:ADAR_Gen3_GCA Ti/Tv % |
| g:Gen2_CCA Ti % | cds:A3Gc G non-syn % | cds:ADAR_Gen3_GCA A:T % |
| g:Gen2_CCA C>T + G>A % | cds:A3Gc MC1 non-syn % | cds:ADAR_Gen3_GCA Ti A:T % |
| g:Gen2_CCA C>A + G>T % | cds:A3Gc MC2 non-syn % | cds:ADAR_Gen3_GCA non-syn % |
| g:Gen2_CCA C>G + G>C % | cds:A3Gc MC3 non-syn % | cds:ADAR_Gen3_GCA A non-syn % |
| nc:Gen2_CCA Hits | g:A3Gc Hits | cds:ADAR_Gen3_GCA T non-syn % |
| nc:Gen2_CCA % | g:A3Gc % | cds:ADAR_Gen3_GCA MC1 non-syn % |
| nc:Gen2_CCA Ti % | g:A3Gc Ti % | cds:ADAR_Gen3_GCA MC2 non-syn % |
| nc:Gen2_CCA C>T + G>A % | g:A3Gc C>T + G>A % | cds:ADAR_Gen3_GCA MC3 non-syn % |
| nc:Gen2_CCA C>A + G>T % | g:A3Gc C>A + G>T % | g:ADAR_Gen3_GCA Hits |
| nc:Gen2_CCA C>G + G>C % | g:A3Gc C>G + G>C % | g:ADAR_Gen3_GCA % |
| cds:Gen2_GCA Hits | nc:A3Gc Hits | g:ADAR_Gen3_GCA Ti % |
| cds:Gen2_GCA % | nc:A3Gc % | g:ADAR_Gen3_GCA A>G + T>C % |
| cds:Gen2_GCA Ti % | nc:A3Gc Ti % | g:ADAR_Gen3_GCA A>C + T>G % |
| cds:Gen2_GCA MC1 % | nc:A3Gc C>T + G>A % | g:ADAR_Gen3_GCA A>T + T>A % |

(continued)

| Metric | Metric | Metric |
|---|---|---|
| cds:Gen2_GCA MC2 % | nc:A3Gc C>A + G>T % | nc:ADAR_Gen3_GCA Hits |
| cds:Gen2_GCA MC3 % | nc:A3Gc C>G + G>C % | nc:ADAR_Gen3_GCA % |
| cds:Gen2_GCA C>T at MC1 % | cds:A3Gd Hits | nc:ADAR_Gen3_GCA Ti % |
| cds:Gen2_GCA C>T at MC2 % | cds:A3Gd % | nc:ADAR_Gen3_GCA A>G + T>C % |
| cds:Gen2_GCA C>T at MC3 % | cds:A3Gd Ti % | nc:ADAR_Gen3_GCA A>C + T>G % |
| cds:Gen2_GCA G>A at MC1 % | cds:A3Gd MC1 % | nc:ADAR_Gen3_GCA A>T + T>A % |
| cds:Gen2_GCA G>A at MC2 % | cds:A3Gd MC2 % | cds:ADAR_Gen3_GGA Hits |
| cds:Gen2_GCA G>A at MC3 % | cds:A3Gd MC3 % | cds:ADAR_Gen3_GGA % |
| cds:Gen2_GCA C>T % | cds:A3Gd C>T at MC1 % | cds:ADAR_Gen3_GGA Ti % |
| cds:Gen2_GCA C>A % | cds:A3Gd C>T at MC2 % | cds:ADAR_Gen3_GGA MC1 % |
| cds:Gen2_GCA C>G % | cds:A3Gd C>T at MC3 % | cds:ADAR_Gen3_GGA MC2 % |
| cds:Gen2_GCA G>A % | cds:A3Gd G>A at MC1 % | cds:ADAR_Gen3_GGA MC3 % |
| cds:Gen2_GCA G>T % | cds:A3Gd G>A at MC2 % | cds:ADAR_Gen3_GGA A>G at MC1 % |
| cds:Gen2_GCA G>C % | cds:A3Gd G>A at MC3 % | cds:ADAR_Gen3_GGA A>G at MC2 % |
| cds:Gen2_GCA Ti/Tv % | cds:A3Gd C>T % | cds:ADAR_Gen3_GGA A>G at MC3 % |
| cds:Gen2_GCA C:G % | cds:A3Gd C>A % | cds:ADAR_Gen3_GGA T>C at MC1 % |
| cds:Gen2_GCA Ti C:G % | cds:A3Gd C>G % | cds:ADAR_Gen3_GGA T>C at MC2 % |
| | cds:A3Gd G>A % | cds:ADAR_Gen3_GGA T>C at MC3 % |
| | cds:A3Gd G>T % | cds:ADAR_Gen3_GGA A>G % |
| cds:Gen2_GCA G non-syn % | cds:A3Gd G>C % | cds:ADAR_Gen3_GGA A>C % |
| cds:Gen2_GCA MC1 non-syn % | cds:A3Gd Ti/Tv % | cds:ADAR_Gen3_GGA A>T % |
| cds:Gen2_GCA MC2 non-syn % | cds:A3Gd C:G % | cds:ADAR_Gen3_GGA T>C % |
| cds:Gen2_GCA MC3 non-syn % | cds:A3Gd Ti C:G % | cds:ADAR_Gen3_GGA T>G % |
| g:Gen2_GCA Hits | cds:A3Gd non-syn % | cds:ADAR_Gen3_GGA T>A % |
| g:Gen2_GCA % | cds:A3Gd C non-syn % | cds:ADAR_Gen3_GGA Ti/Tv % |
| g:Gen2_GCA Ti % | cds:A3Gd G non-syn % | cds:ADAR_Gen3_GGA A:T % |
| g:Gen2_GCA C>T + G>A % | cds:A3Gd MC1 non-syn % | cds:ADAR_Gen3_GGA Ti A:T % |
| g:Gen2_GCA C>A + G>T % | cds:A3Gd MC2 non-syn % | cds:ADAR_Gen3_GGA non-syn % |
| g:Gen2_GCA C>G + G>C % | cds:A3Gd MC3 non-syn % | cds:ADAR_Gen3_GGA A non-syn % |
| nc:Gen2_GCA Hits | g:A3Gd Hits | cds:ADAR_Gen3_GGA T non-syn % |
| nc:Gen2_GCA % | g:A3Gd % | cds:ADAR_Gen3_GGA MC1 non-syn % |
| nc:Gen2_GCA Ti % | g:A3Gd Ti % | cds:ADAR_Gen3_GGA MC2 non-syn % |

(continued)

| Metric | Metric | Metric |
|---|---|---|
| nc:Gen2_GCA C>T + G>A % | g:A3Gd C>T + G>A % | cds:ADAR_Gen3_GGA MC3 non-syn % |
| nc:Gen2_GCA C>A + G>T % | g:A3Gd C>A + G>T % | g:ADAR_Gen3_GGA Hits |
| nc:Gen2_GCA C>G + G>C % | g:A3Gd C>G + G>C % | g:ADAR_Gen3_GGA % |
| cds:Gen2_ACT Hits | nc:A3Gd Hits | g:ADAR_Gen3_GGA Ti % |
| cds:Gen2_ACT % | nc:A3Gd % | g:ADAR_Gen3_GGA A>G + T>C % |
| cds:Gen2_ACT Ti % | nc:A3Gd Ti % | g:ADAR_Gen3_GGA A>C + T>G % |
| cds:Gen2_ACT MC1 % | nc:A3Gd C>T + G>A % | g:ADAR_Gen3_GGA A>T + T>A % |
| cds:Gen2_ACT MC2 % | nc:A3Gd C>A + G>T % | nc:ADAR_Gen3_GGA Hits |
| cds:Gen2_ACT MC3 % | nc:A3Gd C>G + G>C % | nc:ADAR_Gen3_GGA % |
| cds:Gen2_ACT C>T at MC1 % | cds:A3Ge Hits | nc:ADAR_Gen3_GGA Ti % |
| cds:Gen2_ACT C>T at MC2 % | cds:A3Ge % | nc:ADAR_Gen3_GGA A>G + T>C % |
| cds:Gen2_ACT C>T at MC3 % | cds:A3Ge Ti % | nc:ADAR_Gen3_GGA A>C + T>G % |
| cds:Gen2_ACT G>A at MC1 % | cds:A3Ge MC1 % | nc:ADAR_Gen3_GGA A>T + T>A % |
| cds:Gen2_ACT G>A at MC2 % | cds:A3Ge MC2 % | cds:Gen1_CAA Hits |
| cds:Gen2_ACT G>A at MC3 % | cds:A3Ge MC3 % | cds:Gen1_CAA % |
| cds:Gen2_ACT C>T % | cds:A3Ge C>T at MC1 % | cds:Gen1_CAA Ti % |
| cds:Gen2_ACT C>A % | cds:A3Ge C>T at MC2 % | cds:Gen1_CAA MC1 % |
| cds:Gen2_ACT C>G % | cds:A3Ge C>T at MC3 % | cds:Gen1_CAA MC2 % |
| cds:Gen2_ACT G>A % | cds:A3Ge G>A at MC1 % | cds:Gen1_CAA MC3 % |
| cds:Gen2_ACT G>T % | cds:A3Ge G>A at MC2 % | cds:Gen1_CAA C>T at MC1 % |
| cds:Gen2_ACT G>C % | cds:A3Ge G>A at MC3 % | cds:Gen1_CAA C>T at MC2 % |
| cds:Gen2_ACT Ti/Tv % | cds:A3Ge C>T % | cds:Gen1_CAA C>T at MC3 % |
| cds:Gen2_ACT C:G % | cds:A3Ge C>A % | cds:Gen1_CAA G>A at MC1 % |
| cds:Gen2_ACT Ti C:G % | cds:A3Ge C>G % | cds:Gen1_CAA G>A at MC2 % |
| cds:Gen2_ACT non-syn % | cds:A3Ge G>A % | cds:Gen1_CAA G>A at MC3 % |
| cds:Gen2_ACT C non-syn % | cds:A3Ge G>T % | cds:Gen1_CAA C>T % |
| cds:Gen2_ACT G non-syn % | cds:A3Ge G>C % | cds:Gen1_CAA C>A % |
| cds:Gen2_ACT MC1 non-syn % | cds:A3Ge Ti/Tv % | cds:Gen1_CAA C>G % |
| cds:Gen2_ACT MC2 non-syn % | cds:A3Ge C:G % | cds:Gen1_CAA G>A % |
| cds:Gen2_ACT MC3 non-syn % | cds:A3Ge Ti C:G % | cds:Gen1_CAA G>T % |
| g:Gen2_ACT Hits | cds:A3Ge non-syn % | cds:Gen1_CAA G>C % |
| g:Gen2_ACT % | cds:A3Ge C non-syn % | cds:Gen1_CAA Ti/Tv % |
| g:Gen2_ACT Ti % | cds:A3Ge G non-syn % | cds:Gen1_CAA C:G % |
| g:Gen2_ACT C>T + G>A % | cds:A3Ge MC1 non-syn % | cds:Gen1_CAA Ti C:G % |
| g:Gen2_ACT C>A + G>T % | cds:A3Ge MC2 non-syn % | cds:Gen1_CAA non-syn % |
| g:Gen2_ACT C>G + G>C % | cds:A3Ge MC3 non-syn % | cds:Gen1_CAA C non-syn % |
| nc:Gen2_ACT Hits | g:A3Ge Hits | cds:Gen1_CAA G non-syn % |

(continued)

| Metric | Metric | Metric |
|---|---|---|
| nc:Gen2_ACT % | g:A3Ge % | cds:Gen1_CAA MC1 non-syn % |
| nc:Gen2_ACT Ti % | g:A3Ge Ti % | cds:Gen1_CAA MC2 non-syn % |
| nc:Gen2_ACT C>T + G>A % | g:A3Ge C>T + G>A % | cds:Gen1_CAA MC3 non-syn % |
| nc:Gen2_ACT C>A + G>T % | g:A3Ge C>A + G>T % | g:Gen1_CAA Hits |
| nc:Gen2_ACT C>G + G>C % | g:A3Ge C>G + G>C % | g:Gen1_CAA % |
| cds:Gen2_TCT Hits | nc:A3Ge Hits | g:Gen1_CAA Ti % |
| cds:Gen2_TCT % | nc:A3Ge % | g:Gen1_CAA C>T + G>A % |
| cds:Gen2_TCT Ti % | nc:A3Ge Ti % | g:Gen1_CAA C>A + G>T % |
| cds:Gen2_TCT MC1 % | nc:A3Ge C>T + G>A % | g:Gen1_CAA C>G + G>C % |
| cds:Gen2_TCT MC2 % | nc:A3Ge C>A + G>T % | nc:Gen1_CAA Hits |
| cds:Gen2_TCT MC3 % | nc:A3Ge C>G + G>C % | nc:Gen1_CAA % |
| cds:Gen2_TCT C>T at MC1 % | cds:A3Gf Hits | nc:Gen1_CAA Ti % |
| cds:Gen2_TCT C>T at MC2 % | cds:A3Gf % | nc:Gen1_CAA C>T + G>A % |
| cds:Gen2_TCT C>T at MC3 % | cds:A3Gf Ti % | nc:Gen1_CAA C>A + G>T % |
| cds:Gen2_TCT G>A at MC1 % | cds:A3Gf MC1 % | nc:Gen1_CAA C>G + G>C % |
| cds:Gen2_TCT G>A at MC2 % | cds:A3Gf MC2 % | cds:Gen1_CTA Hits |
| cds:Gen2_TCT G>A at MC3 % | cds:A3Gf MC3 % | cds:Gen1_CTA % |
| cds:Gen2_TCT C>T % | cds:A3Gf C>T at MC1 % | cds:Gen1_CTA Ti % |
| cds:Gen2_TCT C>A % | cds:A3Gf C>T at MC2 % | cds:Gen1_CTA MC1 % |
| cds:Gen2_TCT C>G % | cds:A3Gf C>T at MC3 % | cds:Gen1_CTA MC2 % |
| cds:Gen2_TCT G>A % | cds:A3Gf G>A at MC1 % | cds:Gen1_CTA MC3 % |
| cds:Gen2_TCT G>T % | cds:A3Gf G>A at MC2 % | cds:Gen1_CTA C>T at MC1 % |
| cds:Gen2_TCT G>C % | cds:A3Gf G>A at MC3 % | cds:Gen1_CTA C>T at MC2 % |
| cds:Gen2_TCT Ti/Tv % | cds:A3Gf C>T % | cds:Gen1_CTA C>T at MC3 % |
| cds:Gen2_TCT C:G % | cds:A3Gf C>A % | cds:Gen1_CTA G>A at MC1 % |
| cds:Gen2_TCT Ti C:G % | cds:A3Gf C>G % | cds:Gen1_CTA G>A at MC2 % |
| cds:Gen2_TCT non-syn % | cds:A3Gf G>A % | cds:Gen1_CTA G>A at MC3 % |
| cds:Gen2_TCT C non-syn % | cds:A3Gf G>T % | cds:Gen1_CTA C>T % |
| cds:Gen2_TCT G non-syn % | cds:A3Gf G>C % | cds:Gen1_CTA C>A % |
| cds:Gen2_TCT MC1 non-syn % | cds:A3Gf Ti/Tv % | cds:Gen1_CTA C>G % |
| cds:Gen2_TCT MC2 non-syn % | cds:A3Gf C:G % | cds:Gen1_CTA G>A % |
| cds:Gen2_TCT MC3 non-syn % | cds:A3Gf Ti C:G % | cds:Gen1_CTA G>T % |
| g:Gen2_TCT Hits | cds:A3Gf G non-syn % | cds:Gen1_CTA G>C % |
| g:Gen2_TCT % | cds:A3Gf MC1 non-syn % | cds:Gen1_CTA Ti/Tv % |
| g:Gen2_TCT Ti % | cds:A3Gf MC2 non-syn % | cds:Gen1_CTA C:G % |
| g:Gen2_TCT C>T + G>A % | cds:A3Gf MC3 non-syn % | cds:Gen1_CTA Ti C:G % |
| g:Gen2_TCT C>A + G>T % | g:A3Gf Hits | cds:Gen1_CTA non-syn % |
| g:Gen2_TCT C>G + G>C % | g:A3Gf % | cds:Gen1_CTA C non-syn % |
| nc:Gen2_TCT Hits | g:A3Gf Ti % | cds:Gen1_CTA G non-syn % |

(continued)

| Metric | Metric | Metric |
|---|---|---|
| nc:Gen2_TCT % | g:A3Gf C>T + G>A % | cds:Gen1_CTA MC1 non-syn % |
| nc:Gen2_TCT Ti % | g:A3Gf C>A + G>T % | cds:Gen1_CTA MC2 non-syn % |
| nc:Gen2_TCT C>T + G>A % | g:A3Gf C>G + G>C % | cds:Gen1_CTA MC3 non-syn % |
| nc:Gen2_TCT C>A + G>T % | nc:A3Gf Hits | g:Gen1_CTA Hits |
| nc:Gen2_TCT C>G + G>C % | nc:A3Gf % | g:Gen1_CTA % |
| cds:Gen2_CCT Hits | nc:A3Gf Ti % | g:Gen1_CTA Ti % |
| cds:Gen2_CCT % | nc:A3Gf C>T + G>A % | g:Gen1_CTA C>T + G>A % |
| cds:Gen2_CCT Ti % | nc:A3Gf C>A + G>T % | g:Gen1_CTA C>A + G>T % |
| cds:Gen2_CCT MC1 % | nc:A3Gf C>G + G>C % | g:Gen1_CTA C>G + G>C % |
| cds:Gen2_CCT MC2 % | cds:A3Gg Hits | nc:Gen1_CTA Hits |
| cds:Gen2_CCT MC3 % | cds:A3Gg % | nc:Gen1_CTA % |
| cds:Gen2_CCT C>T at MC1 % | cds:A3Gg Ti % | nc:Gen1_CTA Ti % |
| cds:Gen2_CCT C>T at MC2 % | cds:A3Gg MC1 % | nc:Gen1_CTA C>T + G>A % |
| cds:Gen2_CCT C>T at MC3 % | cds:A3Gg MC2 % | nc:Gen1_CTA C>A + G>T % |
| cds:Gen2_CCT G>A at MC1 % | cds:A3Gg MC3 % | nc:Gen1_CTA C>G + G>C % |
| cds:Gen2_CCT G>A at MC2 % | cds:A3Gg C>T at MC1 % | cds:Genl_CCA Hits |
| cds:Gen2_CCT G>A at MC3 % | cds:A3Gg C>T at MC2 % | cds:Gen1_CCA % |
| cds:Gen2_CCT C>T % | cds:A3Gg C>T at MC3 % | cds:Genl_CCA Ti % |
| cds:Gen2_CCT C>A % | cds:A3Gg G>A at MC1 % | cds:Gen1_CCA MC1 % |
| cds:Gen2_CCT C>G % | cds:A3Gg G>A at MC2 % | cds:Gen1_CCA MC2 % |
| cds:Gen2_CCT G>A % | cds:A3Gg G>A at MC3 % | cds:Gen1_CCA MC3 % |
| cds:Gen2_CCT G>T % | cds:A3Gg C>T % | cds:Gen1_CCA C>T at MC1 % |
| cds:Gen2_CCT G>C % | cds:A3Gg C>A % | cds:Genl_CCA C>T at MC2 % |
| cds:Gen2_CCT Ti/Tv % | cds:A3Gg C>G % | cds:Genl_CCA C>T at MC3 % |
| cds:Gen2_CCT C:G % | cds:A3Gg G>A % | cds:Genl_CCA G>A at MC1 % |
| cds:Gen2_CCT Ti C:G % | cds:A3Gg G>T % | cds:Genl_CCA G>A at MC2 % |
| cds:Gen2_CCT non-syn % | cds:A3Gg G>C % | cds:Genl_CCA G>A at MC3 % |
| cds:Gen2_CCT C non-syn % | cds:A3Gg Ti/Tv % | cds:Genl_CCA C>T % |
| cds:Gen2_CCT G non-syn % | cds:A3Gg C:G % | cds:Gen1_CCA C>A % |
| cds:Gen2_CCT MC1 non-syn % | cds:A3Gg Ti C:G % | cds:Gen1_CCA C>G % |
| cds:Gen2_CCT MC2 non-syn % | cds:A3Gg non-syn % | cds:Gen1_CCA G>A % |
| cds:Gen2_CCT MC3 non-syn % | cds:A3Gg C non-syn % | cds:Genl_CCA G>T % |
| g:Gen2_CCT Hits | cds:A3Gg G non-syn % | cds:Gen1_CCA G>C % |
| g:Gen2_CCT % | cds:A3Gg MC1 non-syn % | cds:Genl_CCA Ti/Tv % |
| g:Gen2_CCT Ti % | cds:A3Gg MC2 non-syn % | cds:Gen1_CCA C:G % |
| g:Gen2_CCT C>T + G>A % | cds:A3Gg MC3 non-syn % | cds:Genl_CCA Ti C:G % |
| g:Gen2_CCT C>A + G>T % | g:A3Gg Hits | cds:Genl_CCA non-syn % |
| g:Gen2_CCT C>G + G>C % | g:A3Gg % | cds:Genl_CCA C non-syn % |
| nc:Gen2_CCT Hits | g:A3Gg Ti % | cds:Genl_CCA G non-syn % |

(continued)

| Metric | Metric | Metric |
|---|---|---|
| nc:Gen2_CCT % | g:A3Gg C>T + G>A % | cds:Gen1_CCA MC1 non-syn % |
| nc:Gen2_CCT Ti % | g:A3Gg C>A + G>T % | cds:Genl_CCA MC2 non-syn % |
| nc:Gen2_CCT C>T + G>A % | g:A3Gg C>G + G>C % | cds:Genl_CCA MC3 non-syn % |
| nc:Gen2_CCT C>A + G>T % | nc:A3Gg Hits | g:Gen1_CCA Hits |
| nc:Gen2_CCT C>G + G>C % | nc:A3Gg % | g:Gen1_CCA % |
| cds:Gen2_GCT Hits | nc:A3Gg Ti % | g:Gen1_CCA Ti % |
| cds:Gen2_GCT % | nc:A3Gg C>T + G>A % | g:Gen1_CCA C>T + G>A % |
| cds:Gen2_GCT Ti % | nc:A3Gg C>A + G>T % | g:Gen1_CCA C>A + G>T % |
| cds:Gen2_GCT MC1 % | nc:A3Gg C>G + G>C % | g:Gen1_CCA C>G + G>C % |
| cds:Gen2_GCT MC2 % | cds:A3Gh Hits | nc:Gen1_CCA Hits |
| cds:Gen2_GCT MC3 % | cds:A3Gh % | nc:Gen1_CCA % |
| cds:Gen2_GCT C>T at MC1 % | cds:A3Gh Ti % | nc:Gen1_CCA Ti % |
| cds:Gen2_GCT C>T at MC2 % | cds:A3Gh MC1 % | nc:Gen1_CCA C>T + G>A % |
| cds:Gen2_GCT C>T at MC3 % | cds:A3Gh MC2 % | nc:Gen1_CCA C>A + G>T % |
| cds:Gen2_GCT G>A at MC1 % | cds:A3Gh MC3 % | nc:Gen1_CCA C>G + G>C % |
| cds:Gen2_GCT G>A at MC2 % | cds:A3Gh C>T at MC1 % | cds:Gen1_CGA Hits |
| cds:Gen2_GCT G>A at MC3 % | cds:A3Gh C>T at MC2 % | cds:Gen1_CGA % |
| cds:Gen2_GCT C>T % | cds:A3Gh C>T at MC3 % | cds:Gen1_CGA Ti % |
| cds:Gen2_GCT C>A % | cds:A3Gh G>A at MC1 % | cds:Gen1_CGA MC1 % |
| cds:Gen2_GCT C>G % | cds:A3Gh G>A at MC2 % | cds:Gen1_CGA MC2 % |
| cds:Gen2_GCT G>A % | cds:A3Gh G>A at MC3 % | cds:Gen1_CGA MC3 % |
| cds:Gen2_GCT G>T % | cds:A3Gh C>T % | cds:Gen1_CGA C>T at MC1 % |
| cds:Gen2_GCT G>C % | cds:A3Gh C>A % | cds:Gen1_CGA C>T at MC2 % |
| cds:Gen2_GCT Ti/Tv % | cds:A3Gh C>G % | cds:Gen1_CGA C>T at MC3 % |
| cds:Gen2_GCT C:G % | cds:A3Gh G>A % | cds:Gen1_CGA G>A at MC1 % |
| cds:Gen2_GCT Ti C:G % | cds:A3Gh G>T % | cds:Gen1_CGA G>A at MC2 % |
| cds:Gen2_GCT non-syn % | cds:A3Gh G>C % | cds:Gen1_CGA G>A at MC3 % |
| cds:Gen2_GCT C non-syn % | cds:A3Gh Ti/Tv % | cds:Gen1_CGA C>T % |
| cds:Gen2_GCT G non-syn % | cds:A3Gh C:G % | cds:Gen1_CGA C>A % |
| cds:Gen2_GCT MC1 non-syn % | cds:A3Gh Ti C:G % | cds:Gen1_CGA C>G % |
| cds:Gen2_GCT MC2 non-syn % | cds:A3Gh non-syn % | cds:Gen1_CGA G>A % |
| cds:Gen2_GCT MC3 non-syn % | cds:A3Gh C non-syn % | cds:Gen1_CGA G>T % |
| g:Gen2_GCT Hits | cds:A3Gh G non-syn % | cds:Gen1_CGA G>C % |
| g:Gen2_GCT % | cds:A3Gh MC1 non-syn % | cds:Gen1_CGA Ti/Tv % |
| g:Gen2_GCT Ti % | cds:A3Gh MC2 non-syn % | cds:Gen1_CGA C:G % |
| g:Gen2_GCT C>T + G>A % | cds:A3Gh MC3 non-syn % | cds:Gen1_CGA Ti C:G % |
| g:Gen2_GCT C>A + G>T % | g:A3Gh Hits | cds:Gen1_CGA non-syn % |
| g:Gen2_GCT C>G + G>C % | g:A3Gh % | cds:Gen1_CGA C non-syn % |
| nc:Gen2_GCT Hits | g:A3Gh Ti % | cds:Gen1_CGA G non-syn % |

(continued)

| Metric | Metric | Metric |
|---|---|---|
| nc:Gen2_GCT % | g:A3Gh C>T + G>A % | cds:Gen1_CGA MC1 non-syn % |
| nc:Gen2_GCT Ti % | g:A3Gh C>A + G>T % | cds:Gen1_CGA MC2 non-syn % |
| nc:Gen2_GCT C>T + G>A % | g:A3Gh C>G + G>C % | cds:Gen1_CGA MC3 non-syn % |
| nc:Gen2_GCT C>A + G>T % | nc:A3Gh Hits | g:Gen1_CGA Hits |
| nc:Gen2_GCT C>G + G>C % | nc:A3Gh % | g:Gen1_CGA % |
| cds:Gen2_ACC Hits | nc:A3Gh Ti % | g:Gen1_CGA Ti % |
| cds:Gen2_ACC % | nc:A3Gh C>T + G>A % | g:Gen1_CGA C>T + G>A % |
| cds:Gen2_ACC Ti % | nc:A3Gh C>A + G>T % | g:Gen1_CGA C>A + G>T % |
| cds:Gen2_ACC MC1 % | nc:A3Gh C>G + G>C % | g:Gen1_CGA C>G + G>C % |
| cds:Gen2_ACC MC2 % | cds:A3Gi Hits | nc:Gen1_CGA Hits |
| cds:Gen2_ACC MC3 % | cds:A3Gi % | nc:Gen1_CGA % |
| cds:Gen2_ACC C>T at MC1 % | cds:A3Gi Ti % | nc:Gen1_CGA Ti % |
| cds:Gen2_ACC C>T at MC2 % | cds:A3Gi MC1 % | nc:Gen1_CGA C>T + G>A % |
| cds:Gen2_ACC C>T at MC3 % | cds:A3Gi MC2 % | nc:Gen1_CGA C>A + G>T % |
| cds:Gen2_ACC G>A at MC1 % | cds:A3Gi MC3 % | nc:Gen1_CGA C>G + G>C % |
| cds:Gen2_ACC G>A at MC2 % | cds:A3Gi C>T at MC1 % | cds:Gen1_CAT Hits |
| cds:Gen2_ACC G>A at MC3 % | cds:A3Gi C>T at MC2 % | cds:Gen1_CAT % |
| cds:Gen2_ACC C>T % | cds:A3Gi C>T at MC3 % | cds:Gen1_CAT Ti % |
| cds:Gen2_ACC C>A % | cds:A3Gi G>A at MC1 % | cds:Gen1_CAT MC1 % |
| cds:Gen2_ACC C>G % | cds:A3Gi G>A at MC2 % | cds:Gen1_CAT MC2 % |
| cds:Gen2_ACC G>A % | cds:A3Gi G>A at MC3 % | cds:Gen1_CAT MC3 % |
| cds:Gen2_ACC G>T % | cds:A3Gi C>T % | cds:Gen1_CAT C>T at MC1 % |
| cds:Gen2_ACC G>C % | cds:A3Gi C>A % | cds:Gen1_CAT C>T at MC2 % |
| cds:Gen2_ACC Ti/Tv % | cds:A3Gi C>G % | cds:Gen1_CAT C>T at MC3 % |
| cds:Gen2_ACC C:G % | cds:A3Gi G>A % | cds:Gen1_CAT G>A at MC1 % |
| cds:Gen2_ACC Ti C:G % | cds:A3Gi G>T % | cds:Gen1_CAT G>A at MC2 % |
| cds:Gen2_ACC non-syn % | cds:A3Gi G>C % | cds:Gen1_CAT G>A at MC3 % |
| cds:Gen2_ACC C non-syn % | cds:A3Gi Ti/Tv % | cds:Gen1_CAT C>T % |
| cds:Gen2_ACC G non-syn % | cds:A3Gi C:G % | cds:Gen1_CAT C>A % |
| cds:Gen2_ACC MC1 non-syn % | cds:A3Gi Ti C:G % | cds:Gen1_CAT C>G % |
| cds:Gen2_ACC MC2 non-syn % | cds:A3Gi non-syn % | cds:Gen1_CAT G>A % |
| cds:Gen2_ACC MC3 non-syn % | cds:A3Gi C non-syn % | cds:Gen1_CAT G>T % |
| g:Gen2_ACC Hits | cds:A3Gi G non-syn % | cds:Gen1_CAT G>C % |
| g:Gen2_ACC % | cds:A3Gi MC1 non-syn % | cds:Gen1_CAT Ti/Tv % |
| g:Gen2_ACC Ti % | cds:A3Gi MC2 non-syn % | cds:Gen1_CAT C:G % |
| g:Gen2_ACC C>T + G>A % | cds:A3Gi MC3 non-syn % | cds:Gen1_CAT Ti C:G % |
| g:Gen2_ACC C>A + G>T % | g:A3Gi Hits | cds:Gen1_CAT non-syn % |
| g:Gen2_ACC C>G + G>C % | g:A3Gi % | cds:Gen1_CAT C non-syn % |
| nc:Gen2_ACC Hits | g:A3Gi Ti % | cds:Gen1_CAT G non-syn % |

(continued)

| Metric | Metric | Metric |
| --- | --- | --- |
| nc:Gen2_ACC % | g:A3Gi C>T + G>A % | cds:Gen1_CAT MC1 non-syn % |
| nc:Gen2_ACC Ti % | g:A3Gi C>A + G>T % | cds:Gen1_CAT MC2 non-syn % |
| nc:Gen2_ACC C>T + G>A % | g:A3Gi C>G + G>C % | cds:Gen1_CAT MC3 non-syn % |
| nc:Gen2_ACC C>A + G>T % | nc:A3Gi Hits | g:Gen1_CAT Hits |
| nc:Gen2_ACC C>G + G>C % | nc:A3Gi % | g:Gen1_CAT % |
| cds:Gen2_TCC Hits | nc:A3Gi Ti % | g:Gen1_CAT Ti % |
| cds:Gen2_TCC % | nc:A3Gi C>T + G>A % | g:Gen1_CAT C>T + G>A % |
| cds:Gen2_TCC Ti % | nc:A3Gi C>A + G>T % | g:Gen1_CAT C>A + G>T % |
| cds:Gen2_TCC MC1 % | nc:A3Gi C>G + G>C % | g:Gen1_CAT C>G + G>C % |
| cds:Gen2_TCC MC2 % | cds:A3Bb Hits | nc:Gen1_CAT Hits |
| cds:Gen2_TCC MC3 % | cds:A3Bb % | nc:Gen1_CAT % |
| cds:Gen2_TCC C>T at MC1 % | cds:A3Bb Ti % | nc:Gen1_CAT Ti % |
| cds:Gen2_TCC C>T at MC2 % | cds:A3Bb MC1 % | nc:Gen1_CAT C>T + G>A % |
| cds:Gen2_TCC C>T at MC3 % | cds:A3Bb MC2 % | nc:Gen1_CAT C>A + G>T % |
| cds:Gen2_TCC G>A at MC1 % | cds:A3Bb MC3 % | nc:Gen1_CAT C>G + G>C % |
| cds:Gen2_TCC G>A at MC2 % | cds:A3Bb C>T at MC1 % | cds:Gen1_CTT Hits |
| cds:Gen2_TCC G>A at MC3 % | cds:A3Bb C>T at MC2 % | cds:Gen1_CTT % |
| cds:Gen2_TCC C>T % | cds:A3Bb C>T at MC3 % | cds:Gen1_CTT Ti % |
| cds:Gen2_TCC C>A % | cds:A3Bb G>A at MC1 % | cds:Gen1_CTT MC1 % |
| cds:Gen2_TCC C>G % | cds:A3Bb G>A at MC2 % | cds:Gen1_CTT MC2 % |
| cds:Gen2_TCC G>A % | cds:A3Bb G>A at MC3 % | cds:Gen1_CTT MC3 % |
| cds:Gen2_TCC G>T % | cds:A3Bb C>T % | cds:Gen1_CTT C>T at MC1 % |
| cds:Gen2_TCC G>C % | cds:A3Bb C>A % | cds:Gen1_CTT C>T at MC2 % |
| cds:Gen2_TCC Ti/Tv % | cds:A3Bb C>G % | cds:Gen1_CTT C>T at MC3 % |
| cds:Gen2_TCC C:G % | cds:A3Bb G>A % | cds:Gen1_CTT G>A at MC1 % |
| cds:Gen2_TCC Ti C:G % | cds:A3Bb G>T % | cds:Gen1_CTT G>A at MC2 % |
| cds:Gen2_TCC non-syn % | cds:A3Bb G>C % | cds:Gen1_CTT G>A at MC3 % |
| cds:Gen2_TCC C non-syn % | cds:A3Bb Ti/Tv % | cds:Gen1_CTT C>T % |
| cds:Gen2_TCC G non-syn % | cds:A3Bb C:G % | cds:Gen1_CTT C>A % |
| cds:Gen2_TCC MC1 non-syn % | cds:A3Bb Ti C:G % | cds:Gen1_CTT C>G % |
| cds:Gen2_TCC MC2 non-syn % | cds:A3Bb non-syn % | cds:Gen1_CTT G>A % |
| cds:Gen2_TCC MC3 non-syn % | cds:A3Bb C non-syn % | cds:Gen1_CTT G>T % |
| g:Gen2_TCC Hits | cds:A3Bb G non-syn % | cds:Gen1_CTT G>C % |
| g:Gen2_TCC % | cds:A3Bb MC1 non-syn % | cds:Gen1_CTT Ti/Tv % |
| g:Gen2_TCC Ti % | cds:A3Bb MC2 non-syn % | cds:Gen1_CTT C:G % |
| g:Gen2_TCC C>T + G>A % | cds:A3Bb MC3 non-syn % | cds:Gen1_CTT Ti C:G % |
| g:Gen2_TCC C>A + G>T % | g:A3Bb Hits | cds:Gen1_CTT non-syn % |
| g:Gen2_TCC C>G + G>C % | g:A3Bb % | cds:Gen1_CTT C non-syn % |
| nc:Gen2_TCC Hits | g:A3Bb Ti % | cds:Gen1_CTT G non-syn % |

(continued)

| Metric | Metric | Metric |
|---|---|---|
| nc:Gen2_TCC % | g:A3Bb C>T + G>A % | cds:Gen1_CTT MC1 non-syn % |
| nc:Gen2_TCC Ti % | g:A3Bb C>A + G>T % | cds:Gen1_CTT MC2 non-syn % |
| nc:Gen2_TCC C>T + G>A % | g:A3Bb C>G + G>C % | cds:Gen1_CTT MC3 non-syn % |
| nc:Gen2_TCC C>A + G>T % | nc:A3Bb Hits | g:Gen1_CTT Hits |
| nc:Gen2_TCC C>G + G>C % | nc:A3Bb % | g:Gen1_CTT % |
| cds:Gen2_CCC Hits | nc:A3Bb Ti % | g:Gen1_CTT Ti % |
| cds:Gen2_CCC % | nc:A3Bb C>T + G>A % | g:Gen1_CTT C>T + G>A % |
| cds:Gen2_CCC Ti % | nc:A3Bb C>A + G>T % | g:Gen1_CTT C>A + G>T % |
| cds:Gen2_CCC MC1 % | nc:A3Bb C>G + G>C % | g:Gen1_CTT C>G + G>C % |
| cds:Gen2_CCC MC2 % | cds:A3Bc Hits | nc:Gen1_CTT Hits |
| cds:Gen2_CCC MC3 % | cds:A3Bc % | nc:Gen1_CTT % |
| cds:Gen2_CCC C>T at MC1 % | cds:A3Bc Ti % | nc:Gen1_CTT Ti % |
| cds:Gen2_CCC C>T at MC2 % | cds:A3Bc MC1 % | nc:Gen1_CTT C>T + G>A % |
| cds:Gen2_CCC C>T at MC3 % | cds:A3Bc MC2 % | nc:Gen1_CTT C>A + G>T % |
| cds:Gen2_CCC G>A at MC1 % | cds:A3Bc MC3 % | nc:Gen1_CTT C>G + G>C % |
| cds:Gen2_CCC G>A at MC2 % | cds:A3Bc C>T at MC1 % | cds:Gen1_CCT Hits |
| cds:Gen2_CCC G>A at MC3 % | cds:A3Bc C>T at MC2 % | cds:Gen1_CCT % |
| cds:Gen2_CCC C>T % | cds:A3Bc C>T at MC3 % | cds:Gen1_CCT Ti % |
| cds:Gen2_CCC C>A % | cds:A3Bc G>A at MC1 % | cds:Gen1_CCT MC1 % |
| cds:Gen2_CCC C>G % | cds:A3Bc G>A at MC2 % | cds:Gen1_CCT MC2 % |
| cds:Gen2_CCC G>A % | cds:A3Bc G>A at MC3 % | cds:Gen1_CCT MC3 % |
| cds:Gen2_CCC G>T % | cds:A3Bc C>T % | cds:Gen1_CCT C>T at MC1 % |
| cds:Gen2_CCC G>C % | cds:A3Bc C>A % | cds:Gen1_CCT C>T at MC2 % |
| cds:Gen2_CCC Ti/Tv % | cds:A3Bc C>G % | cds:Gen1_CCT C>T at MC3 % |
| cds:Gen2_CCC C:G % | cds:A3Bc G>A % | cds:Gen1_CCT G>A at MC1 % |
| cds:Gen2_CCC Ti C:G % | cds:A3Bc G>T % | cds:Gen1_CCT G>A at MC2 % |
| cds:Gen2_CCC non-syn % | cds:A3Bc G>C % | cds:Gen1_CCT G>A at MC3 % |
| cds:Gen2_CCC C non-syn % | cds:A3Bc Ti/Tv % | cds:Gen1_CCT C>T % |
| cds:Gen2_CCC G non-syn % | cds:A3Bc C:G % | cds:Gen1_CCT C>A % |
| cds:Gen2_CCC MC1 non-syn % | cds:A3Bc Ti C:G % | cds:Gen1_CCT C>G % |
| cds:Gen2_CCC MC2 non-syn % | cds:A3Bc non-syn % | cds:Gen1_CCT G>A % |
| cds:Gen2_CCC MC3 non-syn % | cds:A3Bc C non-syn % | cds:Gen1_CCT G>T % |
| g:Gen2_CCC Hits | cds:A3Bc G non-syn % | cds:Gen1_CCT G>C % |
| g:Gen2_CCC % | cds:A3Bc MC1 non-syn % | cds:Gen1_CCT Ti/Tv % |
| g:Gen2_CCC Ti % | cds:A3Bc MC2 non-syn % | cds:Gen1_CCT C:G % |
| g:Gen2_CCC C>T + G>A % | cds:A3Bc MC3 non-syn % | cds:Gen1_CCT Ti C:G % |
| g:Gen2_CCC C>A + G>T % | g:A3Bc Hits | cds:Gen1_CCT non-syn % |
| g:Gen2_CCC C>G + G>C % | g:A3Bc % | cds:Gen1_CCT C non-syn % |
| nc:Gen2_CCC Hits | g:A3Bc Ti % | cds:Gen1_CCT G non-syn % |

(continued)

| Metric | Metric | Metric |
|---|---|---|
| nc:Gen2_CCC % | g:A3Bc C>T + G>A % | cds:Gen1_CCT MC1 non-syn % |
| nc:Gen2_CCC Ti % | g:A3Bc C>A + G>T % | cds:Gen1_CCT MC2 non-syn % |
| nc:Gen2_CCC C>T + G>A % | g:A3Bc C>G + G>C % | cds:Gen1_CCT MC3 non-syn % |
| nc:Gen2_CCC C>A + G>T % | nc:A3Bc Hits | g:Gen1_CCT Hits |
| nc:Gen2_CCC C>G + G>C % | nc:A3Bc % | g:Gen1_CCT % |
| cds:Gen2_GCC Hits | nc:A3Bc Ti % | g:Gen1_CCT Ti % |
| cds:Gen2_GCC % | nc:A3Bc C>T + G>A % | g:Gen1_CCT C>T + G>A % |
| cds:Gen2_GCC Ti % | nc:A3Bc C>A + G>T % | g:Gen1_CCT C>A + G>T % |
| cds:Gen2_GCC MC1 % | nc:A3Bc C>G + G>C % | g:Gen1_CCT C>G + G>C % |
| cds:Gen2_GCC MC2 % | cds:A3Bd Hits | nc:Gen1_CCT Hits |
| cds:Gen2_GCC MC3 % | cds:A3Bd % | nc:Gen1_CCT % |
| cds:Gen2_GCC C>T at MC1 % | cds:A3Bd Ti % | nc:Gen1_CCT Ti % |
| cds:Gen2_GCC C>T at MC2 % | cds:A3Bd MC1 % | nc:Gen1_CCT C>T + G>A % |
| cds:Gen2_GCC C>T at MC3 % | cds:A3Bd MC2 % | nc:Gen1_CCT C>A + G>T % |
| cds:Gen2_GCC G>A at MC1 % | cds:A3Bd MC3 % | nc:Gen1_CCT C>G + G>C % |
| cds:Gen2_GCC G>A at MC2 % | cds:A3Bd C>T at MC1 % | cds:Gen1_CGT Hits |
| cds:Gen2_GCC G>A at MC3 % | cds:A3Bd C>T at MC2 % | cds:Gen1_CGT % |
| cds:Gen2_GCC C>T % | cds:A3Bd C>T at MC3 % | cds:Gen1_CGT Ti % |
| cds:Gen2_GCC C>A % | cds:A3Bd G>A at MC1 % | cds:Gen1_CGT MC1 % |
| cds:Gen2_GCC C>G % | cds:A3Bd G>A at MC2 % | cds:Gen1_CGT MC2 % |
| cds:Gen2_GCC G>A % | cds:A3Bd G>A at MC3 % | cds:Gen1_CGT MC3 % |
| cds:Gen2_GCC G>T % | cds:A3Bd C>T % | cds:Gen1_CGT C>T at MC1 % |
| cds:Gen2_GCC G>C % | cds:A3Bd C>A % | cds:Gen1_CGT C>T at MC2 % |
| cds:Gen2_GCC Ti/Tv % | cds:A3Bd C>G % | cds:Gen1_CGT C>T at MC3 % |
| cds:Gen2_GCC C:G % | cds:A3Bd G>A % | cds:Gen1_CGT G>A at MC1 % |
| cds:Gen2_GCC Ti C:G % | cds:A3Bd G>T % | cds:Gen1_CGT G>A at MC2 % |
| cds:Gen2_GCC non-syn % | cds:A3Bd G>C % | cds:Gen1_CGT G>A at MC3 % |
| cds:Gen2_GCC C non-syn % | cds:A3Bd Ti/Tv % | cds:Gen1_CGT C>T % |
| cds:Gen2_GCC G non-syn % | cds:A3Bd C:G % | cds:Gen1_CGT C>A % |
| cds:Gen2_GCC MC1 non-syn % | cds:A3Bd Ti C:G % | cds:Gen1_CGT C>G % |
| cds:Gen2_GCC MC2 non-syn % | cds:A3Bd non-syn % | cds:Gen1_CGT G>A % |
| cds:Gen2_GCC MC3 non-syn % | cds:A3Bd C non-syn % | cds:Gen1_CGT G>T % |
| g:Gen2_GCC Hits | cds:A3Bd G non-syn % | cds:Gen1_CGT G>C % |
| g:Gen2_GCC % | cds:A3Bd MC1 non-syn % | cds:Gen1_CGT Ti/Tv % |
| g:Gen2_GCC Ti % | cds:A3Bd MC2 non-syn % | cds:Gen1_CGT C:G % |
| g:Gen2_GCC C>T + G>A % | cds:A3Bd MC3 non-syn % | cds:Gen1_CGT Ti C:G % |
| g:Gen2_GCC C>A + G>T % | g:A3Bd Hits | cds:Gen1_CGT non-syn % |
| g:Gen2_GCC C>G + G>C % | g:A3Bd % | cds:Gen1_CGT C non-syn % |
| nc:Gen2_GCC Hits | g:A3Bd Ti % | cds:Gen1_CGT G non-syn % |

(continued)

| Metric | Metric | Metric |
|---|---|---|
| nc:Gen2_GCC % | g:A3Bd C>T + G>A % | cds:Gen1_CGT MC1 non-syn % |
| nc:Gen2_GCC Ti % | g:A3Bd C>A + G>T % | cds:Gen1_CGT MC2 non-syn % |
| nc:Gen2_GCC C>T + G>A % | g:A3Bd C>G + G>C % | cds:Gen1_CGT MC3 non-syn % |
| nc:Gen2_GCC C>A + G>T % | nc:A3Bd Hits | g:Gen1_CGT Hits |
| nc:Gen2_GCC C>G + G>C % | nc:A3Bd % | g:Gen1_CGT % |
| cds:Gen2_ACG Hits | nc:A3Bd Ti % | g:Gen1_CGT Ti % |
| cds:Gen2_ACG % | nc:A3Bd C>T + G>A % | g:Gen1_CGT C>T + G>A % |
| cds:Gen2_ACG Ti % | nc:A3Bd C>A + G>T % | g:Gen1_CGT C>A + G>T % |
| cds:Gen2_ACG MC1 % | nc:A3Bd C>G + G>C % | g:Gen1_CGT C>G + G>C % |
| cds:Gen2_ACG MC2 % | cds:A3Be Hits | nc:Gen1_CGT Hits |
| cds:Gen2_ACG MC3 % | cds:A3Be % | nc:Gen1_CGT % |
| cds:Gen2_ACG C>T at MC1 % | cds:A3Be Ti % | nc:Gen1_CGT Ti % |
| cds:Gen2_ACG C>T at MC2 % | cds:A3Be MC1 % | nc:Gen1_CGT C>T + G>A % |
| cds:Gen2_ACG C>T at MC3 % | cds:A3Be MC2 % | nc:Gen1_CGT C>A + G>T % |
| cds:Gen2_ACG G>A at MC1 % | cds:A3Be MC3 % | nc:Gen1_CGT C>G + G>C % |
| cds:Gen2_ACG G>A at MC2 % | cds:A3Be C>T at MC1 % | cds:Gen1_CAC Hits |
| cds:Gen2_ACG G>A at MC3 % | cds:A3Be C>T at MC2 % | cds:Gen1_CAC % |
| cds:Gen2_ACG C>T % | cds:A3Be C>T at MC3 % | cds:Gen1_CAC Ti % |
| cds:Gen2_ACG C>A % | cds:A3Be G>A at MC1 % | cds:Gen1_CAC MC1 % |
| cds:Gen2_ACG C>G % | cds:A3Be G>A at MC2 % | cds:Gen1_CAC MC2 % |
| cds:Gen2_ACG G>A % | cds:A3Be G>A at MC3 % | cds:Gen1_CAC MC3 % |
| cds:Gen2_ACG G>T % | cds:A3Be C>T % | cds:Gen1_CAC C>T at MC1 % |
| cds:Gen2_ACG G>C % | cds:A3Be C>A % | cds:Gen1_CAC C>T at MC2 % |
| cds:Gen2_ACG Ti/Tv % | cds:A3Be C>G % | cds:Gen1_CAC C>T at MC3 % |
| cds:Gen2_ACG C:G % | cds:A3Be G>A % | cds:Gen1_CAC G>A at MC1 % |
| cds:Gen2_ACG Ti C:G % | cds:A3Be G>T % | cds:Gen1_CAC G>A at MC2 % |
| cds:Gen2_ACG non-syn % | cds:A3Be G>C % | cds:Gen1_CAC G>A at MC3 % |
| cds:Gen2_ACG C non-syn % | cds:A3Be Ti/Tv % | cds:Gen1_CAC C>T % |
| cds:Gen2_ACG G non-syn % | cds:A3Be C:G % | cds:Gen1_CAC C>A % |
| cds:Gen2_ACG MC1 non-syn % | cds:A3Be Ti C:G % | cds:Gen1_CAC C>G % |
| cds:Gen2_ACG MC2 non-syn % | cds:A3Be non-syn % | cds:Gen1_CAC G>A % |
| cds:Gen2_ACG MC3 non-syn % | cds:A3Be C non-syn % | cds:Gen1_CAC G>T % |
| g:Gen2_ACG Hits | cds:A3Be G non-syn % | cds:Gen1_CAC G>C % |
| g:Gen2_ACG % | cds:A3Be MC1 non-syn % | cds:Gen1_CAC Ti/Tv % |
| g:Gen2_ACG Ti % | cds:A3Be MC2 non-syn % | cds:Gen1_CAC C:G % |
| g:Gen2_ACG C>T + G>A % | cds:A3Be MC3 non-syn % | cds:Gen1_CAC Ti C:G % |
| g:Gen2_ACG C>A + G>T % | g:A3Be Hits | cds:Gen1_CAC non-syn % |
| g:Gen2_ACG C>G + G>C % | g:A3Be % | cds:Gen1_CAC C non-syn % |
| nc:Gen2_ACG Hits | g:A3Be Ti % | cds:Gen1_CAC G non-syn % |

(continued)

| Metric | Metric | Metric |
|---|---|---|
| nc:Gen2_ACG % | g:A3Be C>T + G>A % | cds:Gen1_CAC MC1 non-syn % |
| nc:Gen2_ACG Ti % | g:A3Be C>A + G>T % | cds:Gen1_CAC MC2 non-syn % |
| nc:Gen2_ACG C>T + G>A % | g:A3Be C>G + G>C % | cds:Gen1_CAC MC3 non-syn % |
| nc:Gen2_ACG C>A + G>T % | nc:A3Be Hits | g:Gen1_CAC Hits |
| nc:Gen2_ACG C>G + G>C % | nc:A3Be % | g:Gen1_CAC % |
| cds:Gen2_TCG Hits | nc:A3Be Ti % | g:Gen1_CAC Ti % |
| cds:Gen2_TCG % | nc:A3Be C>T + G>A % | g:Gen1_CAC C>T + G>A % |
| cds:Gen2_TCG Ti % | nc:A3Be C>A + G>T % | g:Gen1_CAC C>A + G>T % |
| cds:Gen2_TCG MC1 % | nc:A3Be C>G + G>C % | g:Gen1_CAC C>G + G>C % |
| cds:Gen2_TCG MC2 % | cds:A3Bf Hits | nc:Gen1_CAC Hits |
| cds:Gen2_TCG MC3 % | cds:A3Bf % | nc:Gen1_CAC % |
| cds:Gen2_TCG C>T at MC1 % | cds:A3Bf Ti % | nc:Gen1_CAC Ti % |
| cds:Gen2_TCG C>T at MC2 % | cds:A3Bf MC1 % | nc:Gen1_CAC C>T + G>A % |
| cds:Gen2_TCG C>T at MC3 % | cds:A3Bf MC2 % | nc:Gen1_CAC C>A + G>T % |
| cds:Gen2_TCG G>A at MC1 % | cds:A3Bf MC3 % | nc:Gen1_CAC C>G + G>C % |
| cds:Gen2_TCG G>A at MC2 % | cds:A3Bf C>T at MC1 % | cds:Gen1_CTC Hits |
| cds:Gen2_TCG G>A at MC3 % | cds:A3Bf C>T at MC2 % | cds:Gen1_CTC % |
| cds:Gen2_TCG C>T % | cds:A3Bf C>T at MC3 % | cds:Gen1_CTC Ti % |
| cds:Gen2_TCG C>A % | cds:A3Bf G>A at MC1 % | cds:Gen1_CTC MC1 % |
| cds:Gen2_TCG C>G % | cds:A3Bf G>A at MC2 % | cds:Gen1_CTC MC2 % |
| cds:Gen2_TCG G>A % | cds:A3Bf G>A at MC3 % | cds:Gen1_CTC MC3 % |
| cds:Gen2_TCG G>T % | cds:A3Bf C>T % | cds:Gen1_CTC C>T at MC1 % |
| cds:Gen2_TCG G>C % | cds:A3Bf C>A % | cds:Gen1_CTC C>T at MC2 % |
| cds:Gen2_TCG Ti/Tv % | cds:A3Bf C>G % | cds:Gen1_CTC C>T at MC3 % |
| cds:Gen2_TCG C:G % | cds:A3Bf G>A % | cds:Gen1_CTC G>A at MC1 % |
| cds:Gen2_TCG Ti C:G % | cds:A3Bf G>T % | cds:Gen1_CTC G>A at MC2 % |
| cds:Gen2_TCG non-syn % | cds:A3Bf G>C % | cds:Gen1_CTC G>A at MC3 % |
| cds:Gen2_TCG C non-syn % | cds:A3Bf Ti/Tv % | cds:Gen1_CTC C>T % |
| cds:Gen2_TCG G non-syn % | cds:A3Bf C:G % | cds:Gen1_CTC C>A % |
| cds:Gen2_TCG MC1 non-syn % | cds:A3Bf Ti C:G % | cds:Gen1_CTC C>G % |
| cds:Gen2_TCG MC2 non-syn % | cds:A3Bf non-syn % | cds:Gen1_CTC G>A % |
| cds:Gen2_TCG MC3 non-syn % | cds:A3Bf C non-syn % | cds:Gen1_CTC G>T % |
| g:Gen2_TCG Hits | cds:A3Bf G non-syn % | cds:Gen1_CTC G>C % |
| g:Gen2_TCG % | cds:A3Bf MC1 non-syn % | cds:Gen1_CTC Ti/Tv % |
| g:Gen2_TCG Ti % | cds:A3Bf MC2 non-syn % | cds:Gen1_CTC C:G % |
| g:Gen2_TCG C>T + G>A % | cds:A3Bf MC3 non-syn % | cds:Gen1_CTC Ti C:G % |
| g:Gen2_TCG C>A + G>T % | g:A3Bf Hits | cds:Gen1_CTC non-syn % |
| g:Gen2_TCG C>G + G>C % | g:A3Bf % | cds:Gen1_CTC C non-syn % |
| nc:Gen2_TCG Hits | g:A3Bf Ti % | cds:Gen1_CTC G non-syn % |

(continued)

| Metric | Metric | Metric |
|---|---|---|
| nc:Gen2_TCG % | g:A3Bf C>T + G>A % | cds:Gen1_CTC MC1 non-syn % |
| nc:Gen2_TCG Ti % | g:A3Bf C>A + G>T % | cds:Gen1_CTC MC2 non-syn % |
| nc:Gen2_TCG C>T + G>A % | g:A3Bf C>G + G>C % | cds:Gen1_CTC MC3 non-syn % |
| nc:Gen2_TCG C>A + G>T % | nc:A3Bf Hits | g:Gen1_CTC Hits |
| nc:Gen2_TCG C>G + G>C % | nc:A3Bf % | g:Gen1_CTC % |
| cds:Gen2_CCG Hits | nc:A3Bf Ti % | g:Gen1_CTC Ti % |
| cds:Gen2_CCG % | nc:A3Bf C>T + G>A % | g:Gen1_CTC C>T + G>A % |
| cds:Gen2_CCG Ti % | nc:A3Bf C>A + G>T % | g:Gen1_CTC C>A + G>T % |
| cds:Gen2_CCG MC1 % | nc:A3Bf C>G + G>C % | g:Gen1_CTC C>G + G>C % |
| cds:Gen2_CCG MC2 % | cds:A3Bg Hits | nc:Gen1_CTC Hits |
| cds:Gen2_CCG MC3 % | cds:A3Bg % | nc:Gen1_CTC % |
| cds:Gen2_CCG C>T at MC1 % | cds:A3Bg Ti % | nc:Gen1_CTC Ti % |
| cds:Gen2_CCG C>T at MC2 % | cds:A3Bg MC1 % | nc:Gen1_CTC C>T + G>A % |
| cds:Gen2_CCG C>T at MC3 % | cds:A3Bg MC2 % | nc:Gen1_CTC C>A + G>T % |
| cds:Gen2_CCG G>A at MC1 % | cds:A3Bg MC3 % | nc:Gen1_CTC C>G + G>C % |
| cds:Gen2_CCG G>A at MC2 % | cds:A3Bg C>T at MC1 % | cds:Gen1_CCC Hits |
| cds:Gen2_CCG G>A at MC3 % | cds:A3Bg C>T at MC2 % | cds:Gen1_CCC % |
| cds:Gen2_CCG C>T % | cds:A3Bg C>T at MC3 % | cds:Gen1_CCC Ti % |
| cds:Gen2_CCG C>A % | cds:A3Bg G>A at MC1 % | cds:Gen1_CCC MC1 % |
| cds:Gen2_CCG C>G % | cds:A3Bg G>A at MC2 % | cds:Gen1_CCC MC2 % |
| cds:Gen2_CCG G>A % | cds:A3Bg G>A at MC3 % | cds:Gen1_CCC MC3 % |
| cds:Gen2_CCG G>T % | cds:A3Bg C>T % | cds:Gen1_CCC C>T at MC1 % |
| cds:Gen2_CCG G>C % | cds:A3Bg C>A % | cds:Gen1_CCC C>T at MC2 % |
| cds:Gen2_CCG Ti/Tv % | cds:A3Bg C>G % | cds:Gen1_CCC C>T at MC3 % |
| cds:Gen2_CCG C:G % | cds:A3Bg G>A % | cds:Gen1_CCC G>A at MC1 % |
| cds:Gen2_CCG Ti C:G % | cds:A3Bg G>T % | cds:Gen1_CCC G>A at MC2 % |
| cds:Gen2_CCG non-syn % | cds:A3Bg G>C % | cds:Gen1_CCC G>A at MC3 % |
| cds:Gen2_CCG C non-syn % | cds:A3Bg Ti/Tv % | cds:Gen1_CCC C>T % |
| cds:Gen2_CCG G non-syn % | cds:A3Bg C:G % | cds:Gen1_CCC C>A % |
| cds:Gen2_CCG MC1 non-syn % | cds:A3Bg Ti C:G % | cds:Gen1_CCC C>G % |
| cds:Gen2_CCG MC2 non-syn % | cds:A3Bg non-syn % | cds:Gen1_CCC G>A % |
| cds:Gen2_CCG MC3 non-syn % | cds:A3Bg C non-syn % | cds:Gen1_CCC G>T % |
| g:Gen2_CCG Hits | cds:A3Bg G non-syn % | cds:Gen1_CCC G>C % |
| g:Gen2_CCG % | cds:A3Bg MC1 non-syn % | cds:Gen1_CCC Ti/Tv % |
| g:Gen2_CCG Ti % | cds:A3Bg MC2 non-syn % | cds:Gen1_CCC C:G % |
| g:Gen2_CCG C>T + G>A % | cds:A3Bg MC3 non-syn % | cds:Gen1_CCC Ti C:G % |
| g:Gen2_CCG C>A + G>T % | g:A3Bg Hits | cds:Gen1_CCC non-syn % |
| g:Gen2_CCG C>G + G>C % | g:A3Bg % | cds:Gen1_CCC C non-syn % |
| nc:Gen2_CCG Hits | g:A3Bg Ti % | cds:Gen1_CCC G non-syn % |

(continued)

| Metric | Metric | Metric |
|---|---|---|
| nc:Gen2_CCG % | g:A3Bg C>T + G>A % | cds:Gen1_CCC MC1 non-syn % |
| nc:Gen2_CCG Ti % | g:A3Bg C>A + G>T % | cds:Gen1_CCC MC2 non-syn % |
| nc:Gen2_CCG C>T + G>A % | g:A3Bg C>G + G>C % | cds:Gen1_CCC MC3 non-syn % |
| nc:Gen2_CCG C>A + G>T % | nc:A3Bg Hits | g:Gen1_CCC Hits |
| nc:Gen2_CCG C>G + G>C % | nc:A3Bg % | g:Gen1_CCC % |
| cds:Gen2_GCG Hits | nc:A3Bg Ti % | g:Gen1_CCC Ti % |
| cds:Gen2_GCG % | nc:A3Bg C>T + G>A % | g:Gen1_CCC C>T + G>A % |
| cds:Gen2_GCG Ti % | nc:A3Bg C>A + G>T % | g:Gen1_CCC C>A + G>T % |
| cds:Gen2_GCG MC1 % | nc:A3Bg C>G + G>C % | g:Gen1_CCC C>G + G>C % |
| cds:Gen2_GCG MC2 % | cds:A3Bh Hits | nc:Gen1_CCC Hits |
| cds:Gen2_GCG MC3 % | cds:A3Bh % | nc:Gen1_CCC % |
| cds:Gen2_GCG C>T at MC1 % | cds:A3Bh Ti % | nc:Gen1_CCC Ti % |
| cds:Gen2_GCG C>T at MC2 % | cds:A3Bh MC1 % | nc:Gen1_CCC C>T + G>A % |
| cds:Gen2_GCG C>T at MC3 % | cds:A3Bh MC2 % | nc:Gen1_CCC C>A + G>T % |
| cds:Gen2_GCG G>A at MC1 % | cds:A3Bh MC3 % | nc:Gen1_CCC C>G + G>C % |
| cds:Gen2_GCG G>A at MC2 % | cds:A3Bh C>T at MC1 % | cds:Gen1_CGC Hits |
| cds:Gen2_GCG G>A at MC3 % | cds:A3Bh C>T at MC2 % | cds:Gen1_CGC % |
| cds:Gen2_GCG C>T % | cds:A3Bh C>T at MC3 % | cds:Gen1_CGC Ti % |
| cds:Gen2_GCG C>A % | cds:A3Bh G>A at MC1 % | cds:Gen1_CGC MC1 % |
| cds:Gen2_GCG C>G % | cds:A3Bh G>A at MC2 % | cds:Gen1_CGC MC2 % |
| cds:Gen2_GCG G>A % | cds:A3Bh G>A at MC3 % | cds:Gen1_CGC MC3 % |
| cds:Gen2_GCG G>T % | cds:A3Bh C>T % | cds:Gen1_CGC C>T at MC1 % |
| cds:Gen2_GCG G>C % | cds:A3Bh C>A % | cds:Gen1_CGC C>T at MC2 % |
| cds:Gen2_GCG Ti/Tv % | cds:A3Bh C>G % | cds:Gen1_CGC C>T at MC3 % |
| cds:Gen2_GCG C:G % | cds:A3Bh G>A % | cds:Gen1_CGC G>A at MC1 % |
| cds:Gen2_GCG Ti C:G % | cds:A3Bh G>T % | cds:Gen1_CGC G>A at MC2 % |
| cds:Gen2_GCG non-syn % | cds:A3Bh G>C % | cds:Gen1_CGC G>A at MC3 % |
| cds:Gen2_GCG C non-syn % | cds:A3Bh Ti/Tv % | cds:Gen1_CGC C>T % |
| cds:Gen2_GCG G non-syn % | cds:A3Bh C:G % | cds:Gen1_CGC C>A % |
| cds:Gen2_GCG MC1 non-syn % | cds:A3Bh Ti C:G % | cds:Gen1_CGC C>G % |
| cds:Gen2_GCG MC2 non-syn % | cds:A3Bh non-syn % | cds:Gen1_CGC G>A % |
| cds:Gen2_GCG MC3 non-syn % | cds:A3Bh C non-syn % | cds:Gen1_CGC G>T % |
| g:Gen2_GCG Hits | cds:A3Bh G non-syn % | cds:Gen1_CGC G>C % |
| g:Gen2_GCG % | cds:A3Bh MC1 non-syn % | cds:Gen1_CGC Ti/Tv % |
| g:Gen2_GCG Ti % | cds:A3Bh MC2 non-syn % | cds:Gen1_CGC C:G % |
| g:Gen2_GCG C>T + G>A % | cds:A3Bh MC3 non-syn % | cds:Gen1_CGC Ti C:G % |
| g:Gen2_GCG C>A + G>T % | g:A3Bh Hits | cds:Gen1_CGC non-syn % |
| g:Gen2_GCG C>G + G>C % | g:A3Bh % | cds:Gen1_CGC C non-syn % |
| nc:Gen2_GCG Hits | g:A3Bh Ti % | cds:Gen1_CGC G non-syn % |

(continued)

| Metric | Metric | Metric |
|---|---|---|
| nc:Gen2_GCG % | g:A3Bh C>T + G>A % | cds:Gen1_CGC MC1 non-syn % |
| nc:Gen2_GCG Ti % | g:A3Bh C>A + G>T % | cds:Gen1_CGC MC2 non-syn % |
| nc:Gen2_GCG C>T + G>A % | g:A3Bh C>G + G>C % | cds:Gen1_CGC MC3 non-syn % |
| nc:Gen2_GCG C>A + G>T % | nc:A3Bh Hits | g:Gen1_CGC Hits |
| nc:Gen2_GCG C>G + G>C % | nc:A3Bh % | g:Gen1_CGC % |
| cds:ADAR_Gen2_AAA Hits | nc:A3Bh Ti % | g:Gen1_CGC Ti % |
| cds:ADAR_Gen2_AAA % | nc:A3Bh C>T + G>A % | g:Gen1_CGC C>T + G>A % |
| cds:ADAR_Gen2_AAA Ti % | nc:A3Bh C>A + G>T % | g:Gen1_CGC C>A + G>T % |
| cds:ADAR_Gen2_AAA MC1 % | nc:A3Bh C>G + G>C % | g:Gen1_CGC C>G + G>C % |
| cds:ADAR_Gen2_AAA MC2 % | cds:A3F Hits | nc:Gen1_CGC Hits |
| cds:ADAR_Gen2_AAA MC3 % | cds:A3F % | nc:Gen1_CGC % |
| cds:ADAR_Gen2_AAA A>G at MC1 % | cds:A3F Ti % | nc:Gen1_CGC Ti % |
| cds:ADAR_Gen2_AAA A>G at MC2 % | cds:A3F MC1 % | nc:Gen1_CGC C>T + G>A % |
| cds:ADAR_Gen2_AAA A>G at MC3 % | cds:A3F MC2 % | nc:Gen1_CGC C>A + G>T % |
| cds:ADAR_Gen2_AAA T>C at MC1 % | cds:A3F MC3 % | nc:Gen1_CGC C>G + G>C % |
| cds:ADAR_Gen2_AAA T>C at MC2 % | cds:A3F C>T at MC1 % | cds:Gen1_CAG Hits |
| cds:ADAR_Gen2_AAA T>C at MC3 % | cds:A3F C>T at MC2 % | cds:Gen1_CAG % |
| cds:ADAR_Gen2_AAA A>G % | cds:A3F C>T at MC3 % | cds:Gen1_CAG Ti % |
| cds:ADAR_Gen2_AAA A>C % | cds:A3F G>A at MC1 % | cds:Gen1_CAG MC1 % |
| cds:ADAR_Gen2_AAA A>T % | cds:A3F G>A at MC2 % | cds:Gen1_CAG MC2 % |
| cds:ADAR_Gen2_AAA T>C % | cds:A3F G>A at MC3 % | cds:Gen1_CAG MC3 % |
| cds:ADAR_Gen2_AAA T>G % | cds:A3F C>T % | cds:Gen1_CAG C>T at MC1 % |
| cds:ADAR_Gen2_AAA T>A % | cds:A3F C>A % | cds:Gen1_CAG C>T at MC2 % |
| cds:ADAR_Gen2_AAA Ti/Tv % | cds:A3F C>G % | cds:Gen1_CAG C>T at MC3 % |
| cds:ADAR_Gen2_AAA A:T % | cds:A3F G>A % | cds:Gen1_CAG G>A at MC1 % |
| cds:ADAR_Gen2_AAA Ti A:T % | cds:A3F G>T % | cds:Gen1_CAG G>A at MC2 % |
| cds:ADAR_Gen2_AAA non-syn % | cds:A3F G>C % | cds:Gen1_CAG G>A at MC3 % |
| cds:ADAR_Gen2_AAA A non-syn % | cds:A3F Ti/Tv % | cds:Gen1_CAG C>T % |
| cds:ADAR_Gen2_AAA T non-syn % | cds:A3F C:G % | cds:Gen1_CAG C>A % |
| cds:ADAR_Gen2_AAA MC1 non-syn % | cds:A3F Ti C:G % | cds:Gen1_CAG C>G % |
| cds:ADAR_Gen2_AAA MC2 non-syn % | cds:A3F non-syn % | cds:Gen1_CAG G>A % |
| cds:ADAR_Gen2_AAA MC3 non-syn % | cds:A3F C non-syn % | cds:Gen1_CAG G>T % |
| g:ADAR_Gen2_AAA Hits | cds:A3F G non-syn % | cds:Gen1_CAG G>C % |

(continued)

| Metric | Metric | Metric |
|---|---|---|
| g:ADAR_Gen2_AAA % | cds:A3F MC1 non-syn % | cds:Gen1_CAG Ti/Tv % |
| g:ADAR_Gen2_AAA Ti % | cds:A3F MC2 non-syn % | cds:Gen1_CAG C:G % |
| g:ADAR_Gen2_AAA A>G + T>C % | cds:A3F MC3 non-syn % | cds:Gen1_CAG Ti C:G % |
| g:ADAR_Gen2_AAA A>C + T>G % | g:A3F Hits | cds:Gen1_CAG non-syn % |
| g:ADAR_Gen2_AAA A>T + T>A % | g:A3F % | cds:Gen1_CAG C non-syn % |
| nc:ADAR_Gen2_AAA Hits | g:A3F Ti % | cds:Gen1_CAG G non-syn % |
| nc:ADAR_Gen2_AAA % | g:A3F C>T + G>A % | cds:Gen1_CAG MC1 non-syn % |
| nc:ADAR_Gen2_AAA Ti % | g:A3F C>A + G>T % | cds:Gen1_CAG MC2 non-syn % |
| nc:ADAR_Gen2_AAA A>G + T>C % | g:A3F C>G + G>C % | cds:Gen1_CAG MC3 non-syn % |
| nc:ADAR_Gen2_AAA A>C + T>G % | nc:A3F Hits | g:Gen1_CAG Hits |
| nc:ADAR_Gen2_AAA A>T + T>A % | nc:A3F % | g:Gen1_CAG % |
| cds:ADAR_Gen2_TAA Hits | nc:A3F Ti % | g:Gen1_CAG Ti % |
| cds:ADAR_Gen2_TAA % | nc:A3F C>T + G>A % | g:Gen1_CAG C>T + G>A % |
| cds:ADAR_Gen2_TAA Ti % | nc:A3F C>A + G>T % | g:Gen1_CAG C>A + G>T % |
| cds:ADAR_Gen2_TAA MC1 % | nc:A3F C>G + G>C % | g:Gen1_CAG C>G + G>C % |
| cds:ADAR_Gen2_TAA MC2 % | cds:AI Hits | nc:Gen1_CAG Hits |
| cds:ADAR_Gen2_TAA MC3 % | cds:AI % | nc:Gen1_CAG % |
| cds:ADAR_Gen2_TAA A>G at MC1 % | cds:A1 Ti % | nc:Gen1_CAG Ti % |
| cds:ADAR_Gen2_TAA A>G at MC2 % | cds:AI MC1 % | nc:Gen1_CAG C>T + G>A % |
| cds:ADAR_Gen2_TAA A>G at MC3 % | cds:AI MC2 % | nc:Gen1_CAG C>A + G>T % |
| cds:ADAR_Gen2_TAA T>C at MC1 % | cds:AI MC3 % | nc:Gen1_CAG C>G + G>C % |
| cds:ADAR_Gen2_TAA T>C at MC2 % | cds:AI C>T at MC1 % | cds:Gen1_CTG Hits |
| cds:ADAR_Gen2_TAA T>C at MC3 % | cds:AI C>T at MC2 % | cds:Gen1_CTG % |
| cds:ADAR_Gen2_TAA A>G % | cds:AI C>T at MC3 % | cds:Gen1_CTG Ti % |
| cds:ADAR_Gen2_TAA A>C % | cds:AI G>A at MC1 % | cds:Gen1_CTG MC1 % |
| cds:ADAR_Gen2_TAA A>T % | cds:AI G>A at MC2 % | cds:Gen1_CTG MC2 % |
| cds:ADAR_Gen2_TAA T>C % | cds:AI G>A at MC3 % | cds:Gen1_CTG MC3 % |
| cds:ADAR_Gen2_TAA T>G % | cds:AI C>T % | cds:Gen1_CTG C>T at MC1 % |
| cds:ADAR_Gen2_TAA T>A % | cds:AI C>A % | cds:Gen1_CTG C>T at MC2 % |
| cds:ADAR_Gen2_TAA Ti/Tv % | cds:A1 C>G % | cds:Gen1_CTG C>T at MC3 % |
| cds:ADAR_Gen2_TAA A:T % | cds:AI G>A % | cds:Gen1_CTG G>A at MC1 % |
| cds:ADAR_Gen2_TAA Ti A:T % | cds:A1 G>T % | cds:Gen1_CTG G>A at MC2 % |
| cds:ADAR_Gen2_TAA non-syn % | cds:AI G>C % | cds:Gen1_CTG G>A at MC3 % |
| cds:ADAR_Gen2_TAA A non-syn % | cds:AI Ti/Tv % | cds:Gen1_CTG C>T % |
| cds:ADAR_Gen2_TAA T non-syn % | cds:AI C:G % | cds:Gen1_CTG C>A % |

(continued)

| Metric | Metric | Metric |
|---|---|---|
| cds:ADAR_Gen2_TAA MC1 non-syn % | cds:A1 Ti C:G % | cds:Gen1_CTG C>G % |
| cds:ADAR_Gen2_TAA MC2 non-syn % | cds:Al non-syn % | cds:Gen1_CTG G>A % |
| cds:ADAR_Gen2_TAA MC3 non-syn % | cds:A1 C non-syn % | cds:Gen1_CTG G>T % |
| g:ADAR_Gen2_TAA Hits | cds:Al G non-syn % | cds:Gen1_CTG G>C % |
| g:ADAR_Gen2_TAA % | cds:Al MC1 non-syn % | cds:Gen1_CTG Ti/Tv % |
| g:ADAR_Gen2_TAA Ti % | cds:Al MC2 non-syn % | cds:Gen1_CTG C:G % |
| g:ADAR_Gen2_TAA A>G + T>C % | cds:Al MC3 non-syn % | cds:Gen1_CTG Ti C:G % |
| g:ADAR_Gen2_TAA A>C + T>G % | g:A1 Hits | cds:Gen1_CTG non-syn % |
| g:ADAR_Gen2_TAA A>T + T>A % | g:A1 % | cds:Gen1_CTG C non-syn % |
| nc:ADAR_Gen2_TAA Hits | g:A1 Ti % | cds:Gen1_CTG G non-syn % |
| nc:ADAR_Gen2_TAA % | g:A1 C>T + G>A % | cds:Gen1_CTG MC1 non-syn % |
| nc:ADAR_Gen2_TAA Ti % | g:A1 C>A + G>T % | cds:Gen1_CTG MC2 non-syn % |
| nc:ADAR_Gen2_TAA A>G + T>C % | g:A1 C>G + G>C % | cds:Gen1_CTG MC3 non-syn % |
| nc:ADAR_Gen2_TAA A>C + T>G % | nc:Al Hits | g:Gen1_CTG Hits |
| nc:ADAR_Gen2_TAA A>T + T>A % | nc:A1 % | g:Gen1_CTG % |
| cds:ADAR_Gen2_CAA Hits | nc:A1 Ti % | g:Gen1_CTG Ti % |
| cds:ADAR_Gen2_CAA % | nc:Al C>T + G>A % | g:Gen1_CTG C>T + G>A % |
| cds:ADAR_Gen2_CAA Ti % | nc:Al C>A + G>T % | g:Gen1_CTG C>A + G>T % |
| cds:ADAR_Gen2_CAA MC1 % | nc:A1 C>G + G>C % | g:Gen1_CTG C>G + G>C % |
| cds:ADAR_Gen2_CAA MC2 % | cds:ADAR_Gen1_AAA Hits | nc:Gen1_CTG Hits |
| cds:ADAR_Gen2_CAA MC3 % | cds:ADAR_Gen1_AAA % | nc:Gen1_CTG % |
| cds:ADAR_Gen2_CAA A>G at MC1 % | cds:ADAR_Gen1_AAA Ti % | nc:Gen1_CTG Ti % |
| cds:ADAR_Gen2_CAA A>G at MC2 % | cds:ADAR_Gen1_AAA MC1 % | nc:Gen1_CTG C>T + G>A % |
| cds:ADAR_Gen2_CAA A>G at MC3 % | cds:ADAR_Gen1_AAA MC2 % | nc:Gen1_CTG C>A + G>T % |
| cds:ADAR_Gen2_CAA T>C at MC1 % | cds:ADAR_Gen1_AAA MC3 % | nc:Gen1_CTG C>G + G>C % |
| cds:ADAR_Gen2_CAA T>C at MC2 % | cds:ADAR_Gen1_AAA A>G at MC1 % | cds:Gen1_CCG Hits |
| cds:ADAR_Gen2_CAA T>C at MC3 % | cds:ADAR_Gen1_AAA A>G at MC2 % | cds:Gen1_CCG % |
| cds:ADAR_Gen2_CAA A>G % | cds:ADAR_Gen1_AAA A>G at MC3 % | cds:Gen1_CCG Ti % |
| cds:ADAR_Gen2_CAA A>C % | cds:ADAR_Gen1_AAA T>C at MC1 % | cds:Gen1_CCG MC1 % |
| cds:ADAR_Gen2_CAA A>T % | cds:ADAR_Gen1_AAA T>C at MC2 % | cds:Gen1_CCG MC2 % |

(continued)

| Metric | Metric | Metric |
|---|---|---|
| cds:ADAR_Gen2_CAA T>C % | cds:ADAR_Gen1_AAA T>C at MC3 % | cds:Gen1_CCG MC3 % |
| cds:ADAR_Gen2_CAA T>G % | cds:ADAR_Gen1_AAA A>G % | cds:Gen1_CCG C>T at MC1 % |
| cds:ADAR_Gen2_CAA T>A % | cds:ADAR_Gen1_AAA A>C % | cds:Gen1_CCG C>T at MC2 % |
| cds:ADAR_Gen2_CAA Ti/Tv % | cds:ADAR_Gen1_AAA A>T % | cds:Gen1_CCG C>T at MC3 % |
| cds:ADAR_Gen2_CAA A:T % | cds:ADAR_Gen1_AAA T>C % | cds:Gen1_CCG G>A at MC1 % |
| cds:ADAR_Gen2_CAA Ti A:T % | cds:ADAR_Gen1_AAA T>G % | cds:Gen1_CCG G>A at MC2 % |
| cds:ADAR_Gen2_CAA non-syn % | cds:ADAR_Gen1_AAA T>A % | cds:Gen1_CCG G>A at MC3 % |
| cds:ADAR_Gen2_CAA A non-syn % | cds:ADAR_Gen1_AAA Ti/Tv % | cds:Gen1_CCG C>T % |
| cds:ADAR_Gen2_CAA T non-syn % | cds:ADAR_Gen1_AAA A:T % | cds:Gen1_CCG C>A % |
| cds:ADAR_Gen2_CAA MC1 non-syn % | cds:ADAR_Gen1_AAA Ti A:T % | cds:Gen1_CCG C>G % |
| cds:ADAR_Gen2_CAA MC2 non-syn % | cds:ADAR_Gen1_AAA non-syn % | cds:Gen1_CCG G>A % |
| cds:ADAR_Gen2_CAA MC3 non-syn % | cds:ADAR_Gen1_AAA A non-syn % | cds:Gen1_CCG G>T % |
| g:ADAR_Gen2_CAA Hits | cds:ADAR_Gen1_AAA T non-syn % | cds:Gen1_CCG G>C % |
| g:ADAR_Gen2_CAA % | cds:ADAR_Gen1_AAA MC1 non-syn % | cds:Gen1_CCG Ti/Tv % |
| g:ADAR_Gen2_CAA Ti % | cds:ADAR_Gen1_AAA MC2 non-syn % | cds:Gen1_CCG C:G % |
| g:ADAR_Gen2_CAA A>G + T>C % | cds:ADAR_Gen1_AAA MC3 non-syn % | cds:Gen1_CCG Ti C:G % |
| g:ADAR_Gen2_CAA A>C + T>G % | g:ADAR_Gen1_AAA Hits | cds:Gen1_CCG non-syn % |
| g:ADAR_Gen2_CAA A>T + T>A % | g:ADAR_Gen1_AAA % | cds:Gen1_CCG C non-syn % |
| nc:ADAR_Gen2_CAA Hits | g:ADAR_Gen1_AAA Ti % | cds:Gen1_CCG G non-syn % |
| nc:ADAR_Gen2_CAA % | g:ADAR_Gen1_AAA A>G + T>C % | cds:Gen1_CCG MC1 non-syn % |
| nc:ADAR_Gen2_CAA Ti % | g:ADAR_Gen1_AAA A>C + T>G % | cds:Gen1_CCG MC2 non-syn % |
| nc:ADAR_Gen2_CAA A>G + T>C % | g:ADAR_Gen1_AAA A>T + T>A % | cds:Gen1_CCG MC3 non-syn % |
| nc:ADAR_Gen2_CAA A>C + T>G % | nc:ADAR_Gen1_AAA Hits | g:Gen1_CCG Hits |
| nc:ADAR_Gen2_CAA A>T + T>A % | nc:ADAR_Gen1_AAA % | g:Gen1_CCG % |
| cds:ADAR_Gen2_GAA Hits | nc:ADAR_Gen1_AAA Ti % | g:Gen1_CCG Ti % |
| cds:ADAR_Gen2_GAA % | nc:ADAR_Gen1_AAA A>G + T>C % | g:Gen1_CCG C>T + G>A % |
| cds:ADAR_Gen2_GAA Ti % | nc:ADAR_Gen1_AAA A>C + T>G % | g:Gen1_CCG C>A + G>T % |
| cds:ADAR_Gen2_GAA MC1 % | nc:ADAR_Gen1_AAA A>T + T>A % | g:Gen1_CCG C>G + G>C % |
| cds:ADAR_Gen2_GAA MC2 % | cds:ADAR_Gen1_AAT Hits | nc:Gen1_CCG Hits |
| cds:ADAR_Gen2_GAA MC3 % | cds:ADAR_Gen1_AAT % | nc:Gen1_CCG % |
| cds:ADAR_Gen2_GAA A>G at MC1 % | cds:ADAR_Gen1_AAT Ti % | nc:Gen1_CCG Ti % |
| cds:ADAR_Gen2_GAA A>G at MC2 % | cds:ADAR_Gen1_AAT MC1 % | nc:Gen1_CCG C>T + G>A % |

(continued)

| Metric | Metric | Metric |
|---|---|---|
| cds:ADAR_Gen2_GAA A>G at MC3 % | cds:ADAR_Gen1_AAT MC2 % | nc:Gen1_CCG C>A + G>T % |
| cds:ADAR_Gen2_GAA T>C at MC1 % | cds:ADAR_Gen1_AAT MC3 % | nc:Gen1_CCG C>G + G>C % |
| cds:ADAR_Gen2_GAA T>C at MC2 % | cds:ADAR_Gen1_AAT A>G at MC1 % | cds:Gen1_CGG Hits |
| cds:ADAR_Gen2_GAA T>C at MC3 % | cds:ADAR_Gen1_AAT A>G at MC2 % | cds:Gen1_CGG % |
| cds:ADAR_Gen2_GAA A>G % | cds:ADAR_Gen1_AAT A>G at MC3 % | cds:Gen1_CGG Ti % |
| cds:ADAR_Gen2_GAA A>C % | cds:ADAR_Gen1_AAT T>C at MC1 % | cds:Gen1_CGG MC1 % |
| cds:ADAR_Gen2_GAA A>T % | cds:ADAR_Gen1_AAT T>C at MC2 % | cds:Gen1_CGG MC2 % |
| cds:ADAR_Gen2_GAA T>C % | cds:ADAR_Gen1_AAT T>C at MC3 % | cds:Gen1_CGG MC3 % |
| cds:ADAR_Gen2_GAA T>G % | cds:ADAR_Gen1_AAT A>G % | cds:Gen1_CGG C>T at MC1 % |
| cds:ADAR_Gen2_GAA T>A % | cds:ADAR_Gen1_AAT A>C % | cds:Gen1_CGG C>T at MC2 % |
| cds:ADAR_Gen2_GAA Ti/Tv % | cds:ADAR_Gen1_AAT A>T % | cds:Gen1_CGG C>T at MC3 % |
| cds:ADAR_Gen2_GAA A:T % | cds:ADAR_Gen1_AAT T>C % | cds:Gen1_CGG G>A at MC1 % |
| cds:ADAR_Gen2_GAA Ti A:T % | cds:ADAR_Gen1_AAT T>G % | cds:Gen1_CGG G>A at MC2 % |
| cds:ADAR_Gen2_GAA non-syn % | cds:ADAR_Gen1_AAT T>A % | cds:Gen1_CGG G>A at MC3 % |
| cds:ADAR_Gen2_GAA A non-syn % | cds:ADAR_Gen1_AAT Ti/Tv % | cds:Gen1_CGG C>T % |
| cds:ADAR_Gen2_GAA T non-syn % | cds:ADAR_Gen1_AAT A:T % | cds:Gen1_CGG C>A % |
| cds:ADAR_Gen2_GAA MC1 non-syn % | cds:ADAR_Gen1_AAT Ti A:T % | cds:Gen1_CGG C>G % |
| cds:ADAR_Gen2_GAA MC2 non-syn % | cds:ADAR_Gen1_AAT non-syn % | cds:Gen1_CGG G>A % |
| cds:ADAR_Gen2_GAA MC3 non-syn % | cds:ADAR_Gen1_AAT A non-syn % | cds:Gen1_CGG G>T % |
| g:ADAR_Gen2_GAA Hits | cds:ADAR_Gen1_AAT T non-syn % | cds:Gen1_CGG G>C % |
| g:ADAR_Gen2_GAA % | cds:ADAR_Gen1_AAT MC1 non-syn % | cds:Gen1_CGG Ti/Tv % |
| g:ADAR_Gen2_GAA Ti % | cds:ADAR_Gen1_AAT MC2 non-syn % | cds:Gen1_CGG C:G % |
| g:ADAR_Gen2_GAA A>G + T>C % | cds:ADAR_Gen1_AAT MC3 non-syn % | cds:Gen1_CGG Ti C:G % |
| g:ADAR_Gen2_GAA A>C + T>G % | g:ADAR_Gen1_AAT Hits | cds:Gen1_CGG non-syn % |
| g:ADAR_Gen2_GAA A>T + T>A % | g:ADAR_Gen1_AAT % | cds:Gen1_CGG C non-syn % |
| nc:ADAR_Gen2_GAA Hits | g:ADAR_Gen1_AAT Ti % | cds:Gen1_CGG G non-syn % |
| nc:ADAR_Gen2_GAA % | g:ADAR_Gen1_AAT A>G + T>C % | cds:Gen1_CGG MC1 non-syn % |
| nc:ADAR_Gen2_GAA Ti % | g:ADAR_Gen1_AAT A>C + T>G % | cds:Gen1_CGG MC2 non-syn % |
| nc:ADAR_Gen2_GAA A>G + T>C % | g:ADAR_Gen1_AAT A>T + T>A % | cds:Gen1_CGG MC3 non-syn % |

73

(continued)

| Metric | Metric | Metric |
|---|---|---|
| nc:ADAR_Gen2_GAA A>C + T>G % | nc:ADAR_Gen1_AAT Hits | g:Gen1_CGG Hits |
| nc:ADAR_Gen2_GAA A>T + T>A % | nc:ADAR_Gen1_AAT % | g:Gen1_CGG % |
| cds:ADAR_Gen2_AAT Hits | nc:ADAR_Gen1_AAT Ti % | g:Gen1_CGG Ti % |
| cds:ADAR_Gen2_AAT % | nc:ADAR_Gen1_AAT A>G + T>C % | g:Gen1_CGG C>T + G>A % |
| cds:ADAR_Gen2_AAT Ti % | nc:ADAR_Gen1_AAT A>C + T>G % | g:Gen1_CGG C>A + G>T % |
| cds:ADAR_Gen2_AAT MC1 % | nc:ADAR_Gen1_AAT A>T + T>A % | g:Gen1_CGG C>G + G>C % |
| cds:ADAR_Gen2_AAT MC2 % | cds:ADAR_Gen1_AAC Hits | nc:Gen1_CGG Hits |
| cds:ADAR_Gen2_AAT MC3 % | cds:ADAR_Gen1_AAC % | nc:Gen1_CGG % |
| cds:ADAR_Gen2_AAT A>G at MC1 % | cds:ADAR_Gen1_AAC Ti % | nc:Gen1_CGG Ti % |
| cds:ADAR_Gen2_AAT A>G at MC2 % | cds:ADAR_Gen1_AAC MC1 % | nc:Gen1_CGG C>T + G>A % |
| cds:ADAR_Gen2_AAT A>G at MC3 % | cds:ADAR_Gen1_AAC MC2 % | nc:Gen1_CGG C>A + G>T % |
| cds:ADAR_Gen2_AAT T>C at MC1 % | cds:ADAR_Gen1_AAC MC3 % | nc:Gen1_CGG C>G + G>C % |
| cds:ADAR_Gen2_AAT T>C at MC2 % | cds:ADAR_Gen1_AAC A>G at MC1 % | cds:Gen3_AAC Hits |
| cds:ADAR_Gen2_AAT T>C at MC3 % | cds:ADAR_Gen1_AAC A>G at MC2 % | cds:Gen3_AAC % |
| cds:ADAR_Gen2_AAT A>G % | cds:ADAR_Gen1_AAC A>G at MC3 % | cds:Gen3_AAC Ti % |
| cds:ADAR_Gen2_AAT A>C % | cds:ADAR_Gen1_AAC T>C at MC1 % | cds:Gen3_AAC MC1 % |
| cds:ADAR_Gen2_AAT A>T % | cds:ADAR_Gen1_AAC T>C at MC2 % | cds:Gen3_AAC MC2 % |
| cds:ADAR_Gen2_AAT T>C % | cds:ADAR_Gen1_AAC T>C at MC3 % | cds:Gen3_AAC MC3 % |
| cds:ADAR_Gen2_AAT T>G % | cds:ADAR_Gen1_AAC A>G % | cds:Gen3_AAC C>T at MC1 % |
| cds:ADAR_Gen2_AAT T>A % | cds:ADAR_Gen1_AAC A>C % | cds:Gen3_AAC C>T at MC2 % |
| cds:ADAR_Gen2_AAT Ti/Tv % | cds:ADAR_Gen1_AAC A>T % | cds:Gen3_AAC C>T at MC3 % |
| cds:ADAR_Gen2_AAT A:T % | cds:ADAR_Gen1_AAC T>C % | cds:Gen3_AAC G>A at MC1 % |
| cds:ADAR_Gen2_AAT Ti A:T % | cds:ADAR_Gen1_AAC T>G % | cds:Gen3_AAC G>A at MC2 % |
| cds:ADAR_Gen2_AAT non-syn % | cds:ADAR_Gen1_AAC T>A % | cds:Gen3_AAC G>A at MC3 % |
| cds:ADAR_Gen2_AAT A non-syn % | cds:ADAR_Gen1_AAC Ti/Tv % | cds:Gen3_AAC C>T % |
| cds:ADAR_Gen2_AAT T non-syn % | cds:ADAR_Gen1_AAC A:T % | cds:Gen3_AAC C>A % |
| cds:ADAR_Gen2_AAT MC1 non-syn % | cds:ADAR_Gen1_AAC Ti A:T % | cds:Gen3_AAC C>G % |
| cds:ADAR_Gen2_AAT MC2 non-syn % | cds:ADAR_Gen1_AAC non-syn % | cds:Gen3_AAC G>A % |
| cds:ADAR_Gen2_AAT MC3 non-syn % | cds:ADAR_Gen1_AAC A non-syn % | cds:Gen3_AAC G>T % |
| g:ADAR_Gen2_AAT Hits | cds:ADAR_Gen1_AAC T non-syn % | cds:Gen3_AAC G>C % |

(continued)

| Metric | Metric | Metric |
|---|---|---|
| g:ADAR_Gen2_AAT % | cds:ADAR_Gen1_AAC MC1 non-syn % | cds:Gen3_AAC Ti/Tv % |
| g:ADAR_Gen2_AAT Ti % | cds:ADAR_Gen1_AAC MC2 non-syn % | cds:Gen3_AAC C:G % |
| g:ADAR_Gen2_AAT A>G + T>C % | cds:ADAR_Gen1_AAC MC3 non-syn % | cds:Gen3_AAC Ti C:G % |
| g:ADAR_Gen2_AAT A>C + T>G | g:ADAR_Gen1_AAC Hits | cds:Gen3_AAC non-syn % |
| g:ADAR_Gen2_AAT A>T + T>A % | g:ADAR_Gen1_AAC % | cds:Gen3_AAC C non-syn % |
| nc:ADAR_Gen2_AAT Hits | g:ADAR_Gen1_AAC Ti % | cds:Gen3_AAC G non-syn % |
| nc:ADAR_Gen2_AAT % | g:ADAR_Gen1_AAC A>G + T>C % | cds:Gen3_AAC MC1 non-syn % |
| nc:ADAR_Gen2_AAT Ti % | g:ADAR_Gen1_AAC A>C + T>G % | cds:Gen3_AAC MC2 non-syn % |
| nc:ADAR_Gen2_AAT A>G + T>C % | g:ADAR_Gen1_AAC A>T + T>A % | cds:Gen3_AAC MC3 non-syn % |
| nc:ADAR_Gen2_AAT A>C + T>G % | nc:ADAR_Gen1_AAC Hits | g:Gen3_AAC Hits |
| nc:ADAR_Gen2_AAT A>T + T>A % | nc:ADAR_Gen1_AAC % | g:Gen3_AAC % |
| cds:ADAR_Gen2_TAT Hits | nc:ADAR_Gen1_AAC Ti % | g:Gen3_AAC Ti % |
| cds:ADAR_Gen2_TAT % | nc:ADAR_Gen1_AAC A>G + T>C % | g:Gen3_AAC C>T + G>A % |
| cds:ADAR_Gen2_TAT Ti % | nc:ADAR_Gen1_AAC A>C + T>G % | g:Gen3_AAC C>A + G>T % |
| cds:ADAR_Gen2_TAT MC1 % | nc:ADAR_Gen1_AAC A>T + T>A % | g:Gen3_AAC C>G + G>C % |
| cds:ADAR_Gen2_TAT MC2 % | cds:ADAR_Gen1_AAG Hits | nc:Gen3_AAC Hits |
| cds:ADAR_Gen2_TAT MC3 % | cds:ADAR_Gen1_AAG % | nc:Gen3_AAC % |
| cds:ADAR_Gen2_TAT A>G at MC1 % | cds:ADAR_Gen1_AAG Ti % | nc:Gen3_AAC Ti % |
| cds:ADAR_Gen2_TAT A>G at MC2 % | cds:ADAR_Gen1_AAG MC1 % | nc:Gen3_AAC C>T + G>A % |
| cds:ADAR_Gen2_TAT A>G at MC3 % | cds:ADAR_Gen1_AAG MC2 % | nc:Gen3_AAC C>A + G>T % |
| cds:ADAR_Gen2_TAT T>C at MC1 % | cds:ADAR_Gen1_AAG MC3 % | nc:Gen3_AAC C>G + G>C % |
| cds:ADAR_Gen2_TAT T>C at MC2 % | cds:ADAR_Gen1_AAG A>G at MC1 % | cds:Gen3_ATC Hits |
| cds:ADAR_Gen2_TAT T>C at MC3 % | cds:ADAR_Gen1_AAG A>G at MC2 % | cds:Gen3_ATC % |
| cds:ADAR_Gen2_TAT A>G % | cds:ADAR_Gen1_AAG A>G at MC3 % | cds:Gen3_ATC Ti % |
| cds:ADAR_Gen2_TAT A>C % | cds:ADAR_Gen1_AAG T>C at MC1 % | cds:Gen3_ATC MC1 % |
| cds:ADAR_Gen2_TAT A>T % | cds:ADAR_Gen1_AAG T>C at MC2 % | cds:Gen3_ATC MC2 % |
| cds:ADAR_Gen2_TAT T>C % | cds:ADAR_Gen1_AAG T>C at MC3 % | cds:Gen3_ATC MC3 % |
| cds:ADAR_Gen2_TAT T>G % | cds:ADAR_Gen1_AAG A>G % | cds:Gen3_ATC C>T at MC1 % |
| cds:ADAR_Gen2_TAT T>A % | cds:ADAR_Gen1_AAG A>C % | cds:Gen3_ATC C>T at MC2 % |
| cds:ADAR_Gen2_TAT Ti/Tv % | cds:ADAR_Gen1_AAG A>T % | cds:Gen3_ATC C>T at MC3 % |

(continued)

| Metric | Metric | Metric |
|---|---|---|
| cds:ADAR_Gen2_TAT A:T % | cds:ADAR_Gen1_AAG T>C % | cds:Gen3_ATC G>A at MC1 % |
| cds:ADAR_Gen2_TAT Ti A:T % | cds:ADAR_Gen1_AAG T>G % | cds:Gen3_ATC G>A at MC2 % |
| cds:ADAR_Gen2_TAT non-syn % | cds:ADAR_Gen1_AAG T>A % | cds:Gen3_ATC G>A at MC3 % |
| cds:ADAR_Gen2_TAT A non-syn % | cds:ADAR_Gen1_AAG Ti/Tv % | cds:Gen3_ATC C>T % |
| cds:ADAR_Gen2_TAT T non-syn % | cds:ADAR_Gen1_AAG A:T % | cds:Gen3_ATC C>A % |
| cds:ADAR_Gen2_TAT MC1 non-syn % | cds:ADAR_Gen1_AAG Ti A:T % | cds:Gen3_ATC C>G % |
| cds:ADAR_Gen2_TAT MC2 non-syn % | cds:ADAR_Gen1_AAG non-syn % | cds:Gen3_ATC G>A % |
| cds:ADAR_Gen2_TAT MC3 non-syn % | cds:ADAR_Gen1_AAG A non-syn % | cds:Gen3_ATC G>T % |
| g:ADAR_Gen2_TAT Hits | cds:ADAR_Gen1_AAG T non-syn % | cds:Gen3_ATC G>C % |
| g:ADAR_Gen2_TAT % | cds:ADAR_Gen1_AAG MC1 non-syn % | cds:Gen3_ATC Ti/Tv % |
| g:ADAR_Gen2_TAT Ti % | cds:ADAR_Gen1_AAG MC2 non-syn % | cds:Gen3_ATC C:G % |
| g:ADAR_Gen2_TAT A>G + T>C % | cds:ADAR_Gen1_AAG MC3 non-syn % | cds:Gen3_ATC Ti C:G % |
| g:ADAR_Gen2_TAT A>C + T>G % | g:ADAR_Gen1_AAG Hits | cds:Gen3_ATC non-syn % |
| g:ADAR_Gen2_TAT A>T + T>A % | g:ADAR_Gen1_AAG % | cds:Gen3_ATC C non-syn % |
| nc:ADAR_Gen2_TAT Hits | g:ADAR_Gen1_AAG Ti % | cds:Gen3_ATC G non-syn % |
| nc:ADAR_Gen2_TAT % | g:ADAR_Gen1_AAG A>G + T>C % | cds:Gen3_ATC MC1 non-syn % |
| nc:ADAR_Gen2_TAT Ti % | g:ADAR_Gen1_AAG A>C + T>G % | cds:Gen3_ATC MC2 non-syn % |
| nc:ADAR_Gen2_TAT A>G + T>C % | g:ADAR_Gen1_AAG A>T + T>A % | cds:Gen3_ATC MC3 non-syn % |
| nc:ADAR_Gen2_TAT A>C + T>G % | nc:ADAR_Gen1_AAG Hits | g:Gen3_ATC Hits |
| nc:ADAR_Gen2_TAT A>T + T>A % | nc:ADAR_Gen1_AAG % | g:Gen3_ATC % |
| cds:ADAR_Gen2_CAT Hits | nc:ADAR_Gen1_AAG Ti % | g:Gen3_ATC Ti % |
| cds:ADAR_Gen2_CAT % | nc:ADAR_Gen1_AAG A>G + T>C % | g:Gen3_ATC C>T + G>A % |
| cds:ADAR_Gen2_CAT Ti % | nc:ADAR_Gen1_AAG A>C + T>G % | g:Gen3_ATC C>A + G>T % |
| cds:ADAR_Gen2_CAT MC1 % | nc:ADAR_Gen1_AAG A>T + T>A % | g:Gen3_ATC C>G + G>C % |
| cds:ADAR_Gen2_CAT MC2 % | cds:ADAR_Gen1_ATA Hits | nc:Gen3_ATC Hits |
| cds:ADAR_Gen2_CAT MC3 % | cds:ADAR_Gen1_ATA % | nc:Gen3_ATC % |
| cds:ADAR_Gen2_CAT A>G at MC1 % | cds:ADAR_Gen1_ATA Ti % | nc:Gen3_ATC Ti % |
| cds:ADAR_Gen2_CAT A>G at MC2 % | cds:ADAR_Gen1_ATA MC1 % | nc:Gen3_ATC C>T + G>A % |
| cds:ADAR_Gen2_CAT A>G at MC3 % | cds:ADAR_Gen1_ATA MC2 % | nc:Gen3_ATC C>A + G>T % |
| cds:ADAR_Gen2_CAT T>C at MC1 % | cds:ADAR_Gen1_ATA MC3 % | nc:Gen3_ATC C>G + G>C % |
| cds:ADAR_Gen2_CAT T>C at MC2 % | cds:ADAR_Gen1_ATA A>G at MC1 % | cds:Gen3_ACC Hits |

(continued)

| Metric | Metric | Metric |
|---|---|---|
| cds:ADAR_Gen2_CAT T>C at MC3 % | cds:ADAR_Gen1_ATA A>G at MC2 % | cds:Gen3_ACC % |
| cds:ADAR_Gen2_CAT A>G % | cds:ADAR_Gen1_ATA A>G at MC3 % | cds:Gen3_ACC Ti % |
| cds:ADAR_Gen2_CAT A>C % | cds:ADAR_Gen1_ATA T>C at MC1 % | cds:Gen3_ACC MC1 % |
| cds:ADAR_Gen2_CAT A>T % | cds:ADAR_Gen1_ATA T>C at MC2 % | cds:Gen3_ACC MC2 % |
| cds:ADAR_Gen2_CAT T>C % | cds:ADAR_Gen1_ATA T>C at MC3 % | cds:Gen3_ACC MC3 % |
| cds:ADAR_Gen2_CAT T>G % | cds:ADAR_Gen1_ATA A>G % | cds:Gen3_ACC C>T at MC1 % |
| cds:ADAR_Gen2_CAT T>A % | cds:ADAR_Gen1_ATA A>C % | cds:Gen3_ACC C>T at MC2 % |
| cds:ADAR_Gen2_CAT Ti/Tv % | cds:ADAR_Gen1_ATA A>T % | cds:Gen3_ACC C>T at MC3 % |
| cds:ADAR_Gen2_CAT A:T % | cds:ADAR_Gen1_ATA T>C % | cds:Gen3_ACC G>A at MC1 % |
| cds:ADAR_Gen2_CAT Ti A:T % | cds:ADAR_Gen1_ATA T>G % | cds:Gen3_ACC G>A at MC2 % |
| cds:ADAR_Gen2_CAT non-syn % | cds:ADAR_Gen1_ATA T>A % | cds:Gen3_ACC G>A at MC3 % |
| cds:ADAR_Gen2_CAT A non-syn % | cds:ADAR_Gen1_ATA Ti/Tv % | cds:Gen3_ACC C>T % |
| cds:ADAR_Gen2_CAT T non-syn % | cds:ADAR_Gen1_ATA A:T % | cds:Gen3_ACC C>A % |
| cds:ADAR_Gen2_CAT MC1 non-syn % | cds:ADAR_Gen1_ATA Ti A:T % | cds:Gen3_ACC C>G % |
| cds:ADAR_Gen2_CAT MC2 non-syn % | cds:ADAR_Gen1_ATA non-syn % | cds:Gen3_ACC G>A % |
| cds:ADAR_Gen2_CAT MC3 non-syn % | cds:ADAR_Gen1_ATA A non-syn % | cds:Gen3_ACC G>T % |
| g:ADAR_Gen2_CAT Hits | cds:ADAR_Gen1_ATA T non-syn % | cds:Gen3_ACC G>C % |
| g:ADAR_Gen2_CAT % | cds:ADAR_Gen1_ATA MC1 non-syn % | cds:Gen3_ACC Ti/Tv % |
| g:ADAR_Gen2_CAT Ti % | cds:ADAR_Gen1_ATA MC2 non-syn % | cds:Gen3_ACC C:G % |
| g:ADAR_Gen2_CAT A>G + T>C % | cds:ADAR_Gen1_ATA MC3 non-syn % | cds:Gen3_ACC Ti C:G % |
| g:ADAR_Gen2_CAT A>C + T>G % | g:ADAR_Gen1_ATA Hits | cds:Gen3_ACC non-syn % |
| g:ADAR_Gen2_CAT A>T + T>A % | g:ADAR_Gen1_ATA % | cds:Gen3_ACC C non-syn % |
| nc:ADAR_Gen2_CAT Hits | g:ADAR_Gen1_ATA Ti % | cds:Gen3_ACC G non-syn % |
| nc:ADAR_Gen2_CAT % | g:ADAR_Gen1_ATA A>G + T>C % | cds:Gen3_ACC MC1 non-syn % |
| nc:ADAR_Gen2_CAT Ti % | g:ADAR_Gen1_ATA A>C + T>G % | cds:Gen3_ACC MC2 non-syn % |
| nc:ADAR_Gen2_CAT A>G + T>C % | g:ADAR_Gen1_ATA A>T + T>A % | cds:Gen3_ACC MC3 non-syn % |
| nc:ADAR_Gen2_CAT A>C + T>G % | nc:ADAR_Gen1_ATA Hits | g:Gen3_ACC Hits |
| nc:ADAR_Gen2_CAT A>T + T>A % | nc:ADAR_Gen1_ATA % | g:Gen3_ACC % |
| cds:ADAR_Gen2_GAT Hits | nc:ADAR_Gen1_ATA Ti % | g:Gen3_ACC Ti % |
| cds:ADAR_Gen2_GAT % | nc:ADAR_Genl_ATA A>G + T>C % | g:Gen3_ACC C>T + G>A % |
| cds:ADAR_Gen2_GAT Ti % | nc:ADAR_Gen1_ATA A>C + T>G % | g:Gen3_ACC C>A + G>T % |

(continued)

| Metric | Metric | Metric |
|---|---|---|
| cds:ADAR_Gen2_GAT MC1 % | nc:ADAR_Gen1_ATA A>T + T>A % | g:Gen3_ACC C>G + G>C % |
| cds:ADAR_Gen2_GAT MC2 % | cds:ADAR_Gen1_ATT Hits | nc:Gen3_ACC Hits |
| cds:ADAR_Gen2_GAT MC3 % | cds:ADAR_Gen1_ATT % | nc:Gen3_ACC % |
| cds:ADAR_Gen2_GAT A>G at MC1 % | cds:ADAR_Gen1_ATT Ti % | nc:Gen3_ACC Ti % |
| cds:ADAR_Gen2_GAT A>G at MC2 % | cds:ADAR_Gen1_ATT MC1 % | nc:Gen3_ACC C>T + G>A % |
| cds:ADAR_Gen2_GAT A>G at MC3 % | cds:ADAR_Gen1_ATT MC2 % | nc:Gen3_ACC C>A + G>T % |
| cds:ADAR_Gen2_GAT T>C at MC1 % | cds:ADAR_Gen1_ATT MC3 % | nc:Gen3_ACC C>G + G>C % |
| cds:ADAR_Gen2_GAT T>C at MC2 % | cds:ADAR_Gen1_ATT A>G at MC1 % | cds:Gen3_AGC Hits |
| cds:ADAR_Gen2_GAT T>C at MC3 % | cds:ADAR_Gen1_ATT A>G at MC2 % | cds:Gen3_AGC % |
| cds:ADAR_Gen2_GAT A>G % | cds:ADAR_Gen1_ATT A>G at MC3 % | cds:Gen3_AGC Ti % |
| cds:ADAR_Gen2_GAT A>C % | cds:ADAR_Gen1_ATT T>C at MC1 % | cds:Gen3_AGC MC1 % |
| cds:ADAR_Gen2_GAT A>T % | cds:ADAR_Gen1_ATT T>C at MC2 % | cds:Gen3_AGC MC2 % |
| cds:ADAR_Gen2_GAT T>C % | cds:ADAR_Gen1_ATT T>C at MC3 % | cds:Gen3_AGC MC3 % |
| cds:ADAR_Gen2_GAT T>G % | cds:ADAR_Gen1_ATT A>G % | cds:Gen3_AGC C>T at MC1 % |
| cds:ADAR_Gen2_GAT T>A % | cds:ADAR_Gen1_ATT A>C % | cds:Gen3_AGC C>T at MC2 % |
| cds:ADAR_Gen2_GAT Ti/Tv % | cds:ADAR_Gen1_ATT A>T % | cds:Gen3_AGC C>T at MC3 % |
| cds:ADAR_Gen2_GAT A:T % | cds:ADAR_Gen1_ATT T>C % | cds:Gen3_AGC G>A at MC1 % |
| cds:ADAR_Gen2_GAT Ti A:T % | cds:ADAR_Gen1_ATT T>G % | cds:Gen3_AGC G>A at MC2 % |
| cds:ADAR_Gen2_GAT non-syn % | cds:ADAR_Gen1_ATT T>A % | cds:Gen3_AGC G>A at MC3 % |
| cds:ADAR_Gen2_GAT A non-syn % | cds:ADAR_Gen1_ATT Ti/Tv % | cds:Gen3_AGC C>T % |
| cds:ADAR_Gen2_GAT T non-syn % | cds:ADAR_Gen1_ATT A:T % | cds:Gen3_AGC C>A % |
| cds:ADAR_Gen2_GAT MC1 non-syn % | cds:ADAR_Gen1_ATT Ti A:T % | cds:Gen3_AGC C>G % |
| cds:ADAR_Gen2_GAT MC2 non-syn % | cds:ADAR_Gen1_ATT non-syn % | cds:Gen3_AGC G>A % |
| cds:ADAR_Gen2_GAT MC3 non-syn % | cds:ADAR_Gen1_ATT A non-syn % | cds:Gen3_AGC G>T % |
| g:ADAR_Gen2_GAT Hits | cds:ADAR_Gen1_ATT T non-syn % | cds:Gen3_AGC G>C % |
| g:ADAR_Gen2_GAT % | cds:ADAR_Gen1_ATT MC1 non-syn % | cds:Gen3_AGC Ti/Tv % |
| g:ADAR_Gen2_GAT Ti % | cds:ADAR_Gen1_ATT MC2 non-syn % | cds:Gen3_AGC C:G % |
| g:ADAR_Gen2_GAT A>G + T>C % | cds:ADAR_Gen1_ATT MC3 non-syn % | cds:Gen3_AGC Ti C:G % |

(continued)

| Metric | Metric | Metric |
|---|---|---|
| g:ADAR_Gen2_GAT A>C + T>G % | g:ADAR_Gen1_ATT Hits | cds:Gen3_AGC non-syn % |
| g:ADAR_Gen2_GAT A>T + T>A % | g:ADAR_Gen1_ATT % | cds:Gen3_AGC C non-syn % |
| nc:ADAR_Gen2_GAT Hits | g:ADAR_Gen1_ATT Ti % | cds:Gen3_AGC G non-syn % |
| nc:ADAR_Gen2_GAT % | g:ADAR_Gen1_ATT A>G + T>C % | cds:Gen3_AGC MC1 non-syn % |
| nc:ADAR_Gen2_GAT Ti % | g:ADAR_Gen1_ATT A>C + T>G % | cds:Gen3_AGC MC2 non-syn % |
| nc:ADAR_Gen2_GAT A>G + T>C % | g:ADAR_Gen1_ATT A>T + T>A % | cds:Gen3_AGC MC3 non-syn % |
| nc:ADAR_Gen2_GAT A>C + T>G % | nc:ADAR_Gen1_ATT Hits | g:Gen3_AGC Hits |
| nc:ADAR_Gen2_GAT A>T + T>A % | nc:ADAR_Gen1_ATT % | g:Gen3_AGC % |
| cds:ADAR_Gen2_AAC Hits | nc:ADAR_Gen1_ATT Ti % | g:Gen3_AGC Ti % |
| cds:ADAR_Gen2_AAC % | nc:ADAR_Gen1_ATT A>G + T>C % | g:Gen3_AGC C>T + G>A % |
| cds:ADAR_Gen2_AAC Ti % | nc:ADAR_Gen1_ATT A>C + T>G % | g:Gen3_AGC C>A + G>T % |
| cds:ADAR_Gen2_AAC MC1 % | nc:ADAR_Gen1_ATT A>T + T>A % | g:Gen3_AGC C>G + G>C % |
| cds:ADAR_Gen2_AAC MC2 % | cds:ADAR_Gen1_ATC Hits | nc:Gen3_AGC Hits |
| cds:ADAR_Gen2_AAC MC3 % | cds:ADAR_Gen1_ATC % | nc:Gen3_AGC % |
| cds:ADAR_Gen2_AAC A>G at MC1 % | cds:ADAR_Gen1_ATC Ti % | nc:Gen3_AGC Ti % |
| cds:ADAR_Gen2_AAC A>G at MC2 % | cds:ADAR_Gen1_ATC MC1 % | nc:Gen3_AGC C>T + G>A % |
| cds:ADAR_Gen2_AAC A>G at MC3 % | cds:ADAR_Gen1_ATC MC2 % | nc:Gen3_AGC C>A + G>T % |
| cds:ADAR_Gen2_AAC T>C at MC1 % | cds:ADAR_Gen1_ATC MC3 % | nc:Gen3_AGC C>G + G>C % |
| cds:ADAR_Gen2_AAC T>C at MC2 % | cds:ADAR_Gen1_ATC A>G at MC1 % | cds:Gen3_TAC Hits |
| cds:ADAR_Gen2_AAC T>C at MC3 % | cds:ADAR_Gen1_ATC A>G at MC2 % | cds:Gen3_TAC % |
| cds:ADAR_Gen2_AAC A>G % | cds:ADAR_Gen1_ATC A>G at MC3 % | cds:Gen3_TAC Ti % |
| cds:ADAR_Gen2_AAC A>C % | cds:ADAR_Gen1_ATC T>C at MC1 % | cds:Gen3_TAC MC1 % |
| cds:ADAR_Gen2_AAC A>T % | cds:ADAR_Gen1_ATC T>C at MC2 % | cds:Gen3_TAC MC2 % |
| cds:ADAR_Gen2_AAC T>C % | cds:ADAR_Gen1_ATC T>C at MC3 % | cds:Gen3_TAC MC3 % |
| cds:ADAR_Gen2_AAC T>G % | cds:ADAR_Gen1_ATC A>G % | cds:Gen3_TAC C>T at MC1 % |
| cds:ADAR_Gen2_AAC T>A % | cds:ADAR_Gen1_ATC A>C % | cds:Gen3_TAC C>T at MC2 % |
| cds:ADAR_Gen2_AAC Ti/Tv % | cds:ADAR_Gen1_ATC A>T % | cds:Gen3_TAC C>T at MC3 % |
| cds:ADAR_Gen2_AAC A:T % | cds:ADAR_Gen1_ATC T>C % | cds:Gen3_TAC G>A at MC1 % |
| cds:ADAR_Gen2_AAC Ti A:T % | cds:ADAR_Gen1_ATC T>G % | cds:Gen3_TAC G>A at MC2 % |
| cds:ADAR_Gen2_AAC non-syn % | cds:ADAR_Gen1_ATC T>A % | cds:Gen3_TAC G>A at MC3 % |
| cds:ADAR_Gen2_AAC A non-syn % | cds:ADAR_Gen1_ATC Ti/Tv % | cds:Gen3_TAC C>T % |
| cds:ADAR_Gen2_AAC T non-syn % | cds:ADAR_Gen1_ATC A:T % | cds:Gen3_TAC C>A % |

(continued)

| Metric | Metric | Metric |
|---|---|---|
| cds:ADAR_Gen2_AAC MC1 non-syn % | cds:ADAR_Gen1_ATC Ti A:T % | cds:Gen3_TAC C>G % |
| cds:ADAR_Gen2_AAC MC2 non-syn % | cds:ADAR_Gen1_ATC non-syn % | cds:Gen3_TAC G>A % |
| cds:ADAR_Gen2_AAC MC3 non-syn % | cds:ADAR_Gen1_ATC A non-syn % | cds:Gen3_TAC G>T % |
| g:ADAR_Gen2_AAC Hits | cds:ADAR_Gen1_ATC T non-syn % | cds:Gen3_TAC G>C % |
| g:ADAR_Gen2_AAC % | cds:ADAR_Gen1_ATC MC1 non-syn % | cds:Gen3_TAC Ti/Tv % |
| g:ADAR_Gen2_AAC Ti % | cds:ADAR_Gen1_ATC MC2 non-syn % | cds:Gen3_TAC C:G % |
| g:ADAR_Gen2_AAC A>G + T>C % | cds:ADAR_Gen1_ATC MC3 non-syn % | cds:Gen3_TAC Ti C:G % |
| g:ADAR_Gen2_AAC A>C + T>G % | g:ADAR_Gen1_ATC Hits | cds:Gen3_TAC non-syn % |
| g:ADAR_Gen2_AAC A>T + T>A % | g:ADAR_Gen1_ATC % | cds:Gen3_TAC C non-syn % |
| nc:ADAR_Gen2_AAC Hits | g:ADAR_Gen1_ATC Ti % | cds:Gen3_TAC G non-syn % |
| nc:ADAR_Gen2_AAC % | g:ADAR_Gen1_ATC A>G + T>C % | cds:Gen3_TAC MC1 non-syn % |
| nc:ADAR_Gen2_AAC Ti % | g:ADAR_Gen1_ATC A>C + T>G % | cds:Gen3_TAC MC2 non-syn % |
| nc:ADAR_Gen2_AAC A>G + T>C % | g:ADAR_Gen1_ATC A>T + T>A % | cds:Gen3_TAC MC3 non-syn % |
| nc:ADAR_Gen2_AAC A>C + T>G % | nc:ADAR_Gen1_ATC Hits | g:Gen3_TAC Hits |
| nc:ADAR_Gen2_AAC A>T + T>A % | nc:ADAR_Gen1_ATC % | g:Gen3_TAC % |
| cds:ADAR_Gen2_TAC Hits | nc:ADAR_Gen1_ATC Ti % | g:Gen3_TAC Ti % |
| cds:ADAR_Gen2_TAC % | nc:ADAR_Gen1_ATC A>G + T>C % | g:Gen3_TAC C>T + G>A % |
| cds:ADAR_Gen2_TAC Ti % | nc:ADAR_Gen1_ATC A>C + T>G % | g:Gen3_TAC C>A + G>T % |
| cds:ADAR_Gen2_TAC MC1 % | nc:ADAR_Gen1_ATC A>T + T>A % | g:Gen3_TAC C>G + G>C % |
| cds:ADAR_Gen2_TAC MC2 % | cds:ADAR_Gen1_ATG Hits | nc:Gen3_TAC Hits |
| cds:ADAR_Gen2_TAC MC3 % | cds:ADAR_Gen1_ATG % | nc:Gen3_TAC % |
| cds:ADAR_Gen2_TAC A>G at MC1 % | cds:ADAR_Gen1_ATG Ti % | nc:Gen3_TAC Ti % |
| cds:ADAR_Gen2_TAC A>G at MC2 % | cds:ADAR_Gen1_ATG MC1 % | nc:Gen3_TAC C>T + G>A % |
| cds:ADAR_Gen2_TAC A>G at MC3 % | cds:ADAR_Gen1_ATG MC2 % | nc:Gen3_TAC C>A + G>T % |
| cds:ADAR_Gen2_TAC T>C at MC1 % | cds:ADAR_Gen1_ATG MC3 % | nc:Gen3_TAC C>G + G>C % |
| cds:ADAR_Gen2_TAC T>C at MC2 % | cds:ADAR_Gen1_ATG A>G at MC1 % | cds:Gen3_TTC Hits |
| cds:ADAR_Gen2_TAC T>C at MC3 % | cds:ADAR_Gen1_ATG A>G at MC2 % | cds:Gen3_TTC % |
| cds:ADAR_Gen2_TAC A>G % | cds:ADAR_Gen1_ATG A>G at MC3 % | cds:Gen3_TTC Ti % |
| cds:ADAR_Gen2_TAC A>C % | cds:ADAR_Gen1_ATG T>C at MC1 % | cds:Gen3_TTC MC1 % |

(continued)

| Metric | Metric | Metric |
|---|---|---|
| cds:ADAR_Gen2_TAC A>T % | cds:ADAR_Gen1_ATG T>C at MC2 % | cds:Gen3_TTC MC2 % |
| cds:ADAR_Gen2_TAC T>C % | cds:ADAR_Gen1_ATG T>C at MC3 % | cds:Gen3_TTC MC3 % |
| cds:ADAR_Gen2_TAC T>G % | cds:ADAR_Gen1_ATG A>G % | cds:Gen3_TTC C>T at MC1 % |
| cds:ADAR_Gen2_TAC T>A % | cds:ADAR_Gen1_ATG A>C % | cds:Gen3_TTC C>T at MC2 % |
| cds:ADAR_Gen2_TAC Ti/Tv % | cds:ADAR_Gen1_ATG A>T % | cds:Gen3_TTC C>T at MC3 % |
| cds:ADAR_Gen2_TAC A:T % | cds:ADAR_Gen1_ATG T>C % | cds:Gen3_TTC G>A at MC1 % |
| cds:ADAR_Gen2_TAC Ti A:T % | cds:ADAR_Gen1_ATG T>G % | cds:Gen3_TTC G>A at MC2 % |
| cds:ADAR_Gen2_TAC non-syn % | cds:ADAR_Gen1_ATG T>A % | cds:Gen3_TTC G>A at MC3 % |
| cds:ADAR_Gen2_TAC A non-syn % | cds:ADAR_Gen1_ATG Ti/Tv % | cds:Gen3_TTC C>T % |
| cds:ADAR_Gen2_TAC T non-syn % | cds:ADAR_Gen1_ATG A:T % | cds:Gen3_TTC C>A % |
| cds:ADAR_Gen2_TAC MC1 non-syn % | cds:ADAR_Gen1_ATG Ti A:T % | cds:Gen3_TTC C>G % |
| cds:ADAR_Gen2_TAC MC2 non-syn % | cds:ADAR_Gen1_ATG non-syn % | cds:Gen3_TTC G>A % |
| cds:ADAR_Gen2_TAC MC3 non-syn % | cds:ADAR_Gen1_ATG A non-syn % | cds:Gen3_TTC G>T % |
| g:ADAR_Gen2_TAC Hits | cds:ADAR_Gen1_ATG T non-syn % | cds:Gen3_TTC G>C % |
| g:ADAR_Gen2_TAC % | cds:ADAR_Gen1_ATG MC1 non-syn % | cds:Gen3_TTC Ti/Tv % |
| g:ADAR_Gen2_TAC Ti % | cds:ADAR_Gen1_ATG MC2 non-syn % | cds:Gen3_TTC C:G % |
| g:ADAR_Gen2_TAC A>G + T>C % | cds:ADAR_Gen1_ATG MC3 non-syn % | cds:Gen3_TTC Ti C:G % |
| g:ADAR_Gen2_TAC A>C + T>G % | g:ADAR_Gen1_ATG Hits | cds:Gen3_TTC non-syn % |
| g:ADAR_Gen2_TAC A>T + T>A % | g:ADAR_Gen1_ATG % | cds:Gen3_TTC C non-syn % |
| nc:ADAR_Gen2_TAC Hits | g:ADAR_Gen1_ATG Ti % | cds:Gen3_TTC G non-syn % |
| nc:ADAR_Gen2_TAC % | g:ADAR_Gen1_ATG A>G + T>C % | cds:Gen3_TTC MC1 non-syn % |
| nc:ADAR_Gen2_TAC Ti % | g:ADAR_Gen1_ATG A>C + T>G % | cds:Gen3_TTC MC2 non-syn % |
| nc:ADAR_Gen2_TAC A>G + T>C % | g:ADAR_Gen1_ATG A>T + T>A % | cds:Gen3_TTC MC3 non-syn % |
| nc:ADAR_Gen2_TAC A>C + T>G % | nc:ADAR_Gen1_ATG Hits | g:Gen3_TTC Hits |
| nc:ADAR_Gen2_TAC A>T + T>A % | nc:ADAR_Gen1_ATG % | g:Gen3_TTC % |
| cds:ADAR_Gen2_CAC Hits | nc:ADAR_Gen1_ATG Ti % | g:Gen3_TTC Ti % |
| cds:ADAR_Gen2_CAC % | nc:ADAR_Gen1_ATG A>G + T>C % | g:Gen3_TTC C>T + G>A % |
| cds:ADAR_Gen2_CAC Ti % | nc:ADAR_Gen1_ATG A>C + T>G % | g:Gen3_TTC C>A + G>T % |
| cds:ADAR_Gen2_CAC MC1 % | nc:ADAR_Gen1_ATG A>T + T>A % | g:Gen3_TTC C>G + G>C % |
| cds:ADAR_Gen2_CAC MC2 % | cds:ADAR_Gen1_ACA Hits | nc:Gen3_TTC Hits |
| cds:ADAR_Gen2_CAC MC3 % | cds:ADAR_Gen1_ACA % | nc:Gen3_TTC % |
| cds:ADAR_Gen2_CAC A>G at MC1 % | cds:ADAR_Gen1_ACA Ti % | nc:Gen3_TTC Ti % |

(continued)

| Metric | Metric | Metric |
|---|---|---|
| cds:ADAR_Gen2_CAC A>G at MC2 % | cds:ADAR_Gen1_ACA MC1 % | nc:Gen3_TTC C>T + G>A % |
| cds:ADAR_Gen2_CAC A>G at MC3 % | cds:ADAR_Gen1_ACA MC2 % | nc:Gen3_TTC C>A + G>T % |
| cds:ADAR_Gen2_CAC T>C at MC1 % | cds:ADAR_Gen1_ACA MC3 % | nc:Gen3_TTC C>G + G>C % |
| cds:ADAR_Gen2_CAC T>C at MC2 % | cds:ADAR_Gen1_ACA A>G at MC1 % | cds:Gen3_TCC Hits |
| cds:ADAR_Gen2_CAC T>C at MC3 % | cds:ADAR_Gen1_ACA A>G at MC2 % | cds:Gen3_TCC % |
| cds:ADAR_Gen2_CAC A>G % | cds:ADAR_Gen1_ACA A>G at MC3 % | cds:Gen3_TCC Ti % |
| cds:ADAR_Gen2_CAC A>C % | cds:ADAR_Gen1_ACA T>C at MC1 % | cds:Gen3_TCC MC1 % |
| cds:ADAR_Gen2_CAC A>T % | cds:ADAR_Gen1_ACA T>C at MC2 % | cds:Gen3_TCC MC2 % |
| cds:ADAR_Gen2_CAC T>C % | cds:ADAR_Gen1_ACA T>C at MC3 % | cds:Gen3_TCC MC3 % |
| cds:ADAR_Gen2_CAC T>G % | cds:ADAR_Gen1_ACA A>G % | cds:Gen3_TCC C>T at MC1 % |
| cds:ADAR_Gen2_CAC T>A % | cds:ADAR_Gen1_ACA A>C % | cds:Gen3_TCC C>T at MC2 % |
| cds:ADAR_Gen2_CAC Ti/Tv % | cds:ADAR_Gen1_ACA A>T % | cds:Gen3_TCC C>T at MC3 % |
| cds:ADAR_Gen2_CAC A:T % | cds:ADAR_Gen1_ACA T>C % | cds:Gen3_TCC G>A at MC1 % |
| cds:ADAR_Gen2_CAC Ti A:T % | cds:ADAR_Gen1_ACA T>G % | cds:Gen3_TCC G>A at MC2 % |
| cds:ADAR_Gen2_CAC non-syn % | cds:ADAR_Gen1_ACA T>A % | cds:Gen3_TCC G>A at MC3 % |
| cds:ADAR_Gen2_CAC A non-syn % | cds:ADAR_Gen1_ACA Ti/Tv % | cds:Gen3_TCC C>T % |
| cds:ADAR_Gen2_CAC T non-syn % | cds:ADAR_Gen1_ACA A:T % | cds:Gen3_TCC C>A % |
| cds:ADAR_Gen2_CAC MC1 non-syn % | cds:ADAR_Gen1_ACA Ti A:T % | cds:Gen3_TCC C>G % |
| cds:ADAR_Gen2_CAC MC2 non-syn % | cds:ADAR_Gen1_ACA non-syn % | cds:Gen3_TCC G>A % |
| cds:ADAR_Gen2_CAC MC3 non-syn % | cds:ADAR_Gen1_ACA A non-syn % | cds:Gen3_TCC G>T % |
| g:ADAR_Gen2_CAC Hits | cds:ADAR_Gen1_ACA T non-syn % | cds:Gen3_TCC G>C % |
| g:ADAR_Gen2_CAC % | cds:ADAR_Gen1_ACA MC1 non-syn % | cds:Gen3_TCC Ti/Tv % |
| g:ADAR_Gen2_CAC Ti % | cds:ADAR_Gen1_ACA MC2 non-syn % | cds:Gen3_TCC C:G % |
| g:ADAR_Gen2_CAC A>G + T>C % | cds:ADAR_Gen1_ACA MC3 non-syn % | cds:Gen3_TCC Ti C:G % |
| g:ADAR_Gen2_CAC A>C + T>G % | g:ADAR_Gen1_ACA Hits | cds:Gen3_TCC non-syn % |
| g:ADAR_Gen2_CAC A>T + T>A % | g:ADAR_Gen1_ACA % | cds:Gen3_TCC C non-syn % |
| nc:ADAR_Gen2_CAC Hits | g:ADAR_Gen1_ACA Ti % | cds:Gen3_TCC G non-syn % |
| nc:ADAR_Gen2_CAC % | g:ADAR_Gen1_ACA A>G + T>C % | cds:Gen3_TCC MC1 non-syn % |

(continued)

| Metric | Metric | Metric |
|---|---|---|
| nc:ADAR_Gen2_CAC Ti % | g:ADAR_Gen1_ACA A>C + T>G % | cds:Gen3_TCC MC2 non-syn % |
| nc:ADAR_Gen2_CAC A>G + T>C % | g:ADAR_Gen1_ACA A>T + T>A % | cds:Gen3_TCC MC3 non-syn % |
| nc:ADAR_Gen2_CAC A>C + T>G % | nc:ADAR_Gen1_ACA Hits | g:Gen3_TCC Hits |
| nc:ADAR_Gen2_CAC A>T + T>A % | nc:ADAR_Gen1_ACA % | g:Gen3_TCC % |
| cds:ADAR_Gen2_GAC Hits | nc:ADAR_Gen1_ACA Ti % | g:Gen3_TCC Ti % |
| cds:ADAR_Gen2_GAC % | nc:ADAR_Genl_ACA A>G + T>C % | g:Gen3_TCC C>T + G>A % |
| cds:ADAR_Gen2_GAC Ti % | nc:ADAR_Gen1_ACA A>C + T>G % | g:Gen3_TCC C>A + G>T % |
| cds:ADAR_Gen2_GAC MC1 % | nc:ADAR_Gen1_ACA A>T + T>A % | g:Gen3_TCC C>G + G>C % |
| cds:ADAR_Gen2_GAC MC2 % | cds:ADAR_Gen1_ACT Hits | nc:Gen3_TCC Hits |
| cds:ADAR_Gen2_GAC MC3 % | cds:ADAR_Gen1_ACT % | nc:Gen3_TCC % |
| cds:ADAR_Gen2_GAC A>G at MC1 % | cds:ADAR_Gen1_ACT Ti % | nc:Gen3_TCC Ti % |
| cds:ADAR_Gen2_GAC A>G at MC2 % | cds:ADAR_Gen1_ACT MC1 % | nc:Gen3_TCC C>T + G>A % |
| cds:ADAR_Gen2_GAC A>G at MC3 % | cds:ADAR_Gen1_ACT MC2 % | nc:Gen3_TCC C>A + G>T % |
| cds:ADAR_Gen2_GAC T>C at MC1 % | cds:ADAR_Gen1_ACT MC3 % | nc:Gen3_TCC C>G + G>C % |
| cds:ADAR_Gen2_GAC T>C at MC2 % | cds:ADAR_Gen1_ACT A>G at MC1 % | cds:Gen3_TGC Hits |
| cds:ADAR_Gen2_GAC T>C at MC3 % | cds:ADAR_Gen1_ACT A>G at MC2 % | cds:Gen3_TGC % |
| cds:ADAR_Gen2_GAC A>G % | cds:ADAR_Gen1_ACT A>G at MC3 % | cds:Gen3_TGC Ti % |
| cds:ADAR_Gen2_GAC A>C % | cds:ADAR_Gen1_ACT T>C at MC1 % | cds:Gen3_TGC MC1 % |
| cds:ADAR_Gen2_GAC A>T % | cds:ADAR_Gen1_ACT T>C at MC2 % | cds:Gen3_TGC MC2 % |
| cds:ADAR_Gen2_GAC T>C % | cds:ADAR_Gen1_ACT T>C at MC3 % | cds:Gen3_TGC MC3 % |
| cds:ADAR_Gen2_GAC T>G % | cds:ADAR_Gen1_ACT A>G % | cds:Gen3_TGC C>T at MC1 % |
| cds:ADAR_Gen2_GAC T>A % | cds:ADAR_Gen1_ACT A>C % | cds:Gen3_TGC C>T at MC2 % |
| cds:ADAR_Gen2_GAC Ti/Tv % | cds:ADAR_Gen1_ACT A>T % | cds:Gen3_TGC C>T at MC3 % |
| cds:ADAR_Gen2_GAC A:T % | cds:ADAR_Gen1_ACT T>C % | cds:Gen3_TGC G>A at MC1 % |
| cds:ADAR_Gen2_GAC Ti A:T % | cds:ADAR_Gen1_ACT T>G % | cds:Gen3_TGC G>A at MC2 % |
| cds:ADAR_Gen2_GAC non-syn % | cds:ADAR_Gen1_ACT T>A % | cds:Gen3_TGC G>A at MC3 % |
| cds:ADAR_Gen2_GAC A non-syn % | cds:ADAR_Gen1_ACT Ti/Tv % | cds:Gen3_TGC C>T % |
| cds:ADAR_Gen2_GAC T non-syn % | cds:ADAR_Gen1_ACT A:T % | cds:Gen3_TGC C>A % |
| cds:ADAR_Gen2_GAC MC1 non-syn % | cds:ADAR_Gen1_ACT Ti A:T % | cds:Gen3_TGC C>G % |

(continued)

| Metric | Metric | Metric |
|---|---|---|
| cds:ADAR_Gen2_GAC MC2 non-syn % | cds:ADAR_Gen1_ACT non-syn % | cds:Gen3_TGC G>A % |
| cds:ADAR_Gen2_GAC MC3 non-syn % | cds:ADAR_Gen1_ACT A non-syn % | cds:Gen3_TGC G>T % |
| g:ADAR_Gen2_GAC Hits | cds:ADAR_Gen1_ACT T non-syn % | cds:Gen3_TGC G>C % |
| g:ADAR_Gen2_GAC % | cds:ADAR_Gen1_ACT MC1 non-syn % | cds:Gen3_TGC Ti/Tv % |
| g:ADAR_Gen2_GAC Ti % | cds:ADAR_Gen1_ACT MC2 non-syn % | cds:Gen3_TGC C:G % |
| g:ADAR_Gen2_GAC A>G + T>C % | cds:ADAR_Gen1_ACT MC3 non-syn % | cds:Gen3_TGC Ti C:G % |
| g:ADAR_Gen2_GAC A>C + T>G % | g:ADAR_Gen1_ACT Hits | cds:Gen3_TGC non-syn % |
| g:ADAR_Gen2_GAC A>T + T>A % | g:ADAR_Gen1_ACT % | cds:Gen3_TGC C non-syn % |
| nc:ADAR_Gen2_GAC Hits | g:ADAR_Gen1_ACT Ti % | cds:Gen3_TGC G non-syn % |
| nc:ADAR_Gen2_GAC % | g:ADAR_Gen1_ACT A>G + T>C % | cds:Gen3_TGC MC1 non-syn % |
| nc:ADAR_Gen2_GAC Ti % | g:ADAR_Gen1_ACT A>C + T>G % | cds:Gen3_TGC MC2 non-syn % |
| nc:ADAR_Gen2_GAC A>G + T>C % | g:ADAR_Gen1_ACT A>T + T>A % | cds:Gen3_TGC MC3 non-syn % |
| nc:ADAR_Gen2_GAC A>C + T>G % | nc:ADAR_Gen1_ACT Hits | g:Gen3_TGC Hits |
| nc:ADAR_Gen2_GAC A>T + T>A % | nc:ADAR_Gen1_ACT % | g:Gen3_TGC % |
| cds:ADAR_Gen2_AAG Hits | nc:ADAR_Gen1_ACT Ti % | g:Gen3_TGC Ti % |
| cds:ADAR_Gen2_AAG % | nc:ADAR_Gen1_ACT A>G + T>C % | g:Gen3_TGC C>T + G>A % |
| cds:ADAR_Gen2_AAG Ti % | nc:ADAR_Gen1_ACT A>C + T>G % | g:Gen3_TGC C>A + G>T % |
| cds:ADAR_Gen2_AAG MC1 % | nc:ADAR_Gen1_ACT A>T + T>A % | g:Gen3_TGC C>G + G>C % |
| cds:ADAR_Gen2_AAG MC2 % | cds:ADAR_Gen1_ACC Hits | nc:Gen3_TGC Hits |
| cds:ADAR_Gen2_AAG MC3 % | cds:ADAR_Gen1_ACC % | nc:Gen3_TGC % |
| cds:ADAR_Gen2_AAG A>G at MC1 % | cds:ADAR_Gen1_ACC Ti % | nc:Gen3_TGC Ti % |
| cds:ADAR_Gen2_AAG A>G at MC2 % | cds:ADAR_Gen1_ACC MC1 % | nc:Gen3_TGC C>T + G>A % |
| cds:ADAR_Gen2_AAG A>G at MC3 % | cds:ADAR_Gen1_ACC MC2 % | nc:Gen3_TGC C>A + G>T % |
| cds:ADAR_Gen2_AAG T>C at MC1 % | cds:ADAR_Gen1_ACC MC3 % | nc:Gen3_TGC C>G + G>C % |
| cds:ADAR_Gen2_AAG T>C at MC2 % | cds:ADAR_Gen1_ACC A>G at MC1 % | cds:Gen3_CAC Hits |
| cds:ADAR_Gen2_AAG T>C at MC3 % | cds:ADAR_Gen1_ACC A>G at MC2 % | cds:Gen3_CAC % |
| cds:ADAR_Gen2_AAG A>G % | cds:ADAR_Gen1_ACC A>G at MC3 % | cds:Gen3_CAC Ti % |
| cds:ADAR_Gen2_AAG A>C % | cds:ADAR_Gen1_ACC T>C at MC1 % | cds:Gen3_CAC MC1 % |

(continued)

| Metric | Metric | Metric |
|---|---|---|
| cds:ADAR_Gen2_AAG A>T % | cds:ADAR_Gen1_ACC T>C at MC2 % | cds:Gen3_CAC MC2 % |
| cds:ADAR_Gen2_AAG T>C % | cds:ADAR_Gen1_ACC T>C at MC3 % | cds:Gen3_CAC MC3 % |
| cds:ADAR_Gen2_AAG T>G % | cds:ADAR_Gen1_ACC A>G % | cds:Gen3_CAC C>T at MC1 % |
| cds:ADAR_Gen2_AAG T>A % | cds:ADAR_Gen1_ACC A>C % | cds:Gen3_CAC C>T at MC2 % |
| cds:ADAR_Gen2_AAG Ti/Tv % | cds:ADAR_Gen1_ACC A>T % | cds:Gen3_CAC C>T at MC3 % |
| cds:ADAR_Gen2_AAG A:T % | cds:ADAR_Gen1_ACC T>C % | cds:Gen3_CAC G>A at MC1 % |
| cds:ADAR_Gen2_AAG Ti A:T % | cds:ADAR_Gen1_ACC T>G % | cds:Gen3_CAC G>A at MC2 % |
| cds:ADAR_Gen2_AAG non-syn % | cds:ADAR_Gen1_ACC T>A % | cds:Gen3_CAC G>A at MC3 % |
| cds:ADAR_Gen2_AAG A non-syn % | cds:ADAR_Gen1_ACC Ti/Tv % | cds:Gen3_CAC C>T % |
| cds:ADAR_Gen2_AAG T non-syn % | cds:ADAR_Gen1_ACC A:T % | cds:Gen3_CAC C>A % |
| cds:ADAR_Gen2_AAG MC1 non-syn % | cds:ADAR_Gen1_ACC Ti A:T % | cds:Gen3_CAC C>G % |
| cds:ADAR_Gen2_AAG MC2 non-syn % | cds:ADAR_Gen1_ACC non-syn % | cds:Gen3_CAC G>A % |
| cds:ADAR_Gen2_AAG MC3 non-syn % | cds:ADAR_Gen1_ACC A non-syn % | cds:Gen3_CAC G>T % |
| g:ADAR_Gen2_AAG Hits | cds:ADAR_Gen1_ACC T non-syn % | cds:Gen3_CAC G>C % |
| g:ADAR_Gen2_AAG % | cds:ADAR_Gen1_ACC MC1 non-syn % | cds:Gen3_CAC Ti/Tv % |
| g:ADAR_Gen2_AAG Ti % | cds:ADAR_Gen1_ACC MC2 non-syn % | cds:Gen3_CAC C:G % |
| g:ADAR_Gen2_AAG A>G + T>C % | cds:ADAR_Gen1_ACC MC3 non-syn % | cds:Gen3_CAC Ti C:G % |
| g:ADAR_Gen2_AAG A>C + T>G % | g:ADAR_Gen1_ACC Hits | cds:Gen3_CAC non-syn % |
| g:ADAR_Gen2_AAG A>T + T>A % | g:ADAR_Gen1_ACC % | cds:Gen3_CAC C non-syn % |
| nc:ADAR_Gen2_AAG Hits | g:ADAR_Gen1_ACC Ti % | cds:Gen3_CAC G non-syn % |
| nc:ADAR_Gen2_AAG % | g:ADAR_Gen1_ACC A>G + T>C % | cds:Gen3_CAC MC1 non-syn % |
| nc:ADAR_Gen2_AAG Ti % | g:ADAR_Gen1_ACC A>C + T>G % | cds:Gen3_CAC MC2 non-syn % |
| nc:ADAR_Gen2_AAG A>G + T>C % | g:ADAR_Gen1_ACC A>T + T>A % | cds:Gen3_CAC MC3 non-syn % |
| nc:ADAR_Gen2_AAG A>C + T>G % | nc:ADAR_Gen1_ACC Hits | g:Gen3_CAC Hits |
| nc:ADAR_Gen2_AAG A>T + T>A % | nc:ADAR_Gen1_ACC % | g:Gen3_CAC % |
| cds:ADAR_Gen2_TAG Hits | nc:ADAR_Gen1_ACC Ti % | g:Gen3_CAC Ti % |
| cds:ADAR_Gen2_TAG % | nc:ADAR_Genl_ACC A>G + T>C % | g:Gen3_CAC C>T + G>A % |
| cds:ADAR_Gen2_TAG Ti % | nc:ADAR_Gen1_ACC A>C + T>G % | g:Gen3_CAC C>A + G>T % |
| cds:ADAR_Gen2_TAG MC1 % | nc:ADAR_Gen1_ACC A>T + T>A % | g:Gen3_CAC C>G + G>C % |
| cds:ADAR_Gen2_TAG MC2 % | cds:ADAR_Gen1_ACG Hits | nc:Gen3_CAC Hits |
| cds:ADAR_Gen2_TAG MC3 % | cds:ADAR_Gen1_ACG % | nc:Gen3_CAC % |
| cds:ADAR_Gen2_TAG A>G at MC1 % | cds:ADAR_Gen1_ACG Ti % | nc:Gen3_CAC Ti % |

(continued)

| Metric | Metric | Metric |
|---|---|---|
| cds:ADAR_Gen2_TAG A>G at MC2 % | cds:ADAR_Gen1_ACG MC1 % | nc:Gen3_CAC C>T + G>A % |
| cds:ADAR_Gen2_TAG A>G at MC3 % | cds:ADAR_Gen1_ACG MC2 % | nc:Gen3_CAC C>A + G>T % |
| cds:ADAR_Gen2_TAG T>C at MC1 % | cds:ADAR_Gen1_ACG MC3 % | nc:Gen3_CAC C>G + G>C % |
| cds:ADAR_Gen2_TAG T>C at MC2 % | cds:ADAR_Gen1_ACG A>G at MC1 % | cds:Gen3_CTC Hits |
| cds:ADAR_Gen2_TAG T>C at MC3 % | cds:ADAR_Gen1_ACG A>G at MC2 % | cds:Gen3_CTC % |
| cds:ADAR_Gen2_TAG A>G % | cds:ADAR_Gen1_ACG A>G at MC3 % | cds:Gen3_CTC Ti % |
| cds:ADAR_Gen2_TAG A>C % | cds:ADAR_Gen1_ACG T>C at MC1 % | cds:Gen3_CTC MC1 % |
| cds:ADAR_Gen2_TAG A>T % | cds:ADAR_Gen1_ACG T>C at MC2 % | cds:Gen3_CTC MC2 % |
| cds:ADAR_Gen2_TAG T>C % | cds:ADAR_Gen1_ACG T>C at MC3 % | cds:Gen3_CTC MC3 % |
| cds:ADAR_Gen2_TAG T>G % | cds:ADAR_Gen1_ACG A>G % | cds:Gen3_CTC C>T at MC1 % |
| cds:ADAR_Gen2_TAG T>A % | cds:ADAR_Gen1_ACG A>C % | cds:Gen3_CTC C>T at MC2 % |
| cds:ADAR_Gen2_TAG Ti/Tv % | cds:ADAR_Gen1_ACG A>T % | cds:Gen3_CTC C>T at MC3 % |
| cds:ADAR_Gen2_TAG A:T % | cds:ADAR_Gen1_ACG T>C % | cds:Gen3_CTC G>A at MC1 % |
| cds:ADAR_Gen2_TAG Ti A:T % | cds:ADAR_Gen1_ACG T>G % | cds:Gen3_CTC G>A at MC2 % |
| cds:ADAR_Gen2_TAG non-syn % | cds:ADAR_Gen1_ACG T>A % | cds:Gen3_CTC G>A at MC3 % |
| cds:ADAR_Gen2_TAG A non-syn % | cds:ADAR_Gen1_ACG Ti/Tv % | cds:Gen3_CTC C>T % |
| cds:ADAR_Gen2_TAG T non-syn % | cds:ADAR_Gen1_ACG A:T % | cds:Gen3_CTC C>A % |
| cds:ADAR_Gen2_TAG MC1 non-syn % | cds:ADAR_Gen1_ACG Ti A:T % | cds:Gen3_CTC C>G % |
| cds:ADAR_Gen2_TAG MC2 non-syn % | cds:ADAR_Gen1_ACG non-syn % | cds:Gen3_CTC G>A % |
| cds:ADAR_Gen2_TAG MC3 non-syn % | cds:ADAR_Gen1_ACG A non-syn % | cds:Gen3_CTC G>T % |
| g:ADAR_Gen2_TAG Hits | cds:ADAR_Gen1_ACG T non-syn % | cds:Gen3_CTC G>C % |
| g:ADAR_Gen2_TAG % | cds:ADAR_Gen1_ACG MC1 non-syn % | cds:Gen3_CTC Ti/Tv % |
| g:ADAR_Gen2_TAG Ti % | cds:ADAR_Gen1_ACG MC2 non-syn % | cds:Gen3_CTC C:G % |
| g:ADAR_Gen2_TAG A>G + T>C % | cds:ADAR_Gen1_ACG MC3 non-syn % | cds:Gen3_CTC Ti C:G % |
| g:ADAR_Gen2_TAG A>C + T>G % | g:ADAR_Gen1_ACG Hits | cds:Gen3_CTC non-syn % |
| g:ADAR_Gen2_TAG A>T + T>A % | g:ADAR_Gen1_ACG % | cds:Gen3_CTC C non-syn % |
| nc:ADAR_Gen2_TAG Hits | g:ADAR_Gen1_ACG Ti % | cds:Gen3_CTC G non-syn % |
| nc:ADAR_Gen2_TAG % | g:ADAR_Gen1_ACG A>G + T>C % | cds:Gen3_CTC MC1 non-syn % |

(continued)

| Metric | Metric | Metric |
|---|---|---|
| nc:ADAR_Gen2_TAG Ti % | g:ADAR_Gen1_ACG A>C + T>G % | cds:Gen3_CTC MC2 non-syn % |
| nc:ADAR_Gen2_TAG A>G + T>C % | g:ADAR_Gen1_ACG A>T + T>A % | cds:Gen3_CTC MC3 non-syn % |
| nc:ADAR_Gen2_TAG A>C + T>G % | nc:ADAR_Gen1_ACG Hits | g:Gen3_CTC Hits |
| nc:ADAR_Gen2_TAG A>T + T>A % | nc:ADAR_Gen1_ACG % | g:Gen3_CTC % |
| cds:ADAR_Gen2_CAG Hits | nc:ADAR_Gen1_ACG Ti % | g:Gen3_CTC Ti % |
| cds:ADAR_Gen2_CAG % | nc:ADAR_Gen1_ACG A>G + T>C % | g:Gen3_CTC C>T + G>A % |
| cds:ADAR_Gen2_CAG Ti % | nc:ADAR_Gen1_ACG A>C + T>G % | g:Gen3_CTC C>A + G>T % |
| cds:ADAR_Gen2_CAG MC1 % | nc:ADAR_Gen1_ACG A>T + T>A % | g:Gen3_CTC C>G + G>C % |
| cds:ADAR_Gen2_CAG MC2 % | cds:ADAR_Gen1_AGA Hits | nc:Gen3_CTC Hits |
| cds:ADAR_Gen2_CAG MC3 % | cds:ADAR_Gen1_AGA % | nc:Gen3_CTC % |
| cds:ADAR_Gen2_CAG A>G at MC1 % | cds:ADAR_Gen1_AGA Ti % | nc:Gen3_CTC Ti % |
| cds:ADAR_Gen2_CAG A>G at MC2 % | cds:ADAR_Gen1_AGA MC1 % | nc:Gen3_CTC C>T + G>A % |
| cds:ADAR_Gen2_CAG A>G at MC3 % | cds:ADAR_Gen1_AGA MC2 % | nc:Gen3_CTC C>A + G>T % |
| cds:ADAR_Gen2_CAG T>C at MC1 % | cds:ADAR_Gen1_AGA MC3 % | nc:Gen3_CTC C>G + G>C % |
| cds:ADAR_Gen2_CAG T>C at MC2 % | cds:ADAR_Gen1_AGA A>G at MC1 % | cds:Gen3_CCC Hits |
| cds:ADAR_Gen2_CAG T>C at MC3 % | cds:ADAR_Gen1_AGA A>G at MC2 % | cds:Gen3_CCC % |
| cds:ADAR_Gen2_CAG A>G % | cds:ADAR_Gen1_AGA A>G at MC3 % | cds:Gen3_CCC Ti % |
| cds:ADAR_Gen2_CAG A>C % | cds:ADAR_Gen1_AGA T>C at MC1 % | cds:Gen3_CCC MC1 % |
| cds:ADAR_Gen2_CAG A>T % | cds:ADAR_Gen1_AGA T>C at MC2 % | cds:Gen3_CCC MC2 % |
| cds:ADAR_Gen2_CAG T>C % | cds:ADAR_Gen1_AGA T>C at MC3 % | cds:Gen3_CCC MC3 % |
| cds:ADAR_Gen2_CAG T>G % | cds:ADAR_Gen1_AGA A>G % | cds:Gen3_CCC C>T at MC1 % |
| cds:ADAR_Gen2_CAG T>A % | cds:ADAR_Gen1_AGA A>C % | cds:Gen3_CCC C>T at MC2 % |
| cds:ADAR_Gen2_CAG Ti/Tv % | cds:ADAR_Gen1_AGA A>T % | cds:Gen3_CCC C>T at MC3 % |
| cds:ADAR_Gen2_CAG A:T % | cds:ADAR_Gen1_AGA T>C % | cds:Gen3_CCC G>A at MC1 % |
| cds:ADAR_Gen2_CAG Ti A:T % | cds:ADAR_Gen1_AGA T>G % | cds:Gen3_CCC G>A at MC2 % |
| cds:ADAR_Gen2_CAG non-syn % | cds:ADAR_Gen1_AGA T>A % | cds:Gen3_CCC G>A at MC3 % |
| cds:ADAR_Gen2_CAG A non-syn % | cds:ADAR_Gen1_AGA Ti/Tv % | cds:Gen3_CCC C>T % |
| cds:ADAR_Gen2_CAG T non-syn % | cds:ADAR_Gen1_AGA A:T % | cds:Gen3_CCC C>A % |
| cds:ADAR_Gen2_CAG MC1 non-syn % | cds:ADAR_Gen1_AGA Ti A:T % | cds:Gen3_CCC C>G % |

(continued)

| Metric | Metric | Metric |
|---|---|---|
| cds:ADAR_Gen2_CAG MC2 non-syn % | cds:ADAR_Gen1_AGA non-syn % | cds:Gen3_CCC G>A % |
| cds:ADAR_Gen2_CAG MC3 non-syn % | cds:ADAR_Gen1_AGA A non-syn % | cds:Gen3_CCC G>T % |
| g:ADAR_Gen2_CAG Hits | cds:ADAR_Gen1_AGA T non-syn % | cds:Gen3_CCC G>C % |
| g:ADAR_Gen2_CAG % | cds:ADAR_Gen1_AGA MC1 non-syn % | cds:Gen3_CCC Ti/Tv % |
| g:ADAR_Gen2_CAG Ti % | cds:ADAR_Gen1_AGA MC2 non-syn % | cds:Gen3_CCC C:G % |
| g:ADAR_Gen2_CAG A>G + T>C % | cds:ADAR_Gen1_AGA MC3 non-syn % | cds:Gen3_CCC Ti C:G % |
| g:ADAR_Gen2_CAG A>C + T>G % | g:ADAR_Gen1_AGA Hits | cds:Gen3_CCC non-syn % |
| g:ADAR_Gen2_CAG A>T + T>A % | g:ADAR_Gen1_AGA % | cds:Gen3_CCC C non-syn % |
| nc:ADAR_Gen2_CAG Hits | g:ADAR_Gen1_AGA Ti % | cds:Gen3_CCC G non-syn % |
| nc:ADAR_Gen2_CAG % | g:ADAR_Gen1_AGA A>G + T>C % | cds:Gen3_CCC MC1 non-syn % |
| nc:ADAR_Gen2_CAG Ti % | g:ADAR_Gen1_AGA A>C + T>G % | cds:Gen3_CCC MC2 non-syn % |
| nc:ADAR_Gen2_CAG A>G + T>C % | g:ADAR_Gen1_AGA A>T + T>A % | cds:Gen3_CCC MC3 non-syn % |
| nc:ADAR_Gen2_CAG A>C + T>G % | nc:ADAR_Gen1_AGA Hits | g:Gen3_CCC Hits |
| nc:ADAR_Gen2_CAG A>T + T>A % | nc:ADAR_Gen1_AGA % | g:Gen3_CCC % |
| cds:ADAR_Gen2_GAG Hits | nc:ADAR_Gen1_AGA Ti % | g:Gen3_CCC Ti % |
| cds:ADAR_Gen2_GAG % | nc:ADAR_Gen1_AGA A>G + T>C % | g:Gen3_CCC C>T + G>A % |
| cds:ADAR_Gen2_GAG Ti % | nc:ADAR_Gen1_AGA A>C + T>G % | g:Gen3_CCC C>A + G>T % |
| cds:ADAR_Gen2_GAG MC1 % | nc:ADAR_Gen1_AGA A>T + T>A % | g:Gen3_CCC C>G + G>C % |
| cds:ADAR_Gen2_GAG MC2 % | cds:ADAR_Gen1_AGT Hits | nc:Gen3_CCC Hits |
| cds:ADAR_Gen2_GAG MC3 % | cds:ADAR_Gen1_AGT % | nc:Gen3_CCC % |
| cds:ADAR_Gen2_GAG A>G at MC1 % | cds:ADAR_Gen1_AGT Ti % | nc:Gen3_CCC Ti % |
| cds:ADAR_Gen2_GAG A>G at MC2 % | cds:ADAR_Gen1_AGT MC1 % | nc:Gen3_CCC C>T + G>A % |
| cds:ADAR_Gen2_GAG A>G at MC3 % | cds:ADAR_Gen1_AGT MC2 % | nc:Gen3_CCC C>A + G>T % |
| cds:ADAR_Gen2_GAG T>C at MC1 % | cds:ADAR_Gen1_AGT MC3 % | nc:Gen3_CCC C>G + G>C % |
| cds:ADAR_Gen2_GAG T>C at MC2 % | cds:ADAR_Gen1_AGT A>G at MC1 % | cds:Gen3_CGC Hits |
| cds:ADAR_Gen2_GAG T>C at MC3 % | cds:ADAR_Gen1_AGT A>G at MC2 % | cds:Gen3_CGC % |
| cds:ADAR_Gen2_GAG A>G % | cds:ADAR_Gen1_AGT A>G at MC3 % | cds:Gen3_CGC Ti % |
| cds:ADAR_Gen2_GAG A>C % | cds:ADAR_Gen1_AGT T>C at MC1 % | cds:Gen3_CGC MC1 % |

(continued)

| Metric | Metric | Metric |
|---|---|---|
| cds:ADAR_Gen2_GAG A>T % | cds:ADAR_Gen1_AGT T>C at MC2 % | cds:Gen3_CGC MC2 % |
| cds:ADAR_Gen2_GAG T>C % | cds:ADAR_Gen1_AGT T>C at MC3 % | cds:Gen3_CGC MC3 % |
| cds:ADAR_Gen2_GAG T>G % | cds:ADAR_Gen1_AGT A>G % | cds:Gen3_CGC C>T at MC1 % |
| cds:ADAR_Gen2_GAG T>A % | cds:ADAR_Gen1_AGT A>C % | cds:Gen3_CGC C>T at MC2 % |
| cds:ADAR_Gen2_GAG Ti/Tv % | cds:ADAR_Gen1_AGT A>T % | cds:Gen3_CGC C>T at MC3 % |
| cds:ADAR_Gen2_GAG A:T % | cds:ADAR_Gen1_AGT T>C % | cds:Gen3_CGC G>A at MC1 % |
| cds:ADAR_Gen2_GAG Ti A:T % | cds:ADAR_Gen1_AGT T>G % | cds:Gen3_CGC G>A at MC2 % |
| cds:ADAR_Gen2_GAG non-syn % | cds:ADAR_Gen1_AGT T>A % | cds:Gen3_CGC G>A at MC3 % |
| cds:ADAR_Gen2_GAG A non-syn % | cds:ADAR_Gen1_AGT Ti/Tv % | cds:Gen3_CGC C>T % |
| cds:ADAR_Gen2_GAG T non-syn % | cds:ADAR_Gen1_AGT A:T % | cds:Gen3_CGC C>A % |
| cds:ADAR_Gen2_GAG MC1 non-syn % | cds:ADAR_Gen1_AGT Ti A:T % | cds:Gen3_CGC C>G % |
| cds:ADAR_Gen2_GAG MC2 non-syn % | cds:ADAR_Gen1_AGT non-syn % | cds:Gen3_CGC G>A % |
| cds:ADAR_Gen2_GAG MC3 non-syn % | cds:ADAR_Gen1_AGT A non-syn % | cds:Gen3_CGC G>T % |
| g:ADAR_Gen2_GAG Hits | cds:ADAR_Gen1_AGT T non-syn % | cds:Gen3_CGC G>C % |
| g:ADAR_Gen2_GAG % | cds:ADAR_Gen1_AGT MC1 non-syn % | cds:Gen3_CGC Ti/Tv % |
| g:ADAR_Gen2_GAG Ti % | cds:ADAR_Gen1_AGT MC2 non-syn % | cds:Gen3_CGC C:G % |
| g:ADAR_Gen2_GAG A>G + T>C % | cds:ADAR_Gen1_AGT MC3 non-syn % | cds:Gen3_CGC Ti C:G % |
| g:ADAR_Gen2_GAG A>C + T>G % | g:ADAR_Gen1_AGT Hits | cds:Gen3_CGC non-syn % |
| g:ADAR_Gen2_GAG A>T + T>A % | g:ADAR_Gen1_AGT % | cds:Gen3_CGC C non-syn % |
| nc:ADAR_Gen2_GAG Hits | g:ADAR_Gen1_AGT Ti % | cds:Gen3_CGC G non-syn % |
| nc:ADAR_Gen2_GAG % | g:ADAR_Gen1_AGT A>G + T>C % | cds:Gen3_CGC MC1 non-syn % |
| nc:ADAR_Gen2_GAG Ti % | g:ADAR_Gen1_AGT A>C + T>G % | cds:Gen3_CGC MC2 non-syn % |
| nc:ADAR_Gen2_GAG A>G + T>C % | g:ADAR_Gen1_AGT A>T + T>A % | cds:Gen3_CGC MC3 non-syn % |
| nc:ADAR_Gen2_GAG A>C + T>G % | nc:ADAR_Gen1_AGT Hits | g:Gen3_CGC Hits |
| nc:ADAR_Gen2_GAG A>T + T>A % | nc:ADAR_Gen1_AGT % | g:Gen3_CGC % |
| cds:AIDb Hits | nc:ADAR_Gen1_AGT Ti % | g:Gen3_CGC Ti % |
| cds:AIDb % | nc:ADAR_Genl_AGT A>G + T>C % | g:Gen3_CGC C>T + G>A % |
| cds:AIDb Ti % | nc:ADAR_Gen1_AGT A>C + T>G % | g:Gen3_CGC C>A + G>T % |
| cds:AIDb MC1 % | nc:ADAR_Gen1_AGT A>T + T>A % | g:Gen3_CGC C>G + G>C % |
| cds:AIDb MC2 % | cds:ADAR_Gen1_AGC Hits | nc:Gen3_CGC Hits |
| cds:AIDb MC3 % | cds:ADAR_Gen1_AGC % | nc:Gen3_CGC % |

(continued)

| Metric | Metric | Metric |
|---|---|---|
| cds:AIDb C>T at MC1 % | cds:ADAR_Gen1_AGC Ti % | nc:Gen3_CGC Ti % |
| cds:AIDb C>T at MC2 % | cds:ADAR_Gen1_AGC MC1 % | nc:Gen3_CGC C>T + G>A % |
| cds:AIDb C>T at MC3 % | cds:ADAR_Gen1_AGC MC2 % | nc:Gen3_CGC C>A + G>T % |
| cds:AIDb G>A at MC1 % | cds:ADAR_Gen1_AGC MC3 % | nc:Gen3_CGC C>G + G>C % |
| cds:AIDb G>A at MC2 % | cds:ADAR_Gen1_AGC A>G at MC1 % | cds:Gen3_GAC Hits |
| cds:AIDb G>A at MC3 % | cds:ADAR_Gen1_AGC A>G at MC2 % | cds:Gen3_GAC % |
| cds:AIDb C>T % | cds:ADAR_Gen1_AGC A>G at MC3 % | cds:Gen3_GAC Ti % |
| cds:AIDb C>A % | cds:ADAR_Gen1_AGC T>C at MC1 % | cds:Gen3_GAC MC1 % |
| cds:AIDb C>G % | cds:ADAR_Gen1_AGC T>C at MC2 % | cds:Gen3_GAC MC2 % |
| cds:AIDb G>A % | cds:ADAR_Gen1_AGC T>C at MC3 % | cds:Gen3_GAC MC3 % |
| cds:AIDb G>T % | cds:ADAR_Gen1_AGC A>G % | cds:Gen3_GAC C>T at MC1 % |
| cds:AIDb G>C % | cds:ADAR_Gen1_AGC A>C % | cds:Gen3_GAC C>T at MC2 % |
| cds:AIDb Ti/Tv % | cds:ADAR_Gen1_AGC A>T % | cds:Gen3_GAC C>T at MC3 % |
| cds:AIDb C:G % | cds:ADAR_Gen1_AGC T>C % | cds:Gen3_GAC G>A at MC1 % |
| cds:AIDb Ti C:G % | cds:ADAR_Gen1_AGC T>G % | cds:Gen3_GAC G>A at MC2 % |
| cds:AIDb non-syn % | cds:ADAR_Gen1_AGC T>A % | cds:Gen3_GAC G>A at MC3 % |
| cds:AIDb C non-syn % | cds:ADAR_Gen1_AGC Ti/Tv % | cds:Gen3_GAC C>T % |
| cds:AIDb G non-syn % | cds:ADAR_Gen1_AGC A:T % | cds:Gen3_GAC C>A % |
| cds:AIDb MC1 non-syn % | cds:ADAR_Gen1_AGC Ti A:T % | cds:Gen3_GAC C>G % |
| cds:AIDb MC2 non-syn % | cds:ADAR_Gen1_AGC non-syn % | cds:Gen3_GAC G>A % |
| cds:AIDb MC3 non-syn % | cds:ADAR_Gen1_AGC A non-syn % | cds:Gen3_GAC G>T % |
| g:AIDb Hits | cds:ADAR_Gen1_AGC T non-syn % | cds:Gen3_GAC G>C % |
| g:AIDb % | cds:ADAR_Gen1_AGC MC1 non-syn % | cds:Gen3_GAC Ti/Tv % |
| g:AIDb Ti % | cds:ADAR_Gen1_AGC MC2 non-syn % | cds:Gen3_GAC C:G % |
| g:AIDb C>T + G>A % | cds:ADAR_Gen1_AGC MC3 non-syn % | cds:Gen3_GAC Ti C:G % |
| g:AIDb C>A + G>T % | g:ADAR_Gen1_AGC Hits | cds:Gen3_GAC non-syn % |
| g:AIDb C>G + G>C % | g:ADAR_Gen1_AGC % | cds:Gen3_GAC C non-syn % |
| nc:AIDb Hits | g:ADAR_Gen1_AGC Ti % | cds:Gen3_GAC G non-syn % |
| nc:AIDb % | g:ADAR_Gen1_AGC A>G + T>C % | cds:Gen3_GAC MC1 non-syn % |
| nc:AIDb Ti % | g:ADAR_Gen1_AGC A>C + T>G % | cds:Gen3_GAC MC2 non-syn % |

(continued)

| Metric | Metric | Metric |
|---|---|---|
| nc:AIDb C>T + G>A % | g:ADAR_Gen1_AGC A>T + T>A % | cds:Gen3_GAC MC3 non-syn % |
| nc:AIDb C>A + G>T % | nc:ADAR_Gen1_AGC Hits | g:Gen3_GAC Hits |
| nc:AIDb C>G + G>C % | nc:ADAR_Gen1_AGC % | g:Gen3_GAC % |
| cds:AIDc Hits | nc:ADAR_Gen1_AGC Ti % | g:Gen3_GAC Ti % |
| cds:AIDc % | nc:ADAR_Gen1_AGC A>G + T>C % | g:Gen3_GAC C>T + G>A % |
| cds:AIDc Ti % | nc:ADAR_Gen1_AGC A>C + T>G % | g:Gen3_GAC C>A + G>T % |
| cds:AIDc MC1 % | nc:ADAR_Gen1_AGC A>T + T>A % | g:Gen3_GAC C>G + G>C % |
| cds:AIDc MC2 % | cds:ADAR_Gen1_AGG Hits | nc:Gen3_GAC Hits |
| cds:AIDc MC3 % | cds:ADAR_Gen1_AGG % | nc:Gen3_GAC % |
| cds:AIDc C>T at MC1 % | cds:ADAR_Gen1_AGG Ti % | nc:Gen3_GAC Ti % |
| cds:AIDc C>T at MC2 % | cds:ADAR_Gen1_AGG MC1 % | nc:Gen3_GAC C>T + G>A % |
| cds:AIDc C>T at MC3 % | cds:ADAR_Gen1_AGG MC2 % | nc:Gen3_GAC C>A + G>T % |
| cds:AIDc G>A at MC1 % | cds:ADAR_Gen1_AGG MC3 % | nc:Gen3_GAC C>G + G>C % |
| cds:AIDc G>A at MC2 % | cds:ADAR_Gen1_AGG A>G at MC1 % | cds:Gen3_GTC Hits |
| cds:AIDc G>A at MC3 % | cds:ADAR_Gen1_AGG A>G at MC2 % | cds:Gen3_GTC % |
| cds:AIDc C>T % | cds:ADAR_Gen1_AGG A>G at MC3 % | cds:Gen3_GTC Ti % |
| cds:AIDc C>A % | cds:ADAR_Gen1_AGG T>C at MC1 % | cds:Gen3_GTC MC1 % |
| cds:AIDc C>G % | cds:ADAR_Gen1_AGG T>C at MC2 % | cds:Gen3_GTC MC2 % |
| cds:AIDc G>A % | cds:ADAR_Gen1_AGG T>C at MC3 % | cds:Gen3_GTC MC3 % |
| cds:AIDc G>T % | cds:ADAR_Gen1_AGG A>G % | cds:Gen3_GTC C>T at MC1 % |
| cds:AIDc G>C % | cds:ADAR_Gen1_AGG A>C % | cds:Gen3_GTC C>T at MC2 % |
| cds:AIDc Ti/Tv % | cds:ADAR_Gen1_AGG A>T % | cds:Gen3_GTC C>T at MC3 % |
| cds:AIDc C:G % | cds:ADAR_Gen1_AGG T>C % | cds:Gen3_GTC G>A at MC1 % |
| cds:AIDc Ti C:G % | cds:ADAR_Gen1_AGG T>G % | cds:Gen3_GTC G>A at MC2 % |
| cds:AIDc non-syn % | cds:ADAR_Gen1_AGG T>A % | cds:Gen3_GTC G>A at MC3 % |
| cds:AIDc C non-syn % | cds:ADAR_Gen1_AGG Ti/Tv % | cds:Gen3_GTC C>T % |
| cds:AIDc G non-syn % | cds:ADAR_Gen1_AGG A:T % | cds:Gen3_GTC C>A % |
| cds:AIDc MC1 non-syn % | cds:ADAR_Gen1_AGG Ti A:T % | cds:Gen3_GTC C>G % |
| cds:AIDc MC2 non-syn % | cds:ADAR_Gen1_AGG non-syn % | cds:Gen3_GTC G>A % |
| cds:AIDc MC3 non-syn % | cds:ADAR_Gen1_AGG A non-syn % | cds:Gen3_GTC G>T % |
| g:AIDc Hits | cds:ADAR_Gen1_AGG T non-syn % | cds:Gen3_GTC G>C % |

(continued)

| Metric | Metric | Metric |
|---|---|---|
| g:AIDc % | cds:ADAR_Gen1_AGG MC1 non-syn % | cds:Gen3_GTC Ti/Tv % |
| g:AIDc Ti % | cds:ADAR_Gen1_AGG MC2 non-syn % | cds:Gen3_GTC C:G % |
| g:AIDc C>T + G>A % | cds:ADAR_Gen1_AGG MC3 non-syn % | cds:Gen3_GTC Ti C:G % |
| g:AIDc C>A + G>T % | g:ADAR_Gen1_AGG Hits | cds:Gen3_GTC non-syn % |
| g:AIDc C>G + G>C % | g:ADAR_Gen1_AGG % | cds:Gen3_GTC C non-syn % |
| nc:AIDc Hits | g:ADAR_Gen1_AGG Ti % | cds:Gen3_GTC G non-syn % |
| nc:AIDc % | g:ADAR_Gen1_AGG A>G + T>C % | cds:Gen3_GTC MC1 non-syn % |
| nc:AIDc Ti % | g:ADAR_Gen1_AGG A>C + T>G % | cds:Gen3_GTC MC2 non-syn % |
| nc:AIDc C>T + G>A % | g:ADAR_Gen1_AGG A>T + T>A % | cds:Gen3_GTC MC3 non-syn % |
| nc:AIDc C>A + G>T % | nc:ADAR_Gen1_AGG Hits | g:Gen3_GTC Hits |
| nc:AIDc C>G + G>C % | nc:ADAR_Gen1_AGG % | g:Gen3_GTC % |
| cds:AIDd Hits | nc:ADAR_Gen1_AGG Ti % | g:Gen3_GTC Ti % |
| cds:AIDd % | nc:ADAR_Gen1_AGG A>G + T>C % | g:Gen3_GTC C>T + G>A % |
| cds:AIDd Ti % | nc:ADAR_Gen1_AGG A>C + T>G % | g:Gen3_GTC C>A + G>T % |
| cds:AIDd MC1 % | nc:ADAR_Gen1_AGG A>T + T>A % | g:Gen3_GTC C>G + G>C % |
| cds:AIDd MC2 % | cds:ADAR_Gen3_AAA Hits | nc:Gen3_GTC Hits |
| cds:AIDd MC3 % | cds:ADAR_Gen3_AAA % | nc:Gen3_GTC % |
| cds:AIDd C>T at MC1 % | cds:ADAR_Gen3_AAA Ti % | nc:Gen3_GTC Ti % |
| cds:AIDd C>T at MC2 % | cds:ADAR_Gen3_AAA MC1 % | nc:Gen3_GTC C>T + G>A % |
| cds:AIDd C>T at MC3 % | cds:ADAR_Gen3_AAA MC2 % | nc:Gen3_GTC C>A + G>T % |
| cds:AIDd G>A at MC1 % | cds:ADAR_Gen3_AAA MC3 % | nc:Gen3_GTC C>G + G>C % |
| cds:AIDd G>A at MC2 % | cds:ADAR_Gen3_AAA A>G at MC1 % | cds:Gen3_GCC Hits |
| cds:AIDd G>A at MC3 % | cds:ADAR_Gen3_AAA A>G at MC2 % | cds:Gen3_GCC % |
| cds:AIDd C>T % | cds:ADAR_Gen3_AAA A>G at MC3 % | cds:Gen3_GCC Ti % |
| cds:AIDd C>A % | cds:ADAR_Gen3_AAA T>C at MC1 % | cds:Gen3_GCC MC1 % |
| cds:AIDd C>G % | cds:ADAR_Gen3_AAA T>C at MC2 % | cds:Gen3_GCC MC2 % |
| cds:AIDd G>A % | cds:ADAR_Gen3_AAA T>C at MC3 % | cds:Gen3_GCC MC3 % |
| cds:AIDd G>T % | cds:ADAR_Gen3_AAA A>G % | cds:Gen3_GCC C>T at MC1 % |
| cds:AIDd G>C % | cds:ADAR_Gen3_AAA A>C % | cds:Gen3_GCC C>T at MC2 % |
| cds:AIDd Ti/Tv % | cds:ADAR_Gen3_AAA A>T % | cds:Gen3_GCC C>T at MC3 % |
| cds:AIDd C:G % | cds:ADAR_Gen3_AAA T>C % | cds:Gen3_GCC G>A at MC1 % |

(continued)

| Metric | Metric | Metric |
|---|---|---|
| cds:AIDd Ti C:G % | cds:ADAR_Gen3_AAA T>G % | cds:Gen3_GCC G>A at MC2 % |
| cds:AIDd non-syn % | cds:ADAR_Gen3_AAA T>A % | cds:Gen3_GCC G>A at MC3 % |
| cds:AIDd C non-syn % | cds:ADAR_Gen3_AAA Ti/Tv % | cds:Gen3_GCC C>T % |
| cds:AIDd G non-syn % | cds:ADAR_Gen3_AAA A:T % | cds:Gen3_GCC C>A % |
| cds:AIDd MC1 non-syn % | cds:ADAR_Gen3_AAA Ti A:T % | cds:Gen3_GCC C>G % |
| cds:AIDd MC2 non-syn % | cds:ADAR_Gen3_AAA non-syn % | cds:Gen3_GCC G>A % |
| cds:AIDd MC3 non-syn % | cds:ADAR_Gen3_AAA A non-syn % | cds:Gen3_GCC G>T % |
| g:AIDd Hits | cds:ADAR_Gen3_AAA T non-syn % | cds:Gen3_GCC G>C % |
| g:AIDd % | cds:ADAR_Gen3_AAA MC1 non-syn % | cds:Gen3_GCC Ti/Tv % |
| g:AIDd Ti % | cds:ADAR_Gen3_AAA MC2 non-syn % | cds:Gen3_GCC C:G % |
| g:AIDd C>T + G>A % | cds:ADAR_Gen3_AAA MC3 non-syn % | cds:Gen3_GCC Ti C:G % |
| g:AIDd C>A + G>T % | g:ADAR_Gen3_AAA Hits | cds:Gen3_GCC non-syn % |
| g:AIDd C>G + G>C % | g:ADAR_Gen3_AAA % | cds:Gen3_GCC C non-syn % |
| nc:AIDd Hits | g:ADAR_Gen3_AAA Ti % | cds:Gen3_GCC G non-syn % |
| nc:AIDd % | g:ADAR_Gen3_AAA A>G + T>C % | cds:Gen3_GCC MC1 non-syn % |
| nc:AIDd Ti % | g:ADAR_Gen3_AAA A>C + T>G % | cds:Gen3_GCC MC2 non-syn % |
| nc:AIDd C>T + G>A % | g:ADAR_Gen3_AAA A>T + T>A % | cds:Gen3_GCC MC3 non-syn % |
| nc:AIDd C>A + G>T % | nc:ADAR_Gen3_AAA Hits | g:Gen3_GCC Hits |
| nc:AIDd C>G + G>C % | nc:ADAR_Gen3_AAA % | g:Gen3_GCC % |
| cds:AIDe Hits | nc:ADAR_Gen3_AAA Ti % | g:Gen3_GCC Ti % |
| cds:AIDe % | nc:ADAR_Gen3_AAA A>G + T>C % | g:Gen3_GCC C>T + G>A % |
| cds:AIDe Ti % | nc:ADAR_Gen3_AAA A>C + T>G % | g:Gen3_GCC C>A + G>T % |
| cds:AIDe MC1 % | nc:ADAR_Gen3_AAA A>T + T>A % | g:Gen3_GCC C>G + G>C % |
| cds:AIDe MC2 % | cds:ADAR_Gen3_ATA Hits | nc:Gen3_GCC Hits |
| cds:AIDe MC3 % | cds:ADAR_Gen3_ATA % | nc:Gen3_GCC % |
| cds:AIDe C>T at MC1 % | cds:ADAR_Gen3_ATA Ti % | nc:Gen3_GCC Ti % |
| cds:AIDe C>T at MC2 % | cds:ADAR_Gen3_ATA MC1 % | nc:Gen3_GCC C>T + G>A % |
| cds:AIDe C>T at MC3 % | cds:ADAR_Gen3_ATA MC2 % | nc:Gen3_GCC C>A + G>T % |
| cds:AIDe G>A at MC1 % | cds:ADAR_Gen3_ATA MC3 % | nc:Gen3_GCC C>G + G>C % |
| cds:AIDe G>A at MC2 % | cds:ADAR_Gen3_ATA A>G at MC1 % | cds:Gen3_GGC Hits |
| cds:AIDe G>A at MC3 % | cds:ADAR_Gen3_ATA A>G at MC2 % | cds:Gen3_GGC % |
| cds:AIDe C>T % | cds:ADAR_Gen3_ATA A>G at MC3 % | cds:Gen3_GGC Ti % |
| cds:AIDe C>A % | cds:ADAR_Gen3_ATA T>C at MC1 % | cds:Gen3_GGC MC1 % |
| cds:AIDe C>G % | cds:ADAR_Gen3_ATA T>C at MC2 % | cds:Gen3_GGC MC2 % |

(continued)

| Metric | Metric | Metric |
|---|---|---|
| cds:AIDe G>A % | cds:ADAR_Gen3_ATA T>C at MC3 % | cds:Gen3_GGC MC3 % |
| cds:AIDe G>T % | cds:ADAR_Gen3_ATA A>G % | cds:Gen3_GGC C>T at MC1 % |
| cds:AIDe G>C % | cds:ADAR_Gen3_ATA A>C % | cds:Gen3_GGC C>T at MC2 % |
| cds:AIDe Ti/Tv % | cds:ADAR_Gen3_ATA A>T % | cds:Gen3_GGC C>T at MC3 % |
| cds:AIDe C:G % | cds:ADAR_Gen3_ATA T>C % | cds:Gen3_GGC G>A at MC1 % |
| cds:AIDe Ti C:G % | cds:ADAR_Gen3_ATA T>G % | cds:Gen3_GGC G>A at MC2 % |
| cds:AIDe non-syn % | cds:ADAR_Gen3_ATA T>A % | cds:Gen3_GGC G>A at MC3 % |
| cds:AIDe C non-syn % | cds:ADAR_Gen3_ATA Ti/Tv % | cds:Gen3_GGC C>T % |
| cds:AIDe G non-syn % | cds:ADAR_Gen3_ATA A:T % | cds:Gen3_GGC C>A % |
| cds:AIDe MC1 non-syn % | cds:ADAR_Gen3_ATA Ti A:T % | cds:Gen3_GGC C>G % |
| cds:AIDe MC2 non-syn % | cds:ADAR_Gen3_ATA non-syn % | cds:Gen3_GGC G>A % |
| cds:AIDe MC3 non-syn % | cds:ADAR_Gen3_ATA A non-syn % | cds:Gen3_GGC G>T % |
| g:AIDe Hits | cds:ADAR_Gen3_ATA T non-syn % | cds:Gen3_GGC G>C % |
| g:AIDe % | cds:ADAR_Gen3_ATA MC1 non-syn % | cds:Gen3_GGC Ti/Tv % |
| g:AIDe Ti % | cds:ADAR_Gen3_ATA MC2 non-syn % | cds:Gen3_GGC C:G % |
| g:AIDe C>T + G>A % | cds:ADAR_Gen3_ATA MC3 non-syn % | cds:Gen3_GGC Ti C:G % |
| g:AIDe C>A + G>T % | g:ADAR_Gen3_ATA Hits | cds:Gen3_GGC non-syn % |
| g:AIDe C>G + G>C % | g:ADAR_Gen3_ATA % | cds:Gen3_GGC C non-syn % |
| nc:AIDe Hits | g:ADAR_Gen3_ATA Ti % | cds:Gen3_GGC G non-syn % |
| nc:AIDe % | g:ADAR_Gen3_ATA A>G + T>C % | cds:Gen3_GGC MC1 non-syn % |
| nc:AIDe Ti % | g:ADAR_Gen3_ATA A>C + T>G % | cds:Gen3_GGC MC2 non-syn % |
| nc:AIDe C>T + G>A % | g:ADAR_Gen3_ATA A>T + T>A % | cds:Gen3_GGC MC3 non-syn % |
| nc:AIDe C>A + G>T % | nc:ADAR_Gen3_ATA Hits | g:Gen3_GGC Hits |
| nc:AIDe C>G + G>C % | nc:ADAR_Gen3_ATA % | g:Gen3_GGC % |
| cds:AIDf Hits | nc:ADAR_Gen3_ATA Ti % | g:Gen3_GGC Ti % |
| cds:AIDf % | nc:ADAR_Gen3_ATA A>G + T>C % | g:Gen3_GGC C>T + G>A % |
| cds:AIDf Ti % | nc:ADAR_Gen3_ATA A>C + T>G % | g:Gen3_GGC C>A + G>T % |
| cds:AIDf MC1 % | nc:ADAR_Gen3_ATA A>T + T>A % | g:Gen3_GGC C>G + G>C % |
| cds:AIDf MC2 % | cds:ADAR_Gen3_ACA Hits | nc:Gen3_GGC Hits |
| cds:AIDf MC3 % | cds:ADAR_Gen3_ACA % | nc:Gen3_GGC % |
| cds:AIDf C>T at MC1 % | cds:ADAR_Gen3_ACA Ti % | nc:Gen3_GGC Ti % |
| cds:AIDf C>T at MC2 % | cds:ADAR_Gen3_ACA MC1 % | nc:Gen3_GGC C>T + G>A % |
| cds:AIDf C>T at MC3 % | cds:ADAR_Gen3_ACA MC2 % | nc:Gen3_GGC C>A + G>T % |
| cds:AIDf G>A at MC1 % | cds:ADAR_Gen3_ACA MC3 % | nc:Gen3_GGC C>G + G>C % |

### *4. Assessing a nucleic acid molecule for SNVs*

Any method known in the art for obtaining and assessing the sequence of a nucleic acid molecule can be used in accordance with the methods and systems of the present disclosure. The nucleic acid molecule analyzed using the systems and methods of the present disclosure can be any nucleic acid molecule, although is generally DNA (including cDNA). Typically, the nucleic acid is mammalian nucleic acid, such as human nucleic acid, and is from a subject previously diagnosed with cancer. The nucleic acid can be obtained from any biological sample. For example, the biological sample may comprise a bodily fluid, tissue or cells. In particular examples, the biological sample is a bodily fluid, such as saliva or blood. In some examples, the biological sample is a biopsy. A biological sample comprising tissue or cells may from any part of the body and may comprise any type of cells or tissue, such as, for example, cells from the liver.

The nucleic acid molecule can contain a part or all of one gene, or a part or all of two or more genes. Most typically, the nucleic acid molecule comprises the whole genome or whole exome, and it is the sequence of the whole genome or whole exome that is analyzed in the methods of the disclosure. In instances where the whole genome or whole exome is used for analysis, SNVs that are in both the coding region and non-coding region or just one of the two regions may be assessed.

When performing the methods of the present disclosure, the sequence of the nucleic acid molecule may have been predetermined. For example, the sequence may be stored in a database or other storage medium, and it is this sequence that is analyzed according to the methods of the disclosure. In other instances, the sequence of the nucleic acid molecule must be first determined prior to employment of the methods of the disclosure. In particular examples, the nucleic acid molecule must also be first isolated from the biological sample.

The biological sample may be any sample suitable for analysis of the nucleic acid of a subject. Typically, the sample is matched to the type of cancer and may be, for example, a biopsy. By way of an illustration, if the subject suffers from an ovarian cancer, then the sample is derived from ovarian tissue or cells, such as a biopsy from the ovarian cancer. In other examples, the biological sample from which the nucleic acid is obtained is a saliva sample or a blood sample.

Methods for obtaining nucleic acid and/or sequencing the nucleic acid are well known in the art, and any such method can be utilized for the methods described herein. In some instances, the methods include amplification of the isolated nucleic acid prior to sequencing, and suitable nucleic acid amplification techniques are well known to a person of ordinary skill in the art. Nucleic acid sequencing techniques are well known in the art and can be applied to single or multiple genes, or whole exomes, transcriptomes or genomes. These techniques include, for example, capillary sequencing methods that rely upon 'Sanger sequencing' (Sanger et al. (1977) Proc Natl Acad Sci USA 74: 5463-5467) (i.e., methods that involve chain-termination sequencing), as well as "next generation sequencing" techniques that facilitate the sequencing of thousands to millions of molecules at once. Such methods include, but are not limited to, pyrosequencing, which makes use of luciferase to read out signals as individual nucleotides are added to DNA templates; "sequencing by synthesis" technology (Illumina), which uses reversible dye-terminator techniques that add a single nucleotide to the DNA template in each cycle; and SOLiD™ sequencing (Sequencing by Oligonucleotide Ligation and Detection; Life Technologies), which sequences by preferential ligation of fixed-length oligonucleotides. These next generation sequencing techniques are particularly useful for sequencing whole exomes and genomes. Other exemplary sequencing platforms include third generation (or long-read) sequencing platforms, such as single-molecule nanopore sequencing using the MiniION™ or GridION™ sequencers (developed by Oxford Nanopore and involving passing a DNA molecule through a nanoscale pore structure and then measuring changes in electrical field surrounding the pore), or single molecule real time sequencing (SMRT) utilizing a zero-mode waveguide (ZMW), such as developed by Pacific Biosciences.

Once the sequence of the nucleic acid molecule is obtained, SNVs are then identified. SNVs may be identified by comparing the sequence to a reference sequence. The reference sequence may be the sequence of a nucleic acid molecule from a database, such as reference genome. In particular examples, the reference sequence is a reference genome, such as GRCh38 (hg38), GRCh37 (hg19), NCBI Build 36.1 (hg18), NCBI Build 35 (hg17) and NCBI Build 34 (hg16). In some embodiments, the SNVs are reviewed to remove known single nucleotide polymorphisms (SNPs) from further analysis, such as those identified in the various SNP databases that are publically available. In further embodiments, only those SNVs that are within a coding region of an ENSEMBL gene are selected for further analysis. In addition to identifying the SNVs, the codon containing the mutation and the position of the mutation within the codon (MC-1, MC-2 or MC-3) may be identified. Nucleotides in the flanking 5' and 3' codons may also be identified so as to identify the motifs. In some instances of the methods of the present disclosure, the sequence of the non-transcribed strand (equivalent to the cDNA sequence) of the nucleic acid molecules is analyzed. In other instances, the sequence of the transcribed strand is analyzed. In further instances, the sequences of both strands are analyzed.

Having identified on or more SNVs in a nucleic acid molecule, one or metrics (or genetic indicators of deaminase activity) can be determined by making the appropriate calculations, as set forth above.

### 5. Kits and Systems for Detecting SNVs and Determining Metrics

All the essential materials and reagents required for detecting SNVs may be assembled together in a kit. For example, when the methods of the present disclosure include first isolating and/or sequencing the nucleic acid to be analyzed, kits comprising reagents to facilitate that isolation and/or sequencing are envisioned. Such reagents can include, for example, primers for amplification of DNA, polymerase, dNTPs (including labelled dNTPs), positive and negative controls, and buffers and solutions. Such kits will also generally comprise, in suitable means, distinct containers for each individual reagent. The kit can also feature various devices, and/or printed instructions for using the kit.

In some embodiments, the methods described generally herein are performed, at least in part, by a processing system, such as a suitably programmed computer system. For example, a processing system can be used to analyze the nucleic acid sequence, identify SNVs, and/or determine metrics. A stand-alone computer, with the microprocessor executing applications software allowing the above-described methods to be performed, may be used. Alternatively, the methods can be performed, at least in part, by one or more processing systems operating as part of a distributed architecture. For example, a processing system can be used to identify mutation types, the codon context of a mutation and/or motifs within one or more nucleic acid sequences so as to generate the metrics described herein. In some examples, commands inputted to the processing system by a user assist the processing system in making these determinations.

In one example, a processing system includes at least one microprocessor, a memory, an input/output device, such as a keyboard and/or display, and an external interface, interconnected via a bus. The external interface can be utilised for connecting the processing system to peripheral devices, such as a communications network, database, or storage devices. The microprocessor can execute instructions in the form of applications software stored in the memory to allow the methods of the present disclosure to be performed, as well as to perform any other required processes, such as communicating with the computer systems. The applications software may include one or more software modules, and may be executed in a suitable execution environment, such as an operating system environment, or the like.

### 6. Systems for Generating Therapy Indicators

The present disclosure provides systems and processes for generating a therapy indicator for assessing responsiveness to cancer therapy.

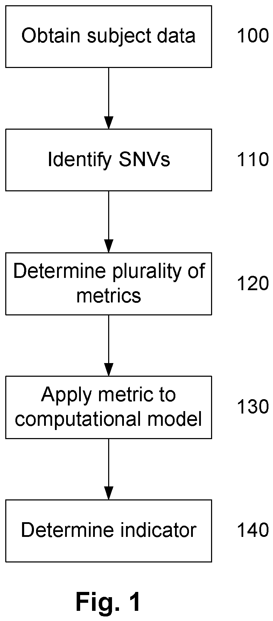

An example of the process for generating a therapy indicator for assessing responsiveness to cancer therapy for a biological subject will now be described with reference to Figure 1.

For the purpose of this example, it is assumed that the method is performed at least in part using one or more electronic processing devices typically forming part of one or more processing systems, such as servers, personal computers or the like and which may optionally be connected to one or more processing systems, data sources or the like via a network architecture as will be described in more detail below.

For the purpose of explanation, the term "reference subject" is used to refer to one or more individuals in a sample population, with "reference subject data" being used to refer to data collected from the reference subjects. The term "subject" refers to any individual that is being assessed for the purpose of identifying a responsiveness to cancer therapy, with "subject data" being used to refer to data collected from the subject. The reference subjects and subjects are animals, and more particularly humans, although this is not intended to be limiting and the techniques could be applied more broadly to other vertebrates and mammals.

In this example, at step 100 subject data is obtained which is at least partially indicative of a sequence of a nucleic acid molecule from the subject. The subject data could be obtained in any appropriate manner, as described above, such as, for example, whole exome sequencing or whole genome sequencing of a biological sample from a subject.

The subject data may also include additional data, such as data regarding subject attributes or other physiological signals measured from the subject, such as measures of physical or mental activity, or the like, as will be described in more detail below.

At step 110 the subject data is analysed to determine identify single nucleotide variations (SNVs) within the nucleic acid molecule, as described above.

At step 120 the identified SNVs are used to determine a plurality of metrics. The metrics used will vary depending upon a range of factors, such as the computational model to be used, subject attributes, the particular type of cancer or cancer therapy being assessed, or the like, as will be described in more detail below. Typically the metrics are selected from groups including i) a coding metric group; ii) a non-coding metric group; iii) a genomic metric group; iv) a codon context metric group; v) a transition/transversion metric group; vi) a synonymous/non-synonymous metric group; vii) a strand bias metric group; viii) a strand specific metric group; ix) an AT/GC metric group; x) a motif metric group; and xi) a motif-independent metric group, as described above, with multiple metrics optionally being selected from across any one or more of these groups. In some examples, the plurality of metrics includes metrics from 3, 4, 5, 6, 7, 8, 9, 10 or all of the metric groups. In other examples, metrics from 1, 2, 3, 4, 5, 6 or all metrics groups selected from i) a motif metric group; ii) a codon context metric group; iii) a transition/transversion metric group; iv) a synonymous/non-synonymous metric group; v) a strand bias metric group; vi) a strand specific metric group; and vii) an AT/GC metric group are used.

At step 130 the one or more metrics are applied to one or more computational models. The computational model(s) typically embody relationship between different responsiveness to therapy and the plurality of metrics, and can be obtained by applying one or more analytical techniques, such as machine learning, conventional clustering, linear regression or Bayesian methods, or any of the other techniques known in the art or described below, to reference metrics derived from a plurality of reference metrics obtained from reference subjects having a known responsiveness to cancer therapy.

**Thus,** it will be appreciated that in practice reference subject data, equivalent to subject data, is collected for a plurality of reference subjects having a different responsiveness to cancer therapy. The collected reference subject data is used to calculate reference metrics, which are then used to train the computational model(s) so that the computational model(s) can discriminate between different responsiveness to cancer therapy, based on metrics derived from the subject's SNVs. The nature of the computational model will vary depending on the implementation and examples will be described in more detail below.

The computational model is used to determine a therapy indicator indicative of the responsiveness to cancer therapy at step 140. Typically the therapy indicator is indicative of whether or not the subject is likely to respond to a particular cancer therapy. This allows a supervising clinician or other medical personnel to assess whether therapy is likely to lead to an improved outcome for the subject.

In one example, the therapy indicator could include a numerical value, for example indicating that there is a 60%, 70%, 80%, 90%, or 95% chance the subject will respond to therapy. However, this is not necessarily essential, and it will be appreciated that any suitable form of therapy indicator could be used.

Accordingly, it will be appreciated that the above described method utilises an analytical technique such as a machine learning technique in order to assess the responsiveness to cancer therapy utilising certain defined metrics relating to specific combinations of metrics. The use of multiple metrics from the different groups can help improve the discriminatory performance of the computational model(s), in turn allowing the responsiveness to cancer therapy to be readily and accurately identified.

In one example, the particular metrics are used in a variety of combinations in order to provide computational models having a discriminatory performance, such as an accuracy, sensitivity, specificity or area under the receiver characteristic operating curve (AUROC) of greater than 70%.

**The** above described approach provides a mechanism for objectively assessing the likelihood of a subject responding to cancer therapy, which can assist in rapidly identifying the most effective therapy, avoiding time being wasted in trying ineffective therapy, and allowing therapies having limited availability to be provided to those who will respond.

A number of further features will now be described.

In one example, the motif metric group comprises a deaminase motif metric group indicative of SNVs in one or more deaminase motifs, such as motifs targeted by a deaminase selected from among activation-induced cytidine deaminase (AID), apolipoprotein B mRNA-editing enzyme, catalytic polypeptide-like (APOBEC) 1 cytosine deaminase (APOBEC1), APOBEC3A, APOBEC3B, APOBEC3C, APOBEC3D, APOBEC3F, APOBEC3G, APOBEC3H and an adenine deaminase acting on RNA (ADAR). The motif metric group can therefore include metrics in any one or more of an ADAR motif group, AID motif group, APOBEC3G motif group, APOBEC3B motif group, APOBEC3F and/or the APOBEC1 motif group, wherein metrics in each group have been determined by assessing SNVs within one or more ADAR, AID, APOBEC3G, APOBEC3B, APOBEC3F or APOBEC1 motifs, respectively.

In one example, the deaminase motif is an AID motif selected from among WRC/GYW, WRCG/CGYW, WRCGS/SCGYW, WRCY/RGYW, WRCGW/WCGYW, WRCR/YGYW and AGCTNT/ANAGCT.

In one example, the deaminase motif is an ADAR motif selected from among WA/TW, WAY/RTW, SWAY/RTWS, CWAY/RTWG, CWAA/TTWG, SWA/TWS, WAA/TTW, WAS/STW, RAWA/TWT and SARA/TYTS.

In one example, the deaminase motif is an APOBEC3G motif selected from among CC/GG, CG/CG, CCGW/WCGG, SCCGW/WCGGS, SCCGS/SCGGS, SCCG/CGGS, CCGS/SCGG, SCGS/SCGS and SGCG/CGCS.

In one example, the deaminase motif is an APOBEC3B motif selected from among TCW/WGA, TCA/TGA, TCWA/TWGA, RTCA/TGAY, YTCA/TGAR, STCG/CGAS, TCGA/TCGA and WTCG/CGAW.

In one example, the deaminase motif is an APOBEC3F motif selected from among TC/GA.

In one example, the deaminase motif is an APOBEC1 motif selected from among CA/TG.

In one example, the motif metric group comprises a 3-mer motif metric group indicative of SNVs in one or more 3-mer motifs, and which can optionally be indicative of SNVs at position 1, 2 and/or 3 of the one or more 3-mer motifs.

In another example, the motif metric group comprises a 5-mer motif metric group indicative of SNVs in one or more 5-mer motifs, which can optionally be indicative of SNVs at position 1, 2, 3, 4 and/or 5 of the one or more 5-mer motifs.

**The** system can use a number of different combinations of computational models, for example depending on the particular discriminatory abilities of the models and the particular cancer therapies of interest.

In one example, the system uses multiple different computational models, which can improve the ability to accurately assess responsiveness to therapy. In this instance, the processing devices apply respective metrics to respective models to determine individual scores, which are then aggregated to determine a therapy indicator.

The nature of the model will vary depending on the implementation, and on example the model could include a decision tree or similar, and in one preferred example, multiple decision trees are used, with results being aggregated. However, it will be appreciated that this is not essential, and other models could be used.

As previously mentioned, multiple metrics are used in order to increase the accuracy of the computational model(s), with these typically being selected from across the groups, in order to maximise the effectiveness of the discriminatory performance of the computational model(s). In one particular example, at least one metric is selected from each of the available groups and optionally at least two metrics are selected for at least some of the available groups.

In general the number of metrics used will vary depending on the implementation and the outcome of training. In a preferred example, the metrics groups include more than five thousand metrics, with the number of metrics used in generating the therapy indicator being a subset of all available metrics. In one example, the number of metrics used includes at least two, at least five, at least ten, at least twenty, at least fifty, at least seventy five, at least one hundred, or at least two hundred. In another example, the system uses at least 0.1%, at least 0.2%, at least 0.3%, at least 0.4%, at least 0.5%, at least 0.75%, at least 1%, at least 1.5%, or at least 2% of all available metrics.

The analysis is also typically performed to take into account subject attributes, such as subject characteristics, possible medical conditions suffered by the subject, possible interventions performed, or the like. In this example, the one or more processing devices can use the one or more subject attributes to apply the computational model so that the metrics are assessed based on reference metrics derived for one or more reference subjects having similar attributes to the subject attributes. This can be achieved in a variety of ways, depending on the preferred implementation, and can include selecting metrics and/or one of a number of different computational models at least in part depending on the subject attributes. Irrespective of how this is achieved, it will be appreciated that taking into account subject attributes can further improve the discriminatory performance by taking into account that subjects with different attributes may react different to the same therapy.

The subject attributes could include subject characteristics such as a subject age, height, weight, sex or ethnicity, body states, such as a healthy or unhealthy body states or one or more disease states, such as whether the subject is obese. The subject attributes could include one or more medical symptoms, such as an elevated temperature, heart rate, or blood pressure, whether the subject is suffering from nausea, or the like. Finally, the subject attributes could include dietary information, such as details of any food or drink consumed, or medication information, including details of any medications taken either as part of a medication regimen or otherwise.

The subject attributes could be determined in any one of a number of ways, for example by way of a clinical assessment, by querying a patient medical record, based on user input commands, or by receiving sensor data from a sensor, such as a weight or heart activity sensor, or the like.

In one example, the one or more processing devices display a representation of the therapy indicator, store the therapy indicator for subsequent retrieval or provide the therapy indicator to a client device for display. Thus, it will be appreciated that the mental state indicator can be used in a variety of manners, depending on the preferred implementation.

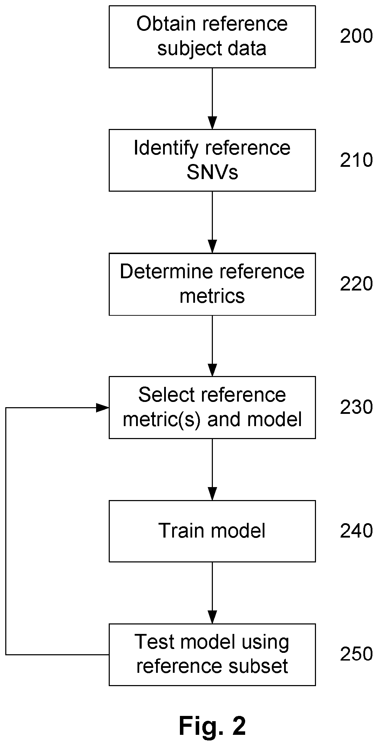

The above described approaches use one or more computational models in order to determine a therapy indicator, and an example of a process for generating such model(s) will now be described with reference to Figure 2.

In this example, reference subject data is obtained at step 200, which is indicative of a sequence of a nucleic acid molecule from the reference subject, as well as responsiveness to a particular cancer therapy. At step 210 the reference subject data is analysed to identify SNVs within the nucleic acid molecule. At step 220 the reference subject data is analysed to determine reference metrics.

Steps 200 to 220 are largely analogous to steps 100 to 120 described with respect to obtaining and analysing subject data of a subject, and it will therefore be appreciated that these can be performed in a largely similar manner, and hence will not be described in further detail.

In contrast to subject data however, as the reference subject data is used in training a computational model, it is typically to determine reference metrics for all available metrics, rather than just selected ones of the metrics, allowing this to be used in order to ascertain which of the metrics are most useful in discriminating between individuals that are responsive or not responsive to cancer therapy. Nevertheless, the reference metrics used are as outlined above, and typically include metrics selected from groups including i) a coding metric group; ii) a non-coding metric group; iii) a genomic metric group; iv) a codon context metric group; v) a transition/transversion metric group; vi) a synonymous/non-synonymous metric group; vii) a strand bias metric group; viii) a strand specific metric group; ix) an AT/GC metric group; x) a motif metric group; and xi) a motif-independent metric group, as described above, with multiple metrics optionally being selected from across these groups.

At step 230 a combination of the reference metrics and one or more generic computational models are selected, with the reference metrics and responsiveness to therapy being used to train the model at step 240. The nature of the model and the training performed can be of any appropriate form and could include any one or more of decision tree learning, random forest, logistic regression, association rule learning, artificial neural networks, deep learning, inductive logic programming, support vector machines, clustering, Bayesian networks, reinforcement learning, representation learning, similarity and metric learning, genetic algorithms, rule-based machine learning, learning classifier systems, or the like. As such schemes are known, these will not be described in any further detail.

Accordingly, the above described process provides a mechanism to develop a computational model that can be used in generating a mental state indicator using the process described above with respect to Figure 1.

In addition to simply generating the model, the process typically includes testing the model at step 250 to assess the discriminatory performance of the trained model. Such testing is typically performed using a subset of the reference subject data, and in particular, different reference subject data to that used to train the model, to avoid model bias. The testing is used to ensure the computational model provides sufficient discriminatory performance. In this regard, the discriminatory performance is typically based on an accuracy, sensitivity, specificity and AUROC, with a discriminatory performance of at least 70% being required in order for the model to be used.

It will be appreciated that if the model meets the discriminatory performance, it can then be used in determining a therapy indicator using the process outlined above with respect to Figure 1. Otherwise, the process returns to step 230 allowing different metrics and/or models to be selected, with training and testing then being repeated as required.

Thus, in one example, the one or more processing devices select a plurality of reference metrics, typically selected as a subset of each of the available metrics listed above, train one or more computational models using the plurality of reference metrics, test the computational models to determine a discriminatory performance of the model(s) and if the discriminatory performance of the model(s) falls below a threshold then selectively retrain the computational model(s) using a different plurality of reference metrics and/or a plurality of metrics from different reference subject data and/or train different computational model(s). Accordingly, it will be appreciated that the above described process can be performed iteratively utilising different metrics and/or different computational models until a required degree of discriminatory power is obtained.

Thus, in one example, the one or more processing devices train the model using at least 1000 metrics, at least 2000 metrics, at least 3000 metrics, at least 4000 metrics or at least 5000 metrics, with the resulting models typically using significantly less metrics, such as less than 500 or the like.

Additionally and/or alternatively, the one or more processing devices can select a plurality of combinations of reference metrics, train a plurality of computational models using each of the combinations, test each computational model to determine a discriminatory performance of the model and select one or more of the computational models with the highest discriminatory performance for use in determining a therapy indicator.

In addition to use the metrics to train the models, the training can also be performed taking into account reference subject attributes, so that models are specific to respective reference subject attributes or can take the subject attributes into account when determining the therapy responsiveness. In one example, this process involves having the one or more processing devices perform clustering using the using the reference subject attributes to determine clusters of reference subjects having similar reference subject attributes, for example using a clustering technique such as k-means clustering, and then training the computational model at least in part using the reference subject clusters. For example clusters of reference individuals suffering from a particular form of cancer, or undertaking particular therapy could be identified, with this being used to train a computational model to identify responsiveness to therapy for particular cancers.

Accordingly, the above described techniques provide a mechanism for training one or more computational models to determine the responsiveness to cancer therapy using a variety of different metrics, and then using the model(s) to generate therapy indicators indicative of the likely effectiveness of therapy.

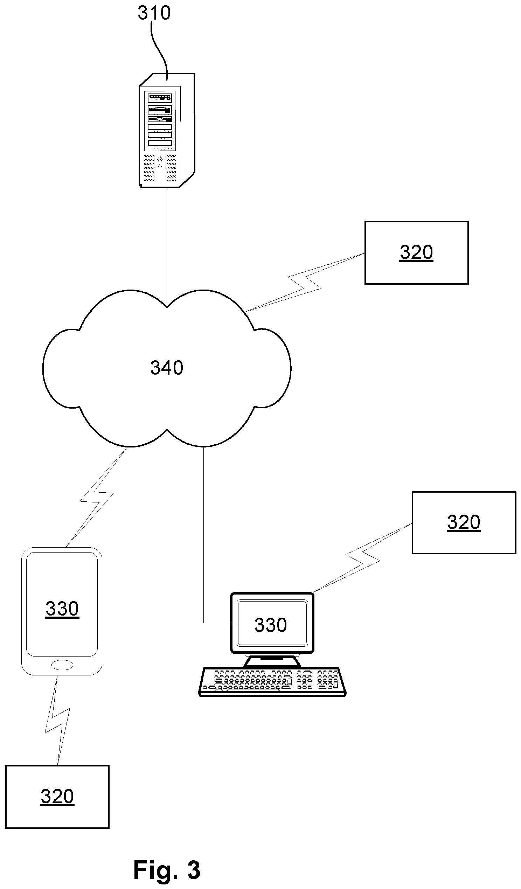

An example of a monitoring system will now be described in more detail with reference to Figure 3.

In this example, one or more processing systems 310 are provided coupled to one or more client devices 330, via one or more communications networks 340, such as the Internet, and/or a number of local area networks (LANs). A number of sequencing devices 320 are provided, with these optionally being connected directly to the processing systems 310 via the communications networks 340, or more typically, with these being coupled to the client devices 330.

Any number of processing systems 310, sequencing devices 320 and client devices 330 could be provided, and the current representation is for the purpose of illustration only. The configuration of the networks 340 is also for the purpose of example only, and in practice the processing systems 310, sequencing devices 320 and client devices 330 can communicate via any appropriate mechanism, such as via wired or wireless connections, including, but not limited to mobile networks, private networks, such as an 802.11 networks, the Internet, LANs, WANs, or the like, as well as via direct or point-to-point connections, such as Bluetooth, or the like.

In this example, the processing systems 310 are adapted to receive and analyse subject data received from the sequencing devices 320 and/or client devices 330, allowing computational models to be generated and used to determine therapy indicators, which can then be displayed via the client devices 330. Whilst the processing systems 310 are shown as single entities, it will be appreciated they could include a number of processing systems distributed over a number of geographically separate locations, for example as part of a cloud based environment. Thus, the above described arrangements are not essential and other suitable configurations could be used.

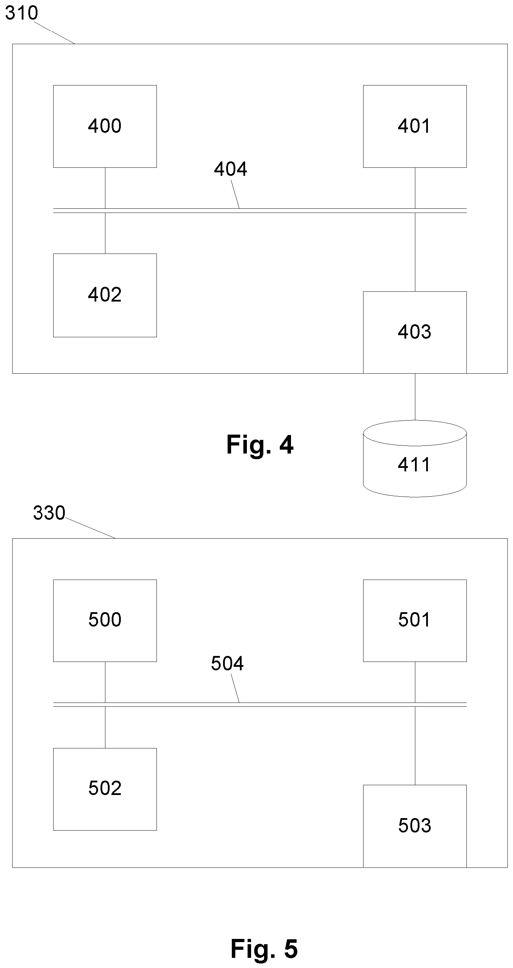

An example of a suitable processing system 310 is shown in Figure 4. In this example, the processing system 310 includes at least one microprocessor 400, a memory 401, an optional input/output device 402, such as a keyboard and/or display, and an external interface 403, interconnected via a bus 404 as shown. In this example the external interface 403 can be utilised for connecting the processing system 310 to peripheral devices, such as the communications networks 340, databases 411, other storage devices, or the like. Although a single external interface 403 is shown, this is for the purpose of example only, and in practice multiple interfaces using various methods (eg. Ethernet, serial, USB, wireless or the like) may be provided.

In use, the microprocessor 400 executes instructions in the form of applications software stored in the memory 401 to allow the required processes to be performed. The applications software may include one or more software modules, and may be executed in a suitable execution environment, such as an operating system environment, or the like.

Accordingly, it will be appreciated that the processing system 310 may be formed from any suitable processing system, such as a suitably programmed PC, web server, network server, or the like. In one particular example, the processing system 310 is a standard processing system such as an Intel Architecture based processing system, which executes software applications stored on non-volatile (e.g., hard disk) storage, although this is not essential. However, it will also be understood that the processing system could be any electronic processing device such as a microprocessor, microchip processor, logic gate configuration, firmware optionally associated with implementing logic such as an FPGA (Field Programmable Gate Array), or any other electronic device, system or arrangement.

As shown in Figure 5, in one example, the client device 330 includes at least one microprocessor 500, a memory 501, an input/output device 502, such as a keyboard and/or display, an external interface 503, interconnected via a bus 504 as shown. In this example the external interface 503 can be utilised for connecting the client device 330 to peripheral devices, such as the communications networks 340, databases, other storage devices, or the like. Although a single external interface 503 is shown, this is for the purpose of example only, and in practice multiple interfaces using various methods (eg. Ethernet, serial, USB, wireless or the like) may be provided. The card reader 504 can be of any suitable form and could include a magnetic card reader, or contactless reader for reading smartcards, or the like.

In use, the microprocessor 500 executes instructions in the form of applications software stored in the memory 501, and to allow communication with one of the processing systems 310 and/or sequencing devices 320.

Accordingly, it will be appreciated that the client device 330 be formed from any suitably programmed processing system and could include suitably programmed PCs, Internet terminal, lap-top, or hand-held PC, a tablet, a smart phone, or the like. However, it will also be understood that the client device 330 can be any electronic processing device such as a microprocessor, microchip processor, logic gate configuration, firmware optionally associated with implementing logic such as an FPGA (Field Programmable Gate Array), or any other electronic device, system or arrangement.

Examples of the processes for generating therapy indicators will now be described in further detail. For the purpose of these examples it is assumed that one or more respective processing systems 310 are servers adapted to receive and analyse subject data, and generate and provide access to therapy indicators. The servers 310 typically execute processing device software, allowing relevant actions to be performed, with actions performed by the server 310 being performed by the processor 400 in accordance with instructions stored as applications software in the memory 401 and/or input commands received from a user via the I/O device 402. It will also be assumed that actions performed by the client devices 330, are performed by the processor 500 in accordance with instructions stored as applications software in the memory 501 and/or input commands received from a user via the I/O device 502.

However, it will be appreciated that the above described configuration assumed for the purpose of the following examples is not essential, and numerous other configurations may be used. It will also be appreciated that the partitioning of functionality between the different processing systems may vary, depending on the particular implementation.

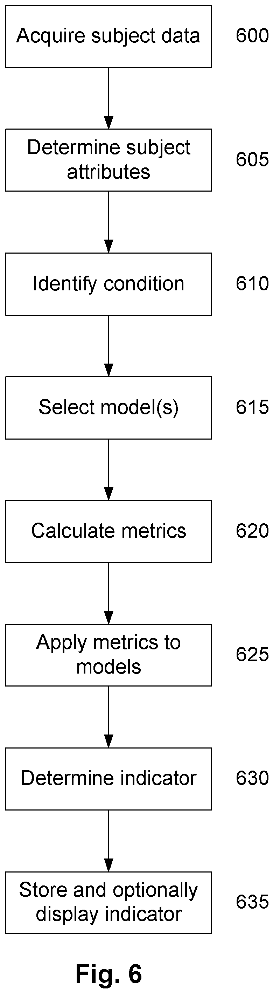

An example of the process for analysing subject data for an individual will now be described in more detail with reference to Figure 6.

In this example, at step 600 the server 310 obtains subject data, either retrieving this from a stored record or receiving this from a sequencing device, optionally via a client device 330, depending upon the preferred implementation.

At step 605, the server 310 determines subject attributes, for example by retrieving these from a database, or obtaining these as part of the subject data. The subject attributes can be used for selecting one or more computational models to be used and/or may be combined with the metrics in order to allow the computational model(s) to be applied. In this regard, the metrics for the subject are typically analysed based on reference metrics for reference subjects having similar attributes to the subject. This could be achieved by using different computational models for different combinations of attributes, or by using the attributes as inputs to the computational model.

At step 610, the server 310 determines a cancer type of the cancer suffered by the subject, using this to select one or more computational models at step 615. In this regard, different computational models will typically be used to assess responsiveness for different types of cancer.

Having selected a model, at step 620, the server 310 then calculates the relevant metrics required by the model.

At step 625 metrics are applied to the computational model(s), for example by using the relevant metrics, optionally together with one or more subject attributes, to perform a decision tree assessment, resulting in the generation of an indicator that is indicative of the effectiveness of therapy at step 630.

At step 635, the server 310 stores the therapy indicator, typically as part of the subject data, optionally allowing the therapy indicator to be displayed, for example by forwarding this to the client device for display.

A specific example of a machine learning approach will now be described in more detail.

In this example, sequencing data are run through the above described process and metrics of interest, including for example those associated with deaminase activity (i.e. genetic indicators of deaminase activity), are identified and quantified with these being collated patient to build a profile.

This is then used to identify patient profiles that are 'normal' (e.g. those who respond to cancer immunotherapy) and those that are dysfunctional (e.g. those that are not able to respond). There are many ways the data can be analysed and the following approach described herein is tailored to cancer immunotherapy. These techniques provide an effective method to analyse the large amount of data produced, and analysis has shown these techniques provide the best predictive accuracy.

Initially, the sequence data is collected and used to produce approximately 5,500 metrics for each patient, depending on the type of sequence data available (i.e. coding, non-coding, genomic) (see Table G). The raw results can be exported and analysed by cleaning the data (e.g. metadata not required for analysis are removed) before patients are grouped for analysis.

To prove effectiveness of the process, a number of cancer patients from various clinical trials for different immunotherapies have been analysed, with the patients being grouped into three categories: training data, tuning data and validation data. The training and tuning datasets are comprised of a large number of patients from multiple clinical trials ('studies'), with patients split into each group randomly; the validation dataset is comprised of patients from a single study (this is the data being predicted).

A typical experimental approach is to 'set aside' the validation dataset (the data being predicted) and collate the rest of the patients together. The collated patients are then split 75:25 (with an ~equal proportion of Responders / Non-Responders) into training (~75%) and tuning (~25%) datasets.

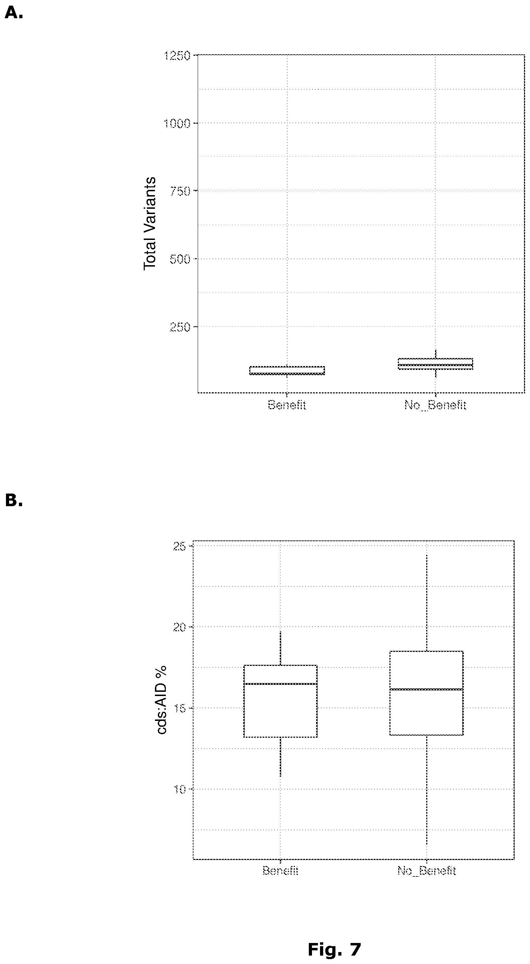

Once the data are grouped, responders/non-responders can be plotted for each metric for patients in the validation dataset. Plotting the data provides a method for further investigating metrics identified by the machine learning analysis as being important, although isn't directly involved in any of the calculations/analyses. Example plots are shown in Figures 7A and 7B.

Each dot represents the value for each patient for the total number of variants (metric #1; left) and for the proportion of variants associated with a specific deaminase motif (metric #4; right). It will be appreciated that in practice some graphs could be constructed for each metric, and this allows further analysis of metrics to be performed. For example, if a metric is determined to be important for predicting response to immunotherapy, the plot for that metric can be examined and used to assess whether there are potential confounding factors e.g. if there are outliers influencing the result.

Once the data are grouped and formatted appropriately, the machine learning algorithm is applied to generate the computational model. In one example, the algorithm used is XGBoost, which is an implementation of 'gradient boosting decision trees', which are specifically designed for speed and performance on large datasets (millions of data points).

The approach calculates a large number of decision trees and checks each decision tree to find the one that maximizes the predictive score on the training dataset. The predictive model can then be applied for predictive purposes. In practice, the preferred approach uses an 'ensemble' of decision trees, each using different combinations of metrics, to make predictions, thereby increasing accuracy.

This approach can be very computationally expensive and can result in many millions of possible trees and many possible ensembles. In general, to optimise this approach each model is trained using a subset of metrics, typically >100 in each case, and whilst a single metric could be used, in practice there are generally >50 for models with a reasonable level of accuracy.

There are a number of parameters that can be adjusted when building the XGBoost model and so multiple passes can be conducted to optimize settings, with the optimized settings then being used. The optimisation is conducted without human interference (various combinations of settings are tested and the computer identifies which settings are optimal) making this approach consistent, reproducible, and minimises susceptibility to experimenter bias.

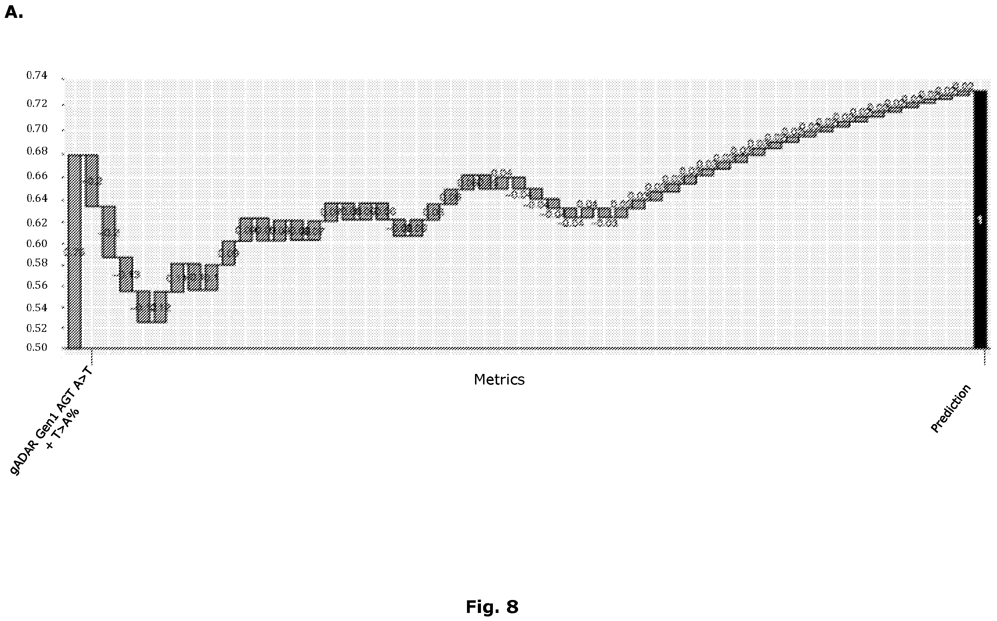

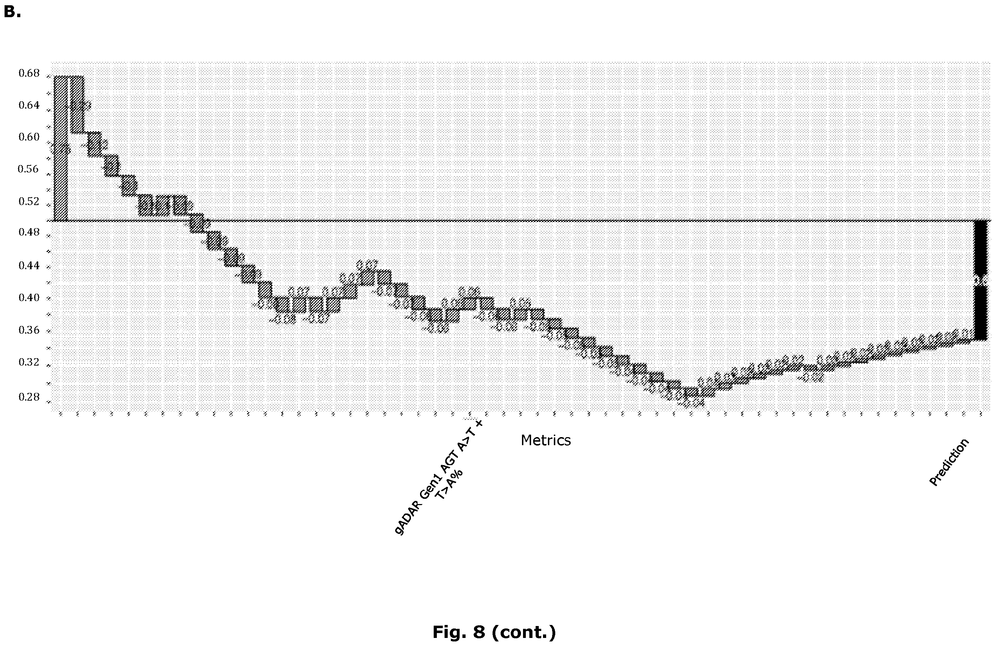

Once the model is built, tuned and applied to the data, it is possible to determine which metrics were important for the predictions made. The contribution of each metric to the overall prediction is cumulative, with the score for specific variables contributing in a 'weighted' manner to the overall prediction (i.e. the score for one metric may indicate the subject is responder, but the score for another metric may indicate that the subject is not a responder). Waterfall plots illustrating this are shown in Figures 8A and 8B, with a number of metrics plotted on the x-axis but only a single highlighted metric identified for simplicity.

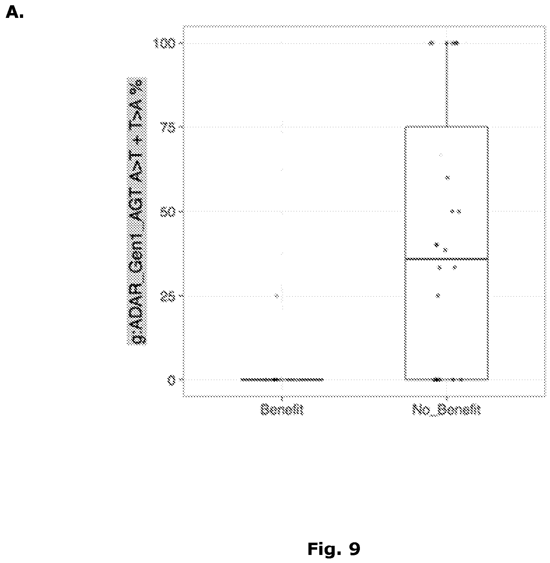

In these examples, the highlighted metric (gADAR Gen1 AGT A>T + T>A%) was important for each patient, but the scores (0 compared to 40) suggest one patient is a responder, and one patient is a non-responder. Examination of the plot shown in Figure 9A shows that there is a difference between patients who responded to treatment ("Benefit") and those that did not ("No_Benefit").

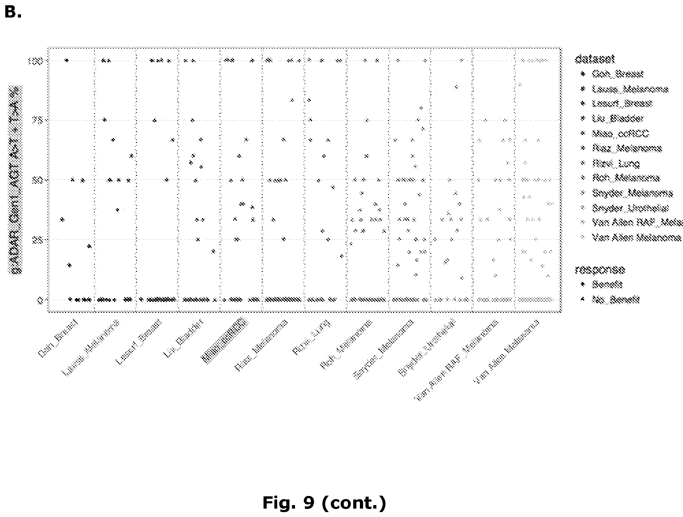

In this example (for this specific metric), patients that had a score >25% were all non-responders. This shows the metric is important for predicting patient response if the score is >25% (as shown in Figure 8A), but less important if the score is 0 (as shown in Figure 8B). This is not a biomarker, as a score of 0 could place the patient in either category, but this metric can differentiate between patients. Looking at this metric in multiple datasets, as shown in Figure 9B, this metric is relatively important for predicting patient response to immunotherapy across the board.

Using this machine learning approach, patient outcome can be predicted with good accuracy when applied to 'real world' datasets (see Example 8).

## 7. Diagnostic and Therapeutic Applications

Using the methods and systems described herein to detect SNVs in the nucleic acid molecule of a subject, generate one or more metrics (or genetic indicators of endogenous deaminase activity) and/or generate a therapy indicator, the likelihood that a subject with cancer will respond to cancer therapy, or will continue to respond to cancer therapy, can be determined. Thus, the methods and systems described herein can also be used to determine the likelihood of disease progression in a subject already on cancer therapy (i.e. whether they will continue to respond to therapy or whether the cancer will develop resistance to the therapy and the subject will cease responding to the therapy, thereby resulting in disease progression, e.g. relapse). Such determinations can facilitate the prescribing of a management program or treatment regimen for a subject. For example, if it is determined that the subject is likely to respond to a particular therapy, then treatment of the subject with that therapy can be initiated. Conversely, if it is determined that the subject is unlikely to respond to a particular therapy, then other therapies can be considered and utilized for that subject. Similarly, if it is determined that a subject already on therapy is likely to cease responding to the therapy (i.e. is likely to develop resistance to that therapy), then the subject can be taken off that therapy and a new treatment regime can be initiated.

As demonstrated in the examples below, subjects who respond to cancer therapy have a different profile of metrics compared to those that do not respond to cancer therapy. A profile of metrics or genetic indicators of deaminase activity for a subject, i.e. a sample profile, can therefore be generated and compared to a reference profile of metrics or genetic indicators of deaminase activity so as to determine whether the subject is likely or unlikely to respond to cancer therapy. Profiles of the present disclosure reflect an evaluation of at least any 1, 2, 3, 4, 5, 6, 7, 8, 9, 10, 20, 30, 40 or more metrics or genetic indicators of endogenous deaminase activity as described above. Reference profiles correlate with a likelihood of responsiveness to cancer therapy and are typically predetermined, although can also be determined at the time of or after determining a sample profile.

Reference profiles are determined based on data obtained in the evaluation of reference metrics (or genetic indicators of deaminase activity) in individuals that have a known responsiveness to cancer therapy. Thus, for example, the reference profiles can be based on the data obtained in the evaluation of metrics or indicators in individuals that responded to cancer therapy. Accordingly, the reference profile can correlate with, for example, a likelihood of responsiveness to therapy. In other examples, the reference profile is based on the data obtained in the evaluation of metrics or indicators in individuals that did not respond to cancer therapy. In such instances, the reference profile correlates to, for example, a likelihood of non-responsiveness to therapy. The individuals used to generate the reference profile may be age, gender and/or ethnicity matched or not.

A profile of metrics or genetic indicators of deaminase activity comprises an assessment of at least 2 metrics or indicators (e.g., 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 25 or more, 30 or more, 40 or more, 50 or more, 60 or more, 70 or more, 80 or more, 90 or more, 100 or more, 200 or more, 300 or more or 400 or more). The indicators or metrics can be associated with the same or different groups of indicators or metrics, as described above.

### 7.1 Range intervals

In some embodiments, reference profiles are generated based on predetermined therapy-responsive range intervals for each metric or indicator assessed. In such embodiments, response to therapy or continued response to therapy is likely when no metrics (or genetic indicators of endogenous deaminase activity) are outside a predetermined therapy-responsive range interval for the metrics (or genetic indicators of endogenous deaminase activity), i.e. all metrics or genetic indicators are within a predetermined therapy-responsive range interval for the metric or genetic indicator of endogenous deaminase activity. Conversely, response to therapy or continued response to therapy is unlikely (or non-response to therapy or development of resistance to therapy is likely) when one or more metrics or genetic indicators of endogenous deaminase activity are outside a predetermined therapy-responsive range interval for the metric or genetic indicator of endogenous deaminase activity. In some examples, 1, 2, 3, 4, 5, 6, 7, 8, 9, 10, 20, 30 40 or more metrics or genetic indicators of endogenous deaminase activity outside a predetermined therapy-responsive range interval indicates that response to therapy or continued response to therapy is unlikely (or that non-response to therapy or development of resistance to therapy is likely).

In some examples, a score is attributed to each metric or genetic indicator of endogenous deaminase activity that is outside a predetermined range interval, and the total score is then calculated by combining all of the scores. Response to therapy or continued response to therapy is then determined to be likely when the score is equal to or above a threshold score. Conversely, response to therapy or continued response to therapy is determined to be unlikely (or non-response to therapy or development of resistance to therapy is determined to be likely) when the score is equal to or below a threshold score. The threshold score represents a score that differentiates those likely to respond or likely to continue to respond to therapy, and those unlikely to respond or unlikely to continue to respond to therapy, and can be readily established by those skilled in the art based on values and scores obtained using control subjects (e.g. positive control subjects known to have responded to therapy or to have continued to respond to therapy, and/or negative control subjects known to have not responded to therapy or to have discontinued response to therapy). The score for each metric or genetic indicator of endogenous deaminase activity may be the same or may be different (e.g. may be "weighted" such that one metric or genetic indicator of endogenous deaminase activity that is outside a predetermined range interval might be given a score that is more or less than another metric or genetic indicator of endogenous deaminase activity that is outside a predetermined range interval). In a particular example, each metric or genetic indicator of endogenous deaminase activity that is outside a predetermined range interval is given a score of 1. In some embodiments, the threshold score is 1 such that any score of 1 and above results in a determination that response to therapy or continued response to therapy is unlikely (or that non-response to therapy or development of resistance to therapy is likely).

The predetermined therapy-responsive range interval for a metric or genetic indicator of endogenous deaminase activity can be determined by assessing a metric or genetic indicator of deaminase activity in two or more subjects that are known to be responding to a particular therapy, or that are known to have responding to a therapy over an extended period of time, e.g. weeks, months or years (i.e. the positive control subjects). A therapy-responsive range interval for the metric or genetic indicator is then calculated to set the upper and lower limits of what would be considered target values for that metric or indicator, i.e. values that reflect a level or quality of deaminase activity that is associated with or that supports response to a therapy. In a particular example, the therapy-responsive range interval is set using the maximum value for that metric or indicator observed in control subjects as the upper limit of the interval, and the minimum observed value for that indicator as the lower limit of the interval. These upper and lower limits may be adjusted, such as by increasing or decreasing the limit by 2.5%, 5%, 10%, 15%, 20% or more. In a further example, the therapy-responsive range interval is calculated by measuring the average plus or minus 2 standard deviations, whereby the lower limit of the range interval is the average minus 2 standard deviations and the upper limit of the range interval is the average plus 2 standard deviations. In other examples, less than or more than 2 standard deviations are used to set the upper and lower limits of the interval, such as 1, 1.5, 2.5, 3, 3.5 or more standard deviations. In still further examples, the upper and lower limits of the predetermined normal range interval are established using receiver operating characteristic (ROC) curves. In still further examples, the upper and lower limits of the predetermined normal range interval are established using receiver operating characteristic (ROC) curves. The subjects used to determine the predetermined therapy-responsive range interval can be of any age, sex or background, or may be of a particular age, sex, ethnic background or other subpopulation. Thus, in some embodiments, two or more predetermined normal range intervals can be calculated for the same metric or genetic indicator of deaminase activity, whereby each range interval is specific for a particular subpopulation, e.g. a particular sex, age group, ethnic background and/or other subpopulation. As would be appreciated, predetermined therapy-responsive range intervals may be specific for the type of cancer and the cancer therapy. Thus, typically, the predetermined therapy-responsive range intervals are established using subjects with the same type of cancer and undergoing the same type of cancer therapy as the subject that is being assessed using the methods of the present disclosure. The predetermined therapy-responsive range interval can be determined using any technique know to those skilled in the art, including manual methods of calculation, an algorithm, a neural network, a support vector machine, deep learning, logistic regression with linear models, machine learning, artificial intelligence and/or a Bayesian network.

7.2 <u>Machine learning</u>

In particular embodiments, reference profiles are produced using, and encompass, computational models, such as those formed using various analytical techniques such as machine learning techniques. Computational models can be formed using any suitable statistical classification or learning method that attempts to segregate bodies of data into classes based on objective parameters present in the data. Classification methods may be either supervised or unsupervised. Examples of supervised and unsupervised classification processes are described in Jain, "Statistical Pattern Recognition: A Review", IEEE Transactions on Pattern Analysis and Machine Intelligence, Vol. 22, No. 1, January 2000.

Non-limiting examples of techniques that can be used to produce classification moedles include deep learning techniques such as Deep Boltzmann Machine, Deep Belief Networks, Convolutional Neural Networks, Stacked Auto Encoders; ensemble techniques such as Random Forest, Gradient Boosting Machines, Boosting, Bootstrapped Aggregation, AdaBoost, Stacked Generalization, Gradient Boosted Regression Trees; neural network techniques such as Radial Basis Function Network, Perceptron, Back-Propagation, Hopfield Network; regularization methods such as Ridge Regression, Least Absolute Shrinkage and Selection Operator, Elastic Net, Least Angle Regression; regression methods such as Linear Regression, Ordinary Least Squares Regression, Multiple Regression, Probit Regression, Stepwise Regression, Multivariate Adaptive Regression Splines, Locally Estimated Scatterplot Smoothing, Logistic Regression, Support Vector Machines, Poisson Regression, Negative Binomial Regression, Multinomial Logistic Regression; Bayesian techniques such as Naïve Bayes, Average One-Dependence Estimators, Gaussian Naive Bayes, Multinomial Naive Bayes, Bayesian Belief Network, Bayesian Network; decision trees such as Classification and Regression Tree, Iterative Dichotomiser, C4.5, C5.0, Chi-squared Automatic Interaction Detection, Decision Stump, Conditional Decision Trees, M5; dimensionality reduction such as Principle Component Analysis, Partial Least Squares Regression, Sammon Mapping, Multidimensional Scaling, Projection Pursuit, Principle Component Regression, Partial Least Squares Discriminant Analysis, Mixture Discriminant Analysis, Quadratic Discriminant Analysis, Regularized Discriminant Analysis, Flexible Discriminant Analysis, Linear Discriminant Analysis, t-Distributed Stochastic Neighbour Embedding; instance-based techniques such as K-Nearest Neighbour, Learning Vector Quantization, Self-Organizing Map, Locally Weighted Learning; clustering methods such as k-Means, k-Modes, k-Medians, DBSCAN, Expectations Maximization, Heirarchical Clustering; adaptations, extensions, and combinations of the previously mentioned approaches.

Data from individuals who are known to have responded to therapy or not responded to therapy can be used to train a computational model as described in more detail above. Such data is typically referred to as a training data set. Once trained, the computational model can recognize patterns in data generated using unknown samples, e.g. the data from patients with cancer used to generate the sample profiles. The sample profile can then be applied to the computational model to classify the sample profile into classes, e.g. likely to respond to cancer therapy or unlikely to respond to cancer therapy.

7.3 <u>Cancers and cancer therapies</u>

The methods and systems of the present disclosure can be used to determine the likelihood of response, or continued response (or the likelihood of non-response or the development of resistance) to any cancer therapy, including but not limited to radiation, non-targeted chemotherapy, hormone therapy, targeted therapy and immunotherapy. The cancer therapy may be monovalent (i.e. a single therapy) or combination therapy. In particular embodiments, the methods are used in relation to targeted therapy and immunotherapy, including antibody-based targeted therapies and immunotherapies (i.e. targeted therapies and immunotherapies that comprise an antibody).

Exemplary cancers include, but are not limited to, breast, prostate, liver, colorectal, gastrointestinal, pancreatic, skin, thyroid, cervical, lymphoid (e.g. non-Hodgkin lymphoma, Hodgkin lymphoma, myeloma and lymphocytic leukemia), haematopoietic (i.e. blood), bladder, lung, renal, ovarian, uterine, and head or neck cancer.

Radiotherapies include radiation and waves that induce DNA damage for example, $\gamma$-irradiation, X-rays, UV irradiation, microwaves, electronic emissions, radioisotopes, and the like. Therapy may be achieved by irradiating the localized tumour site with the above described forms of radiations. It is most likely that all of these factors effect a broad range of damage DNA, on the precursors of DNA, the replication and repair of DNA, and the assembly and maintenance of chromosomes.

Non-targeted chemotherapy (i.e. traditional chemotherapy that does not involve targeting of a particular molecule or cell type, as compared to targeted therapy, but instead broadly affects rapidly-dividing cells) includes, but is not limited to, alkylating agents such as altretamine, busulfan, carboplatin, carmustine, chlorambucil, cisplatin, cyclophosphamide, dacarbazine, lomustine, melphalan, oxaliplatin, temozolomide and thiotepa; antimetabolites such as 5-fluorouracil (5-FU), 6-mercaptopurine (6-MP), aapecitabine (Xeloda®), aytarabine (Ara-C®), floxuridine, fludarabine, gemcitabine (Gemzar®), hydroxyurea, methotrexate, and pemetrexed (Alimta®); anti-tumor antibiotics such as anthracyclines (e.g. daunorubicin, doxorubicin (Adriamycin®), epirubicin and idarubici), actinomycin-D, bleomycin, mitomycin-C and mitoxantrone; topoisomerase inhibitors, topotecan, irinotecan (CPT-11), etoposide (VP-16), teniposide and mitoxantrone; mitotic inhibitors such as docetaxel, estramustine, ixabepilone, paclitaxel, vinblastine, vincristine, vinorelbine; and corticosteroids such as prednisone, methylprednisolone (Solumedrol®) and dexamethasone (Decadron®).

Hormone therapy serves to block or lower the level of a hormone, such as estrogen and/or progesterone, in the subject. Non-limiting examples or hormone therapies include tamoxifen, raloxifene, anastrozole, letrozole and exemestane.

Targeted cancer therapies include drugs (i.e. small molecules) and proteins (including antibodies) that interact with molecules that are specific for or associated with a cancer cell or cancer cell proliferation. Targeted therapies typically act in a cytostatic manner to inhibit cancer cell proliferation, although can also be cytotoxic. Targeted therapies include, for example, drugs (e.g. tyrosine kinase inhibitors) and monoclonal antibodies (including chimeric, humanized or fully human antibodies, whether naked or conjugated with a toxic moiety) specific for ABL, Anaplastic lymphoma kinase (ALK), Beta-1,4 N-acetylgalactosaminyltransferase 1 (B4GALNT1), B-cell activating factor (BAFF), B-Raf, Bruton's tyrosine kinase (BTK), CD19, CD20, CD27, CD30, CD38, CD52, CD137 cytotoxic T-Lymphocyte associated protein 4 (CTLA-4), epidermal growth factor receptor (EGFR), FMS-like tyrosine kinase-3 (FLT3), histone deacetylase (HDAC), human epidermal growth factor receptor 2 (HER-2), isocitrate dehydrogenase 1 (IDH1), IDH2, interleukin 1 beta (IL-1β), IL-6, IL-6R, c-KIT, MEK, MET, mTOR, Poly (ADP-ribose) polymerase (PARP), programmed cell death protein 1 (PD-1), Nectin-4, platelet-derived growth factor receptors a (PDGFRa), PDGFRβ, programmed death-ligand 1 (PD-L1), phosphatidylinositol-3-kinase delta (PI3Kδ), receptor activator of nuclear factor kappa-B ligand (RANKL), RET, ROS1, signaling lymphocytic activation molecule F7 (SLAMF7), vascular endothelial growth factor (VEGF), VEGF receptor (VEGFR) and VEGFR2. In some instances, these targeted therapies target or exploit the immune system and can therefore also be considered cancer immunotherapies.

Cancer immunotherapy functions by exploiting or utilizing the immune system of the patient to treat cancer. This can be through several mechanisms and by using different strategies, including non-specific stimulation of immune responses by stimulating effector cells and/or inhibiting regulatory cells (e.g. by administration of cytokines such as IL-2 and IFN-a, or drugs such as thalidomide (Thalomid®), ienalidomide (Revlimid®), pomalidomide (Pomalyst®) and miquimod (Zyclara®)), active immunization to stimulate or enhance specific anti-cancer immune responses (i.e. using cancer vaccines such as the HPV vaccines Gardasil® and Cevarix® for the prevention of cervical cancer, Sipuleucel-T (Provenge®) for the treatment of prostate cancer, and the Bacillus Calmette-Guérin (BCG) vaccine for the treatment of bladder cancer), and passive transfer of antibodies or activated immune cells with anti-cancer activity (adoptive cell therapy (ACT); e.g. Chimeric antigen receptor (CAR) T-cell therapy).

Antibodies that have been developed as cancer immunotherapies include, for example, immune checkpoint inhibitor antibodies and antibodies that target molecules on cancer cells so as to induce an immune response to the cancer cell (e.g. anti-CD52 antibodies). Immune checkpoint inhibitor antibodies target immune checkpoints that naturally inhibit or dampen an immune response and which are co-opted by cancers to evade the host immune system. These immune checkpoints involve cytotoxic T lymphocyte-associated 4 (CTLA-4) and programmed cell death protein 1 (PD-1), which function as negative regulators of the immune response. CTLA-4 attenuates the early activation of naïve and memory T cells, while PD-1 typically modulates T cell activity in peripheral tissues through interaction with its ligands, PD-L1 and PD-L2. Immune checkpoint inhibitor antibodies target these molecules, and in particular CTLA-4, PD-1 and PD-L1, to inhibit the immune attenuating activity of these molecules and stimulate or enhance the antitumour immunity. Antibodies that have been developed as cancer immunotherapies also include antibodies that induce complement dependent cytotoxicity (CDC) or antibody-dependent cellular cytotoxicity (ADCC). Such antibodies may target a molecule expressed on the cancer cell (e.g. HER-2) and induce CDC or ADCC so as to kill the cancer cell.

In particular embodiments, the cancer therapy is an antibody-based targeted therapy and/or immunotherapy, such as an antibody specific for CTLA-4, PD-1, PD-L1, CD-52, CD19, CD20, CD27, CD30, CD38, CD137, HER-2, EGFR, VEGF, VEGFR, RANKL, BAFF, OX40, gpNMB, SLAM7, B4GALNT1, Nectin-4, PDGFRa, IL-1β, IL-6 and IL-6R. These include monoclonal antibodies, which may be chimeric, humanized or fully human, and fragments thereof. The antibodies may be of any isotype and may be complement fixing (e.g. IgG1 IgG2 antibodies) and may have the ability to elicit complement dependent cytotoxicity (CDC) or antibody-dependent cellular cytotoxicity (ADCC). Exemplary antibodies include, but are not limited to Ado-trastuzumab emtansine (Kadcyla®; HER2), Alemtuzumab (Campath®; CD52), Atezolizumab (Tecentriq®; PD-L1), Avelumab (Bavencio®; PD-L1), Belimumab (Benlysta®; BAFF), Belinostat (Beleodaq®; HDAC), Bevacizumab (Avastin®; VEGF ligand), Blinatumomab (Blincyto®; CD19/CD3), Brentuximab vedotin (Adcetris®; CD30), Canakinumab (Ilaris®; IL-1β), Cetuximab (Erbitux®; EGFR), Daratumumab (Darzalex®; CD38, Denosumab (Xgeva®; RANKL), Dinutuximab (Unituxin®; B4GALNT1 (GD2), Durvalumab (Imfinzi®; PD-L1), Elotuzumab (Emplicit®; SLAMF7), Enfortumab (Nectin-4), Glembatumumab (gpNMB), GSK3174998 (CD134/OX40), Ibritumomab tiuxetan (Zevalin®; CD20), Ipilimumab (Yervoy®; CTLA-4), Necitumumab (Portrazza®; EGFR), Nivolumab (Opdivo®; PD-1), Obinutuzumab (Gazyva®; CD20), Ofatumumab (Arzerra®; CD20), Olaratumab (Lartruvo®; PDGFRa), Panitumumab (Vectibix®; EGFR), Pembrolizumab (Keytruda®; PD-1), Pertuzumab (Perjeta®; HER2), PF-04518600 (CD134/OX40), Pidilizumab (PD-1), Pogalizumab (CD134/OX40), Ramucirumab (Cyramza®; VEGFR2), Rituximab (Rituxan®; CD20), Siltuximab (Sylvant®; IL-6), Tavolixizumab (CD134/OX40), Tocilizumab (Actemra®; IL-6R), Tositumomab (Bexxar®; CD20), Trastuzumab (Herceptin®; HER2), Tremelimumab (CTLA-4), Urelumab (CD137), and Varlilumab IgG1 (CD27).

In some examples, the antibodies are useful for the treatment of a selected few specific cancers, while in other instance, the antibodies are useful for a broad range of cancers. For example, bladder cancer may be treated with atezolizumab, avelumab, durvalumab, nivolumab, or pembrolizumab; brain cancer may be treated with bevacizumab or dinutuximab; breast cancer may be treated with pertuzumab, trastuzumab or trastuzumab emtansine; cervical cancer may be treated with bevacizumab; colorectal cancer may be treated with bevacizumab, cetuximab, ramucirumab, panitumumab & nivolumab or pembrolizumab; esophageal cancer may be treated with trastuzumab or ramucirumab & pembrolizumab; head & neck cancer may be treated with nivolumab, orpembrolizumab or cetuximab; lung cancer may be treated with bevacizumab, ramucirumab, necitumumab, atezolizumab, nivolumab, or pembrolizumab; lymphoma may be treated with rituximab, obinutuzumab, brentuximab vedotin or ibritumomab tiuxetan; melanoma may be treated with ipilimumab, pembrolizumab or nivolumab; myeloma may be treated with daratumumab or elotuzumab, ovarian cancer may be treated with bevacizumab; sarcoma may be treated with denosumab or olaratumab; and stomach cancer may be treated with trastuzumab, ramucirumab or pembrolizumab.

7.4 <u>Exemplary combinations for diagnostic applications</u>

The particular combination of metrics or genetic indicators of deaminase activity used in the systems and methods of the disclosure for any given cancer therapy, cancer and/or population of patients can be determined by those skilled in the art using the teachings herein and as exemplified in Examples 1-8. It will be understood that the combinations that are effective for differentiating "responder" and "non-responder" subjects may be different depending on the cancer therapy or the type of cancer therapy, the type of cancer, or the patient population. However, the optimal combination of metrics or genetic indicators of deaminase activity for differentiating "responder" and "non-responder" subjects can be readily determined as described herein, i.e. by assessing groups of subjects (optionally with particular characteristics (e.g. age, sex etc.) and a particular cancer) known to respond to a particular therapy or known to be non-responsive to a particular therapy to determine which combination of indicators can differentiate between the groups. As demonstrated herein, more than one combination of metrics (or genetic indicators of deaminase activity) will typically be able to be used in the systems and methods of the disclosure.

In some embodiments, the methods and systems include an assessment of metrics (or genetic indicators of deaminase activity) in the motif metric group, and in particular of metrics in the deaminase metrics group including metrics in one or more of the AID, ADAR, APOBEC3G, APOBEC3B, APOBEC3F and APOBEC1 motif metric groups. Metrics in the three-mer metric group may also be used. Typically, the methods and systems further include an assessment of metrics (or genetic indicators of deaminase activity) in the codon context metric group, and/or the motif-independent metric group, and/or the strand specific metric group, and/or the transition/transversion metric group, and/or the strand bias metric group, and/or the AT/GC metric group.

In particular embodiments, the methods include an assessment of at least one or the percentage of SNVs at an AID motif (e.g. WR<u>C</u>/<u>G</u>YW), the percentage of SNVs at an ADAR motif (e.g. W<u>A</u>/<u>T</u>W), the percentage of SNVs at an APOBEC3G motif (e.g. C<u>C</u>/<u>G</u>G); and the percentage of SNVs at an APOBEC3B motif (e.g. T<u>C</u>A/TG<u>A</u>).

In particular embodiments, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of a thymine nucleotide that occur at a MC-3 site; the percentage of the SNVs resulting from a mutation of a guanine nucleotide that occur at a MC-3 site; the percentage of the SNVs resulting from a mutation of a cytosine nucleotide that occur at a MC-1 site; the percentage of the SNVs resulting from a mutation of a cytosine nucleotide that occur at a MC-2 site; the percentage of SNVs at MC-1 sites; the percentage of SNVs at MC-2 sites; the percentage of SNVs at MC-3 sites; the percentage of SNVs resulting from mutation of an adenine nucleotide; the ratio of the percentage of SNVs resulting from mutation of an adenine nucleotide to the percentage of SNVs resulting from a mutation of a thymine nucleotide (A:T ratio); transition-transversion ratio of SNVs resulting from mutation of a cytosine or guanine. Such a combination of indicators can be used, for example, to determine whether the subject is likely to respond to an immunotherapy, for example an antibody-based immunotherapy, such as an anti-CTLA-4 antibody (e.g. Ipilimumab).

In some embodiments, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of a cytosine nucleotide which occur at a MC-2 site; the percentage of the SNVs resulting from a mutation of a thymine nucleotide which occur at a MC-1 site; the percentage of the SNVs resulting from a mutation of a thymine nucleotide which occur at a MC-2 site; the percentage of the SNVs resulting from a mutation of a thymine nucleotide which occur at a MC-3 site; the percentage of SNVs at MC-1 sites; the percentage of SNVs at MC-2 sites; the percentage of SNVs at MC-3 sites; the percentage of SNVs resulting from mutation of a guanine nucleotide; the percentage of SNVs resulting from mutation of an adenine nucleotide; the ratio of the percentage of SNVs resulting from mutation of an adenine nucleotide to the percentage of SNVs resulting from a mutation of a thymine nucleotide (A:T ratio); transition-transversion ratio of SNVs resulting from mutation of a cytosine or guanine. Such a combination of indicators can be used, for example, to determine whether the subject is likely to respond or to continue to respond to an immunotherapy or targeted therapy, for example an antibody-based immunotherapy or targeted therapy, such as an anti-PD-1 antibody (e.g. pembrolizumab).

In other embodiments, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of a cytosine nucleotide which occur at a MC-1 site (C_MC1%); the percentage of the SNVs resulting from a mutation of a cytosine nucleotide which occur at a MC-2 site; the percentage of the SNVs resulting from a mutation of a cytosine nucleotide which occur at a MC-3 site; the percentage of SNVs at MC-1 sites; the percentage of SNVs at MC-2 sites; the percentage of SNVs at MC-3 sites; the percentage of SNVs resulting from mutation of a thymine nucleotide; the percentage of SNVs resulting from mutation of an adenine nucleotide; the ratio of the percentage of SNVs resulting from mutation of an adenine nucleotide to the percentage of SNVs resulting from a mutation of a thymine nucleotide (A:T ratio); transition-transversion ratio of SNVs resulting from mutation of a guanine. Such a combination of indicators can be used, for example, to determine whether the subject is likely to respond or to continue to respond to an immunotherapy or targeted therapy, for example an antibody-based immunotherapy or targeted therapy, such as an anti-PD-L1 antibody (e.g. atezolizumab).

In further embodiments, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs at the AID motif WRC/GYW which occurred at a MC-2 site; the percentage of the SNVs at an AID motif GYW which involve a G>A mutation and which occur at a MC-3 site; the percentage of the SNVs at the APOBEC3B motif TCA which involve a C>T mutation and which occur at a MC-1 site; the percentage of the SNVs at the APOBEC3B motif TCA which involve a C>T mutation and which occur at a MC-3 site; the percentage of SNVs at MC-1 sites; the percentage of SNVs at MC-2 sites; the percentage of SNVs at MC-3 sites; the percentage of SNVs resulting from mutation of a guanine nucleotide; the percentage of SNVs resulting from mutation of a cytosine nucleotide; the ratio of the number of SNVs resulting from mutation of an adenine nucleotide that are not in the deaminase motif WA to the number of SNVs resulting from a mutation of a thymine nucleotide that are not in the deaminase motif TW; transition-transversion ratio of SNVs resulting from mutation of a guanine or cytosine. Such a combination of indicators can be used, for example, to determine whether the subject is likely to respond or to continue to respond to an immunotherapy or targeted therapy, for example an antibody-based immunotherapy or targeted therapy, such as an anti-PD-1 therapy.

In a particular embodiment, the one or more genetic indicators of endogenous deaminase activity are selected from or include the percentage of SNVs at an AID motif; the percentage of SNVs at an ADAR motif; the percentage of SNVs at an APOBEC3G motif; the percentage of SNVs at an APOBEC3B motif; the percentage of the SNVs resulting from a mutation of an adenine nucleotide which occur at a MC-1 site (A_MC1%); the percentage of the SNVs resulting from a mutation of an adenine nucleotide which occur at a MC-2 site (A_MC2%); the percentage of the SNVs resulting from a mutation of an adenine nucleotide which occur at a MC-3 site (A_MC3%); the percentage of SNVs at MC-1 sites; the percentage of SNVs at MC-2 sites; the percentage of SNVs at MC-3 sites; the percentage of SNVs resulting from mutation of an thymine nucleotide; the percentage of SNVs resulting from mutation of an adenine nucleotide; the ratio of the percentage of SNVs resulting from mutation of an cytosine nucleotide to the percentage of SNVs resulting from a mutation of a guanine nucleotide; transition-transversion ratio of SNVs resulting from mutation of a guanine or cytosine. Such a combination of indicators can be used, for example, to determine whether the subject is likely to respond or continue to respond to an targeted therapy, for example a tyrosine kinase inhibitor (e.g. afatinib).

### 7.5 Therapeutic applications

The methods of the present disclosure also extend to therapeutic protocols. In instances where a subject is determined to be likely to respond to a cancer therapy, the methods may involve administering the cancer therapy to the subject. In some examples, combination therapy is utilized, whereby the subject is administered the cancer therapy under assessment and one or more other cancer therapies, such as radiotherapy, surgery, chemotherapy, hormone therapy, immunotherapy and targeted therapy. In instances where a subject is determined to be unlikely to respond to a cancer therapy, the methods may involve administering a different cancer therapy to the subject. In some examples, the different cancer therapy is a combination therapy that includes one or more of radiotherapy, surgery, chemotherapy, hormone therapy, immunotherapy and targeted therapy. Where the methods of the present disclosure are used to determine that a subject is unlikely to continue responding to a cancer therapy (i.e. is likely to develop resistance to that cancer therapy), the methods of the disclosure extend to ceasing treatment of the subject with that cancer therapy and initiating a new treatment regimen, such as administering a different cancer therapy to the subject. In some examples, the different cancer therapy is a combination therapy that includes one or more of radiotherapy, surgery, chemotherapy, hormone therapy, immunotherapy and targeted therapy.

In order that the disclosure may be readily understood and put into practical effect, particular preferred embodiments will now be described by way of the following non-limiting examples.

**EXAMPLES**

**Example 1**

**Methods for detecting genetic indicators of deaminase activity**

Whole exome or full genome sequences from patients were analyzed to identify single nucleotide variants (SNVs). Briefly, sequences were formatted in a .vcf file using the hg38 genome coordinates as a reference. The Batch Coordinate Conversion (liftOver; University of California Santa Cruz) program tool was used to transform between different genome references.

Each somatic variant in the .vcf file was analyzed and selected for further consideration if it was a simple SNV (e.g. A >T or G >A, but not G >A and T); it was not an insertion or deletion; and it was in a coding region of an ENSEMBL gene and had three exonic base pairs either side of the mutated position. The following steps were then performed:

a) the codon context within the structure of the mutated codon (MC) was determined, *i.e.* the position of the SNV within the encoding triplet was determined, wherein the first position (read from 5' to 3') is referred to as MC1 (or MC-1 site), the second position is referred to as MC2 (or MC-2 site) and the third position is referred to as MC3 (or MC-3 site);

b) a nine-base window was extracted from the surrounding genome sequence such that the sequence of three complete codons was obtained. The direction of the gene was used for determining 5' and 3' directions, and for determining the correct strand of the nine bases. The nine-base window was always reported according to the direction of the gene such that bases in the window around variants in genes on the reverse strand of the genome are reverse complimented in relation to the genome, but in the forward direction in relation to the gene. By convention, this context is always reported in the same strand of the gene. Positive strand genes will have codon context bases from the positive strand of the reference genome, and negative strand genes will have codon context bases from the negative strand of the reference genome;

c) motif searching was performed using known motifs for the four main deaminases (AID, ADAR, APOBEC3G (A3G) and APOBEC3B (A3B)) to determine whether the variation was within such a motif. The four main deaminase motifs identified were as follows wherein the underlined base corresponds to the targeted/mutated base, and the target motif to the right of the forward slash is the reverse compliment of the forward strand motif that is used for searching on the reverse strand of the gene (wherein mutations in the forward and reverse motif were identified):

AID - WR<u>C</u>/<u>G</u>YW

ADAR - W<u>A</u>/<u>T</u>W

APOBEC3G - C<u>C</u>/<u>G</u>G

APOBEC3B - T<u>C</u>A/<u>TGA</u>

Other motifs for these deaminases were used as described in the Examples below.

Mutations at these motifs (both the forward and reverse motifs) were identified and considered as mutations attributable to the respective deaminase. For each deaminase motif considered, any one or more of the following metrics were calculated:

- Deaminase% (e.g. AID%): total number of SNVs attributable to a deaminase (e.g. AID) as represented by mutation at a particular deaminase motif (e.g. the AID motif WR<u>C</u>/<u>G</u>YW, in both the forward and reverse motif) divided by the total number of SNVs in the patient, and represented as a percentage

- Deaminase_True% (e.g. AID_True%): total number of SNVs attributable to a deaminase (e.g. AID) as represented by mutation at a particular deaminase motif (e.g. the AID motif WR<u>C</u>/<u>G</u>YW, in both the forward and reverse motif) that are transition mutations divided by total number of SNVs in the patient, and represented as a percentage (wherein transition mutations are defined as C>T, G>A, T>C and A>G).

- Deaminase _MC1% (e.g. AID_MC1%): percentage of all SNVs attributable to a deaminase (e.g. AID) using a particular deaminase motif (e.g. the AID motif WR<u>C</u>/<u>G</u>YW) which occurred at the MC1 site.

- Deaminase _MC2%: percentage of all SNVs attributable to a deaminase which occurred at the MC2 site.

- Deaminase _MC3%: percentage of all SNVs attributable to a deaminase which occurred at the MC3 site.

- C>T_MC1%: percentage of C>T mutations at the MC1 site (metric used for SNVs attributable to AID, A3B or A3G)

- C>T_MC2%: percentage of C>T mutations at the MC2 site (metric used for SNVs attributable to AID, A3B or A3G)

- C>T_MC3%: percentage of C>T mutations at the MC3 site (metric used for SNVs attributable to AID, A3B or A3G)

- G>A_MC1%: percentage of G>A mutations at the MC1 site (metric used for SNVs attributable to AID, A3B or A3G)

- G>A_MC2%: percentage of G>A mutations at the MC2 site (metric used for SNVs attributable to AID, A3B or A3G)

- G>A_MC3%: percentage of G>A mutations at the MC3 site (metric used for SNVs attributable to AID, A3B or A3G)

- T>C_MC1%: percentage of T>C mutations at the MC1 site (metric used for SNVs attributable to ADAR)

- T>C_MC2%: percentage of T>C mutations at the MC2 site (metric used for SNVs attributable to ADAR)

- T>C_MC3%: percentage of T>C mutations at the MC3 site (metric used for SNVs attributable to ADAR)

- A>G_MC1%: percentage of A>G mutations at the MC1 site (metric used for SNVs attributable to ADAR)

- A>G_MC2%: percentage of A>G mutations at the MC2 site (metric used for SNVs attributable to ADAR)

- A>G_MC3%: percentage of A>G mutations at the MC3 site (metric used for SNVs attributable to ADAR)

- Deaminase_Ti/Tv: transition/transversion ratio given by total number of SNVs attributable to a deaminase that are transition mutations divided by the total number of SNVs attributable to a deaminase that are transversion mutations

- Deaminase_C:G: strand bias ratio given by the number of SNVs attributable to a deaminase that are mutations of a cytosine divided by the number of SNVs attributable to a deaminase that are mutations of a guanine.

- Deaminase_True_C:G: strand bias ratio given by total of transition mutations at a C divided by total number of transition mutations at a G.

Other deaminase-related metrics calculated included:

- Deaminase%: total number of SNVs attributable to the four primary deaminase motifs for AID, ADAR, A3G, A3B, as described above, divided by the total number of SNVs in the patient, represented as percentage.

- Other%: total number of SNVs that are not attributable to the four primary deaminase motifs for AID, ADAR, A3G, A3B, as described above, divided by the total number of SNVs in the patient, represented as percentage.

Other metrics not related to particular motifs but that are an indirect consequence of aberrant deaminase activity include:

- All_A%: total number of SNVs resulting from mutation of an adenine (i.e. A>T, C or G), represented as a percentage of total number of SNVs.

- A_MC1%: percentage of the SNVs resulting from mutation of an adenine which are at the MC1 position

- A_MC2%: percentage of the SNVs resulting from mutation of an adenine which are at the MC2 position

- A_MC3%: percentage of the SNVs resulting from mutation of an adenine which are at the MC3 position

$$i.e.\ A\_MC1\% + A\_MC2\% + A\_MC3\% = 100\%$$

- All_T%: total number of SNVs resulting from mutation of a thymine (i.e. T>A, C or G), represented as a percentage of total number of SNVs.

- T_MC1%: percentage of the SNVs resulting from mutation of a thymine that occurred at the MC1 position

- T_MC2%: percentage of the SNVs resulting from mutation of a thymine that occurred at the MC2 position.

- T_MC3%: percentage of the SNVs resulting from mutation of a thymine that occurred at the MC3 position

$$i.e.\ T\_MC1\% + T\_MC2\% + T\_MC3\% = 100\%$$

- All_C%: total number of the SNVs resulting from mutation of a cytosine (i.e. C>G, A or T), represented as a percentage of total number of SNVs.

- C_MC1%: percentage of the SNVs resulting from mutation of a cytosine that occurred at the MC1 position.

- C_MC2%: percentage of the SNVs resulting from mutation of a cytosine that occurred at the MC2 position.

- C_MC3%: percentage of the SNVs resulting from mutation of a cytosine that occurred at the MC3 position.

$$i.e.\ C\_MC1\% + C\_MC2\% + C\_MC3\% = 100\%$$

*i.e.*

- All_G%: total number of SNVs resulting from mutation of a guanine (i.e. G>C, A or T), represented as a percentage of total number of SNVs.

- G_MC1%: percentage of the SNVs resulting from mutation of a guanine that occurred at the MC1 position

- G_MC2%: percentage of the SNVs resulting from mutation of a guanine that occurred at MC2 position

- G_MC3%: percentage of the SNVs resulting from mutation of a guanine that occurred at MC3 position

$$i.e.\ G\_MC1\% + G\_MC2\% + G\_MC3\% = 100\%$$

- all_MC1%: percentage of all SNVs that are at MC1 position

- all_MC2%: percentage of all SNVs that are at MC2 position

- all_MC3%: percentage of all SNVs that are at MC3 position

- all_A_Ti/Tv: transition-transversion ratio of all SNVs resulting from mutation of an adenine, i.e. ratio of all SNVs resulting from a transition mutation of an adenine (i.e. A>G) to all SNVs resulting from a transversion mutation of an adenine (i.e. A>T or C).

- all_T_Ti/Tv: transition-transversion ratio of all SNVs resulting from mutation of a thymine i.e. ratio of all SNVs resulting from a transition mutation of a thymine (i.e. T>C) to all SNVs resulting from a transversion mutation of a thymine (i.e. T>G or A).

- all_C_Ti/Tv: transition-transversion ratio of all SNVs resulting from mutation of a cytosine i.e. ratio of all SNVs resulting from a transition mutation of a cytosine (i.e. C>T) to all SNVs resulting from a transversion mutation of a cytosine (i.e. C>G or A).

- all_G_Ti/Tv: transition-transversion ratio of all SNVs resulting from mutation of an guanine, i.e. ratio of all SNVs resulting from a transition mutation of a guanine (i.e. G>A) to all SNVs resulting from a transversion mutation of a guanine (i.e. G>C or T).

- all_AT_Ti/Tv: transition-transversion ratio of all SNVs resulting from mutation of an adenine or thymine, i.e. ratio of all SNVs resulting from a transition mutation of an adenine (i.e. A>G) or thymine (i.e. T>C) to all SNVs resulting from a transversion mutation of an adenine (i.e. A>T or C) or thymine (i.e. T>G or A).

- all_GC_Ti/Tv: transition-transversion ratio of SNVs resulting from mutation of an cytosine or guanine, i.e. ratio of all SNVs resulting from a transition mutation of a guanine (i.e. G>A) or cytosine (i.e. C>T) to all SNVs resulting from a transversion mutation of a guanine (i.e. G>C or T) or cytosine (i.e. C>G or A).

- all_C:G: ratio of all SNVs resulting from mutation of a cytosine to all SNVs resulting from mutation of a guanine

- all_A:T: ratio of all SNVs resulting from mutation of an adenine to all SNVs resulting from mutation of a thymine

- all_AT:GC: ratio of all SNVs resulting from mutation of an adenine or a thymine to all SNVs resulting from mutation of a guanidine or cytosine

**Example 2**

**Genetic indicators of deaminase activity for predicting response to ipilimumab treatment**

A clinical trial assessing the efficacy of ipilimumab (Yervoy™), which is a monoclonal antibody directed against cytotoxic T lymphocyte-associated antigen-4 (CTLA-4), in patients with metastatic melanoma found that only 27 of the 110 patients (i.e. 24.5%) responded to therapy (Van Allen et al (2015) Science 350(6257): 207-211). No features exclusive to the responders that could be used to predict patient response to treatment were identified.

The whole exome sequence of each of the patients was analyzed as described in Example 1 to determine whether genetic indicators of deaminase activity were associated with response or non-response to therapy. In particular, the genetic indicators of deaminase activity that were assessed included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs resulting from a mutation of a thymine nucleotide that occurred at a MC-3 site (T_MC3%); the percentage of the SNVs resulting from a mutation of a guanine nucleotide that occurred at a MC-3 site (G_MC3%); the percentage of the SNVs resulting from a mutation of a cytosine nucleotide that occurred at a MC-1 site (C_MC1%); the percentage of the SNVs resulting from a mutation of a cytosine nucleotide that occurred at a MC-2 site (C_MC2%); the percentage of SNVs that were at a MC-1 site (all_MC1%); the percentage of SNVs that were at a MC-2 site (all_MC2%); and the percentage of SNVs that were at a MC-3 site (all_MC3%)

- and those indicators assessing strand bias and/or nucleotide targeting, including the percentage of SNVs resulting from mutation of an adenine nucleotide (all_A%); the ratio of the percentage of SNVs resulting from mutation of an adenine nucleotide to the percentage of SNVs resulting from a mutation of a thymine nucleotide (all A:T); and the transition-transversion ratio of SNVs resulting from mutation of an cytosine or guanine all_GC TiTv.

Table 1 shows the results of the analysis of genetic indicators of deaminase activity for the 27 'responders'. The data from these responders was used to calculate the Range Intervals (RIs) for each indicator to which the data from the "non-responders" was then compared. In this example, the RI for each indicator was set by the maximum observed value and the minimum observed value for that indicator. The exception was the RI for the percentage of total SNVs that were at the APOBEC3B motif TCA/TGA (A3B%), which was set as the maximum observed value minus 5% and the minimum observed value plus 5%. Where the observed value of a genetic indicator of deaminase activity was outside the RI (H - high or L - low compared to the RI), a score of 1 was attributed to that indicator. Where the observed value of a genetic indicator of deaminase activity was within the RI, a score of 0 was attributed to that indicator. The score provided in the right hand column of the Table is the total score for the patient. Thus, where the patient has no genetic indicators outside of the RI, i.e.

no "outliers", the total score was 0. This total score is also called the predicted test score. As can be seen, only 2/27 of the patients responding to ipilimumab treatment had a score of 1 (i.e. each of these two patients had one outlier), while 25/27 patients had a score of 0. Thus, using the assessment of the genetic indicators of deaminase activity, it could be predicted that 25/27 (or 92.6%) of the patients were suitable for treatment with ipilimumab and would be likely to respond to therapy.

Table 2 shows the results for the 73 'non-responders', and Table 3 shows the results for the 10 non-responders that nonetheless had long term survival. The results for the pooled non-responder group show that 60/83 patients had at least one genetic indicator of deaminase activity that was outside the RI. This indicates that an assessment of genetic indicators of deaminase activity as described here prior to treatment would have predicted that 60/83 (72.3%) of these patients would not have been suitable for treatment.

TABLE 1

| Patient | total cds | DEAMINASES | | | | CODON CONTEXT TARGETTING | | | | | | | STRAND BIAS | | Ti/Tv | score |
|---|---|---|---|---|---|---|---|---|---|---|---|---|---|---|---|---|
| | | AID % | ADAR % | A3G % | A3B % | T_MC3 % | G_MC3 % | C_MC1 % | C_MC2 % | all_MC1 % | all_MC2 % | all_MC3 % | all_A % | all_A:T | all_GC TiTv | |
| RESPONDERS | | | | | | | | | | | | | | | | |
| Pat02 | 275 | 6.545 | 8.000 | 22.546 | 9.455 | 41.177 | 31.579 | 25.781 | 36.719 | 32.000 | 33.091 | 34.909 | 5.818 | 0.941 | 12.444 | 0 |
| Pat04 | 420 | 6.190 | 11.905 | 28.810 | 5.714 | 25.000 | 31.210 | 33.117 | 22.727 | 37.619 | 27.857 | 34.524 | 14.524 | 1.271 | 3.937 | 0 |
| Pat07 | 192 | 5.729 | 9.375 | 26.563 | 5.729 | 0.000 | 35.616 | 27.957 | 29.032 | 32.813 | 31.771 | 35.417 | 8.333 | 1.600 | 4.355 | 0 |
| Pat103 | 774 | 2.972 | 5.556 | 27.003 | 11.499 | 34.286 | 31.655 | 30.516 | 29.812 | 34.367 | 29.070 | 36.563 | 4.522 | 1.000 | 34.200 | 0 |
| Pat105 | 155 | 4.516 | 7.097 | 34.839 | 6.452 | 25.000 | 33.929 | 27.500 | 26.250 | 34.839 | 26.452 | 38.710 | 7.097 | 1.375 | 21.667 | 0 |
| Pat113 | 403 | 4.467 | 13.896 | 25.558 | 7.940 | 43.902 | 39.437 | 32.778 | 25.000 | 36.228 | 23.325 | 40.447 | 9.926 | 0.976 | 16.889 | 0 |
| Pat117 | 922 | 5.423 | 4.013 | 31.236 | 10.629 | 42.424 | 29.191 | 34.961 | 26.758 | 38.720 | 26.790 | 34.490 | 3.362 | 0.939 | 34.750 | 0 |
| Pat123 | 698 | 4.871 | 5.731 | 23.496 | 10.602 | 40.000 | 35.227 | 27.717 | 25.815 | 33.811 | 25.358 | 40.831 | 4.441 | 0.886 | 17.057 | 0 |
| Pat126 | 506 | 3.557 | 7.312 | 28.854 | 6.917 | 33.333 | 35.714 | 29.508 | 32.787 | 33.202 | 31.028 | 35.771 | 6.917 | 0.778 | 14.214 | 0 |
| Pat132 | 2544 | 23.860 | 0.825 | 22.091 | 2.634 | 37.500 | 27.960 | 31.287 | 35.072 | 37.539 | 31.643 | 30.818 | 0.865 | 0.917 | 82.267 | 0 |
| Pat138 | 6311 | 17.034 | 2.583 | 30.249 | 2.884 | 33.140 | 31.045 | 30.599 | 32.573 | 35.953 | 30.534 | 33.513 | 2.947 | 1.081 | 56.796 | 0 |
| Pat174 | 807 | 4.089 | 4.585 | 27.262 | 13.383 H | 33.333 | 30.539 | 32.614 | 31.175 | 39.529 | 26.642 | 33.829 | 3.594 | 1.074 | 29.040 | 1 |
| Pat21 | 1968 | 3.608 | 3.201 | 34.705 | 8.181 | 41.509 | 33.472 | 30.986 | 28.609 | 34.502 | 27.998 | 37.500 | 2.846 | 1.057 | 32.196 | 0 |
| Pat24 | 36 | 25.000 | 8.333 | 25.000 | 8.333 | inf | 36.364 | 36.364 | 54.546 | 41.667 | 25.000 | 33.333 | 8.333 | inf | 1.750 | 0 |
| Pat29 | 54 | 3.704 | 5.556 | 25.926 | 14.815 H | 0.000 | 47.826 | 23.077 | 34.615 | 31.482 | 25.926 | 42.593 | 7.407 | 4.000 | 23.500 | 1 |
| Pat38 | 3330 | 23.153 | 0.751 | 24.114 | 2.072 | 36.667 | 31.427 | 29.792 | 34.066 | 34.234 | 32.072 | 33.694 | 1.081 | 1.200 | 119.889 | 0 |
| Pat39 | 89 | 6.742 | 6.742 | 35.955 | 6.742 | 50.000 | 45.000 | 36.842 | 23.684 | 37.079 | 22.472 | 40.449 | 7.865 | 1.750 | 3.588 | 0 |
| Pat47 | 181 | 6.630 | 6.077 | 35.912 | 6.630 | 40.000 | 49.351 | 34.524 | 32.143 | 34.807 | 25.967 | 39.227 | 8.287 | 3.000 | 6.667 | 0 |
| Pat49 | 896 | 5.357 | 6.585 | 26.228 | 9.152 | 28.302 | 31.921 | 34.298 | 26.503 | 37.500 | 27.121 | 35.380 | 4.464 | 0.755 | 25.767 | 0 |
| Pat63 | 281 | 2.847 | 2.847 | 33.808 | 7.473 | 37.500 | 40.187 | 31.373 | 30.719 | 34.875 | 27.402 | 37.722 | 4.626 | 1.625 | 19.000 | 0 |
| Pat66 | 388 | 3.608 | 7.990 | 36.083 | 9.536 | 48.148 | 27.211 | 32.821 | 33.846 | 37.629 | 31.443 | 30.928 | 4.897 | 0.704 | 13.250 | 0 |
| Pat73 | 313 | 7.029 | 8.626 | 26.837 | 10.543 | 44.444 | 35.514 | 35.571 | 28.859 | 33.866 | 32.268 | 33.866 | 9.585 | 1.111 | 22.273 | 0 |
| Pat77 | 472 | 3.390 | 4.449 | 28.602 | 11.441 | 35.000 | 34.146 | 32.340 | 35.319 | 35.381 | 31.568 | 33.051 | 2.542 | 0.600 | 21.000 | 0 |
| Pat79 | 400 | 4.250 | 3.500 | 29.000 | 10.250 | 12.500 | 31.013 | 35.514 | 26.636 | 39.750 | 26.750 | 33.500 | 3.000 | 0.750 | 52.143 | 0 |
| Pat80 | 253 | 11.858 | 9.091 | 25.296 | 1.976 | 33.333 | 30.263 | 25.490 | 30.392 | 29.249 | 35.178 | 35.573 | 16.601 | 1.273 | 4.563 | 0 |
| Pat88 | 1494 | 3.548 | 4.284 | 29.183 | 11.781 | 27.778 | 33.394 | 33.650 | 26.873 | 35.609 | 27.912 | 36.479 | 3.213 | 0.889 | 24.778 | 0 |
| Pat90 | 279 | 4.660 | 4.301 | 26.882 | 12.186 | 33.333 | 27.273 | 23.529 | 32.941 | 32.617 | 30.824 | 36.559 | 4.301 | 1.333 | 20.500 | 0 |
| RANGE | 36 | 2.847 | 0.751 | 22.091 | 1.976 | 0.000 | 27.211 | 23.077 | 22.727 | 29.249 | 22.472 | 30.818 | 0.865 | 0.600 | 1.750 | |
| INTERVAL | 6311 | 25.000 | 13.896 | 36.083 | 12.186 | 50.000 | 49.351 | 36.842 | 54.546 | 41.667 | 35.178 | 42.593 | 16.601 | 4.000 | 119.889 | |

TABLE 2

| Patient | total cds | AID % | DEAMINASES | | | T_MC3 % | G_MC3 % | CODON CONTEXT TARGETING | | | | | STRAND BIAS | | Ti/Tv | score |
|---|---|---|---|---|---|---|---|---|---|---|---|---|---|---|---|---|
| | | | ADAR % | A3G % | A3B % | | | C_MC1 % | C_MC2 % | all_MC1 % | all_MC2 % | all_MC3 % | all_A % | all_A:T | all_GC TiTv | |
| **NON-RESPONDERS** | | | | | | | | | | | | | | | | |
| Pat03 | 473 | 8.034 | 1.057 | 39.746 H | 2.960 | 25.000 | 34.742 | 32.653 | 34.286 | 36.998 | 28.753 | 34.250 | 1.480 | 0.875 | 40.636 | 1 |
| Pat06 | 187 | 5.882 | 4.278 | 35.829 | 7.487 | 25.000 | 29.032 | 38.393 H | 22.321 L | 43.316 H | 21.390 L | 35.294 | 4.813 | 2.250 | 14.818 | 4 |
| Pat08 | 365 | 4.932 | 3.288 | 29.315 | 9.589 | 35.294 | 34.074 | 28.079 | 29.557 | 31.233 | 30.137 | 38.630 | 2.740 | 0.588 L | 18.882 | 1 |
| Pat100 | 708 | 4.379 | 4.944 | 27.966 | 9.463 | 29.412 | 33.333 | 32.546 | 26.772 | 35.028 | 28.107 | 36.864 | 4.096 | 0.853 | 14.732 | 0 |
| Pat101 | 49 | 16.327 | 6.122 | 32.653 | 4.082 | 20.000 | 41.177 | 19.048 L | 28.571 | 36.735 | 24.490 | 38.776 | 12.245 | 1.200 | 3.222 | 1 |
| Pat104 | 32 | 12.500 | 18.750 H | 25.000 | 3.125 | 0.000 | 7.143 L | 44.444 H | 22.222 L | 40.625 | 37.500 | 21.875 L | 18.750 H | 2.000 | 22.000 | 6 |
| Pat106 | 10 | 20.000 | 10.000 | 40.000 H | 0.000 L | 0.000 | 20.000 L | 0.000 L | 0.000 L | 30.000 | 30.000 | 40.000 | 10.000 | 1.000 | inf | 5 |
| Pat109 | 356 | 5.899 | 5.618 | 28.090 | 8.146 | 60.000 H | 35.526 | 27.545 | 27.545 L | 29.494 | 29.494 | 41.011 | 6.180 | 1.467 | 18.938 | 2 |
| Pat110 | 8338 | 3.082 | 6.476 | 29.324 | 10.398 | 27.778 | 34.694 | 32.866 | 28.029 | 35.740 | 27.776 | 36.484 | 4.605 | 0.889 | 32.580 | 0 |
| Pat115 | 80 | 7.500 | 8.750 | 26.250 | 8.750 | 37.500 | 33.333 | 28.947 | 28.947 | 33.750 | 28.750 | 37.500 | 5.000 | 0.500 L | 6.556 | 1 |
| Pat118 | 147 | 6.122 | 4.762 | 23.810 | 7.483 | 50.000 | 31.667 | 38.571 H | 32.857 | 39.456 | 28.571 | 31.973 | 3.401 | 0.417 L | 17.571 | 2 |
| Pat121 | 46 | 17.391 | 8.696 | 15.217 L | 4.348 | 0.000 | 18.182 L | 35.294 | 35.294 | 41.304 | 34.783 | 23.913 L | 13.044 | 6.000 | 4.571 | 3 |
| Pat124 | 526 | 4.943 | 5.323 | 25.285 | 7.795 | 52.174 H | 31.720 | 31.250 | 26.042 L | 36.122 | 26.046 | 37.833 | 5.513 | 1.261 | 19.609 | 2 |
| Pat127 | 139 | 6.475 | 4.317 | 33.094 | 8.633 | 62.500 H | 32.692 | 27.027 | 31.081 | 30.935 | 30.216 | 38.849 | 3.597 | 0.625 | 6.000 | 1 |
| Pat128 | 99 | 12.121 | 10.101 | 24.242 | 7.071 | 12.500 | 36.111 | 17.778 L | 35.556 | 24.242 L | 36.364 H | 39.394 | 10.101 | 1.250 | 5.750 | 3 |
| Pat129 | 116 | 9.483 | 6.897 | 25.862 | 7.759 | 28.571 | 33.333 | 32.143 | 33.929 | 35.345 | 31.035 | 33.621 | 6.897 | 1.143 | 15.833 | 0 |
| Pat130 | 257 | 3.891 | 2.724 | 29.183 | 7.004 | 57.143 H | 35.644 | 31.206 | 29.787 | 35.020 | 26.459 | 38.521 | 3.113 | 1.143 | 19.167 | 1 |
| Pat131 | 118 | 9.322 | 11.017 | 27.119 | 6.780 | 18.182 | 26.471 L | 25.000 | 28.125 | 33.051 | 27.966 | 38.983 | 7.627 | 0.818 | 11.250 | 1 |
| Pat133 | 258 | 8.527 | 9.302 | 31.783 | 5.039 | 18.519 | 41.000 | 33.333 | 32.143 | 34.109 | 31.008 | 34.884 | 17.054 H | 1.630 | 1.968 | 0 |
| Pat135 | 174 | 6.897 | 5.172 | 28.161 | 5.172 | 28.571 | 25.000 L | 30.337 | 38.202 | 40.230 | 29.310 | 30.460 L | 8.046 | 2.000 | 29.600 | 2 |
| Pat139 | 1850 | 3.351 | 3.405 | 24.973 | 12.487 H | 23.636 | 30.844 | 29.941 | 29.452 | 35.351 | 28.595 | 36.054 | 2.703 | 0.909 | 53.531 | 1 |
| Pat14 | 54 | 11.111 | 1.852 | 31.482 | 0.000 L | 28.000 | 52.381 H | 23.810 | 38.889 | 31.482 | 29.630 L | 1.852 | 0.143 | 1.190 L | | 4 |
| Pat140 | 363 | 5.234 | 4.408 | 26.722 | 12.672 H | 35.714 | 30.935 | 24.366 | 31.980 | 32.507 | 29.201 | 38.292 | 3.581 | 0.929 | 21.400 | 1 |
| Pat143 | 1171 | 5.295 | 6.746 | 26.388 | 8.625 | 35.211 | 35.000 | 30.298 | 29.636 | 34.586 | 28.523 | 36.892 | 6.490 | 1.070 | 12.474 | 0 |
| Pat147 | 739 | 7.172 | 3.924 | 25.169 | 8.119 H | 21.875 | 31.438 | 31.200 | 31.200 | 37.077 | 29.364 | 33.559 | 4.465 | 1.031 | 25.960 | 1 |
| Pat148 | 158 | 5.063 | 11.392 | 18.987 L | 15.190 H | 14.286 | 33.333 | 33.750 | 31.250 | 41.139 | 26.582 | 32.279 | 8.228 | 0.929 | 9.077 | 2 |
| Pat15 | 305 | 5.574 | 7.541 | 35.082 | 7.869 | 33.333 | 32.759 | 35.099 | 32.450 | 39.344 | 28.853 | 31.803 | 6.557 | 1.111 | 11.136 | 0 |
| Pat151 | 1937 | 23.748 | 0.568 L | 22.767 | 2.375 | 40.000 | 31.195 | 31.891 | 33.257 | 36.861 | 30.201 | 32.938 | 0.516 L | 0.500 L | 89.810 | 3 |
| Pat157 | 134 | 12.687 | 3.731 | 21.642 L | 6.716 | 20.000 | 28.000 | 28.125 | 28.125 | 32.090 | 34.328 | 33.582 | 7.463 | 1.000 | 6.600 | 1 |
| Pat160 | 37 | 27.027 H | 10.811 | 27.027 | 2.703 | 33.333 | 17.647 L | 42.857 H | 28.571 | 35.135 | 43.243 | 21.622 L | 8.108 | 1.000 | 0.722 L | 5 |
| Pat162 | 60 | 23.333 | 6.667 | 18.333 L | 3.333 | 40.000 | 29.167 | 39.130 H | 21.739 L | 35.000 | 26.667 | 38.333 | 13.333 | 1.600 | 1.938 | 3 |
| Pat165 | 30 | 10.000 | 16.667 H | 33.333 | 10.000 | 25.000 | 30.000 | 25.000 | 50.000 | 43.333 H | 33.333 | 23.333 L | 13.333 | 1.000 | 2.143 | 3 |
| Pat166 | 25 | 28.000 H | 8.000 | 24.000 | 0.000 L | 0.000 | 44.444 | 30.769 | 30.769 | 32.000 | 32.000 | 36.000 | 12.000 | inf | 10.000 | 2 |
| Pat167 | 41 | 17.073 | 4.878 | 24.390 | 7.317 | 0.000 | 47.826 | 45.455 H | 36.364 | 46.342 H | 19.512 L | 34.146 | 12.195 | 2.500 | 2.091 | 3 |

| Patient | total cds | AID % | DEAMINASES | | | T_MC3 % | G_MC3 % | CODON CONTEXT TARGETING | | | | | STRAND BIAS | | Ti/Tv | score |
|---|---|---|---|---|---|---|---|---|---|---|---|---|---|---|---|---|
| | | | ADAR % | A3G % | A3B % | | | C_MC1 % | C_MC2 % | all_MC1 % | all_MC2 % | all_MC3 % | all_A % | all_A:T | all_GC TiTv | |
| **NON-RESPONDERS** | | | | | | | | | | | | | | | | |
| Pat168 | 229 | 6.987 | 12.227 | 28.821 | 5.240 | 46.429 | 29.333 | 36.083 | 35.052 | 33.188 | 35.371 | 31.441 | 12.664 | 1.036 | 4.931 | 0 |
| Pat17 | 280 | 2.143 L | 3.929 | 28.929 | 13.571 H | 33.333 | 32.231 | 32.609 | 29.710 | 36.429 | 28.214 | 35.357 | 3.214 | 0.750 | 20.583 | 2 |
| Pat170 | 239 | 5.439 | 7.113 | 21.757 L | 11.297 | 18.182 | 26.000 L | 32.743 | 28.319 | 38.494 | 28.870 | 32.636 | 6.276 | 1.364 | 8.261 | 2 |
| Pat171 | 91 | 25.275 H | 12.088 | 19.780 L | 2.198 | 28.571 | 32.609 | 43.333 H | 13.333 L | 40.659 | 23.077 | 36.264 | 8.791 | 1.143 | 1.714 L | 5 |
| Pat175 | 108 | 13.889 | 10.185 | 18.519 L | 9.259 | 26.667 | 50.000 H | 41.177 H | 29.412 | 41.667 | 24.074 | 34.259 | 9.259 | 0.667 | 6.545 | 3 |
| Pat19 | 705 | 5.106 | 2.979 | 28.085 | 10.071 | 41.177 | 34.050 | 31.105 | 27.249 | 38.298 | 23.121 | 38.582 | 2.837 | 1.176 | 40.750 | 0 |
| Pat25 | 53 | 18.868 | 18.868 H | 18.868 L | 0.000 L | 60.000 H | 50.000 H | 45.000 H | 30.000 | 24.528 L | 37.736 | 37.736 | 16.981 H | 0.900 | 2.400 | 9 |
| Pat32 | 947 | 4.541 | 3.485 | 28.300 | 11.193 | 35.135 | 35.422 | 29.365 | 31.548 | 36.325 | 26.399 | 37.276 | 4.118 | 1.054 | 25.394 | 0 |
| Pat33 | 15 | 13.333 | 13.333 | 33.333 | 13.333 H | 0.000 | 16.667 L | 14.286 L | 42.857 | 26.667 L | 46.667 H | 26.667 L | 0.000 L | 0.000 L | inf | 8 |
| Pat36 | 8 | 12.500 | 50.000 H | 25.000 | 0.000 L | 100.000 H | 33.333 | 0.000 L | 100.000 | 25.000 L | 25.000 | 50.000 H | 25.000 H | 1.000 | 3.000 | 7 |
| Pat37 | 160 | 8.750 | 7.500 | 27.500 | 5.625 | 27.273 | 29.412 | 23.529 | 32.941 H | 28.750 L | 34.375 | 36.875 | 8.125 | 1.182 | 9.462 | 2 |
| Pat40 | 31 | 6.452 | 19.355 H | 25.807 | 16.129 H | 40.000 | 50.000 H | 42.857 H | 14.286 L | 41.936 H | 12.903 L | 45.161 H | 6.452 | 0.400 L | 5.000 | 8 |
| Pat41 | 488 | 4.098 | 4.508 | 33.607 | 9.221 | 50.000 | 40.212 | 34.587 | 28.571 | 36.066 | 25.820 | 38.115 | 2.664 | 0.650 | 49.556 | 0 |
| Pat43 | 221 | 8.597 | 19.005 H | 15.385 L | 6.335 | 16.279 | 29.412 | 31.081 | 25.676 | 33.937 | 35.294 H | 23.913 L | 23.982 H | 1.233 | 5.250 | 4 |
| Pat44 | 71 | 8.451 | 2.817 | 33.803 | 11.268 | 33.333 | 36.667 | 27.778 | 30.556 | 33.803 | 26.761 | 39.437 | 2.817 | 0.667 | 15.500 | 0 |
| Pat45 | 1290 | 3.023 | 2.868 | 28.140 | 12.868 H | 30.769 | 30.579 | 31.836 | 28.270 | 36.667 | 27.442 | 35.892 | 1.783 | 0.885 | 43.321 | 1 |
| Pat46 | 350 | 6.286 | 5.143 | 32.857 | 8.571 | 15.000 | 40.602 | 27.808 | 29.947 | 32.000 | 28.857 | 39.143 | 2.857 | 0.500 L | 12.913 | 1 |
| Pat50 | 586 | 5.461 | 3.584 | 30.887 | 9.386 | 23.810 | 39.269 | 30.303 | 31.818 | 34.130 | 28.498 | 37.372 | 2.730 | 0.762 | 23.955 | 0 |
| Pat54 | 1179 | 4.326 | 4.071 | 29.347 | 8.821 | 27.907 | 34.460 | 28.832 | 32.625 | 34.266 | 29.262 | 36.472 | 2.799 | 0.767 | 28.026 | 0 |
| Pat55 | 613 | 5.873 | 5.710 | 29.690 | 10.114 | 25.926 | 30.078 | 32.782 | 27.483 L | 37.520 | 28.059 | 34.421 | 4.568 | 1.037 | 13.308 | 1 |
| Pat56 | 47 | 8.511 | 12.766 | 23.404 | 2.128 | 0.000 | 22.222 L | 21.053 L | 47.368 | 38.298 | 34.043 | 27.660 L | 12.766 | 1.500 | 2.364 | 3 |
| Pat57 | 69 | 10.145 | 13.044 | 17.391 L | 8.696 | 25.000 | 40.000 | 33.333 | 25.926 L | 34.783 | 28.986 | 36.232 | 11.594 | 2.000 | 4.182 | 2 |
| Pat58 | 2272 | 20.555 | 0.748 | 24.956 | 3.609 | 14.286 | 31.712 | 31.733 | 32.444 | 36.004 | 30.414 | 33.583 | 0.704 L | 0.762 | 61.083 | 1 |
| Pat59 | 397 | 5.038 | 1.763 | 33.753 | 8.564 | 66.667 H | 37.297 | 29.146 | 24.623 L | 36.776 | 21.663 L | 41.562 | 1.763 | 1.167 | 24.600 | 3 |
| Pat60 | 1039 | 4.427 | 3.754 | 29.740 | 8.758 | 36.842 | 35.349 | 32.584 | 30.712 | 36.862 | 27.623 | 35.515 | 3.561 | 0.974 | 24.368 | 0 |
| Pat62 | 1826 | 8.105 | 3.614 | 27.382 | 8.817 | 26.984 | 32.543 | 28.571 | 29.646 | 34.940 | 28.368 | 36.692 | 3.998 | 1.159 | 30.296 | 0 |
| Pat64 | 874 | 3.776 | 7.666 | 24.600 | 10.984 | 36.667 | 31.875 | 30.787 | 23.820 | 34.325 | 27.346 | 38.330 | 5.606 | 0.817 | 20.250 | 0 |
| Pat67 | 24 | 12.500 | 20.833 H | 25.000 | 4.167 | 40.000 | 37.500 | 33.333 | 66.667 H | 25.000 L | 45.833 | 29.167 L | 8.333 | 0.400 L | 1.125 L | 6 |
| Pat70 | 95 | 5.263 | 7.368 | 28.421 | 9.474 | 37.500 | 31.035 | 46.296 H | 22.222 L | 41.053 | 26.316 | 32.632 | 4.211 | 0.500 L | 40.500 | 3 |
| Pat71 | 936 | 5.128 | 2.885 | 30.235 | 10.684 | 15.385 | 32.113 | 31.391 | 28.196 | 35.256 | 28.846 | 35.897 | 2.457 | 0.885 | 34.480 | 0 |
| Pat74 | 701 | 4.565 | 6.419 | 27.817 | 8.131 | 33.333 | 33.453 | 32.303 | 26.966 | 35.378 | 27.817 | 36.805 | 4.422 | 0.861 | 36.294 | 0 |
| Pat76 | 249 | 5.622 | 4.016 | 29.317 | 10.040 | 44.444 | 31.683 | 30.233 | 31.783 | 34.137 | 30.121 | 35.743 | 4.016 | 1.111 | 13.375 | 0 |
| Pat78 | 19 | 15.790 | 5.263 | 26.316 | 15.790 H | 0.000 | 14.286 L | 40.000 | 20.000 L | 57.895 | 15.790 L | 26.316 | 5.263 | 1.000 | 1.833 | 4 |
| Pat81 | 110 | 14.546 | 10.909 | 29.091 | 7.273 | 33.333 | 30.500 | 30.333 | 35.714 | 32.727 | 33.636 | 33.636 | 7.273 | 0.667 | 1.045 L | 1 |
| Pat82 | 581 | 4.131 | 6.196 | 27.711 | 9.983 | 35.484 | 34.952 | 29.870 | 25.325 | 36.145 | 24.613 | 39.243 | 6.196 | 1.161 | 24.700 | 0 |
| Pat85 | 441 | 3.628 | 7.256 | 25.397 | 8.163 | 31.429 | 37.423 | 35.135 | 31.982 | 37.642 | 27.891 | 34.467 | 4.762 | 0.600 | 24.667 | 0 |
| Pat86 | 65 | 12.308 | 9.231 | 20.000 L | 4.615 | 14.286 | 21.429 L | 37.037 | 33.333 | 36.923 | 40.000 H | 23.077 L | 4.615 | 0.429 L | 12.750 | 5 |

| | | DEAMINASES | | | | CODON CONTEXT TARGETING | | | | | | | STRAND BIAS | | Ti/Tv | |
|---|---|---|---|---|---|---|---|---|---|---|---|---|---|---|---|---|
| Patient | total cds | AID % | ADAR % | A3G % | A3B % | T_MC3 % | G_MC3 % | C_MC1 % | C_MC2 % | all_MC1 % | all_MC2 % | all_MC3 % | all_A % | all_A:T | all_GC TiTv | score |
| **NON-RESPONDERS** | | | | | | | | | | | | | | | | |
| Pat92 | 27 | 22.222 | 7.407 | 22.222 | 7.407 | 0.000 | 25.000 L | 50.000 H | 28.571 | 51.852 H | 29.630 | 18.519 L | 14.815 | 4.000 | 1.750 | 4 |
| Pat98 | 40 | 20.000 | 12.500 | 20.000 L | 5.000 | 66.667 H | 31.579 | 57.143 H | 28.571 | 47.500 H | 22.500 | 30.000 L | 10.000 | 1.333 | 3.714 | 5 |

TABLE 3

| | | DEAMINASES | | | | CODON CONTEXT TARGETING | | | | | | | STRAND BIAS | | Ti/Tv | |
|---|---|---|---|---|---|---|---|---|---|---|---|---|---|---|---|---|
| Patient | total cds | AID % | ADAR % | A3G % | A3B % | T_MC3 % | G_MC3 % | C_MC1 % | C_MC2 % | all_MC1 % | all_MC2 % | all_MC3 % | all_A % | all_A:T | all_GC TiTv | score |
| **NON-RESPONDERS, LONGTERM SURVIVAL** | | | | | | | | | | | | | | | | |
| Pat11 | 2936 | 4.564 | 1.941 | 31.710 | 10.797 | 29.167 | 34.167 | 31.943 | 28.963 | 35.899 | 27.146 | 36.955 | 1.737 | 1.063 | 41.343 | 0 |
| Pat119 | 374 | 5.615 | 5.348 | 34.225 | 6.684 | 37.931 | 32.667 | 31.707 | 31.098 | 37.166 | 28.075 | 34.759 | 8.289 | 1.069 | 7.051 | 0 |
| Pat13 | 140 | 5.000 | 4.286 | 29.286 | 11.429 | 28.571 | 35.556 | 40.000 H | 22.500 L | 40.714 | 23.571 | 35.714 | 5.714 | 1.143 | 12.889 | 2 |
| Pat159 | 306 | 6.536 | 4.902 | 27.778 | 12.418 H | 18.182 L | 26.230 L | 32.716 | 26.543 | 35.294 | 31.046 | 33.660 | 3.595 | 1.000 | 11.909 | 2 |
| Pat16 | 2646 | 3.590 | 5.026 | 30.763 | 9.033 | 34.400 | 32.629 | 30.833 | 29.242 | 36.092 | 27.929 | 35.979 | 4.271 | 0.904 | 21.296 | 0 |
| Pat163 | 62 | 12.903 | 8.065 | 20.968 L | 4.839 | 50.000 | 21.053 L | 22.857 L | 40.000 | 30.645 | 37.097 H | 32.258 | 6.452 | 1.000 | 9.800 | 4 |
| Pat18 | 37 | 10.811 | 8.108 | 27.027 | 2.703 | 40.000 | 16.667 L | 25.000 | 43.750 | 24.324 L | 48.649 | 27.027 L | 10.811 | 0.800 | 1.800 | 2 |
| Pat27 | 34 | 14.706 | 8.824 | 20.588 L | 14.706 H | 75.000 H | 35.000 | 62.500 H | 12.500 L | 35.294 | 29.412 | 35.294 | 5.882 | 0.500 L | 2.500 L | 6 |
| Pat28 | 864 | 19.792 | 0.347 L | 22.569 | 3.125 | 25.000 | 34.532 | 27.397 | 36.530 | 34.144 | 30.787 | 35.069 | 0.579 L | 1.250 | 56.000 | 2 |
| Pat83 | 43 | 6.977 | 9.302 | 25.581 | 9.302 | 0.000 | 38.889 | 15.790 L | 31.579 | 27.907 L | 30.233 | 41.861 | 9.302 | 2.000 | 17.500 | 2 |

## Example 3

### Genetic indicators of deaminase activity for predicting response to pembrolizumab treatment

Monoclonal antibodies directed against programmed cell death 1 receptor (PD-1), such as pembrolizumab, yield considerable clinical benefit for patients with lung cancer by inhibiting immune checkpoint activity. However, clinical predictors of patient response to this immunotherapy are lacking. A clinical trial assessing the efficacy of pembrolizumab (Keytruda™) in patients with non-small cell lung cancer demonstrated a clinical benefit in 14 patients of 34 patients treated (41.2%; durable clinical benefit defined as partial or stable response lasting >6 months) (Rizvi et al. (2015) Science 348 6230: 124-128). No features exclusive to the responders were identified that could be used to predict patient response to treatment.

The whole exome sequence of each of the patients in this clinical study was analyzed as described in Example 1 to determine whether genetic indicators of deaminase activity were associated with response or non-response to therapy. In particular, the genetic indicators of deaminase activity that were assessed included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs resulting from a mutation of a cytosine nucleotide which were at a MC-2 site (C_MC2%); the percentage of the SNVs resulting from a mutation of a thymine nucleotide which were at a MC-1 site (T_MC1%); the percentage of the SNVs resulting from a mutation of a thymine nucleotide which were at a MC-2 site (T_MC2%); the percentage of the SNVs resulting from a mutation of a thymine nucleotide which were at a MC-3 site (T_MC3%); the percentage of SNVs that were at a MC-1 site (all_MC1%); the percentage of SNVs that were at a MC-2 site (all_MC2%); and the percentage of SNVs that were at a MC-3 site (all_MC3%)

- and those indicators assessing strand bias and/or nucleotide targeting, including the percentage of SNVs resulting from mutation of an guanine nucleotide (all_G%); the percentage of SNVs resulting from mutation of an adenine nucleotide (all_A%); the ratio of the percentage of SNVs resulting from mutation of an adenine nucleotide to the percentage of SNVs resulting from a mutation of a thymine nucleotide (all A:T); and the transition-transversion ratio of SNVs resulting from mutation of a cytosine or guanine (all_GC_TiTv).

Table 4 shows the results of the analysis of genetic indicators of deaminase activity for the 14 'responders'. The data from these responders was used to calculate the Range Intervals (RIs) for each indicator to which the data from the "non-responders" was then compared. In this example, the RI for each indicator was set by the maximum observed value and the minimum observed value for that indicator. Where the observed value of a genetic indicator of deaminase activity was outside the RI (H - high or L - low compared to the RI), a score of 1 was attributed to that indicator. Where the observed value of a genetic indicator of deaminase activity was within the RI, a score of 0 was attributed to that indicator. The score provided in the right hand column of the Table is the total score for the patient. Thus, where the patient has no genetic indicators outside of the RI, i.e. no "outliers", the total score was 0. This total score is also called the predicted test score. As can be seen, all of the patients responding to pembrolizumab treatment had a score of 0.

In contrast, and as shown in Table 5, all 17 patients within the non-responder group had at least one value outside the RI, with the number of outliers added to comprise each patient's score. This indicates that an assessment of genetic indicators of deaminase activity as described here prior to treatment would have predicted that none of these patients would have been suitable for treatment, i.e. predicted that all of the patients would have been non-responders.

TABLE 4

| Patient | total cds | DEAMINASES | | | | CODON CONTEXT TARGETTING | | | | | | | NUCLEOTIDE RATIOS | | STRAND BIAS all_A:T | TI/Tv all_GC TiTv | SCORE |
|---------|-----------|------------|---------|--------|--------|----------|----------|----------|----------|------------|------------|------------|---------|---------|-----|-----|-----|
| | | AID % | ADAR % | A3G % | A3B % | C_MC2 % | T_MC1 % | T_MC2 % | T_MC3 % | all_MC1 % | all_MC2 % | all_MC3 % | all_G % | all_A % | | | |
| **Responders** | | | | | | | | | | | | | | | | | |
| AL4602 | 227 | 12.33 | 6.17 | 33.48 | 2.20 | 48.00 | 31.82 | 59.09 | 9.09 | 45.37 | 42.73 | 11.89 | 44.93 | 12.33 | 1.27 | 0.64 | 0 |
| CA9903 | 292 | 19.86 | 8.22 | 24.32 | 3.42 | 42.35 | 50.00 | 50.00 | 0.00 | 46.92 | 39.73 | 13.36 | 48.29 | 13.01 | 1.36 | 0.35 | 0 |
| DI6359 | 203 | 16.75 | 14.29 | 17.24 | 4.43 | 51.02 | 36.84 | 52.63 | 10.53 | 41.38 | 43.35 | 15.27 | 43.35 | 23.15 | 2.47 | 0.54 | 0 |
| GR0134 | 49 | 18.37 | 6.12 | 26.53 | 2.04 | 35.71 | 40.00 | 60.00 | 0.00 | 36.73 | 57.14 | 6.12 | 46.94 | 14.29 | 1.40 | 0.85 | 0 |
| HE3202 | 695 | 16.26 | 8.49 | 24.32 | 9.06 | 45.29 | 47.50 | 40.00 | 12.50 | 51.37 | 36.12 | 12.52 | 50.79 | 11.37 | 1.98 | 0.47 | 0 |
| KA3947 | 275 | 14.55 | 9.82 | 17.82 | 9.45 | 42.42 | 22.73 | 77.27 | 0.00 | 45.82 | 40.36 | 13.82 | 42.18 | 13.82 | 1.73 | 0.38 | 0 |
| M4945 | 399 | 17.04 | 8.27 | 22.31 | 1.50 | 42.52 | 35.00 | 52.50 | 12.50 | 45.61 | 40.60 | 13.78 | 43.86 | 14.29 | 1.43 | 0.75 | 0 |
| RH090935 | 188 | 13.83 | 7.45 | 12.77 | 12.23 | 46.30 | 36.36 | 54.55 | 9.09 | 50.53 | 36.70 | 12.77 | 56.38 | 9.04 | 1.55 | 1.19 | 0 |
| RI1933 | 444 | 15.77 | 8.11 | 29.96 | 4.05 | 49.07 | 48.15 | 40.74 | 11.11 | 46.40 | 40.54 | 13.06 | 45.95 | 11.71 | 1.93 | 0.38 | 0 |
| SA9755 | 1118 | 13.77 | 8.41 | 17.53 | 12.25 | 42.90 | 36.26 | 48.35 | 15.38 | 48.93 | 39.18 | 11.90 | 50.36 | 10.64 | 1.31 | 0.48 | 0 |
| SB010944 | 187 | 17.11 | 17.65 | 14.44 | 2.67 | 40.00 | 29.17 | 62.50 | 8.33 | 45.45 | 48.66 | 5.88 | 40.64 | 22.46 | 1.75 | 2.27 | 0 |
| SC0899 | 276 | 15.58 | 8.70 | 22.83 | 6.52 | 38.30 | 26.09 | 60.87 | 13.04 | 38.41 | 46.74 | 14.86 | 43.12 | 14.49 | 1.74 | 0.26 | 0 |
| SC6470 | 168 | 20.24 | 2.98 | 26.79 | 4.76 | 41.67 | 30.77 | 53.85 | 15.38 | 49.40 | 38.10 | 12.50 | 48.81 | 7.74 | 1.00 | 0.33 | 0 |
| Y2087 | 479 | 18.79 | 8.56 | 25.05 | 4.80 | 48.95 | 46.15 | 51.28 | 2.56 | 50.31 | 43.42 | 6.26 | 40.92 | 11.27 | 1.38 | 1.70 | 0 |
| **RANGE** | **1118** | **20.24** | **17.65** | **33.48** | **12.25** | **51.02** | **50.00** | **77.27** | **15.38** | **51.37** | **57.14** | **15.27** | **56.38** | **23.15** | **2.47** | **2.27** | **0** |
| **INTERVAL** | **49** | **12.33** | **2.98** | **12.77** | **1.50** | **35.71** | **22.73** | **40.00** | **0.00** | **36.73** | **36.12** | **5.88** | **40.64** | **7.74** | **1.00** | **0.26** | **0** |

TABLE 5

| Patient | total cds | DEAMINASES | | | | CODON CONTEXT TARGETING | | | | | | | NUCLEOTIDE RATIOS | | STRAND BIAS | TI/Tv | SCORE |
|---|---|---|---|---|---|---|---|---|---|---|---|---|---|---|---|---|---|
| | | AID % | ADAR % | A3G % | A3B % | C_MC2 % | T_MC1 % | T_MC2 % | T_MC3 % | all_MC1 % | all_MC2 % | all_MC3 % | all_G % | all_A % | all_A:T | all_GC TiTv | |
| **Non-Responders** | | | | | | | | | | | | | | | | | |
| AU5884 | 30 | 10.00 L | 3.33 | 30.00 | 6.67 | 54.55 R | 0.00 L | 100.00 H | 0.00 | 36.67 L | 53.33 | 10.00 | 40.00 L | 13.33 | 1.33 | 1.30 | 6 |
| BL3403 | 140 | 12.86 | 7.86 | 27.14 | 1.43 L | 44.68 | 50.00 | 37.50 L | 12.50 | 53.57 H | 40.71 | 5.71 L | 46.43 | 14.29 | 2.50 R | 3.48 H | 6 |
| DM123062 | 120 | 20.00 | 13.33 | 13.33 | 9.17 | 40.91 | 31.25 | 62.50 | 6.25 | 44.17 | 43.33 | 12.50 | 38.33 L | 11.67 | 0.88 L | 1.00 | 2 |
| GR4788 | 158 | 17.09 | 6.96 | 26.58 | 3.16 | 43.86 | 16.67 L | 75.00 | 8.33 | 43.04 | 43.67 | 13.29 | 46.84 | 9.49 | 1.25 | 0.52 | 1 |
| JB112852 | 175 | 15.43 | 6.86 | 20.57 | 6.86 | 50.00 | 50.00 | 31.25 L | 18.75 H | 49.71 | 42.29 | 8.00 | 47.43 | 8.00 | 0.88 L | 2.54 H | 4 |
| LO3793 | 104 | 16.35 | 9.62 | 15.38 | 11.54 | 52.94 R | 80.00 H | 20.00 L | 0.00 | 44.23 | 43.27 | 12.50 | 45.19 | 12.50 | 1.30 | 1.13 | 3 |
| LO5004 | 68 | 19.12 | 1.47 L | 25.00 | 1.47 L | 51.61 R | 0.00 L | 80.00 H | 20.00 H | 39.71 | 45.59 | 14.71 | 32.35 L | 14.71 | 2.00 | 0.61 | 7 |
| MA7027 | 274 | 14.23 | 10.95 | 27.74 | 6.57 | 40.86 | 42.86 | 39.29 L | 17.86 H | 47.45 | 40.51 | 12.04 | 42.34 | 13.50 | 1.32 | 0.42 | 2 |
| NI9507 | 31 | 25.81 H | 3.23 | 16.13 | 6.45 | 33.33 L | 0.00 L | 75.00 | 25.00 H | 48.39 | 48.39 | 3.23 L | 58.06 H | 0.00 L | 0.00 L | 4.40 H | 9 |
| R7495 | 118 | 13.56 | 13.56 | 22.88 | 3.39 | 41.30 | 21.43 L | 78.57 H | | 44.07 | 45.76 | 10.17 | 30.51 L | 18.64 | 1.57 | 0.78 | 3 |
| RO3338 | 99 | 16.16 | 5.05 | 20.20 | 4.04 | 46.88 | 69.23 H | 23.08 L | 7.69 | 46.46 | 45.45 | 8.08 | 42.42 | 12.12 | 0.92 L | 1.24 | 3 |
| SR070761 | 169 | 15.38 | 11.24 | 21.30 | 4.14 | 46.67 | 26.09 | 65.22 | 8.70 | 42.60 | 48.52 | 8.88 | 37.28 L | 13.61 | 1.00 | 1.80 | 1 |
| TU0428 | 591 | 16.07 | 11.51 | 18.95 | 3.05 | 45.73 | 37.68 | 53.62 | 8.70 | 47.72 | 41.96 | 10.32 | 37.39 L | 17.26 | 1.48 | 0.83 | 1 |
| VA1330 | 36 | 11.11 L | 19.44 R | 22.22 | 5.56 | 50.00 | 44.44 | 33.33 L | 22.22 H | 33.33 L | 44.44 | 22.22 H | 27.78 L | 25.00 H | 1.00 | 1.25 | 8 |
| VA7859 | 8 | 25.00 H | 0.00 L | 12.50 L | 0.00 L | 33.33 L | 0.00 L | 100.00 H | 0.00 | 37.50 | 50.00 | 12.50 | 50.00 | 0.00 L | 0.00 L | 2.50 H | 10 |
| WA7899 | 117 | 25.64 H | 12.82 | 15.38 | 0.85 L | 53.66 R | 33.33 | 61.11 | 5.56 | 38.46 | 52.14 | 9.40 | 28.21 L | 21.37 | 1.39 | 0.85 | 4 |
| ZA6505 | 351 | 17.38 | 16.81 | 11.68 L | 2.85 | 52.58 R | 28.57 | 61.22 | 10.20 | 41.88 | 50.43 | 7.69 | 35.90 L | 22.51 | 1.61 | 1.79 | 3 |

## Example 4

### Genetic indicators of deaminase activity for predicting relapse in responders to pembrolizumab treatment

To determine whether the genetic indicators of deaminase activity could be used to identify differences in individual patient profiles before and after immunotherapy treatment in patients currently on effective therapy, the whole exome sequences of patients from a clinical trial assessing the efficacy of pembrolizumab treatment were assessed.

The clinical trial was conducted on 78 patients with metastatic melanoma being treated with pembrolizumab (Keytruda™). As described in Zaretsky et al. (New England Journal of Medicine (2016) 375(9): 819-829), 42 of these patients had an objective response (53.8%). From this subgroup, 15 then went on to have disease progression, and 4 of these patients were considered to have had an objective response followed by recurring tumour growth ("late acquired resistance"). Whole exome sequencing was performed on skin biopsies in these 4 'recurrent' patients obtained before and after treatment. Zaretsky et al. did not identify any factors that predicted this 'recurrent' response to treatment, although they did suggest that *JAK* mutations were implicated in the aetiology of the disease.

The whole exome sequences from these 4 'recurrent' patients, from samples taken before and after treatment, were assessed as described in Example 1 to determine whether genetic indicators of deaminase activity were associated with relapse. In particular, the genetic indicators of deaminase activity that were assessed included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs resulting from a mutation of a cytosine nucleotide which were at a MC-2 site (C_MC2%); the percentage of the SNVs resulting from a mutation of a thymine nucleotide which were at a MC-1 site (T_MC1%); the percentage of the SNVs resulting from a mutation of a thymine nucleotide which were at a MC-2 site (T_MC2%); the percentage of the SNVs resulting from a mutation of a thymine nucleotide which were at a MC-3 site (T_MC3%); the percentage of SNVs that were at a MC-1 site (all_MC1%); the percentage of SNVs that were at a MC-2 site (all_MC2%); and the percentage of SNVs that were at a MC-3 site (all_MC3%)

- and those indicators assessing strand bias and/or nucleotide targeting, including the percentage of SNVs resulting from mutation of an guanine nucleotide (all_G%); the percentage of SNVs resulting from mutation of an adenine nucleotide (all_A%); the ratio of the percentage of SNVs resulting from mutation of an adenine nucleotide to the percentage of SNVs resulting from a mutation of a thymine nucleotide (all A:T); and the transition-transversion ratio of SNVs resulting from mutation of an cytosine or guanine (all_GCTiTv).

Table 6 shows the results of the analysis of genetic indicators of deaminase activity for the 4 patients prior to relapse (or prior to the development of resistance to therapy). This data was used to calculate the Range Intervals (RIs) for each indicator to which the data from the "post-relapse" samples were then compared. In this example, the RI for each indicator was set by the maximum observed value and the minimum observed value for that indicator. Where the observed value of a genetic indicator of deaminase activity was outside the RI (H - high or L - low compared to the RI), a score of 1 was attributed to that indicator. Where the observed value of a genetic indicator of deaminase activity was within the RI, a score of 0 was attributed to that indicator. The score provided in the right hand column of the Table is the total score for the patient. Thus, where the patient has no genetic indicators outside of the RI, i.e. no "outliers", the total score was 0. This total score is also called the predicted test score. As can be seen, all of the patients that were responding to pembrolizumab treatment at the time the sample was taken had a score of 0.

In contrast, and as shown in Table 7, all 4 patients had at least one value outside the RI following relapse, with the number of outliers added to comprise each patient's score. This indicates that an assessment of genetic indicators of deaminase activity as described here during treatment would have predicted that none of these patients would have continued to respond to treatment, i.e. all would have developed resistance to treatment and would have relapsed.

TABLE 6

| Patient | total cds | DEAMINASES | | | | CODON CONTEXT TARGETTING | | | | | | | NUCLEOTIDE RATIOS | | STRAND BIAS all_A:T | TI/Tv all_GC TiTv | SCORE |
|---|---|---|---|---|---|---|---|---|---|---|---|---|---|---|---|---|---|
| | | AID % | ADAR % | A3G % | A3B % | C_MC2 % | T_MC1 % | T_MC2 % | T_MC3 % | all_MC1 % | all_MC2 % | all_MC3 % | all_G % | all_A % | | | |
| Patients at baseline | | | | | | | | | | | | | | | | | |
| Relapse_1 | 1029 | 4.66 | 3.69 | 21.67 | 10.98 | 52.16 | 40.54 | 40.54 | 18.92 | 51.61 | 41.94 | 6.45 | 46.26 | 3.01 | 0.84 | 30.00 | 0 |
| Relapse_2 | 209 | 5.26 | 4.31 | 19.62 | 10.53 | 39.78 | 33.33 | 66.67 | 0.00 | 27.27 | 72.73 | 0.00 | 47.37 | 5.26 | 1.83 | 13.77 | 0 |
| Relapse_3 | 384 | 5.47 | 4.17 | 30.47 | 7.29 | 55.03 | 29.41 | 52.94 | 17.65 | 56.67 | 40.00 | 3.33 | 43.75 | 7.81 | 1.76 | 8.63 | 0 |
| Relapse_4 | 314 | 8.92 | 4.14 | 23.57 | 8.60 | 49.30 | 25.00 | 37.50 | 37.50 | 37.50 | 62.50 | 0.00 | 44.59 | 7.64 | 3.00 | 9.85 | 0 |
| RANGE | 1029 | 8.92 | 4.31 | 30.47 | 10.98 | 55.03 | 40.54 | 66.67 | 37.50 | 56.67 | 72.73 | 6.45 | 47.37 | 7.81 | 3.00 | 30.00 | 0 |
| INTERVAL | 209 | 4.66 | 3.69 | 19.62 | 7.29 | 39.78 | 25.00 | 37.50 | 0.00 | 27.27 | 40.00 | 0.00 | 43.75 | 3.01 | 0.84 | 8.63 | 0 |

TABLE 7

| Patient | total cds | DEAMINASES | | | | CODON CONTEXT TARGETTING | | | | | | | NUCLEOTIDE RATIOS | | STRAND BIAS | TI/Tv | SCORE |
|---|---|---|---|---|---|---|---|---|---|---|---|---|---|---|---|---|---|
| | | AID % | ADAR % | A3G % | A3B % | C_MC2 % | T_MC1 % | T_MC2 % | T_MC3 % | all_MC1 % | all_MC2 % | all_MC3 % | all_G % | all_A % | all_A:T | all_GC TiTv | |
| Patients after relapse | | | | | | | | | | | | | | | | | |
| Relapse_1 | 1010 | 4.46 L | 4.75 H | 21.09 | 10.59 | 52.13 | 36.17 | 51.06 | 12.77 | 54.75 | 41.19 | 4.06 | 45.35 | 3.47 | 0.74 L | 28.00 | 3 |
| Relapse_2 | 530 | 12.45 H | 5.85 H | 19.06 L | 8.68 | 39.29 L | 38.46 | 50.00 | 11.54 | 50.38 | 41.70 | 7.92 H | 44.34 | 8.49 H | 1.73 | 4.88 L | 7 |
| Relapse_3 | 272 | 3.31 L | 1.84 L | 29.78 | 7.35 | 57.25 H | 30.00 | 60.00 | 10.00 | 48.53 | 46.69 | 4.78 | 46.32 | 1.84 L | 0.50 L | 22.36 | 5 |
| Relapse_4 | 372 | 9.14 H | 4.84 H | 23.12 | 7.80 | 50.66 | 31.58 | 47.37 | 21.05 | 51.08 | 43.55 | 5.38 | 44.35 | 9.68 H | 1.89 | 5.89 L | 4 |

## Example 5

### Genetic indicators of deaminase activity for predicting response to atezolizumab treatment

Inhibition of programmed death-ligand 1 (PD-L1) with atezolizumab can induce durable clinical benefit in patients with metastatic urothelial cancers. Mutation load and PD-L1 immune cell (IC) staining have been associated with response to therapy but they lack sufficient sensitivity and specificity for clinical use. In a clinical trial assessing the efficacy of atezolizumab (Tecentriq™), which was performed on 29 patients, 9 patients had an objective response (31%) and 20 had no durable clinical response (69%) (Snyder et al. PLoS Medicine, (2017) 14(5), e1002309). Whole exome sequencing of tissue biopsies from these patients was performed in order to identify factors associated with response. While it was found that patients with durable clinical benefit (i.e. the 9 "responders") displayed a higher proportion of tumor-infiltrating T lymphocytes (TIL), Snyder et al. concluded this measure would not be suitable as a biomarker and further investigation is required.

The whole exome sequence of each of the patients in this clinical study was analyzed as described in Example 1 to determine whether genetic indicators of deaminase activity were associated with response or non-response to atezolizumab therapy, and could thus be used as predictors of response or non-response. In particular, the genetic indicators of deaminase activity that were assessed included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs resulting from a mutation of a cytosine nucleotide which were at a MC-1 site (C_MC1%); the percentage of the SNVs resulting from a mutation of a cytosine nucleotide which were at a MC-2 site (C_MC2%); the percentage of the SNVs resulting from a mutation of a cytosine nucleotide which were at a MC-3 site (C_MC3%); the percentage of SNVs that were at a MC-1 site (all_MC1%); the percentage of SNVs that were at a MC-2 site (all_MC2%); and the percentage of SNVs that were at a MC-3 site (all_MC3%)

- and those indicators assessing strand bias and/or nucleotide targeting, including the percentage of SNVs resulting from mutation of an thymine nucleotide (all_T%); the percentage of SNVs resulting from mutation of an adenine nucleotide (all_A%); the ratio of the percentage of SNVs resulting from mutation of an adenine nucleotide to the percentage of SNVs resulting from a mutation of a thymine nucleotide (all A:T); and the transition-transversion ratio of SNVs resulting from mutation of a guanine (all_G_TiTv).

Table 8 shows the results of the analysis of genetic indicators of deaminase activity for the 9 'responders'. The data from these responders was used to calculate the Range Intervals (RIs) for each indicator to which the data from the "non-responders" was then compared. In this example, the RI for each indicator was set by the maximum observed value and the minimum observed value for that indicator. Where the observed value of a genetic indicator of deaminase activity was outside the RI (H - high or L - low compared to the RI), a score of 1 was attributed to that indicator. Where the observed value of a genetic indicator of deaminase activity was within the RI, a score of 0 was attributed to that indicator. The score provided in the right hand column of the Table is the total score for the patient. Thus, where the patient has no genetic indicators outside of the RI, i.e. no "outliers", the total score was 0. This total score is also called the predicted test score. As can be seen, all of the patients responding to atezolizumab treatment had a score of 0.

In contrast, and as shown in Table 9, all 29 patients within the non-responder group had at least one value outside the RI, with the number of outliers added to comprise each patient's total score. This indicates that an assessment of genetic indicators of deaminase activity as described here prior to treatment would have predicted that none of these patients would have been suitable for treatment, i.e. predicted at all of the patients would have been non-responders.

TABLE 8

| Patient | total cds | DEAMINASES | | | | CODON CONTEXT TARGETTING | | | | | | NUCLEOTIDE RATIOS | | STRAND BIAS all_A:T | Ti/Tv all_G | SCORE |
|---|---|---|---|---|---|---|---|---|---|---|---|---|---|---|---|---|
| | | AID % | ADAR % | A3G % | A3B % | C_MC1 % | C_MC2 % | C_MC3 % | a all_MC1 % | all_MC2 % | all_MC3 % | all_T % | all_A % | | | |
| **Responders** | | | | | | | | | | | | | | | | |
| p_1233 | 4 | 50.00 | 0.00 | 25.00 | 0.00 | 50.00 | 0.00 | 50.00 | 25.00 | 25.00 | 50.00 | 0.00 | 0.00 | 0.00 | 1.00 | **0** |
| p_1849 | 292 | 7.19 | 1.03 | 10.96 | 32.53 | 26.39 | 26.39 | 47.22 | 36.64 | 22.95 | 40.41 | 1.03 | 2.05 | 2.00 | 1.96 | **0** |
| p_2131 | 985 | 2.23 | 1.12 | 4.57 | 37.67 | 28.05 | 28.74 | 43.22 | 40.81 | 20.91 | 38.27 | 1.02 | 1.12 | 1.10 | 0.76 | **0** |
| p_2278 | 136 | 4.41 | 2.21 | 11.76 | 28.68 | 26.15 | 27.69 | 46.15 | 37.50 | 21.32 | 41.18 | 2.21 | 2.94 | 1.33 | 1.06 | **0** |
| p_2389 | 318 | 3.14 | 0.94 | 7.23 | 30.19 | 30.92 | 30.26 | 38.82 | 40.25 | 24.53 | 35.22 | 1.89 | 0.63 | 0.33 | 1.05 | **0** |
| p_5037 | 1170 | 25.73 | 1.88 | 26.41 | 0.09 | 32.95 | 26.49 | 40.56 | 33.50 | 34.10 | 32.39 | 1.62 | 2.22 | 1.37 | 29.65 | **0** |
| p_5122 | 347 | 10.66 | 8.65 | 20.46 | 12.68 | 28.87 | 28.17 | 42.96 | 34.58 | 28.24 | 37.18 | 6.34 | 12.68 | 2.00 | 1.36 | **0** |
| p_6229 | 83 | 24.10 | 10.84 | 18.07 | 8.43 | 34.48 | 20.69 | 44.83 | 36.14 | 21.69 | 42.17 | 7.23 | 13.25 | 1.83 | 1.06 | **0** |
| p_6800 | 593 | 3.88 | 4.05 | 8.94 | 26.98 | 28.13 | 26.79 | 45.09 | 39.46 | 23.95 | 36.59 | 4.55 | 5.23 | 1.15 | 1.66 | **0** |
| **RANGE** | **1170** | **50.00** | **10.84** | **26.41** | **37.67** | **50.00** | **30.26** | **50.00** | **40.81** | **34.10** | **50.00** | **7.23** | **13.25** | **2.00** | **29.65** | **0** |
| **INTERVAL** | **4** | **2.23** | **0.00** | **4.57** | **0.00** | **26.15** | **0.00** | **38.82** | **25.00** | **20.91** | **32.39** | **0.00** | **0.00** | **0.00** | **0.76** | **0** |

TABLE 9

| Patient | total cds | DEAMINASES | | | | CODON CONTEXT TARGETING | | | | | | NUCLEOTIDE RATIOS | | STRAND BIAS | Ti/Tv | SCORE |
|---|---|---|---|---|---|---|---|---|---|---|---|---|---|---|---|---|
| | | AID % | ADAR % | A3G % | A3B % | C_MC1 % | C_MC2 % | C_MC3 % | all_MC1 % | all_MC2 % | all_MC3 % | all_T % | all_A % | all_A:T % | all_G | |
| Non-responders | | | | | | | | | | | | | | | | |
| p_0040 | 917 | 50.00 | 4.58 | 11.56 | 20.50 | 33.33 | 27.68 | 38.98 | 38.06 | 23.23 | 38.71 | 5.45 | 6.65 | 1.22 | 0.75 L | 1 |
| p_0471 | 326 | 7.19 | 1.53 | 7.36 | 34.97 | 28.67 | 25.17 | 46.15 | 40.80 | 16.87 L | 42.33 | 2.15 | 0.92 | 0.43 | 1.19 | 1 |
| p_0522 | 184 | 2.23 | 7.61 | 21.74 | 15.76 | 27.27 | 27.27 | 45.45 | 34.24 | 27.17 | 38.59 | 8.15 H | 7.61 | 0.93 | 1.87 | 1 |
| p_1249 | 956 | 4.41 | 14.85 H | 23.12 | 2.72 | 27.38 | 17.85 | 54.77 H | 28.45 | 22.28 | 49.27 | 15.80 H | 19.46 H | 1.23 | 2.42 | 4 |
| p_1994 | 28 | 3.14 | 0.00 | 10.71 | 10.71 | 25.00 L | 41.67 H | 33.33 L | 39.29 | 25.00 | 35.71 | 3.57 | 10.71 | 3.00 H | 1.40 | 4 |
| p_2849 | 624 | 25.73 | 5.13 | 7.05 | 30.45 | 29.93 | 25.18 | 44.89 | 41.99 H | 20.83 L | 37.18 | 3.85 | 5.77 | 1.50 | 1.07 | 2 |
| p_2937 | 574 | 10.66 | 1.74 | 29.62 H | 4.70 | 31.11 | 25.00 | 43.89 | 39.55 | 26.13 | 34.32 | 1.22 | 2.26 | 1.86 | 0.30 L | 2 |
| p_3529 | 266 | 24.10 | 5.64 | 12.41 | 21.80 | 28.07 | 33.33 H | 38.60 L | 38.72 | 25.56 | 35.71 | 4.51 | 5.64 | 1.25 | 1.12 | 2 |
| p_4072 | 140 | 3.88 | 7.14 | 25.71 | 6.43 | 37.14 | 22.86 | 40.00 | 37.14 | 25.00 | 37.86 | 3.57 | 7.86 | 2.20 H | 2.00 | 1 |
| p_5338 | 68 | 50.00 | 10.29 | 26.47 H | 4.41 | 32.14 | 17.86 | 50.00 | 38.24 | 26.47 | 35.29 | 8.82 H | 8.82 | 1.00 | 2.11 | 2 |
| p_6428 | 13 | 7.19 | 30.77 H | 7.69 | 15.38 | 0.00 L | 33.33 | 66.67 H | 15.38 L | 30.77 | 53.85 H | 23.08 H | 30.77 H | 1.33 | 0.50 L | 9 |
| p_7577 | 55 | 2.23 | 10.91 H | 32.73 H | 7.27 | 37.50 | 16.67 | 45.83 | 36.36 | 23.64 | 40.00 | 10.91 H | 7.27 | 0.67 | 3.20 | 3 |
| p_7729 | 98 | 4.41 | 2.04 | 12.24 | 20.41 | 27.50 | 37.50 H | 35.00 L | 39.80 | 27.55 | 32.65 | 4.08 | 4.08 | 1.00 | 1.50 | 2 |
| p_8728 | 64 | 3.14 | 10.94 H | 17.19 | 7.81 | 33.33 | 27.78 | 38.89 | 35.94 | 31.25 | 32.81 | 21.88 H | 10.94 | 0.50 | 1.50 | 2 |
| p_9517 | 373 | 25.73 | 1.07 | 13.67 | 21.45 | 26.70 | 32.98 H | 40.31 | 39.41 | 25.20 | 35.39 | 2.41 | 1.88 | 0.78 | 1.16 | 1 |
| p_9723 | 33 | 10.66 | 18.18 H | 15.15 | 0.00 | 45.45 | 9.09 | 45.45 | 39.39 | 15.15 L | 45.45 | 24.24 H | 15.15 L | 0.63 | 8.00 | 4 |
| p_9881 | 17 | 24.10 | 17.65 H | 35.29 H | 0.00 | 28.57 | 14.29 | 57.14 H | 35.29 | 17.65 L | 47.06 | 5.88 | 23.53 H | 4.00 H | 1.50 | 6 |

## Example 6

**Genetic indicators of deaminase activity for predicting response to anti-PD-1 therapy treatment**

PD-1 immune checkpoint blockade provides significant clinical benefits for melanoma patients. In a clinical trial investigating sensitivity or resistance to anti-PD-1 therapy, 21 of 38 patients (55%) had an objective response, and 17 of 38 had no objective response (45%) (Hugo et al. (2016) Cell, 165: 35-44). Whole exome sequencing of tissue biopsies from these patients was performed by Hugo et al. in order to identify factors associated with response, however specific factors predicting outcome were not identified..

The whole exome sequence of each of the patients in this clinical study was analyzed as described in Example 1 to determine whether genetic indicators of deaminase activity were associated with response or non-response to anti-PD-1 therapy, and could thus be used as predictors of response or non-response. In particular, the genetic indicators of deaminase activity that were assessed included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs at the AID motif WRC/GYW which occurred at a MC-1 site (AID_MC1%); the percentage of the SNVs at the AID motif GYW which involved a G>A mutation and which occurred at a MC-3 site (AIDa-_GA3%); the percentage of the SNVs at the APOBEC3B motif TCA which involved a C>T mutation and which occurred at a MC-1 site (A3Ba_CT1%); the percentage of the SNVs at the APOBEC3B motif TCA which involved a C>T mutation and which occurred at a MC-3 site (A3Ba-_CT3%); the percentage of SNVs that were at a MC-1 site (all_MC1%); the percentage of SNVs that were at a MC-2 site (all_MC2%); and the percentage of SNVs that were at a MC-3 site (all_MC3%)

- and those indicators assessing strand bias and/or nucleotide targeting, including the percentage of SNVs resulting from mutation of an cytosine nucleotide (all_C%); the percentage of SNVs resulting from mutation of an guanine nucleotide (all_G%); the ratio of the number of SNVs resulting from mutation of an adenine nucleotide that are not in the deaminase motif WA to the number of SNVs resulting from a mutation of a thymine nucleotide that are not in the deaminase motif TW (Other A:T); and the transition-transversion ratio of SNVs resulting from mutation of a guanine or cytosine (all_GC_TiTv).

Table 10 shows the results of the analysis of genetic indicators of deaminase activity for the 19 'responders'. The data from these responders was used to calculate the Range Intervals (RIs) for each indicator to which the data from the "non-responders" was then compared. In this example, the RI for each indicator was set by the maximum observed value and the minimum observed value for that indicator. Where the observed value of a genetic indicator of deaminase activity was outside the RI (H - high or L - low compared to the RI), a score of 1 was attributed to that indicator. Where the observed value of a genetic indicator of deaminase activity was within the RI, a score of 0 was attributed to that indicator. The score provided in the right hand column of the Table is the total score for the patient. Thus, where the patient has no genetic indicators outside of the RI, i.e. no "outliers", the total score was 0. This total score is also called the predicted test score. As can be seen, all of the patients responding to trastuzumab treatment had a score of 0.

In contrast, and as shown in Table 11, 14 of the 17 patients (82%) within the non-responder group had at least one value outside the RI, with the number of outliers added to comprise each patient's total score. This indicates that an assessment of genetic indicators of deaminase activity as described here prior to treatment would have predicted that 14 of the 17 patients would not have been suitable for treatment, i.e. would have been non-responders.

TABLE 10

| Patient | total cds | DEAMINASES | | | | CODON CONTEXT TARGETTING | | | | | | | NUCLEOTIDE RATIOS | | STRAND BIAS | TiTv | SCORE |
|---|---|---|---|---|---|---|---|---|---|---|---|---|---|---|---|---|---|
| | | AID % | ADAR % | A3G % | A3B % | AID_MC1 % | AiDa-GA3 % | A3Ba_CT1 % | A3Ba_CT3 % | all_MC1 % | all_MC2 all % | all_Mc3 % | all_G % | all_C % | other_A:T | all_GC TiTv | |
| **Responders** | | | | | | | | | | | | | | | | | |
| patient_2 | 1730 | 3.58 | 6.13 | 25.32 | 9.19 | 32.26 | 4.55 | 61.54 | 0.00 | 52.77 | 43.06 | 4.16 | 43.53 | 47.46 | 1.08 | 27.11 | 0 |
| patient_3 | 1039 | 4.43 | 3.56 | 23.39 | 10.78 | 47.83 | 13.33 | 68.63 | 0.00 | 56.02 | 40.04 | 3.95 | 45.81 | 48.03 | 0.59 | 28.55 | 0 |
| patient_4 | 3431 | 4.17 | 5.19 | 23.96 | 11.08 | 40.56 | 2.00 | 56.25 | 1.25 | 55.17 | 40.13 | 4.69 | 44.65 | 46.52 | 1.02 | 27.44 | 0 |
| patient_5 | 288 | 7.29 | 4.17 | 25.69 | 9.03 | 38.10 | 16.67 | 60.00 | 0.00 | 55.56 | 40.28 | 4.17 | 44.79 | 45.83 | 2.00 | 12.74 | 0 |
| patient_6 | 190 | 2.63 | 2.63 | 32.63 | 5.79 | 40.00 | 0.00 | 40.00 | 0.00 | 50.53 | 45.79 | 3.68 | 42.63 | 50.00 | 1.25 | 16.60 | 0 |
| patient_8 | 722 | 12.47 | 5.82 | 22.58 | 4.16 | 48.89 | 2.08 | 53.85 | 0.00 | 51.39 | 43.91 | 4.71 | 50.55 | 36.98 | 1.67 | 29.10 | 0 |
| patient_9 | 435 | 5.75 | 3.22 | 28.05 | 9.43 | 40.00 | 28.57 | 57.89 | 0.00 | 54.02 | 40.69 | 5.29 | 47.59 | 45.98 | 1.80 | 19.35 | 0 |
| patient_13 | 399 | 5.01 | 5.26 | 27.32 | 6.27 | 35.00 | 0.00 | 72.73 | 0.00 | 46.37 | 48.12 | 5.51 | 39.35 | 52.13 | 0.63 | 35.50 | 0 |
| patient_15 | 471 | 4.03 | 4.03 | 30.57 | 10.40 | 42.11 | 12.50 | 81.48 | 0.00 | 54.56 | 39.28 | 6.16 | 48.41 | 44.37 | 0.88 | 28.13 | 0 |
| patient_18 | 277 | 6.50 | 4.33 | 28.52 | 8.30 | 27.78 | 0.00 | 70.00 | 0.00 | 45.85 | 46.93 | 7.22 | 44.40 | 46.93 | 0.33 | 18.46 | 0 |
| patient_19 | 552 | 3.44 | 4.71 | 24.09 | 8.15 | 42.11 | 0.00 | 73.68 | 0.00 | 56.88 | 38.41 | 4.71 | 46.92 | 44.57 | 1.63 | 20.04 | 0 |
| patient_21 | 217 | 5.53 | 4.61 | 21.20 | 10.14 | 41.67 | 0.00 | 77.78 | 0.00 | 51.61 | 42.40 | 5.99 | 46.54 | 45.62 | 0.40 | 15.67 | 0 |
| patient_24 | 349 | 4.58 | 3.15 | 31.23 | 7.45 | 12.50 | 0.00 | 55.56 | 0.00 | 50.14 | 44.99 | 4.87 | 45.27 | 45.56 | 0.50 | 12.78 | 0 |
| patient_26 | 1011 | 3.46 | 5.74 | 25.72 | 10.98 | 34.29 | 0.00 | 53.19 | 0.00 | 53.61 | 39.96 | 6.43 | 45.30 | 45.99 | 0.67 | 18.23 | 0 |
| patient_27 | 2073 | 4.78 | 5.35 | 24.89 | 10.18 | 49.49 | 0.00 | 58.62 | 0.00 | 53.40 | 42.45 | 4.15 | 44.72 | 46.12 | 1.82 | 25.15 | 0 |
| patient_28 | 414 | 5.80 | 9.18 | 28.26 | 6.52 | 37.50 | 0.00 | 58.33 | 0.00 | 52.66 | 43.00 | 4.35 | 43.96 | 41.30 | 1.09 | 11.61 | 0 |
| patient_33 | 596 | 4.53 | 3.52 | 34.73 | 4.87 | 40.74 | 0.00 | 60.00 | 0.00 | 52.35 | 43.62 | 4.03 | 45.47 | 48.15 | 0.70 | 17.00 | 0 |
| patient_34 | 68 | 16.18 | 8.82 | 17.65 | 0.00 | 45.45 | 20.00 | 0.00 | 0.00 | 57.35 | 38.24 | 4.41 | 41.18 | 30.88 | 1.60 | 2.77 | 0 |
| patient_35 | 283 | 8.48 | 6.71 | 20.85 | 9.54 | 45.83 | 11.11 | 37.50 | 0.00 | 45.94 | 46.29 | 7.77 | 51.94 | 33.92 | 1.33 | 7.68 | 0 |
| patient_37 | 498 | 3.61 | 3.41 | 24.30 | 11.04 | 61.11 | 0.00 | 56.67 | 0.00 | 56.22 | 40.16 | 3.61 | 42.57 | 50.60 | 0.89 | 22.20 | 0 |
| patient_38 | 73 | 6.85 | 4.11 | 30.14 | 5.48 | 40.00 | 0.00 | 33.33 | 0.00 | 42.47 | 47.95 | 9.59 | 39.73 | 54.79 | 0.00 | 6.67 | 0 |
| **RANGE** | **3431** | **16.18** | **9.18** | **34.73** | **11.08** | **61.11** | **28.57** | **81.48** | **1.25** | **57.35** | **48.12** | **9.59** | **51.94** | **54.79** | **2.00** | **35.50** | **0** |
| **INTERVAL** | **68** | **2.63** | **2.63** | **17.65** | **0.00** | **12.50** | **0.00** | **33.33** | **0.00** | **42.47** | **38.24** | **3.61** | **39.35** | **30.88** | **0.00** | **2.77** | **0** |

TABLE 11

| Patient | total cds | DEAMINASES | | | | CODON CONTEXT TARGETTING | | | | | | | NUCLEOTIDE RATIOS | | STRAND BIAS | TiTv | SCORE |
|---|---|---|---|---|---|---|---|---|---|---|---|---|---|---|---|---|---|
| | | AID % | ADAR % | A3G % | A3B % | AID_MC1 % | AIDa_GA3 % | A3Ba_CT1 % | A3Ba_CT3 % | all_MC1 % | all_MC2 % | all_MC3 % | all_G % | all_C % | other_A:T | all_GC TiTv | |
| Non-responders | | | | | | | | | | | | | | | | | |
| patient_1 | 1500 | 4.13 | 3.73 | 24.53 | 11.00 | 45.16 | 5.26 | 53.95 | 0.00 | 52.60 | 42.27 | 5.13 | 46.20 | 47.47 | 1.17 | 28.89 | 0 |
| patient_7 | 1092 | 4.67 | 3.48 | 27.66 | 9.34 | 52.94 | 0.00 | 50.98 | 0.00 | 51.37 | 43.77 | 4.85 | 45.88 | 47.34 | 1.57 | 22.14 | 0 |
| patient_10 | 204 | 4.90 | 5.88 | 26.47 | 9.31 | 40.00 | 0.00 | 36.36 | 0.00 | 50.98 | 43.14 | 5.88 | 37.75 L | 49.02 | 0.88 | 6.70 | 1 |
| patient_11 | 241 | 5.81 | 8.71 | 29.05 | 6.64 | 57.14 | 50.00 H | 33.33 | 0.00 | 48.13 | 46.89 | 4.98 | 41.91 | 43.98 | 0.86 | 8.86 | 1 |
| patient_12 | 44 | 9.09 | 11.36 H | 27.27 | 4.55 | 100.00 H | 0.00 | 0.00 L | 0.00 | 45.45 | 45.45 | 9.09 | 27.27 L | 50.00 | 1.50 | 4.67 | 4 |
| patient_14 | 1341 | 3.80 | 4.10 | 25.28 | 10.81 | 45.10 | 5.26 | 52.94 | 1.47 H | 51.60 | 42.51 | 5.89 | 46.16 | 46.68 | 0.78 | 24.41 | 1 |
| patient_16 | 476 | 4.83 | 5.67 | 25.42 | 9.87 | 47.83 | 44.44 H | 77.78 | 0.00 | 54.83 | 39.29 | 5.88 | 47.06 | 44.75 | 0.20 | 20.85 | 1 |
| patient_17 | 774 | 4.26 | 6.20 | 25.45 | 10.34 | 42.42 | 18.18 | 51.61 | 0.00 | 51.55 | 43.02 | 5.43 | 42.12 | 46.12 | 1.15 | 20.34 | 0 |
| patient_20 | 487 | 6.78 | 6.98 | 25.46 | 8.83 | 36.36 | 8.33 | 58.82 | 0.00 | 49.69 | 45.79 | 4.52 | 46.41 | 42.30 | 2.50 H | 15.62 | 1 |
| patient_22 | 64 | 1.56 L | 7.81 | 21.88 | 3.13 | 0.00 L | 0.00 | 0.00 L | 0.00 | 50.00 | 48.44 H | 1.56 L | 43.75 | 34.38 | 0.50 | 24.00 | 5 |
| patient_23 | 216 | 6.02 | 4.63 | 22.69 | 8.33 | 46.15 | 0.00 | 33.33 | 0.00 | 47.69 | 48.61 H | 3.70 | 44.44 | 45.37 | 2.00 | 18.40 | 1 |
| patient_25 | 96 | 13.54 | 10.42 H | 27.08 | 3.13 | 38.46 | 0.00 | 0.00 L | 0.00 | 36.46 L | 56.25 H | 7.29 | 36.46 L | 42.71 | 4.00 H | 1.45 L | 7 |
| patient_29 | 212 | 16.51 H | 9.43 H | 27.36 | 4.72 | 40.00 | 9.09 | 0.00 L | 0.00 | 45.28 | 42.45 | 12.26 H | 45.75 | 35.85 | 2.80 H | 0.80 L | 6 |
| patient_30 | 513 | 3.70 | 7.99 | 27.88 | 6.63 | 57.89 | 0.00 | 30.00 L | 0.00 | 46.39 | 45.81 | 7.80 | 42.50 | 43.47 | 0.72 | 14.21 | 1 |
| patient_31 | 538 | 4.65 | 3.90 | 29.74 | 7.62 | 64.00 H | 0.00 | 33.33 | 0.00 | 54.28 | 42.19 | 3.53 L | 42.19 | 50.93 | 0.60 | 24.05 | 2 |
| patient_32 | 234 | 2.56 L | 7.69 | 30.34 | 10.68 | 50.00 | 0.00 | 53.85 | 0.00 | 56.84 | 37.61 L | 5.56 | 43.59 | 45.30 | 0.60 | 12.87 | 2 |
| patient_36 | 62 | 3.23 | 1.61 L | 20.97 | 4.84 | 50.00 | 0.00 | 50.00 | 0.00 | 58.06 H | 38.71 | 3.23 L | 43.55 | 54.84 H | 0.00 | 19.33 | 4 |

## Example 7

### Genetic indicators of deaminase activity for predicting response to afatinib treatment

Inflammatory breast cancer (IBC) is a rare, aggressive form of breast cancer that has a high risk of metastasis and an estimated median survival of only 2.9 years. In a single arm phase II clinical trial to investigate the efficacy and safety of afatinib, an irreversible ErbB family inhibitor, alone and in combination with vinorelbine, in patients with HER2-positive IBC, 7 of 22 patient samples (32%) were associated with an objective response (32%) and 15 (68%) had no durable clinical response (Goh et al. (2017) PLoS Medicine, 13(12), e1002136). Whole exome sequencing of tissue biopsies from these patients before and after immunotherapy was performed and the sequences analyzed to determine if there were any factors that were associated with response or non-response to therapy. Goh et al. were not able to identify any differences between the patients.

The whole exome sequence of each of the patients in this clinical study was analyzed as described in Example 1 to determine whether genetic indicators of deaminase activity were associated with response or non-response to afatinib therapy, and could thus be used as predictors of response or non-response. In particular, the genetic indicators of deaminase activity that were assessed included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs resulting from a mutation of an adenine nucleotide which were at a MC-1 site (A_MC1%); the percentage of the SNVs resulting from a mutation of an adenine nucleotide which were at a MC-2 site (A_MC2%); the percentage of the SNVs resulting from a mutation of an adenine nucleotide which were at a MC-3 site (A_MC3%); the percentage of SNVs that were at a MC-1 site (all_MC1%); the percentage of SNVs that were at a MC-2 site (all_MC2%); and the percentage of SNVs that were at a MC-3 site (all_MC3%)

- and those indicators assessing strand bias and/or nucleotide targeting, including the percentage of SNVs resulting from mutation of a thymine nucleotide (all_T%); the percentage of SNVs resulting from mutation of an adenine nucleotide (all_A%); the ratio of the percentage of SNVs resulting from mutation of an cytosine nucleotide to the percentage of SNVs resulting from a mutation of a guanine nucleotide (all_C:G); and the transition-transversion ratio of SNVs resulting from mutation of a guanine or cytosine (all_GC_TiTv).

Table 12 shows the results of the analysis of genetic indicators of deaminase activity for the 7 'responders'. The data from these responders was used to calculate the Range Intervals (RIs) for each indicator to which the data from the "non-responders" was then compared. In this example, the RI for each indicator was set by the maximum observed value and the minimum observed value for that indicator. Where the observed value of a genetic indicator of deaminase activity was outside the RI (H - high or L - low compared to the RI), a score of 1 was attributed to that indicator. Where the observed value of a genetic indicator of deaminase activity was within the RI, a score of 0 was attributed to that indicator. The score provided in the right hand column of the Table is the total score for the patient. Thus, where the patient has no genetic indicators outside of the RI, i.e. no "outliers", the total score was 0. This total score is also called the predicted test score. As can be seen, all of the patients responding to afatinib treatment had a score of 0.

In contrast, and as shown in Table 13, all 15 samples associated with the non-responder group had at least one value outside the RI, with the number of outliers added to comprise each patient's total score. This indicates that an assessment of genetic indicators of deaminase activity as described here prior to treatment with afatinib would have predicted that none of these patients would have been suitable for treatment, i.e. predicted at all of the patients would have been non-responders.

A different combination of genetic indicators of deaminase activity were then used to determine whether the same distinction (and thus potential for prediction) could made with this different combination. The genetic indicators of deaminase activity that were assessed in this alternative analysis included:

- those indicators directly assessing deaminase activity, including the percentage of SNVs at the AID motif WRC/GYW (AID%); the percentage of total SNVs that were at the ADAR motif WA/TW (ADAR%); the percentage of total SNVs that were at the APOBEC3G motif CC/GG (A3G%); and the percentage of total SNVs that were at the APOBEC3B motif TCA/TGA (A3B%)

- those indicators assessing codon context, including the percentage of the SNVs at the AID motif WRC/GYW which occurred at a MC-1 site (AID_MC1%), the percentage of the SNVs at the AID motif WRC/GYW which occurred at a MC-2 site (AID_MC2%), the percentage of the SNVs at the AID motif WRC/GYW which occurred at a MC-3 site (AID_MC3%), the percentage of the SNVs resulting from a mutation of a guanine nucleotide which were at a MC-1 site (G_MC1%); the percentage of the SNVs resulting from a mutation of a guanine nucleotide which were at a MC-2 site (G_MC2%); the percentage of the SNVs resulting from a mutation of a guanine nucleotide which were at a MC-3 site (G_MC3%)

- and those indicators assessing strand bias, including the percentage of SNVs resulting from mutation of a thymine nucleotide (all_T%); the percentage of SNVs resulting from mutation of an adenine nucleotide (all_A%); the ratio of the percentage of SNVs resulting from mutation of an adenine or thymine nucleotide to the percentage of SNVs resulting from a mutation of a guanine or cytosine nucleotide (all_AT:GC); and the transition-transversion ratio of SNVs resulting from mutation of a cytosine (all_C_TiTv).

As can be seen from Table 14 (responders) and Table 15 (non-responders), a different set of genetic indicators of deaminase activity can be used to identify/classify patients that respond to afatinib and patients that do not respond to afatinib.

TABLE 12

| Patient | total cds | DEAMINASES | | | | CODON CONTEXT TARGETTING | | | | | | NUCLEOTIDE RATIOS | | STRAND BIAS all_C:G | TiTv all_GC TiTv | SCORE |
|---|---|---|---|---|---|---|---|---|---|---|---|---|---|---|---|---|
| | | AID % | ADAR % | A3G % | A3B % | A_MC1 % | A_MC2 % | A_MC3 % | all_MC1 % | all_MC2 % | all_MC3 % | all_A % | all_T % | | | |
| Responders | | | | | | | | | | | | | | | | |
| patient_5 | 45 | 13.33 | 13.33 | 24.44 | 6.67 | 33.33 | 33.33 | 33.33 | 40.00 | 26.67 | 33.33 | 13.33 | 8.89 | 1.06 | 1.50 | 0 |
| patient_9 | 48 | 18.75 | 6.25 | 16.67 | 0.00 | 28.57 | 57.14 | 14.29 | 43.75 | 29.17 | 27.08 | 14.58 | 4.17 | 0.95 | 3.88 | 0 |
| patient_10 | 38 | 13.16 | 7.89 | 18.42 | 7.89 | 28.57 | 42.86 | 28.57 | 39.47 | 21.05 | 39.47 | 18.42 | 5.26 | 0.93 | 3.14 | 0 |
| patient_14 | 36 | 22.22 | 11.11 | 19.44 | 2.78 | 50.00 | 33.33 | 16.67 | 38.89 | 38.89 | 22.22 | 16.67 | 2.78 | 0.71 | 0.93 | 0 |
| patient_17 | 29 | 13.79 | 3.45 | 13.79 | 3.45 | 66.67 | 33.33 | 0.00 | 44.83 | 17.24 | 37.93 | 10.34 | 13.79 | 0.69 | 1.75 | 0 |
| patient_21 | 218 | 6.88 | 2.29 | 9.17 | 28.90 | 60.00 | 20.00 | 20.00 | 44.50 | 21.56 | 33.95 | 2.29 | 2.75 | 1.01 | 1.88 | 0 |
| patient_24 | 135 | 10.37 | 2.22 | 5.93 | 28.89 | 25.00 | 75.00 | 0.00 | 37.78 | 25.19 | 37.04 | 2.96 | 2.22 | 0.80 | 2.28 | 0 |
| RANGE | 218 | 22.2222 | 13.33 | 24.4444 | 28.8991 | 66.6667 | 75 | 33.3333 | 44.8276 | 38.8889 | 39.4737 | 18.4211 | 13.7931 | 1.05882 | 3.875 | 0 |
| INTERVAL | 29 | 6.88073 | 2.22 | 5.92593 | 0 | 25 | 20 | 0 | 37.7778 | 17.2414 | 22.2222 | 2.29358 | 2.22222 | 0.692308 | 0.933333 | 0 |

TABLE 13

| Patient | total cds | DEAMINASES | | | | CODON CONTEXT TARGETING | | | | | | NUCLEOTIDE RATIOS | | STRAND BIAS | TiTv | SCORE |
|---|---|---|---|---|---|---|---|---|---|---|---|---|---|---|---|---|
| | | AID % | ADAR % | A3G % | A3B % | A_MC1 % | A_MC2 % | A_MC3 % | all_MC1 % | all_MC2 % | all_MC3 % | all_A % | all_T % | all_C:G | all_GC TiTv | |
| Non-responders | | | | | | | | | | | | | | | | |
| patient_1 | 361 | 3.88 L | 4.99 | 7.20 | 35.73 H | 29.41 | 35.29 | 35.29 H | 41.55 | 21.05 | 37.40 | 4.71 | 3.32 | 0.84 | 1.04 | 3 |
| patient_4 | 191 | 10.47 | 4.71 | 7.33 | 25.65 | 20.00 L | 40.00 | 40.00 H | 37.70 L | 18.32 | 43.98 H | 5.24 | 4.71 | 0.85 | 1.29 | 4 |
| patient_6 | 28 | 7.14 | 7.14 | 35.71 H | 0.00 | 50.00 | 50.00 | 0.00 | 32.14 L | 28.57 | 39.29 | 21.43 H | 14.29 H | 0.80 | 2.60 | 4 |
| patient_7 | 425 | 6.59 L | 3.29 | 8.71 | 27.29 | 35.00 | 55.00 | 10.00 | 44.71 | 21.18 | 34.12 | 4.71 | 3.76 | 0.79 | 0.74 L | 2 |
| patient_8 | 84 | 15.48 | 9.52 | 13.10 | 11.90 | 75.00 H | 25.00 | 0.00 | 44.05 | 26.19 | 29.76 | 9.52 | 9.52 | 1.43 H | 1.62 | 2 |
| patient_11 | 159 | 16.98 | 13.84 H | 18.87 | 6.29 | 36.36 | 40.91 | 22.73 | 38.36 | 31.45 | 30.19 | 13.84 | 14.47 H | 1.07 H | 0.58 L | 4 |
| patient_13 | 106 | 7.55 | 11.32 | 11.32 | 21.70 | 23.53 L | 52.94 | 23.53 | 32.08 L | 35.85 | 32.08 | 16.04 | 6.60 | 0.95 | 1.05 | 2 |
| patient_15 | 172 | 11.63 | 15.12 H | 16.86 | 5.81 | 36.00 | 48.00 | 16.00 | 36.05 L | 30.81 | 33.14 | 14.53 | 13.95 H | 0.78 | 0.52 L | 4 |
| patient_16 | 166 | 9.04 | 6.63 | 9.64 | 24.10 | 50.00 | 16.67 L | 33.33 | 36.75 L | 25.90 | 37.35 | 7.23 | 5.42 | 1.01 | 1.27 | 2 |
| patient_20 | 92 | 11.96 | 6.52 | 21.74 | 7.61 | 10.00 L | 70.00 | 20.00 | 30.43 L | 38.04 | 31.52 | 10.87 | 8.70 | 1.39 H | 1.18 | 3 |
| patient_25 | 83 | 16.87 | 18.07 H | 18.07 | 2.41 | 26.67 | 53.33 | 20.00 | 34.94 L | 26.51 | 38.55 | 18.07 | 16.87 H | 1.16 H | 2.60 | 4 |
| patient_26 | 35 | 22.86 H | 8.57 | 8.57 | 8.57 | 50.00 | 25.00 | 25.00 | 40.00 | 31.43 | 28.57 | 11.43 | 8.57 | 1.00 | 2.11 | 1 |
| patient_27 | 81 | 18.52 | 11.11 | 16.05 | 12.35 | 66.67 | 16.67 L | 16.67 | 53.09 H | 23.46 | 23.46 | 7.41 | 7.41 | 1.09 H | 2.63 | 3 |
| patient_28 | 75 | 8.00 | 6.67 | 9.33 | 22.67 | 33.33 | 50.00 | 16.67 | 45.33 H | 22.67 | 32.00 | 8.00 | 4.00 | 0.74 | 1.87 | 1 |
| patient_29 | 99 | 12.12 | 14.14 H | 22.22 | 9.09 | 44.44 | 44.44 | 11.11 | 43.43 | 29.29 | 27.27 | 9.09 | 14.14 H | 0.62 L | 1.00 | 3 |

TABLE 14

| Patient | total cds | DEAMINASES | | | | CODON CONTEXT TARGETTING | | | | | | NUCLEOTIDE RATIOS | | STRAND BIAS all_C:G | TiTv all_C TiTv | SCORE |
|---|---|---|---|---|---|---|---|---|---|---|---|---|---|---|---|---|
| | | AID % | ADAR % | A3G % | A3B % | AID_MC1 % | AID_MC2 % | AID_MC3 % | G_MC1 % | G_MC2 % | G_MC3 % | all_T % | all_AT:CG % | | | |
| Responders | | | | | | | | | | | | | | | | |
| patient_5 | 45 | 13.33 | 13.33 | 24.44 | 6.67 | 33.33 | 50.00 | 16.67 | 58.82 | 29.41 | 11.76 | 8.89 | 0.29 | 1.06 | 1.57 | 0 |
| patient_9 | 48 | 18.75 | 6.25 | 16.67 | 0.00 | 33.33 | 22.22 | 44.44 | 35.00 | 40.00 | 25.00 | 4.17 | 0.23 | 0.95 | 5.33 | 0 |
| patient_10 | 38 | 13.16 | 7.89 | 18.42 | 7.89 | 40.00 | 20.00 | 40.00 | 33.33 | 26.67 | 40.00 | 5.26 | 0.31 | 0.93 | 13.00 | 0 |
| patient_14 | 36 | 22.22 | 11.11 | 19.44 | 2.78 | 25.00 | 37.50 | 37.50 | 41.18 | 29.41 | 29.41 | 2.78 | 0.24 | 0.71 | 1.40 | 0 |
| patient_17 | 29 | 13.79 | 3.45 | 13.79 | 3.45 | 75.00 | 0.00 | 25.00 | 30.77 | 23.08 | 46.15 | 13.79 | 0.32 | 0.69 | 1.25 | 0 |
| patient_21 | 218 | 6.88 | 2.29 | 9.17 | 28.90 | 53.33 | 13.33 | 33.33 | 54.37 | 15.53 | 30.10 | 2.75 | 0.05 | 1.01 | 1.81 | 0 |
| patient_24 | 135 | 10.37 | 2.22 | 5.93 | 28.89 | 42.86 | 50.00 | 7.14 | 45.07 | 18.31 | 36.62 | 2.22 | 0.05 | 0.80 | 2.56 | 0 |
| RANGE | 218 | 22.22 | 13.33 | 24.4 | 28.9 | 75 | 50 | 44.4444 | 58.8235 | 40 | 46.15 | 13.79 | 0.318182 | 1.05882 | 13 | 0 |
| INTERVAL | 29 | 6.88 | 2.22 | 5.93 | 0 | 25 | 0 | 7.14286 | 30.7692 | 15.53 | 11.76 | 2.222 | 0.05314 | 0.692308 | 1.25 | 0 |

TABLE 15

| Patient | total cds | DEAMINASES | | | | CODON CONTEXT TARGETING | | | | | | NUCLEOTIDE RATIOS | | STRAND BIAS | TiTv | SCORE |
|---|---|---|---|---|---|---|---|---|---|---|---|---|---|---|---|---|
| | | AID % | ADAR % | A3G % | A3B % | AID_MC1 % | AID_MC2 % | AID_MC3 % | G_MC1 % | G_MC2 % | G_MC3 % | all_T % | all_AT:CG % | all_C:G | all_C TiTv | |
| **Non-responders** | | | | | | | | | | | | | | | | |
| patient_1 | 361 | 3.88 L | 4.99 | 7.20 | 35.73 H | 35.71 | 14.29 | 50.00 H | 54.44 | 10.00 L | 35.56 | 3.32 | 0.09 | 0.84 | 1.03 L | 5 |
| patient_4 | 191 | 10.47 | 4.71 | 7.33 | 25.65 | 40.00 | 25.00 | 35.00 | 44.09 | 12.90 L | 43.01 | 4.71 | 0.11 | 0.85 | 1.19 L | 2 |
| patient_6 | 28 | 7.14 | 7.14 | 35.71 H | 0.00 | 50.00 | 0.00 | 50.00 H | 30.00 L | 10.00 L | 60.00 H | 14.29 H | 0.56 H | 0.80 | 7.00 | 7 |
| patient_7 | 425 | 6.59 L | 3.29 | 8.71 | 27.29 | 39.29 | 28.57 | 32.14 | 54.84 | 14.75 L | 30.41 | 3.76 | 0.09 | 0.79 | 0.69 L | 3 |
| patient_8 | 84 | 15.48 | 9.52 | 13.10 | 11.90 | 30.77 | 38.46 | 30.77 | 46.43 | 32.14 | 21.43 | 9.52 | 0.24 | 1.43 H | 1.67 | 1 |
| patient_11 | 159 | 16.98 | 13.84 H | 18.87 | 6.29 | 37.04 | 33.33 | 29.63 | 32.73 | 36.36 | 30.91 | 14.47 H | 0.39 H | 1.07 H | 0.84 L | 5 |
| patient_13 | 106 | 7.55 | 11.32 | 11.32 | 21.70 | 50.00 | 12.50 | 37.50 | 42.86 | 26.19 | 30.95 | 6.60 | 0.29 | 0.95 | 1.00 L | 1 |
| patient_15 | 172 | 11.63 | 15.12 H | 16.86 | 5.81 | 35.00 | 25.00 | 40.00 | 46.38 | 23.19 | 30.43 | 13.95 H | 0.40 H | 0.78 | 0.59 L | 4 |
| patient_16 | 166 | 9.04 | 6.63 | 9.64 | 24.10 | 13.33 L | 60.00 H | 26.67 | 48.61 | 22.22 | 29.17 | 5.42 | 0.14 | 1.01 | 1.28 | 2 |
| patient_20 | 92 | 11.96 | 6.52 | 21.74 | 7.61 | 36.36 | 27.27 | 36.36 | 35.48 | 45.16 H | 19.35 | 8.70 | 0.24 | 1.39 H | 1.26 | 2 |
| patient_25 | 83 | 16.87 | 18.07 H | 18.07 | 2.41 | 50.00 | 21.43 | 28.57 | 52.00 | 28.00 | 20.00 | 16.87 H | 0.54 H | 1.16 H | 3.14 | 4 |
| patient_26 | 35 | 22.86 H | 8.57 | 8.57 | 8.57 | 25.00 | 37.50 | 37.50 | 42.86 | 21.43 | 35.71 | 8.57 | 0.25 | 1.00 | 1.80 | 1 |
| patient_27 | 81 | 18.52 | 11.11 | 16.05 | 12.35 | 46.67 | 26.67 | 26.67 | 63.64 H | 18.18 | 18.18 | 7.41 | 0.17 | 1.09 H | 3.50 | 2 |
| patient_28 | 75 | 8.00 | 6.67 | 9.33 | 22.67 | 33.33 | 33.33 | 33.33 | 57.89 | 10.53 L | 31.58 | 4.00 | 0.14 | 0.74 | 1.33 | 1 |
| patient_29 | 99 | 12.12 | 14.14 H | 22.22 | 9.09 | 41.67 | 8.33 | 50.00 H | 57.45 | 23.40 | 19.15 | 14.14 H | 0.30 | 0.62 L | 0.71 L | 5 |

## Example 8

Predicting response to cancer therapy using computational modeling

Computation modelling was performed using patient genomic data obtained from 'Supplementary' sections of publications reporting results of immunotherapy trials. SNVs were obtained from Whole Exome Sequencing data with studies categorised according to the variants reported i.e. all variants ("Whole exome"), only those variants in the coding region of the gene ("CDS-only"), and only variants corresponding to an amino acid change ("Non-Synonymous"). Table 16 provides an overview of the datasets obtained.

Table 16. Overview of datasets obtained and processed using these methods

| ID | Primary cancer therapy | Cancer type | # resp | # non-resp | Data category |
|---|---|---|---|---|---|
| Riaz (Riaz et al. Cell 2017, 171:934-949) | Nivolumab | melanoma | 14 | 57 | Non-synonymous |
| Lauss (Lauss et al. Nature Comm 2017 8: 1738) | Adoptive T-cell therapy | melanoma | 10 | 14 | Whole exome |
| Roh (Roh et al. Sci Trans Med 2017, 9:3560) | CTLA-4 & PD-1 blockade | melanoma | 18 | 35 | Whole exome |
| Snyder (Synder et al. PLoS Med 2017, 14(5): e1002309) | Atezolizumab | urothelial | 9 | 17 | Whole exome |
| Hellmann (Hellmann et al. Cancer Cell 2018, 33:843-852) | PD-1 plus CTLA-4 blockade | NSCLC | 37 | 38 | Whole exome |
| Hugo (Hugo et al. Cell 2016, 165:35-44) | anti-PD-1 | melanoma | 21 | 17 | Whole exome |
| Miao_ccRCC (Miao et al., Science 2018, 359: 801-806) | anti-PD-1 | renal | 7 | 28 | Whole exome |
| Miao_multi_bladder (Miao et al. Nature Genetics 2018, 50:1271-1281) | anti PD-1 (n=74), PD-L1 (n=20), CTLA-4 (n = 145), combo (n=10) | bladder | 13 | 14 | CDS-only |
| Miao_multi_HNsCC (Miao et al. Nature Genetics 2018, 50:1271-1281) | anti PD-1 (n=74), PD-L1 (n=20), CTLA-4 (n = 145), combo (n=10) | HNSCC | 2 | 10 | CDS-only |
| Miao_multi_lung (Miao et al. Nature Genetics 2018, 50:1271-1281) | anti PD-1 (n=74), PD-L1 (n=20), CTLA-4 (n = 145), combo (n=10) | lung | 7 | 22 | CDS-only |

(continued)

| ID | Primary cancer therapy | Cancer type | # resp | # non-resp | Data category |
|---|---|---|---|---|---|
| Miao_multi_melanom a (Miao et al. Nature Genetics 2018, 50:1271-1281) | anti PD-1 (n=74), PD-L1 (n=20), CTLA-4 (n = 145), combo (n=10) | melanoma | 4 | 2 | CDS-only |
| Miao_multi_snyder_M (Miao et al. Nature Genetics 2018, 50:1271-1281) | anti PD-1 (n=74), PD-L1 (n=20), CTLA-4 (n = 145), combo (n=10) | melanoma | 29 | 17 | CDS-only |
| Miao_multi_Van_Allen _Yervoy (Miao et al. Nature Genetics 2018, 50:1271-1281) | anti PD-1 (n=74), PD-L1 (n=20), CTLA-4 (n = 145), combo (n=10) | lung | 10 | 18 | CDS-only |
| Liu (Liu et al. Nat Comm 2017, 8:2193) | Cisplatin chemotherapy | bladder | 10 | 20 | Whole exome |
| Ganly (Ganly et al. Cancer Cell 2018, 34:256-270) | Surgery/ radioactive iodine | Hurthle cell | 39 | 10 | Whole exome |
| Lesurf (Lesurf et al. Ann Oncol 2017, 28:1070-1077) | Trastuzumab | breast | 24 | 24 | Non-synonymous |
| Goh (Goh et al. PLoS Med 2016, 13(12): e1002136) | Afatinib | breast | 7 | 15 | Whole exome |

Patients were categorized according to the type of variants reported from the whole exome sequencing: all variants ("Whole exome"), only variants occurring in the coding region of the gene ("CDS-only"), and only variants corresponding to an amino acid change ("Non-Synonymous"). Patients were further stratified into groups of interest to enable investigation into differences in 'Responder' and 'Non-Responder' patient profiles. Grouped patients were separated into a 'training dataset' a 'test dataset' and a 'validation dataset'. The 'validation dataset' represents the cohort of patients being assessed and is comprised of patients from a single study. The 'training' and 'test' datasets are comprised of several datasets collated together and split approximately 75:25.

SNVs were obtained for each patient in the VCFv4.1 format. The Batch Coordinate Conversion (liftOver; University of California Santa Cruz) program tool was used to transform between different genome references to use GRCh38 as a reference. Each somatic variant in the .vcf file was analyzed and selected for further consideration if it was a simple SNV (e.g. A >T or G >A). Complex variants such as G>(A and T) and insertion or deletions were not analysed.

The datasets collated into the training/test dataset are specified in each example below. Metrics from Table G were used. For each example, a machine learning model was built using a gradient boosting decision tree algorithm. This is a supervised machine learning technique that produces a prediction model based on 'training' data. The models can then be applied to patient data that was not used to train the model to predict response to therapy. In these examples the models are an ensemble of weak prediction models (decision trees) with stochastic gradient descent used for optimisation. The "XGBoost" algorithm was used in these examples (Chen, T., & Guestrin, C. (2016). Xgboost: A scalable tree boosting system. In Proceedings of the 22nd acm sigkdd international conference on knowledge discovery and data mining (pp. 785-794). ACM).

The parameters used to train the XGBoost models were optimised using standard methods employing the 'MLR' software package (Bischl B, Lang M, Kotthoff L, Schiffner J, Richter J, Studerus E, Casalicchio G, Jones Z (2016). "mir: Machine Learning in R." Journal of Machine Learning Research, 17(170), 1-5. http://jmlr.org/papersjv17/15-066.html).

The models were built using optimised parameters for each training dataset described below. Models were evaluated for accuracy, sensitivity and specificity using the test dataset. In each example, the trained model was used to predict response to immunotherapy for each patient in the corresponding validation dataset.

**A.** Predicting patient response in the "Miao multi lung" dataset

Patient genomic data was processed as described to quantify various metrics from Table G. The accuracy of models improved when additional metrics were utilized (when variants were not restricted to the coding sequence of genes), when patients with melanoma were excluded from the training set, and further improved when a specific dataset (Snyder_U) was excluded.

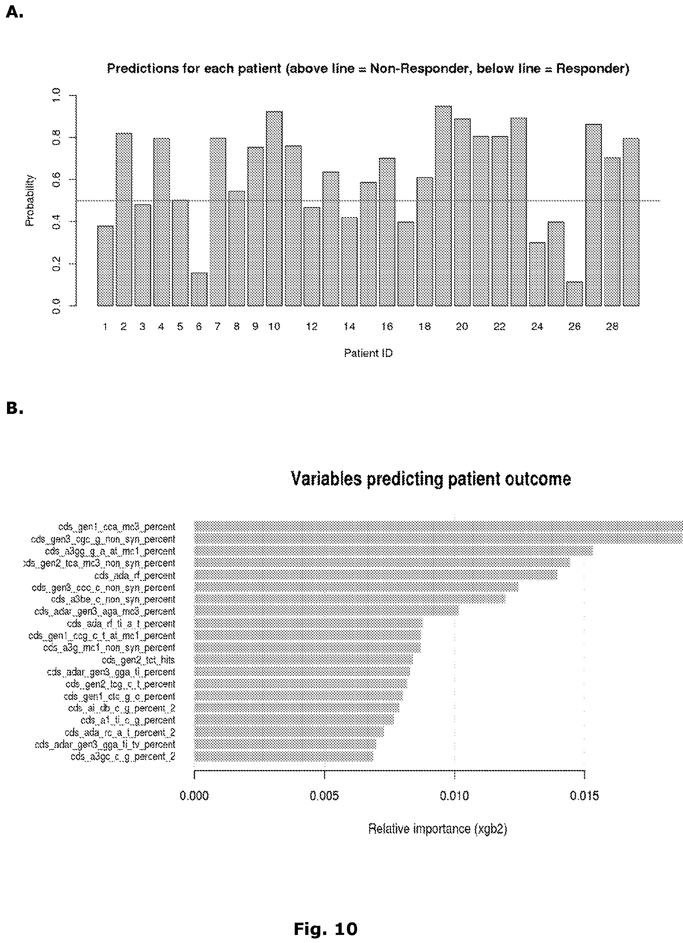

Results for all coding metrics are shown in Table 17, and in Figures 10A and 10B. All coding (cds) metrics from Table G were used to train this model, with 366 metrics being used in the final model. Datasets included in this training set: Hellmann, Hugo, Lauss, Miao_ccRCC, Miao_multi_bladder, Miao_multi_HNSCC, Miao_multi_melanoma, Miao_Rizvi, Miao_Snyder_M, Miao_Van_Allen, Riaz, Roh, Snyder_U.

**Table 17:** Metrics corresponding to the coding region of genes only

|  | Patients (Accuracy) | Non-Responders (Specificity) | Responders (Sensitivity) |
|---|---|---|---|
| Total | 29 | 22 | 7 |
| Correct Prediction | 19 | 16 | 3 |
| Accuracy | 0.6552 | 0.7273 | 0.4286 |

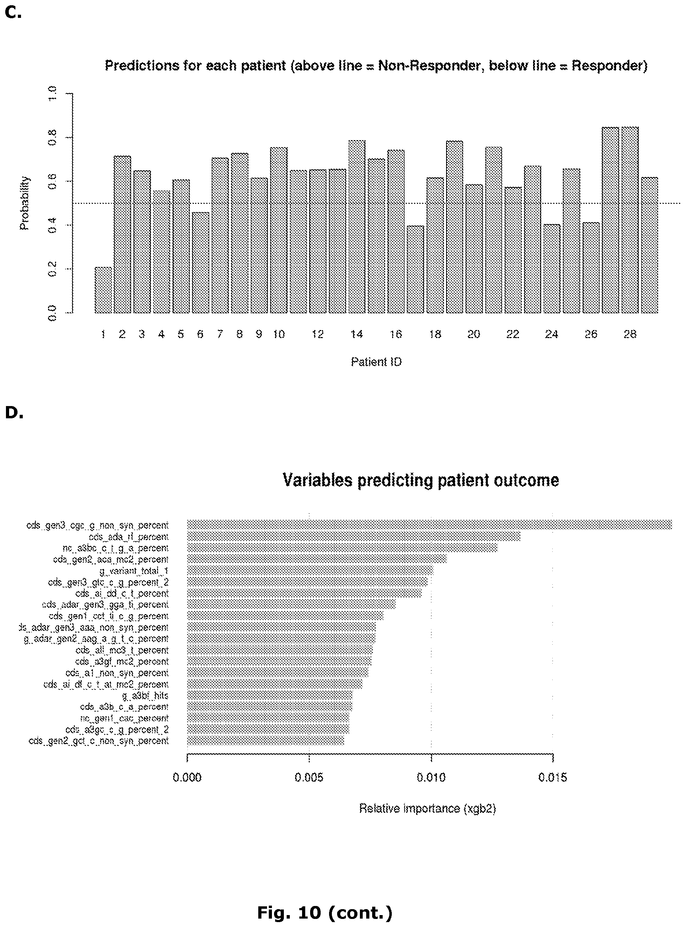

Results for all coding and non-coding metrics are shown in Table 18, and in Figures 10C and 10D. All coding (cds), non-coding (nc) and genomic (g) metrics from Table G were used to train this model, with 411 metrics being used in the final model. Datasets included in this training set: Hellmann, Hugo, Lauss, Miao_ccRCC, Miao_multi_bladder, Miao_multi_HNSCC, Miao_multi_melanoma, Miao_Rizvi, Miao_Snyder_M, Miao_Van_Allen, Riaz, Roh, Snyder_U.

**Table 18:** All metrics used

|  | Patients (Accuracy) | Non-Responders (Specificity) | Responders (Sensitivity) |
|---|---|---|---|
| Total | 29 | 22 | 7 |
| Correct Prediction | 21 | 19 | 2 |
| Accuracy | 0.7241 | 0.86364 | 0.28571 |

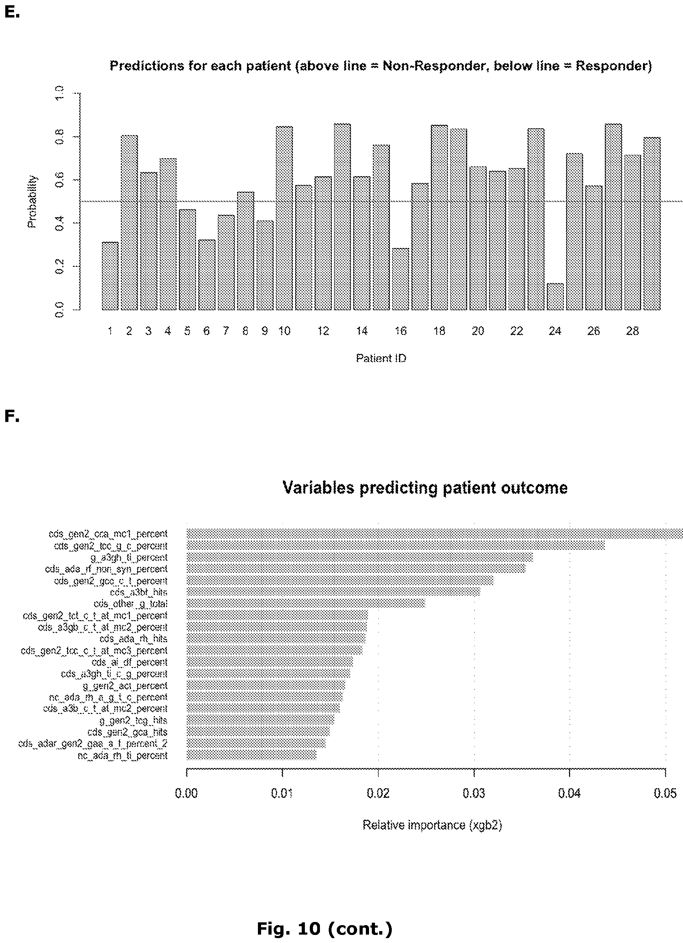

Results for all coding and non-coding metrics, excluding Non-Melanoma datasets from training are shown in Table 19, and in Figures 10E and 10F. All coding (cds), non-coding (nc) and genomic (g) metrics from Table G were used to train this model, with 95 metrics being used in the final model. Datasets included in this training set: Hellmann, Miao_ccRCC, Miao_multi_bladder, Miao_multi_HNSCC, Miao_Rizvi, Snyder_U.

**Table 19:** Non-Melanoma datasets

|  | Patients (Accuracy) | Non-Responders (Specificity) | Responders (Sensitivity) |
|---|---|---|---|
| Total | 29 | 22 | 7 |
| Correct Prediction | 23 | 19 | 4 |
| Accuracy | 0.7931 | 0.8636 | 0.5714 |

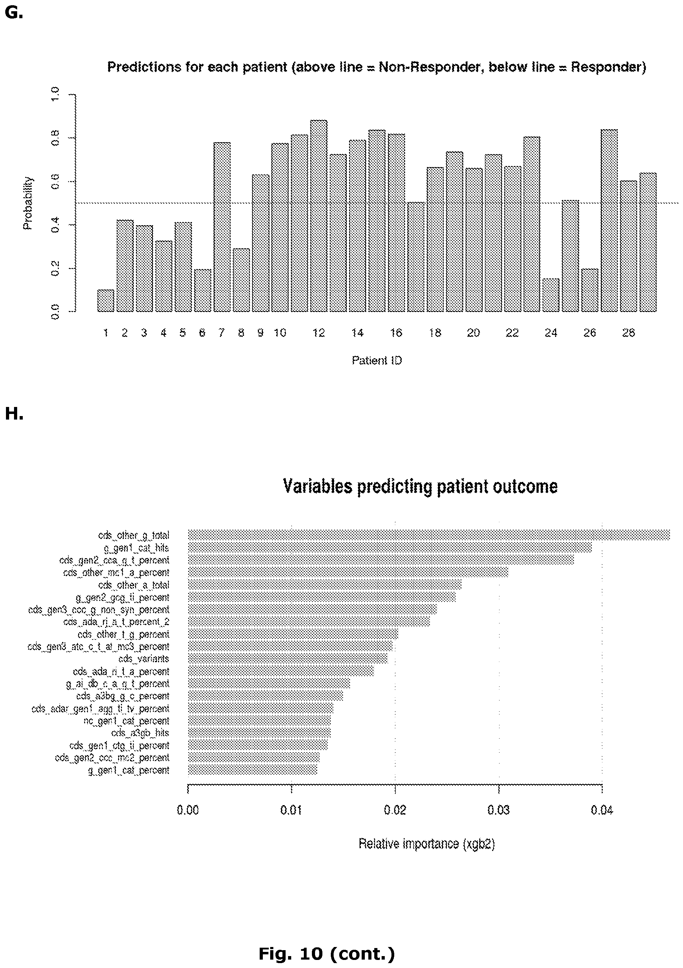

Results for all coding and non-coding metrics, excluding Non-Melanoma datasets and outliers from training are shown in Table 20, and in Figures 10G and 10H. All coding (cds), non-coding (nc) and genomic (g) metrics from Table G were used to train this model, with 132 metrics being used in the final model. Datasets included in the training set: Hellmann, Miao_ccRCC, Miao_multi_bladder, Miao_multi_HNsCC, Miao_Rizvi.

**Table 20:** Non-Melanoma datasets excluding Snyder_U

|  | Patients (Accuracy) | Non-Responders (Specificity) | Responders (Sensitivity) |
|---|---|---|---|
| Total | 29 | 22 | 7 |
| Correct Prediction | 25 | 19 | 6 |
| Accuracy | 0.8621 | 0.8636 | 0.8571 |

**B.** Predicting patient response in the "Miao multi bladder" dataset

A second example dataset was used for predicting patient response in the "Miao_multi_bladder" dataset using metrics from Table G.

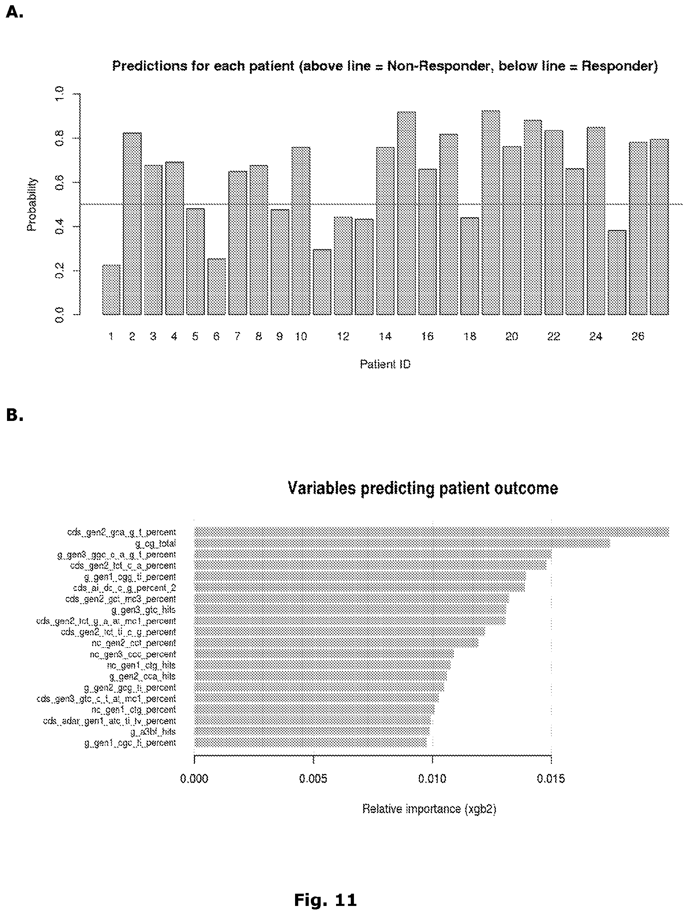

Results excluding outliers from training are shown in Table 21, and in Figures 11A and 11B. All coding (cds), non-coding (nc) and genomic (g) metrics from Table G were used to train this model, with 316 metrics being used in the final model. Datasets included in the training set: Hellmann, Hugo, Lauss, Miao_ccRCC, Miao_multi_HNSCC, Miao_multi_lung, Miao_multi_melanoma, Miao_Rizvi, Miao_Snyder_M, Riaz, Roh, Snyder_U.

**Table 21:** All datasets, excluding Miao_Van_Allen

|  | Patients (Accuracy) | Non-Responders (Specificity) | Responders (Sensitivity) |
|---|---|---|---|
| Total | 27 | 14 | 13 |
| Correct Prediction | 19 | 12 | 7 |
| Accuracy | 0.7037 | 0.8571 | 0.5385 |

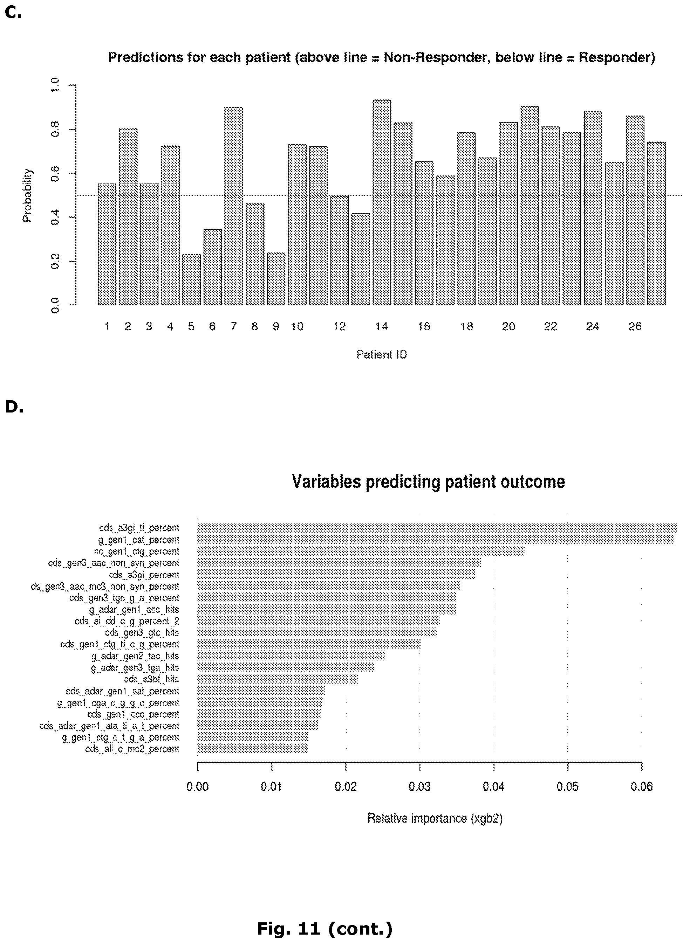

Results for excluding Non-Melanoma datasets and outliers from training are shown in Table 22, and in Figures 11C and 11D. All coding (cds), non-coding (nc) and genomic (g) metrics from Table G were used to train this model, with 65 metrics being used in the final model. Datasets included in the training set: Hellmann, Miao_multi_HNSCC, Miao_multi_lung, Miao_Rizvi, Snyder_U.

**Table 22:** Non-Melanoma datasets excluding Miao_ccRCC

|  | Patients (Accuracy) | Non-Responders (Specificity) | Responders (Sensitivity) |
|---|---|---|---|
| Total | 27 | 14 | 13 |
| Correct Prediction | 20 | 14 | 6 |
| Accuracy | 0.7407 | 1 | 0.4615 |

C. Predicting patient response in the "Miao ccRCC"

A third example dataset was used for predicting patient response in the "Miao_ccRCC" dataset using metrics from Table G.

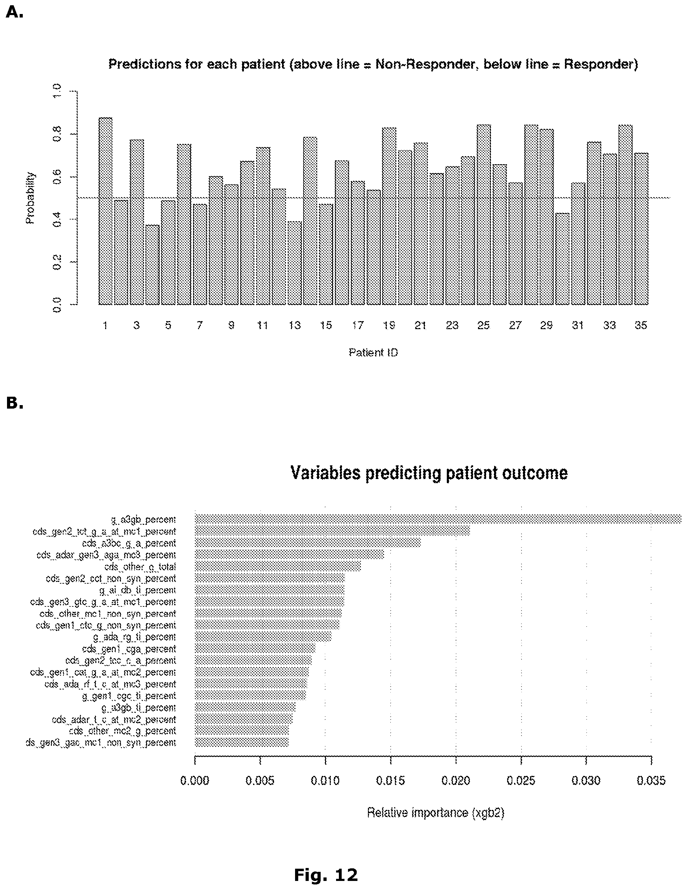

Results excluding outliers from training are shown in Table 23, and in Figures 12A and 12B. All coding (cds), non-coding (nc) and genomic (g) metrics from Table G were used to train this model, with 322 metrics being used in the final model. Datasets included in the training set: Hellmann, Hugo, Lauss, Miao_multi_bladder, Miao_multi_HNSCC, Miao_multi_lung, Miao_multi_melanoma, Miao_Rizvi, Miao_Snyder_M, Miao_Van_Allen, Roh, Snyder_U.

**Table 23:** All datasets excluding Riaz

|  | Patients (Accuracy) | Non-Responders (Specificity) | Responders (Sensitivity) |
|---|---|---|---|
| Total | 35 | 28 | 7 |
| Correct Prediction | 29 | 25 | 4 |
| Accuracy | 0.8286 | 0.8929 | 0.5714 |

D. Predicting patient response in the "Miao multi HNSCC" dataset

A fourth example dataset was used for predicting patient response in the "Miao_multi_HNsCC" dataset using metrics from Table G.

Results are shown in Table 24, and in Figures 12A and 12B. All coding (cds), non-coding (nc) and genomic (g) metrics from Table G were used to train this model, with 200 metrics being used in the final model. Datasets included in the training set: Hellmann, Hugo, Lauss, Miao_ccRCC, Miao_multi_bladder, Miao_multi_lung, Miao_multi_melanoma, Miao_Rizvi, Miao_Snyder_M, Miao_Van_Allen, Riaz, Roh, Snyder_U.

**Table 24:** All datasets

|  | Patients (Accuracy) | Non-Responders (Specificity) | Responders (Sensitivity) |
|---|---|---|---|
| Total | 12 | 10 | 2 |
| Correct Prediction | 10 | 9 | 1 |
| Accuracy | 0.8333 | 0.9 | 0.5 |

Results excluding Non-synonymous datasets are shown in Table 25. All coding (cds), non-coding (nc) and genomic (g) metrics from Table G were used to train this model, with 318 metrics being used in the final model. Datasets included in the training set: Hellmann, Lauss, Miao_ccRCC, Miao_multi_bladder, Miao_multi_lung, Miao_multi_melanoma, Miao_Rizvi, Miao_snyder_M, Miao_Van_Allen, Roh, Snyder_U.

**Table 25:** All datasets excluding Non-synonymous datasets

|  | Patients (Accuracy) | Non-Responders (Specificity) | Responders (Sensitivity) |
|---|---|---|---|
| Total | 12 | 10 | 2 |
| Correct Prediction | 11 | 10 | 1 |
| Accuracy | 0.9167 | 1 | 0.5 |

**E.** Predicting patient response in the "Miao Rizvi" dataset

**A** fifth example dataset is used for predicting patient response in the "Miao_Rizvi" dataset using metrics from Table G.

Results excluding an outlier (Hugo dataset) are shown in Table 26. All coding (cds), non-coding (nc) and genomic (g) metrics from Table G were used to train this model, with 301 metrics being used in the final model. Datasets included in the training set: Hellmann, Lauss, Miao_ccRCC, Miao_multi_bladder, Miao_multi_HNSCC, Miao_multi_lung, Miao_multi_melanoma, Miao_snyder_M, Miao_Van_Allen, Riaz, Roh, Snyder_U.

**Table 26:** All datasets excluding Hugo

|  | Patients (Accuracy) | Non-Responders (Specificity) | Responders (Sensitivity) |
|---|---|---|---|
| Total | 28 | 18 | 10 |
| Correct Prediction | 23 | 17 | 6 |
| Accuracy | 0.8214 | 0.9444 | 0.6 |

**F.** Predicting patient response in the "Lauss" dataset

A sixth example dataset was used for predicting patient response in the "Lauss" dataset using metrics from Table G.

Results for melanoma datasets excluding an outlier (Miao_Van_Allen data set) are shown in Table 27. All coding (cds), non-coding (nc) and genomic (g) metrics from Table G were used to train this model, with 100 metrics being used in the final model. Datasets included in the training set: Hugo, Miao_multi_melanoma, Miao_Snyder_M, Riaz, Roh.

**Table 27:** Melanoma datasets excluding Miao_Van_Allen

|  | Patients (Accuracy) | Non-Responders (Specificity) | Responders (Sensitivity) |
|---|---|---|---|
| Total | 24 | 14 | 10 |
| Correct Prediction | 18 | 11 | 7 |
| Accuracy | 0.75 | 0.7857 | 0.7 |

Results for melanoma datasets excluding another outlier (Roh dataset) are shown in Table 28. All coding (cds), non-coding (nc) and genomic (g) metrics from Table G were used to train this model, with 114 metrics being used in the final model. Datasets included in the training set: Hugo, Miao_multi_melanoma, Miao_snyder_M, Miao_Van_Allen, Riaz.

**Table 28:** Melanoma datasets excluding Roh

|  | Patients (Accuracy) | Non-Responders (Specificity) | Responders (Sensitivity) |
|---|---|---|---|
| Total | 24 | 14 | 10 |
| Correct Prediction | 18 | 12 | 6 |
| Accuracy | 0.75 | 0.8571 | 0.6 |

G. Predicting patient response in the "Miao multi bladder" dataset

A seventh example dataset is used for predicting patient response in the "Miao_multi_bladder" dataset using metrics from Table G.

Results excluding non-synonymous and an outlier (Lauss dataset) are shown in Table 29. All coding (cds), non-coding (nc) and genomic (g) metrics from Table G were used to train this model, with 241 metrics being used in the final model. Datasets included in the training set: Hellmann, Miao_ccRCC, Miao_multi_HNSCC, Miao_multi_lung, Miao_multi_melanoma, Miao_Rizvi, Miao_Snyder_M, Miao_Van_Allen, Roh, Snyder_U.

**Table 29:** Excluding Non-Synonymous datasets and Lauss

|  | Patients (Accuracy) | Non-Responders (Specificity) | Responders (Sensitivity) |
|---|---|---|---|
| Total | 17 | 9 | 26 |
| Correct Prediction | 18 | 10 | 8 |
| Accuracy | 0.6923 | 0.5882 | 0.8889 |

H. Predicting patient response in the "Liu" dataset

An eighth example dataset is used for predicting patient response in the "Liu" dataset (comprised of patients receiving cisplatin therapy) using metrics from Table G.

Results are shown in Table 30. All coding (cds), non-coding (nc) and genomic (g) metrics from Table G were used to train this model, with 290 metrics being used in the final model. Datasets included in the training set: Hellmann, Lauss, Hugo, Miao_ccRCC, Miao_multi_bladder, Miao_multi_lung, Miao_multi_melanoma, Miao_Snyder_M, Miao_Van_Allen, Riaz, Roh, Rizvi, Snyder_U.

Table 30:

|  | Patients (Accuracy) | Non-Responders (Specificity) | Responders (Sensitivity) |
|---|---|---|---|
| Total | 28 | 20 | 10 |
| Correct Prediction | 23 | 16 | 5 |
| Accuracy | 0.700 | 0.800 | 0.500 |

I. Predicting patient response in the "Lesurf" dataset

A ninth example dataset is used for predicting patient response in the "Lesurf" dataset (comprised of patients receiving Trastuzumab therapy) using metrics from Table G.

Results are shown in Table 31. All coding (cds), non-coding (nc) and genomic (g) metrics from Table G were used to train this model, with 213 metrics being used in the final model. Datasets included in the training set: Hellmann, Lauss, Hugo, Miao_multi_bladder, Miao_multi_HNSCC, Miao_multi_melanoma, Miao_Snyder_M, Miao_Van_Allen, Riaz, Rizvi, Roh, Snyder_U.

Table 31:

|  | Patients (Accuracy) | Non-Responders (Specificity) | Responders (Sensitivity) |
|---|---|---|---|
| Total | 48 | 24 | 24 |
| Correct Prediction | 30 | 22 | 8 |
| Accuracy | 0.625 | 0.917 | 0.333 |

J. Predicting patient response in the "Ganly" dataset

A tenth example dataset is used for predicting patient response in the "Ganly" dataset (patients having had surgery and receiving radioactive iodine) using metrics from Table G.

Results are shown in Table 32. All coding (cds), non-coding (nc) and genomic (g) metrics from Table G were used to train this model, with 24 metrics being used in the final model. Miao_multi_melanoma, Miao_Snyder_M, Roh.

Table 32:

|  | Patients (Accuracy) | Non-Responders (Specificity) | Responders (Sensitivity) |
|---|---|---|---|
| Total | 49 | 10 | 39 |
| Correct Prediction | 33 | 2 | 31 |
| Accuracy | 0.674 | 0.200 | 0.795 |

The citation of any reference herein should not be construed as an admission that such reference is available as "Prior Art" to the instant application.

The reference in this specification to any prior publication (or information derived from it), or to any matter which is known, is not, and should not be taken as an acknowledgement or admission or any form of suggestion that the prior publication (or information derived from it) or known matter forms part of the common general knowledge in the field of endeavour to which this specification relates.

Throughout the specification the aim has been to describe the preferred embodiments of the invention without limiting the invention to any one embodiment or specific collection of features. Those of skill in the art will therefore appreciate that, in light of the instant invention, various modifications and changes can be made in the particular embodiments exemplified without departing from the scope of the present invention.

**Claims**

1. A system for generating a therapy indicator for use in assessing responsiveness to cancer therapy for a subject, the system including one or more electronic processing devices that:

   a) obtain subject data indicative of a sequence of a nucleic acid molecule from the subject;
   b) analyze the subject data to identify single nucleotide variations (SNVs) within the nucleic acid molecule;
   c) determine a plurality of metrics using the identified SNVs, the plurality of metrics including metrics from a deaminase motif metric group associated with SNVs in one or more deaminase motifs and a codon context metric group wherein the metrics of this group are associated with a codon context of SNVs, and optionally from one or more of other metric groups including:

      i) a transition/transversion metric group including metrics associated with SNVs that are transitions or transversions;
      ii) a synonymous/non-synonymous metric group including metrics associated with SNVs that are synonymous or non-synonymous;
      iii) a strand bias metric group wherein the metrics of this group are associated with strand bias of SNVs;
      iv) a strand specific metric group that includes metrics associated with SNVs on a specific strand; and
      v) an AT/GC metric group wherein the metrics of this group are associated the number, percentage or ratio of SNVs that target adenine and thymine (AT), and/or guanine and cytidine (GC);

   d) apply the plurality of metrics to at least one computational model to determine a therapy indicator indicative of a predicted responsiveness to cancer therapy, the at least one computational model embodying a relationship between a responsiveness to cancer therapy and the plurality of metrics and being derived by applying machine learning to a plurality of reference metrics obtained from reference subjects having a known responsiveness to cancer therapy.

2. The system of claim 1, wherein the plurality of metrics includes metrics from the deaminase motif metric group, the codon context metric group and the transition/transversion metric group.

3. The system of claim 1 or claim 2, wherein the deaminase motif metric group comprises a group selected from among an activation-induced cytidine deaminase (AID), apolipoprotein B mRNA-editing enzyme, catalytic polypeptide-like (APOBEC) 1 cytosine deaminase (APOBEC1), APOBEC3A, APOBEC3B, APOBEC3C, APOBEC3D, APOBEC3F, APOBEC3G, APOBEC3H and an adenine deaminase acting on RNA (ADAR) motif metric group, wherein each group is associated with SNVs in one or more AID, APOBEC1, APOBEC3A, APOBEC3B, APOBEC3C, APOBEC3D, APOBEC3F, APOBEC3G, APOBEC3H or ADAR motifs, respectively.

4. The system of claim 3, wherein:

the deaminase motif is an AID motif selected from among WRC/GYW, WRCG/CGYW, WRCGS/SCGYW, WRCY/RGYW, WRCGW/WCGYW, WRCR/YGYW and AGCTNT/ANAGCT;
the deaminase motif is an ADAR motif selected from among WA/TW, WAY/RTW, SWAY/RTWS, CWAY/RTWG, CWAA/TTWG, SWA/TWS, WAA/TTW, WAS/STW, RAWA/TWT and SARA/TYTS;
the deaminase motif is an APOBEC3G motif selected from among CC/GG, CG/CG, CCGW/WCGG, SCCGW/WCGGS, SCCGS/SCGGS, SCCG/CGGS, CCGS/SCGG, SCGS/SCGS and SGCG/CGCS;
the deaminase motif is an APOBEC3B motif selected from among TCW/WGA, TCA/TGA, TCWA/TWGA, RTCA/TGAY, YTCA/TGAR, STCG/CGAS, TCGA/TCGA and WTCG/CGAW;
the deaminase motif is the APOBEC3F motif TC/GA; or
the deaminase motif is the APOBEC1 motif CA/TG.

5. The system of any one of claims 1 to 4, wherein at least one of the following applies:

a) the at least one computational model includes a decision tree;
b) the at least one computational model includes a plurality of decision trees, and wherein the therapy indicator is generated by aggregating results from the plurality of decision trees; and
c) when b) applies, at least one metric is used in multiple ones of the plurality of decision trees.

6. The system of any one of claims 1 to 5, wherein the one or more processing devices determine at least one of:

a) at least one metric from each available group; and,
b) at least two metrics from at least some available groups.

7. The system of any one of claims 1 to 6, wherein the one or more processing devices determines at least one of:

a) at least 2 metrics;
b) at least 5 metrics;
c) at least 10 metrics;
d) at least 20 metrics;
e) at least 50 metrics;
f) at least 75 metrics;
g) at least 100 metrics;
h) at least 200 metrics;
i) at least 0.1% of all metrics in the metric groups;
j) at least 0.2% of all metrics in the metric groups;
k) at least 0.3% of all metrics in the metric groups;
l) at least 0.4% of all metrics in the metric groups;
m) at least 0.5% of all metrics in the metric groups;
n) at least 0.75% of all metrics in the metric groups;
o) at least 1% of all metrics in the metric groups;
p) at least 1.5% of all metrics in the metric groups; and,
q) at least 2% of all metrics in the metric groups.

8. The system of claim 1, wherein the one or more electronic processing devices use the plurality of reference metrics and known responsiveness for a number of reference subjects to train at least one computational model, the at least one computational model embodying a relationship between a responsiveness to cancer therapy and the plurality of metrics.

9. The system of claim 8, wherein the plurality of metrics includes metrics from the deaminase motif metric group, the codon context metric group and the transition/transversion metric group.

10. The system of claim 8 or 9, wherein the one or more processing devices test the at least one computational model to determine a discriminatory performance of the model.

11. The system of any one of claims 8 to 10, wherein the one or more processing devices:

a) select a plurality of reference metrics;
b) train at least one computational model using the plurality of reference metrics;

c) test the at least one computational model to determine a discriminatory performance of the model; and,
d) if the discriminatory performance of the model falls below a threshold, at least one of:

i) selectively retrain the at least one computational model using a different plurality of reference metrics; and,
ii) train a different computational model.

12. The system of any one of claims 8 to 11, wherein the one or more processing devices:

a) select a plurality of combinations of reference metrics;
b) train a plurality of computational models using each of the combinations;
c) test each computational model to determine a discriminatory performance of the model; and,
d) selecting the at least one computational model with the highest discriminatory performance for use in determining the therapy indicator.

13. The system of any one of claims 9 to 12, wherein the at least one computational model includes a decision tree.

14. The system of any one of claims 9 to 13, wherein:

(1) the one or more processing devices train the model using at least one of:

a) at least 1000 metrics;
b) at least 2000 metrics;
c) at least 3000 metrics;
d) at least 4000 metrics; and,
e) at least 5000 metrics; and/or

(2) the resulting model uses at least one of:

a) at least 2 metrics;
b) at least 5 metrics;
c) at least 10 metrics;
d) at least 20 metrics;
e) at least 50 metrics;
f) at least 75 metrics;
g) at least 100 metrics;
h) at least 200 metrics;
i) at least 0.1% of all metrics in the metric groups;
j) at least 0.2% of all metrics in the metric groups;
k) at least 0.3% of all metrics in the metric groups;
l) at least 0.4% of all metrics in the metric groups;
m) at least 0.5% of all metrics in the metric groups;
n) at least 0.75% of all metrics in the metric groups;
o) at least 1% of all metrics in the metric groups;
p) at least 1.5% of all metrics in the metric groups; and,
q) at least 2% of all metrics in the metric groups.

15. A method for determining the likelihood that a subject with cancer will respond to a cancer therapy or will continue to respond to a cancer therapy, the method comprising:

analyzing the sequence of a nucleic acid molecule from a subject with cancer to detect SNVs within the nucleic acid molecule;
determining a plurality of metrics based on the number and/or type of SNVs detected so as to obtain a subject profile of metrics, wherein the plurality of metrics includes metrics from a deaminase motif metric group associated with SNVs in one or more deaminase motifs and a codon context metric group wherein the metrics of this group are associated with a codon context of SNVs, and optionally from one or more of the following metric groups:

i) a transition/transversion metric group including metrics associated with SNVs that are transitions or transversions;

ii) a synonymous/non-synonymous metric group including metrics associated with SNVs that are synonymous or non-synonymous;

iii) a strand bias metric group associated with strand bias of SNVs;

iv) a strand specific metric group that includes metrics associated with SNVs on a specific strand; and

v) an AT/GC metric group associated with the number, percentage or ratio of SNVs that target adenine and thymine (AT), and/or guanine and cytidine (GC); and,

determining the likelihood of a subject responding to cancer therapy based on a comparison between the subject profile and a reference profile of metrics.

**Patentansprüche**

1. System zum Erzeugen eines Therapieindikators zur Verwendung beim Beurteilen des Ansprechverhaltens eines Subjekts auf Krebstherapie, wobei das System eine oder mehrere elektronische Verarbeitungsvorrichtungen beinhaltet, die:

a) von dem Subjekt Subjektdaten erhalten, die auf eine Sequenz eines Nukleinsäuremoleküls hinweisen;

b) die Subjektdaten analysieren, um Einzelnukleotid-Variationen (SNVs) innerhalb des Nukleinsäuremoleküls zu identifizieren;

c) unter Verwendung der identifizierten SNVs eine Vielzahl von Metriken bestimmen, wobei die Vielzahl von Metriken Metriken aus einer Deaminasemotiv-Metrikgruppe, die mit SNVs in einem oder mehreren Deaminasemotiven verknüpft sind, und einer Codonkontext-Metrikgruppe, wobei die Metriken dieser Gruppe mit einem Codonkontext von SNVs verknüpft sind, und optional aus einer oder mehreren anderen Metrikgruppen beinhaltet, welche beinhalten:

i) eine Transition-/Transversion-Metrikgruppe, die Metriken beinhaltet, die mit SNVs verknüpft sind, die Transitionen oder Transversionen sind;

ii) eine Synonym-/Nichtsynonym-Metrikgruppe, die Metriken beinhaltet, die mit SNVs verknüpft sind, die synonym oder nichtsynonym sind;

iii) eine Strang-Bias-Metrikgruppe, wobei die Metriken dieser Gruppe mit Strang-Bias von SNVs verknüpft sind;

iv) eine strangspezifische Metrikgruppe, die Metriken beinhaltet, die mit SNVs auf einem spezifischen Strang verknüpft sind; und

v) eine AT/GC-Metrikgruppe, wobei die Metriken dieser Gruppe mit der Anzahl, dem Prozentsatz oder dem Verhältnis von SNVs, die auf Adenin und Thymin (AT), und/oder Guanin und Cytidin (GC) abzielen, verknüpft sind;

d) die Vielzahl von Metriken auf mindestens ein Rechenmodell anwenden, um einen Therapieindikator zu bestimmen, der auf ein vorhergesagtes Ansprechverhalten auf Krebstherapie hinweist, wobei das mindestens eine Rechenmodell eine Beziehung zwischen einem Ansprechverhalten auf Krebstherapie und der Vielzahl von Metriken verkörpert und durch Anwenden von maschinellem Lernen auf eine Vielzahl von Referenzmetriken, die von Referenzsubjekten erhalten wurden, welche ein bekanntes Ansprechverhalten auf Krebstherapie aufweisen, abgeleitet wird.

2. System nach Anspruch 1, wobei die Vielzahl von Metriken Metriken aus der Deaminasemotiv-Metrikgruppe, der Codonkontext-Metrikgruppe und der Transition-/Transversion-Metrikgruppe beinhaltet.

3. System nach Anspruch 1 oder Anspruch 2, wobei die Deaminasemotiv-Metrikgruppe eine Gruppe umfasst, ausgewählt aus einer aktivierungsinduzierten Cytidindeaminase (AID), einem Apolipoprotein B-mRNA-Editierungsenzym, einer katalytischen polypeptidähnlichen (APOBEC) 1-Cytosindeaminase (APOBEC1), APOBEC3A, APOBEC3B, APOBEC3C, APOBEC3D, APOBEC3F, APOBEC3G, APOBEC3H und einer auf RNA wirkenden Adenindeaminase-(ADAR-) Motiv-Metrikgruppe, wobei jede Gruppe jeweils mit SNVs in einem oder mehreren AID-, APOBEC1-, APOBEC3A-, APOBEC3B-, APOBEC3C-, APOBEC3D-, APOBEC3F-, APOBEC3G-, APOBEC3H- oder ADAR-Motiven verknüpft ist.

4. System nach Anspruch 3, wobei:

das Deaminasemotiv ein AID-Motiv ist, ausgewählt aus WRC/GYW, WRCG/CGYW, WRCGS/SCGYW, WRCY/RGYW, WRCGW/WCGYW, WRCR/YGYW und AGCTNT/ANAGCT;

das Deaminasemotiv ein ADAR-Motiv ist, ausgewählt aus WA/TW, WAY/RTW, SWAY/RTWS, CWAY/RTWG, CWAA/TTWG, SWA/TWS, WAA/TTW, WAS/STW, RAWA/TWT und SARA/TYTS;

das Deaminasemotiv ein APOBEC3G-Motiv ist, ausgewählt aus CC/GG, CG/CG, CCGW/WCGG, SCCGW/WCGGS, SCCGS/SCGGS, SCCG/CGGS, CCGS/SCGG, SCGS/SCGS und SGCG/CGCS;

das Deaminasemotiv ein APOBEC3B-Motiv ist, ausgewählt aus TCW/WGA, TCA/TGA, TCWA/TWGA, RTCA/TGAY, YTCA/TGAR, STCG/CGAS, TCGA/TCGA und WTCG/CGAW;

das Deaminasemotiv das APOBEC3F-Motiv TC/GA ist; oder

das Deaminasemotiv das APOBEC1-Motiv CA/TG ist.

5. System nach einem der Ansprüche 1 bis 4, wobei mindestens eines von Folgendem zutrifft:

a) das mindestens eine Rechenmodell beinhaltet einen Entscheidungsbaum;

b) das mindestens eine Rechenmodell beinhaltet eine Vielzahl von Entscheidungsbäumen, und wobei der Therapieindikator durch Aggregieren von Ergebnissen aus der Vielzahl von Entscheidungsbäumen erzeugt wird; und

c) wenn b) zutrifft, wird mindestens eine Metrik in mehreren der Vielzahl von Entscheidungsbäumen verwendet.

6. System nach einem der Ansprüche 1 bis 5, wobei die eine oder mehrere Verarbeitungsvorrichtungen mindestens eines bestimmen von:

a) mindestens einer Metrik aus jeder verfügbaren Gruppe; und

b) mindestens zwei Metriken aus mindestens einigen verfügbaren Gruppen.

7. System nach einem der Ansprüche 1 bis 6, wobei die eine oder mehrere Verarbeitungsvorrichtungen mindestens eines bestimmen von:

a) mindestens 2 Metriken;

b) mindestens 5 Metriken;

c) mindestens 10 Metriken;

d) mindestens 20 Metriken;

e) mindestens 50 Metriken;

f) mindestens 75 Metriken;

g) mindestens 100 Metriken;

h) mindestens 200 Metriken;

i) mindestens 0,1% aller Metriken in den Metrikgruppen;

j) mindestens 0,2% aller Metriken in den Metrikgruppen;

k) mindestens 0,3% aller Metriken in den Metrikgruppen;

l) mindestens 0,4% aller Metriken in den Metrikgruppen;

m) mindestens 0,5% aller Metriken in den Metrikgruppen;

n) mindestens 0,75% aller Metriken in den Metrikgruppen;

o) mindestens 1% aller Metriken in den Metrikgruppen;

p) mindestens 1,5% aller Metriken in den Metrikgruppen; und

q) mindestens 2% aller Metriken in den Metrikgruppen.

8. System nach Anspruch 1, wobei die eine oder mehrere elektronische Verarbeitungsvorrichtungen die Vielzahl von Referenzmetriken und bekannten Ansprechverhalten für eine Anzahl von Referenzsubjekten verwenden, um mindestens ein Rechenmodell zu trainieren, wobei das mindestens eine Rechenmodell eine Beziehung zwischen einem Ansprechverhalten auf Krebstherapie und der Vielzahl von Metriken verkörpert.

9. System nach Anspruch 8, wobei die Vielzahl von Metriken Metriken aus der Deaminasemotiv-Metrikgruppe, der Codonkontext-Metrikgruppe und der Transition-/Transversion-Metrikgruppe beinhaltet.

10. System nach Anspruch 8 oder 9, wobei die eine oder mehrere Verarbeitungsvorrichtungen das mindestens eine Rechenmodell testen, um eine Unterscheidungsleistung des Modells zu bestimmen.

11. System nach einem der Ansprüche 8 bis 10, wobei die eine oder mehrere Verarbeitungsvorrichtungen:

a) eine Vielzahl von Referenzmetriken auswählen;

b) mindestens ein Rechenmodell unter Verwendung der Vielzahl von Referenzmetriken trainieren;

c) das mindestens eine Rechenmodell testen, um eine Unterscheidungsleistung des Modells zu bestimmen; und

d) wenn die Unterscheidungsleistung des Modells eine Schwelle unterschreitet, mindestens eines von:

i) das mindestens eine Rechenmodell unter Verwendung einer anderen Vielzahl von Referenzmetriken selektiv neu trainieren; und

ii) ein anderes Rechenmodell trainieren.

**12.** System nach einem der Ansprüche 8 bis 11, wobei die eine oder mehrere Verarbeitungsvorrichtungen:

a) eine Vielzahl von Kombinationen von Referenzmetriken auswählen;

b) eine Vielzahl von Rechenmodellen unter Verwendung jeder der Kombinationen trainieren;

c) jedes Rechenmodell testen, um eine Unterscheidungsleistung des Modells zu bestimmen; und

d) das mindestens eine Rechenmodell mit der höchsten Unterscheidungsleistung zur Verwendung beim Bestimmen des Therapieindikators auswählen.

**13.** System nach einem der Ansprüche 9 bis 12, wobei das mindestens eine Rechenmodell einen Entscheidungsbaum beinhaltet.

**14.** System nach einem der Ansprüche 9 bis 13, wobei:

(1) die eine oder mehrere Verarbeitungsvorrichtungen trainieren das Modell unter Verwendung von mindestens einem von:

a) mindestens 1000 Metriken;

b) mindestens 2000 Metriken;

c) mindestens 3000 Metriken;

d) mindestens 4000 Metriken; und

e) mindestens 5000 Metriken; und/oder

(2) das resultierende Modell verwendet mindestens eines von:

a) mindestens 2 Metriken;

b) mindestens 5 Metriken;

c) mindestens 10 Metriken;

d) mindestens 20 Metriken;

e) mindestens 50 Metriken;

f) mindestens 75 Metriken;

g) mindestens 100 Metriken;

h) mindestens 200 Metriken;

i) mindestens 0,1% aller Metriken in den Metrikgruppen;

j) mindestens 0,2% aller Metriken in den Metrikgruppen;

k) mindestens 0,3% aller Metriken in den Metrikgruppen;

l) mindestens 0,4% aller Metriken in den Metrikgruppen;

m) mindestens 0,5% aller Metriken in den Metrikgruppen;

n) mindestens 0,75% aller Metriken in den Metrikgruppen;

o) mindestens 1% aller Metriken in den Metrikgruppen;

p) mindestens 1,5% aller Metriken in den Metrikgruppen; und

q) mindestens 2% aller Metriken in den Metrikgruppen.

**15.** Verfahren zum Bestimmen der Wahrscheinlichkeit, dass ein Subjekt mit Krebs auf eine Krebstherapie ansprechen wird oder weiterhin auf eine Krebstherapie ansprechen wird, wobei das Verfahren umfasst:

Analysieren der Sequenz eines Nukleinsäuremoleküls von einem Subjekt mit Krebs, um SNVs innerhalb des Nukleinsäuremoleküls zu erkennen;

Bestimmen einer Vielzahl von Metriken auf Basis der Anzahl und/oder Art von erkannten SNVs, um ein Subjektprofil von Metriken zu erhalten, wobei die Vielzahl von Metriken Metriken aus einer Deaminasemotiv-Metrikgruppe, die mit SNVs in einem oder mehreren Deaminasemotiven verknüpft sind, und einer Codonkontext-Metrikgruppe, wobei die Metriken dieser Gruppe mit einem Codonkontext von SNVs verknüpft sind, und optional aus einer oder mehreren der folgenden Metrikgruppen:

> i) eine Transition-/Transversion-Metrikgruppe, die Metriken beinhaltet, die mit SNVs verknüpft sind, die Transitionen oder Transversionen sind;
> ii) eine Synonym-/Nichtsynonym-Metrikgruppe, die Metriken beinhaltet, die mit SNVs verknüpft sind, die synonym oder nichtsynonym sind;
> iii) eine Strang-Bias-Metrikgruppe, die mit Strang-Bias von SNVs verknüpft ist;
> iv) eine strangspezifische Metrikgruppe, die Metriken beinhaltet, die mit SNVs auf einem spezifischen Strang verknüpft sind; und
> v) eine AT/GC-Metrikgruppe, die mit der Anzahl, dem Prozentsatz oder dem Verhältnis von SNVs, die auf Adenin und Thymin (AT), und/oder Guanin und Cytidin (GC) abzielen, verknüpft ist; und,

Bestimmen der Wahrscheinlichkeit, dass ein Subjekt auf Krebstherapie anspricht, basierend auf einem Vergleich zwischen dem Subjektprofil und einem Referenzprofil von Metriken.

## Revendications

1. Système de génération d'un indicateur de thérapie destiné à être utilisé pour évaluer la réactivité d'un sujet à une thérapie contre le cancer, le système incluant un ou plusieurs dispositifs de traitement électroniques qui :

   a) obtiennent des données de sujet indiquant une séquence d'une molécule d'acide nucléique du sujet ;
   b) analysent les données de sujet pour identifier les variations d'un seul nucléotide (SNV) au sein de la molécule d'acide nucléique ;
   c) déterminent une pluralité de mesures en utilisant les SNV identifiés, la pluralité de mesures incluant des mesures d'un groupe de mesures de motifs désaminase associé à des SNV dans un ou plusieurs motifs désaminase et un groupe de mesures de contexte de codons dans lequel les mesures de ce groupe sont associées à un contexte de codons de SNV, et éventuellement d'un ou plusieurs autres groupes de mesures incluant :

   > i) un groupe de mesures de transition/transversion incluant des mesures associées aux SNV qui sont des transitions ou des transversions ;
   > ii) un groupe de mesures de synonyme/non-synonyme incluant des mesures associées à des SNV qui sont des synonymes ou des non-synonymes ;
   > iii) un groupe de mesures de biais de brin dans lequel les mesures de ce groupe sont associées au biais de brin des SNV ;
   > iv) un groupe de mesures spécifique à un brin qui inclut des mesures associées aux SNV sur un brin spécifique ; et
   > v) un groupe de mesures AT/GC dans lequel les mesures de ce groupe sont associées au nombre, au pourcentage ou à la proportion de SNV qui ciblent l'adénine et la thymine (AT), et/ou la guanine et la cytidine (GC) ;

   d) appliquent la pluralité de mesures à au moins un modèle informatique pour déterminer un indicateur de thérapie indiquant une réactivité prédite à une thérapie contre le cancer, le au moins un modèle informatique incarnant une relation entre une réactivité à une thérapie contre le cancer et la pluralité de mesures et étant obtenu en appliquant l'apprentissage automatique à une pluralité de mesures de référence obtenues à partir de sujets de référence présentant une réactivité connue à une thérapie contre le cancer.

2. Système selon la revendication 1, dans lequel la pluralité de mesures incluent des mesures du groupe de mesures de motif désaminase, du groupe de mesures de contexte de codon et du groupe de mesures de transition/transversion.

3. Système selon la revendication 1 ou la revendication 2, dans lequel le groupe de mesures de motif désaminase comprend un groupe choisi parmi une cytidine désaminase induite par activation (AID), une enzyme d'édition d'ARNm d'apolipoprotéine B, une cytosine désaminase de type polypeptide catalytique (APOBEC) 1 (APOBEC1), APOBEC3A, APOBEC3B, APOBEC3C, APOBEC3D, APOBEC3F, APOBEC3G, APOBEC3H et un groupe de mesures de motif d'adénine désaminase agissant sur l'ARN (ADAR), dans lequel chaque groupe est associé à des SNV dans un ou plusieurs motifs AID, APOBEC1, APOBEC3A, APOBEC3B, APOBEC3C, APOBEC3D, APOBEC3F, APOBEC3G, APOBEC3H ou ADAR, respectivement.

**4.** Système selon la revendication 3, dans lequel :

le motif désaminase est un motif AID choisi parmi WRC/GYW, WRCG/CGYW, WRCGS/SCGYW, WRCY/RGYW, WRCGW/WCGYW, WRCR/YGYW et AGCTNT/ANAGCT ;
le motif désaminase est un motif ADAR choisi parmi WA/TW, WAY/RTW, SWAY/RTWS, CWAY/RTWG, CWAA/TTWG, SWA/TWS, WAA/TTW, WAS/STW, RAWA/TWT et SARA/TYTS ;
le motif désaminase est un motif APOBEC3G choisi parmi CC/GG, CG/CG, CCGW/WCGG, SCCGW/WCGGS, SCCGS/SCGGS, SCCG/CGGS, CCGS/SCGG, SCGS/SCGS et SGCG/CGCS ;
le motif désaminase est un motif APOBEC3B sélectionné parmi TCW/WGA, TCA/TGA, TCWA/TWGA, RTCA/TGAY, YTCA/TGAR, STCG/CGAS, TCGA/TCGA et WTCG/CGAW ;
le motif désaminase est le motif APOBEC3F TC/GA ; ou
le motif désaminase est le motif APOBEC 1 CA/TG.

**5.** Système selon l'une quelconque des revendications 1 à 4, dans lequel au moins l'un des points suivants s'applique :

a) le au moins un modèle informatique inclut un arbre de décision ;
b) le au moins un modèle informatique inclut une pluralité d'arbres de décision, et dans lequel l'indicateur de thérapie est généré en agrégeant les résultats de la pluralité d'arbres de décision ; et
c) lorsque b) s'applique, au moins une mesure est utilisée dans plusieurs arbres de décision de la pluralité d'arbres de décision.

**6.** Système selon l'une quelconque des revendications 1 à 5, dans lequel les un ou plusieurs dispositifs de traitement déterminent au moins l'un des éléments suivants :

a) au moins une mesure de chaque groupe disponible ; et,
b) au moins deux mesures provenant d'au moins certains groupes disponibles.

**7.** Système selon l'une quelconque des revendications 1 à 6, dans lequel les un ou plusieurs dispositifs de traitement déterminent au moins l'un des éléments suivants :

a) au moins 2 mesures ;
b) au moins 5 mesures ;
c) au moins 10 mesures ;
d) au moins 20 mesures ;
e) au moins 50 mesures ;
f) au moins 75 mesures ;
g) au moins 100 mesures ;
h) au moins 200 mesures ;
i) au moins 0,1 % de toutes les mesures dans les groupes de mesure ;
j) au moins 0,2 % de toutes les mesures dans les groupes de mesure ;
k) au moins 0,3 % de toutes les mesures dans les groupes de mesure ;
l) au moins 0,4 % de toutes les mesures dans les groupes de mesure ;
m) au moins 0,5 % de toutes les mesures dans les groupes de mesure ;
n) au moins 0,75 % de toutes les mesures dans les groupes de mesure ;
o) au moins 1 % de toutes les mesures dans les groupes de mesure ;
p) au moins 1,5 % de toutes les mesures dans les groupes de mesure ; et,
q) au moins 2 % de toutes les mesures dans les groupes de mesure.

**8.** Système selon la revendication 1, dans lequel les un ou plusieurs dispositifs de traitement électronique utilisent la pluralité de mesures de référence et une réactivité connue pour un certain nombre de sujets de référence pour former au moins un modèle informatique, le au moins un modèle informatique incarnant une relation entre une réactivité à une thérapie contre le cancer et la pluralité de mesures.

**9.** Système selon la revendication 8, dans lequel la pluralité de mesures incluent des mesures du groupe de mesures de motif désaminase, du groupe de mesures de contexte de codon et du groupe de mesures de transition/transversion.

**10.** Système selon la revendication 8 ou 9, dans lequel les un ou plusieurs dispositifs de traitement testent le au moins un modèle informatique pour déterminer une performance discriminatoire du modèle.

**11.** Système selon l'une quelconque des revendications 8 à 10, dans lequel les un ou plusieurs des dispositifs de traitement :

a) sélectionnent une pluralité de mesures de référence ;
b) forment au moins un modèle informatique en utilisant la pluralité de mesures de référence ;
c) testent le au moins un modèle informatique pour déterminer une performance discriminante du modèle ; et,
d) si la performance discriminatoire du modèle tombe au-dessous d'un seuil, au moins l'un de :

i) reforment de manière sélective le au moins un modèle informatique en utilisant une pluralité différente de mesures de référence ; et,
ii) forment un modèle informatique différent.

**12.** Système selon l'une quelconque des revendications 8 à 11, dans lequel les un ou plusieurs des dispositifs de traitement :

a) sélectionnent une pluralité de combinaisons de mesures de référence ;
b) forment une pluralité de modèles informatiques en utilisant chacune des combinaisons ;
c) testent chaque modèle informatique pour déterminer une performance discriminante du modèle ; et,
d) sélectionnent le au moins un modèle informatique présentant la performance discriminatoire la plus élevée pour l'utiliser dans la détermination de l'indicateur de thérapie.

**13.** Système selon l'une quelconque des revendications 9 à 12, dans lequel le au moins un modèle informatiques inclut un arbre de décision.

**14.** Système selon l'une quelconque des revendications 9 à 13, dans lequel :

(1) les un ou plusieurs dispositifs de traitement forment le modèle en utilisant au moins l'un des éléments suivants :

a) au moins 1000 mesures ;
b) au moins 2 000 mesures ;
c) au moins 3 000 mesures ;
d) au moins 4 000 mesures ; et,
e) au moins 5 000 mesures ; et/ou

(2) le modèle résultant utilise au moins l'un des éléments suivants :

a) au moins 2 mesures ;
b) au moins 5 mesures ;
c) au moins 10 mesures ;
d) au moins 20 mesures ;
e) au moins 50 mesures ;
f) au moins 75 mesures ;
g) au moins 100 mesures ;
h) au moins 200 mesures ;
i) au moins 0,1 % de toutes les mesures dans les groupes de mesure ;
j) au moins 0,2 % de toutes les mesures dans les groupes de mesure ;
k) au moins 0,3 % de toutes les mesures dans les groupes de mesure ;
l) au moins 0,4 % de toutes les mesures dans les groupes de mesure ;
m) au moins 0,5 % de toutes les mesures dans les groupes de mesure ;
n) au moins 0,75 % de toutes les mesures dans les groupes de mesure ;
o) au moins 1 % de toutes les mesures dans les groupes de mesure ;
p) au moins 1,5 % de toutes les mesures dans les groupes de mesure ; et,
q) au moins 2 % de toutes les mesures dans les groupes de mesure.

15. Procédé de détermination de la probabilité qu'un sujet atteint d'un cancer réponde à une thérapie contre le cancer ou continue de répondre à une thérapie contre le cancer, le procédé comprenant :

l'analyse de la séquence d'une molécule d'acide nucléique provenant d'un sujet atteint de cancer pour détecter des SNV dans la molécule d'acide nucléique ;

la détermination d'une pluralité de mesures en fonction du nombre et/ou du type de SNV détectés de manière à obtenir un profil de mesures de sujet, dans lequel la pluralité de mesures incluent des mesures provenant d'un groupe de mesures de motifs désaminase associé à des SNV dans un ou plusieurs motifs désaminase et un groupe de mesures de contexte de codons dans lequel les mesures de ce groupe sont associées à un contexte de codons de SNV, et éventuellement provenant d'un ou plusieurs des groupes de mesures suivants :

i) un groupe de mesures de transition/transversion incluant les mesures associées aux SNV qui sont des transitions ou des transversions ;

ii) un groupe de mesures de synonyme/non-synonyme incluant des mesures associées à des SNV qui sont des synonymes ou des non-synonymes ;

iii) un groupe de mesures de biais de brin associé au biais de brin des SNV ;

iv) un groupe de mesures spécifique à un brin qui inclut des mesures associées aux SNV sur un brin spécifique ; et

v) un groupe de mesures AT/GC associé au nombre, au pourcentage ou à la proportion de SNV qui ciblent l'adénine et la thymine (AT), et/ou la guanine et la cytidine (GC) ; et,

la détermination de la probabilité qu'un sujet réponde à une thérapie contre le cancer en se basant sur une comparaison entre le profil de sujet et un profil de référence de mesures.

| | |
|---|---|
| Obtain subject data | 100 |
| Identify SNVs | 110 |
| Determine plurality of metrics | 120 |
| Apply metric to computational model | 130 |
| Determine indicator | 140 |

**Fig. 1**

Fig. 2

310

320

340

320

330

330

320

**Fig. 3**

310

**Fig. 4**

411

330

**Fig. 5**

Acquire subject data — 600

Determine subject attributes — 605

Identify condition — 610

Select model(s) — 615

Calculate metrics — 620

Apply metrics to models — 625

Determine indicator — 630

Store and optionally display indicator — 635

**Fig. 6**

**A.**

**B.**

**Fig. 7**

**A.**

**Fig. 8**

**B.**

EP 3 710 600 B1

**Fig. 8 (cont.)**

**A.**

**Fig. 9**

**B.**

**Fig. 9 (cont.)**

**A.**

Predictions for each patient (above line = Non-Responder, below line = Responder)

**B.**

Variables predicting patient outcome

**Fig. 10**

**C.**

Predictions for each patient (above line = Non-Responder, below line = Responder)

**D.**

Variables predicting patient outcome

**Fig. 10 (cont.)**

**E.**

**F.**

**Fig. 10 (cont.)**

**G.**

### Predictions for each patient (above line = Non-Responder, below line = Responder)

**H.**

### Variables predicting patient outcome

**Fig. 10 (cont.)**

**A.**

Predictions for each patient (above line = Non-Responder, below line = Responder)

**B.**

Variables predicting patient outcome

**Fig. 11**

## C.

Predictions for each patient (above line = Non-Responder, below line = Responder)

## D.

Variables predicting patient outcome

**Fig. 11 (cont.)**

**A.**

Predictions for each patient (above line = Non-Responder, below line = Responder)

**B.**

Variables predicting patient outcome

**Fig. 12**

**A.**

**B.**

**Fig. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016081947 A2

- WO 2014066955 A


### Non-patent literature cited in the description

- **HANLEY et al.** *Radiology*, 1982, vol. 143, 29-36
- **SAMUEL.** *Curr Top Microbiol Immunol.*, 2012, 353
- **VIEIRA ; SOARES.** *BioMed Research International*, 2013, 683095
- **HE et al.** *Mol Med Rep.*, 2015, vol. 12 (5), 6405-6414
- **SAMUEL.** *Virology*, 2011, vol. 411, 180-193
- **CHAN et al.** *Hepatology*, 2014, vol. 63, 832-843
- **LEONARD et al.** *Cancer Res.*, 2013, vol. 73, 7222-7231
- **LINDLEY et al.** *Cancer Med.*, 2016, vol. 5, 2629-2640
- **TOMASETTI et al.** *Proc. Natl. Acad. Sci. U. S. A.*, 2013, vol. 110, 1999-2004
- **VOGELSTEIN et al.** *Science*, 2013, vol. 339, 1456-1558
- **LINDLEY et al.** *Cancer Med.*, September 2016, vol. 5 (9), 2629-2640
- **LIANG et al.** *Proc Natl Acad Sci U S A.*, vol. 110 (6), 2246-51
- **OKAZAKI et al.** *Adv Immunol.*, 2007, vol. 94, 245-73
- **SMITH et al.** *Semin. Cell. Dev. Biol.*, 2012, vol. 23, 258-268
- **TURELLI et al.** *Science*, 2004, vol. 303, 1829
- **SUSPÈNE et al.** *Proc Natl Acad Sci USA*, 2005, vol. 102 (23), 8321-8326
- **NGUYEN et al.** *J. Virol.*, 2007, vol. 81, 4465-4472
- **KOCK ; BLUM.** *J Gen. Virol.*, 2008, vol. 89, 1184-1191
- **WILLEMS et al.** *Viruses*, 2015, vol. 7, 2999-3018
- **ROBERTS et al.** *Nature Genetics*, 2013, vol. 45, 970-976
- **HARRIS et al.** *Breast Cancer Res.*, 2015, vol. 17, 8
- **NIK-ZAINAL et al.** *Cell*, 2012, vol. 149, 979-993
- **SAMUEL.** *Virology*, 2011, vol. 411 (2), 180-93

- **GALLO ; GALLARDI.** *RNA Biol.*, 2008, vol. 5, 135-139
- **GALEANO et al.** *Semin Cell Dev Biol.*, 2012, vol. 23 (3), 244-50
- **AMIN et al.** *Sci. Signal*, 2017, vol. 10, 3941
- **SANGER et al.** *Proc Natl Acad Sci USA*, 1977, vol. 74, 5463-5467
- **JAIN.** Statistical Pattern Recognition: A Review. *IEEE Transactions on Pattern Analysis and Machine Intelligence*, January 2000, vol. 22 (1)
- **VAN ALLEN et al.** *Science*, 2015, vol. 350 (6257), 207-211
- **RIZVI et al.** *Science*, 2015, vol. 348 (6230), 124-128
- **ZARETSKY et al.** *New England Journal of Medicine*, 2016, vol. 375 (9), 819-829
- **SNYDER et al.** *PLoS Medicine*, 2017, vol. 14 (5), e1002309
- **HUGO et al.** *Cell*, 2016, vol. 165, 35-44
- **GOH et al.** *PLoS Medicine*, 2017, vol. 13 (12), e1002136
- **RIAZ et al.** *Cell*, 2017, vol. 171, 934-949
- **LAUSS et al.** *Nature Comm*, 2017, vol. 8, 1738
- **ROH et al.** *Sci Trans Med*, 2017, vol. 9, 3560
- **SYNDER et al.** *PLoS Med*, 2017, vol. 14 (5), e1002309
- **HELLMANN et al.** *Cancer Cell*, 2018, vol. 33, 843-852
- **MIAO et al.** *Science*, 2018, vol. 359, 801-806
- **MIAO et al.** *Nature Genetics*, 2018, vol. 50, 1271-1281
- **MIAO et al.** *Nature Genetics*, 2018
- **LIU et al.** *Nat Comm*, 2017, vol. 8, 2193
- **GANLY et al.** *Cancer Cell*, 2018, vol. 34, 256-270
- **LESURF et al.** *Ann Oncol*, 2017, vol. 28, 1070-1077
- **GOH et al.** *PLoS Med*, 2016, vol. 13 (12), e1002136
- Xgboost: A scalable tree boosting system. **CHEN, T. ; GUESTRIN, C.** Proceedings of the 22nd acm sigkdd international conference on knowledge discovery and data mining. ACM, 2016, 785-794

- **BISCHL B** ; **LANG M** ; **KOTTHOFF L** ; **SCHIFFNER J** ; **RICHTER J** ; **STUDERUS E** ; **CASALICCHIO G** ; **JONES Z**. mir: Machine Learning in R. *Journal of Machine Learning Research*, 2016, vol. 17 (170), 1-5, http://jmlr.org/papersjv17/15-066.html